# EUROPEAN PATENT SPECIFICATION

(11) **EP 3 280 739 B1**
(45) Date of publication and mention of the grant of the patent: **30.12.2020**
(21) Application number: 16775984.4
(22) Date of filing: 08.04.2016
(51) Int. Cl.: C07K 19/00, A61K 47/66, C07K 14/00, C07K 7/06, C07K 7/08, C12N 5/07

(54) **POLYPEPTIDE-BASED SHUTTLE AGENTS FOR IMPROVING THE TRANSDUCTION EFFICIENCY OF POLYPEPTIDE CARGOS TO THE CYTOSOL OF TARGET EUKARYOTIC CELLS, USES THEREOF, METHODS AND KITS RELATING TO SAME**
POLYPEPTIDBASIERTE TRANSPORTWIRKSTOFFE ZUR VERBESSERUNG DER TRANSDUKTIONSEFFIZIENZ VON POLYPEPTIDFRACHTEN ZUM ZYTOSOL VON EUKARYOTISCHEN ZIELZELLEN, VERWENDUNGEN DAVON, ENTSPRECHENDE VERFAHREN UND KITS
AGENTS NAVETTES À BASE DE POLYPEPTIDES POUR L'AMÉLIORATION DE L'EFFICACITÉ DE LA TRANSDUCTION DE CARGOS POLYPEPTIDIQUES DANS LE CYTOSOL DE CELLULES EUCARYOTES CIBLES, LEURS UTILISATIONS, PROCÉDÉS ET TROUSSES LES CONCERNANT

(30) Priority: 10.04.2015 US 201562145760 P; 27.10.2015 US 201562246892 P
(43) Date of publication of application: 14.02.2018
(73) Proprietor: Feldan Bio Inc., Québec, QC G1P 4S6 (CA)
(72) Inventor: GUAY, David, Québec, Québec G1M 3P5 (CA); DEL'GUIDICE, Thomas, Québec, Québec G1C 5S6 (CA); LEPETIT-STOFFAES, Jean-Pascal, Québec, Québec G1R 4W9 (CA)
(74) Representative: HGF
(86) International application number: PCT/CA2016/050403
(87) International publication number: WO 2016/161516

(56) References cited:
- WO-A1-2015/038662
- YA-JUNG LEE ET AL: "Delivery of Macromolecules into Live Cells by Simple Co-incubation with a Peptide", CHEMBIOCHEM - A EUROPEAN JOURNAL OF CHEMICAL BIOLOGY., vol. 11, no. 3, 15 February 2010 (2010-02-15), pages 325-330, XP055523692, DE ISSN: 1439-4227, DOI: 10.1002/cbic.200900527
- JI-SING LIOU ET AL: "Protein transduction in human cells is enhanced by cell-penetrating peptides fused with an endosomolytic HA2 sequence", PEPTIDES, vol. 37, no. 2, 1 October 2012 (2012-10-01), pages 273-284, XP055523627, AMSTERDAM, NL ISSN: 0196-9781, DOI: 10.1016/j.peptides.2012.07.019
- HONGMEI LI ET AL: "Delivery of Intracellular-Acting Biologics in Pro-Apoptotic Therapies", CURRENT PHARMACEUTICAL DESIGN, vol. 17, no. 3, 1 January 2011 (2011-01-01), pages 293-319, XP055031559, ISSN: 1381-6128, DOI: 10.2174/138161211795049642
- SALOMONE F ET AL.: 'A novel chimeric cell -penetrating peptide with membrane-disruptive properties for efficient endosomal escape''.' JOURNAL OF CONTROLLED RELEASE. vol. 163, no. 3, 10 November 2012, ISSN 0168-3659 pages 293 - 303, XP055321151
- FARKHANI SM ET AL.: 'Cell penetrating peptides: Efficient vectors for delivery of nanoparticles, nanocarriers, therapeutic and diagnostic molecules''.' PEPTIDES. vol. 57, 31 July 2014, ISSN 0196-9781 pages 78 - 94, XP028854855
- ERAZO-OLIVERAS A ET AL.: 'Improving the endosomal escape of cell -penetrating peptides and their cargos: Strategies and challenges''.' PHARMACEUTICALS. vol. 5, no. 12, 01 November 2012, ISSN 1424-8247 pages 1177 - 1209, XP055321157
- DATABASE MEDLINE [Online] US NATIONAL LIBRARY OF MEDICINE (NLM), BETHESDA, MD, US; 2 July 2014 (2014-07-02), FASOLI ANNA ET AL: "Mechanistic insight into CM18-Tat11 peptide membrane-perturbing action by whole-cell patch-clamp recording.", Database accession no. NLM24991756 & FASOLI ANNA ET AL: "Mechanistic insight into CM18-Tat11 peptide membrane-perturbing action by whole-cell patch-clamp recording.", MOLECULES (BASEL, SWITZERLAND) 02 JUL 2014, vol. 19, no. 7, 2 July 2014 (2014-07-02), pages 9228-9239, ISSN: 1420-3049
- DATABASE BIOSIS [Online] BIOSCIENCES INFORMATION SERVICE, PHILADELPHIA, PA, US; June 2018 (2018-06), BROCK DAKOTA J ET AL: "Efficient cell delivery mediated by lipid-specific endosomal escape of supercharged branched peptides", Database accession no. PREV201800469681 & TRAFFIC, vol. 19, no. 6, June 2018 (2018-06), pages 421-435, ISSN: 1398-9219(print), DOI: 10.1111/TRA.12566
- DATABASE BIOSIS [Online] BIOSCIENCES INFORMATION SERVICE, PHILADELPHIA, PA, US; 29 July 2013 (2013-07-29), SALOMONE FABRIZIO ET AL: "In Vitro Efficient Transfection by CM18-Tat(11) Hybrid Peptide: A New Tool for Gene-Delivery Applications", Database accession no. PREV201300751414 & PLOS ONE, vol. 8, no. 7, 29 July 2013 (2013-07-29), page Article No.: e70108, ISSN: 1932-6203(print), DOI: 10.1371/JOURNAL.PONE.0070108
- DATABASE BIOSIS [Online] BIOSCIENCES INFORMATION SERVICE, PHILADELPHIA, PA, US; July 2014 (2014-07), SALOMONE FABRIZIO ET AL: "High-Yield Nontoxic Gene Transfer through Conjugation of the CM18-Tat(11) Chimeric Peptide with Nanosecond Electric Pulses", Database accession no. PREV201400585926 & MOLECULAR PHARMACEUTICS, vol. 11, no. 7, July 2014 (2014-07), pages 2466-2474, ISSN: 1543-8384, DOI: 10.1021/MP500223T

## Description

The present description relates to synthetic peptides and polypeptide-based shuttle agents comprising an endosome leakage domain (ELD) operably linked to a cell penetrating domain (CPD), or an ELD operably linked to a histidine-rich domain and a CPD, for use in a method of increasing the transduction efficiency of polypeptide cargos to the cytosol of target eukaryotic cells. Also provided is an *in vitro* method for increasing the transduction efficiency of polypeptide cargos to the cytosol of target eukaryotic cells.

### BACKGROUND

Cell delivery technologies to transport large molecules inside eukaryotic cells have a wide range of applications, particularly in the biopharmaceutical industry. While some soluble chemical substances (e.g., small molecule drugs) may passively diffuse through the eukaryotic cell membrane, larger cargos (e.g., biologics, polynucleotides, and polypeptides) require the help of shuttle agents to reach their intracellular targets.

An area that would greatly benefit from advances in cell delivery technologies is the field of cell therapy, which has made enormous leaps over the last two decades. Deciphering the different growth factors and molecular cues that govern cell expansion, differentiation and reprogramming open the door to many therapeutic possibilities for the treatment of unmet medical needs. For example, induction of pluripotent stem cells directly from adult cells, direct cell conversion (trans-differentiation), and genome editing (Zinc finger nuclease, TALEN™ and CRISPR/Cas9 technologies) are examples of methods that have been developed to maximize the therapeutic value of cells for clinical applications. Presently, the production of cells with high therapeutic activity usually requires *ex vivo* manipulations, mainly achieved by viral transduction, raising important safety and economical concerns for human applications. The ability to directly deliver active proteins such as transcription factors or artificial nucleases, inside these cells, may advantageously circumvent the safety concerns and regulatory hurdles associated with more risky gene transfer methods.

In this regard, polypeptide-based transduction agents may be useful for introducing purified recombinant proteins directly into target cells, for example, to help bypass safety concerns regarding the introduction of foreign DNA. Lipid- or cationic polymer-based transduction agents exist, but introduce safety concerns regarding chemical toxicity and efficiency, which hamper their use in human therapy. Protein transduction approaches involving fusing a recombinant protein cargo directly to a cell-penetrating peptide (e.g., HIV transactivating protein TAT) require large amounts of the recombinant protein and often fail to deliver the cargo to the proper subcellular location, leading to massive endosomal trapping and eventual degradation. Several endosomal membrane disrupting peptides have been developed to try and facilitate the escape of endosomally-trapped cargos to the cytosol. However, many of these endosomolytic peptides are intended to alleviate endosomal entrapment of cargos that have already been delivered intracellularly, and do not by themselves aid in the initial step of shuttling the cargos intracellularly across the plasma membrane (Salomone et al., 2012; Salomone et al., 2013; Erazo-Oliveras et al., 2014; Fasoli et al., 2014). Thus, there is a need for improved shuttle agents capable of increasing the transduction efficiency of polypeptide cargos, and delivering the cargos to the cytosol of target eukaryotic cells.

### SUMMARY

The present invention stems from the surprising discovery that synthetic peptides comprising an endosome leakage domain (ELD) operably linked to a cell penetrating domain (CPD) and optionally a histidine-rich domain, have the ability to increase the proportion of cells that can be transduced with a polypeptide cargo of interest, without the synthetic peptide being covalently bound to the polypeptide cargo. Following successful transduction, the synthetic peptides may facilitate the ability of endosomally-trapped polypeptide cargos to gain access to the cytosol, and optionally be targeted to various subcellular comparts (e.g., the nucleus).

Accordingly, the present invention relates to the following aspects:
**(1)** A polypeptide-based shuttle agent comprising an endosome leakage domain (ELD) operably linked to a cell penetrating domain (CPD), or an ELD operably linked to a histidine-rich domain and a CPD, for use in a clinical or therapeutic *in vivo* method for increasing the transduction efficiency of an independent polypeptide cargo to the cytosol of a target eukaryotic cell, wherein the cargo is a biologically active polypeptide, and wherein the polypeptide-based shuttle agent has a predicted net charge of at least +6 at a physiological pH and is used at a concentration sufficient to increase the transduction efficiency of the independent polypeptide cargo at the cytosol of target eukaryotic cell.
**(2)** An *in vitro* method for increasing the transduction efficiency of an independent polypeptide cargo to the cytosol of target eukaryotic cells, the method comprising contacting the target eukaryotic cells with a polypeptide-based shuttle agent comprising an endosome leakage domain (ELD) operably linked to a cell penetrating domain (CPD), or an ELD operably linked to a histidine rich domain and a CPD, wherein the polypeptide-based shuttle agent has a predicted net charge of +6 at physiological pH and is used at a concentration sufficient to increase the transduction efficiency of the independent polypeptide cargo to the cytosol of target eukaryotic cell.
**(3)** The polypeptide-based shuttle agent of **(1)** or the *in vitro* method of **(2)**, wherein the polypeptide-based shuttle agent: (a) comprises a minimum length of 20, 21, 22, 23, 24, 25, 26, 27, 28, 29, or 30 amino acid residues and a maximum length of 35, 40, 45, 50, 55, 65, 70, 75, 80, 85, 90, 95, 100, 105, 110, 115, 120, 125, 130, 135, 140, 145, or 150 amino acid residues; (b) has a predicted net charge of at least +7, +8, +9, +10, +11, +12, +13, +14, or +15 at physiological pH; (c) is soluble in aqueous solution; or (d) any combination of (a) to (c).
**(4)** The polypeptide-based shuttle agent of **(1) or (3)** or the *in vitro* method of **(2)**, wherein: (a) the ELD is or is from: an endosomolytic peptide; an antimicrobial peptide (AMP); a linear cationic alpha-helical antimicrobial peptide; a Cecropin-A/Melittin hybrid (CM series) peptide; pH-dependent membrane active peptide (PAMP); a peptide amphiphile; a peptide derived from the N terminus of the HA2 subunit of influenza hemagglutinin (HA); CM18; Diphtheria toxin T domain (DT); GALA; PEA; INF-7; LAH4; HGP; H5WYG; HA2; EB1; VSVG; *Pseudomonas* toxin; melittin; KALA; JST-1; C(LLKK)₃C; G(LLKK)₃G; or any combination thereof; (b) the CPD is or is from: a cell-penetrating peptide or the protein transduction domain from a cell-penetrating peptide; TAT; PTD4; Penetratin (Antennapedia); pVEC; M918; Pep-1; Pep-2; Xentry; arginine stretch; transportan; SynB1; SynB3; or any combination thereof; (c) the histidine-rich domain is a stretch of at least 3, at least 4, at least 5, or at least 6 amino acids comprising at least 50%, at least 55%, at least 60%, at least 65%, at least 70%, at least 75%, at least 80%, at least 85%, or at least 90% histidine residues; and/or comprises at least 2, at least 3, at least 4, at least 5, at least 6, at least 7, at least 8, or at least 9 consecutive histidine residues; or (d) any combination of (a) to (c).
**(5)** The polypeptide-based shuttle agent of any one of **(1), (3) or (4)** or the *in vitro* method of **(2) to (4),** wherein the polypeptide-based shuttle agent comprises: (a) an ELD comprising the amino acid sequence of any one of **SEQ ID NOs: 1-15, 63, or 64,** or a variant or fragment thereof having endosomolytic activity; (b) a CPD comprising the amino acid sequence of any one of **SEQ ID NOs: 16-27 or 65,** or a variant or fragment thereof having cell penetrating activity; (c) a histidine-rich domain having at least 2, at least 3, at least 4, at least 5, or at least 6 consecutive histidine residues; (d) of any combination of (a) to (c).
**(6)** The polypeptide-based shuttle agent of any one of **(1), or (3) to (5),** or the *in vitro* method of any one of **(2) to (5),** wherein the domains are operably linked via one or more linker domains.
(7) The polypeptide-based shuttle agent of any one of **(1) or (3) to (6),** or the *in vitro* method of any one of **(2) to (6),** wherein the polypeptide-based shuttle agent comprises: (a) an ELD which is CM18, KALA, or C(LLKK)₃C having the amino acid sequence of **SEQ ID NO: 1,14, or 63,** or a variant thereof having at least 85%, 90%, or 95% identity to **SEQ ID NO: 1** and having endosomolytic activity; (b) a CPD which is TAT or PTD4 having the amino acid sequence of **SEQ ID NO: 17 or 65,** or a variant thereof having at least 85%, 90%, or 95% identity to **SEQ ID NO: 17 or 65,** and having cell penetrating activity; or Penetratin having the amino acid sequence of **SEQ ID NO: 18,** or a variant thereof having at least 85%, 90%, or 95% identity to **SEQ ID NO: 18** and having cell penetrating activity; (c) a histidine-rich domain comprising at least 6 consecutive histidine residues; or (d) any combination of (a) to (c).
**(8)** The polypeptide-based shuttle agent of any one of **(1) or (3) to (7),** or the *in vitro* method of any one of **(2) to (7),** wherein the polypeptide-based shuttle agent comprises or consists of the amino acid sequence of any one of **SEQ ID NOs: 57-59, 66-73, or 82-102,** or a functional variant thereof having at least 85%, 90%, or 95% identity to any one of **SEQ ID NOs: 57-59, 66-73, or 82-102.**
**(9)** The polypeptide-based shuttle agent of any one of **(1) or (3) to (8)** or the *in vitro* method of any one of **(2) to (8),** wherein the polypeptide cargo: (a) lacks a CPD or a CPD as defined in **(4)(b);** (b) is a recombinant protein; (c) comprises a subcellular targeting domain; (d) is complexed with a DNA and/or RNA molecule; or (e) any combination of (a) to (d).
**(10)** The polypeptide-based shuttle agent or method of **(9),** wherein the subcellular targeting domain is: (a) a nuclear localization signal (NLS); (b) a nucleolar signal sequence; (c) a mitochondrial signal sequence; or (d) a peroxisome signal sequence.
**(11)** The polypeptide-based shuttle agent, or method of **(10),** wherein: (a) the NLS is from: E1a, T-Ag, c-myc, T-Ag, op-T-NLS, Vp3, nucleoplasmin, histone 2B, Xenopus N1, PARP, PDX-1, QKI-5, HCDA, H2B, v-Rel, Amida, RanBP3, Pho4p, LEF-1, TCF-1, BDV-P, TR2, SOX9, or Max; (b) the nucleolar signal sequence is from BIRC5 or RECQL4; (c) the mitochondrial signal sequence is from Tim9 or Yeast cytochrome c oxidase subunit IV; or (d) the peroxisome signal sequence is from PTS1.
**(12)** The polypeptide-based shuttle agent or method of any one of **(2) to (11),** wherein the polypeptide cargo is a transcription factor, a nuclease, a cytokine, a hormone, a growth factor, or an antibody.
**(13)** The polypeptide-based shuttle agent or method of **(12),** wherein: (a) the transcription factor is: HOXB4, NUP98-HOXA9, Oct3/4, Sox2, Sox9, Klf4, c-Myc, MyoD, Pdx1, Ngn3, MafA, Blimp-1, Eomes, T-bet, FOXO3A, NF-YA, SALL4, ISL1, FoxA1, Nanog, Esrrb, Lin28, HIF1-alpha, Hlf, Runx1t1, Pbx1, Lmo2, Zfp37, Prdm5, Bcl-6, or any combination thereof; and/or the nuclease is: an RNA-guided endonuclease, a CRISPR endonuclease, a type I CRISPR endonuclease, a type II CRISPR endonuclease, a type III CRISPR endonuclease, a type IV CRISPR endonuclease, a type V CRISPR endonuclease, a type VI CRISPR endonuclease, CRISPR associated protein 9 (Cas9), Cpf1, a zinc-finger nuclease (ZFN), a Transcription activator-like effector nucleases (TALENs), a homing endonuclease, a meganuclease, or any combination thereof.
**(14)** The polypeptide-based shuttle agent or method of any one of **(1) to (13),** for increasing the transduction efficiency of a polypeptide cargo to the cytosol of target eukaryotic cells intended for use in cell therapy, genome editing, adoptive cell transfer, and/or regenerative medicine.
**(15)** The polypeptide-based shuttle agent or method of any one of **(1) to (14),** wherein the target eukaryotic cell is a stem cell, a primary cell, an immune cell, a T cell, or a dendritic cell.
**(16)** A synthetic peptide comprising an endosome leakage domain (ELD) operably linked to a cell penetrating domain (CPD), or an ELD operably linked to a histidine-rich domain and a CPD, wherein the synthetic peptide is the polypeptide based shuttle agent as defined in any one of (1) or (3) to (15) and wherein the CPD is or is from:
   PTD4, Penetratin (Antennapedia); pVEC; M918; Pep-1; Pep-2; Xentry; arginine stretch; transportan; SynB1; SynB3; orany combination thereof, and wherein the polypeptide based shuttle agent has a predicted net charge of at least +6 at physiological pH.

Described herein is also the method as described in items 1 to 30 below:
1. A method for increasing the transduction efficiency of an independent polypeptide cargo to the cytosol of a target eukaryotic cell, said method comprising contacting said target eukaryotic cell with a synthetic peptide and said independent polypeptide cargo, wherein said synthetic peptide:
   (a) comprises an endosome leakage domain (ELD), or a variant or fragment thereof having endosomolytic activity, operably linked to a cell penetrating domain (CPD), wherein said ELD comprises the amino acid sequence of any one of **SEQ ID NOs: 1-15, 63, or 64;**
   (b) is not covalently bound to said independent polypeptide cargo;
   (c) has an overall length of between 20 and 100 amino acid residues;
   (d) has a net charge of at least +6 at physiological pH; and
   (e) is soluble in aqueous solution at physiological pH,
   wherein said CPD enables intracellular delivery of said synthetic peptide, and said ELD enables escape of endosomally trapped independent polypeptide cargo to the cytosol of the target eukaryotic cell.
2. The method of item 1, wherein said synthetic peptide has an overall length of between 20 and 70 amino acid residues.
3. The method of item 1, wherein said CPD comprises the amino acid sequence of any one of **SEQ ID NOs: 16-27 or 65,** or is a variant or fragment thereof having cell penetrating activity.
4. The method of item 1, wherein said synthetic peptide further comprises a histidine-rich domain consisting of a stretch of at least 6 amino acids comprising at least 50%, at least 55%, at least 60%, at least 65%, at least 70%, at least 75%, at least 80%, at least 85%, or at least 90% histidine residues; and/or comprises at least 2, at least 3, at least 4, at least 5, or at least 6 consecutive histidine residues.
5. The method of item 1, wherein said ELD variant or ELD fragment has at least 70%, at least 75%, at least 80%, at least 85%, at least 90%, or at least 95% sequence identity to any one of **SEQ ID NOs: 1-15, 63, or 64.**
6. The method of item 3, wherein said CPD variant or CPD fragment has at least 70%, at least 75%, at least 80%, at least 85%, at least 90%, or at least 95% sequence identity to any one of **SEQ ID NOs: 16-27 or 65.**
7. The method of item 1, wherein said ELD and CPD are operably linked via one or more linker domains.
8. The method of item 1, wherein said synthetic peptide is chemically synthesized without an N-terminal methionine residue.
9. The method of item 1, wherein the synthetic peptide comprises the amino acid sequence of any one of **SEQ ID NOs: 57-59, 66-72, or 82-102,** or a functional variant thereof having at least 70%, at least 85%, at least 90%, or at least 95% identity to any one of **SEQ ID NOs: 57-59, 66-72, or 82-102.**
10. The method of item 1, wherein said independent polypeptide cargo is a recombinant protein lacking a CPD.
11. The method of item 1, wherein said independent polypeptide cargo is a transcription factor, a nuclease, a cytokine, a hormone, a growth factor, or an antibody.
12. The method of item 11, wherein:
   (b) said transcription factor is: HOXB4, NUP98-HOXA9, Oct3/4, Sox2, Sox9, Klf4, c-Myc, MyoD, Pdx1, Ngn3, MafA, Blimp-1, Eomes, T-bet, FOXO3A, NF-YA, SALL4, ISL1, FoxA1, Nanog, Esrrb, Lin28, HIF1-alpha, Hlf, Runx1t1, Pbx1, Lmo2, Zfp37, Prdm5, Bcl-6, or any combination thereof; or
   (b) said nuclease is an RNA-guided endonuclease, a CRISPR endonuclease, a type I CRISPR endonuclease, a type II CRISPR endonuclease, a type III CRISPR endonuclease, a type IV CRISPR endonuclease, a type V CRISPR endonuclease, a type VI CRISPR endonuclease, CRISPR associated protein 9 (Cas9), Cpf1, a zinc-finger nuclease (ZFNs), a Transcription activator-like effector nucleases (TALENs), a homing endonuclease, or a meganuclease.
13. The method of item 11, wherein said nuclease is Cas9 or Cpf1.
14. The method of item 13, wherein said nuclease further comprises a guide RNA, a crRNA, a tracrRNA, or both a crRNA and a tracrRNA.
15. The method of item 1, wherein said independent polypeptide cargo comprises a nuclear localization signal or a further nuclear localization signal.
16. The method of item 15, wherein said independent polypeptide cargo is a transcription factor or a nuclease.
17. The method of item 16 wherein:
   (a) said transcription factor is: HOXB4, NUP98-HOXA9, Oct3/4, Sox2, Sox9, Klf4, c-Myc, MyoD, Pdx1, Ngn3, MafA, Blimp-1, Eomes, T-bet, FOXO3A, NF-YA, SALL4, ISL1, FoxA1, Nanog, Esrrb, Lin28, HIF1-alpha, Hlf, Runx1t1, Pbx1, Lmo2, Zfp37, Prdm5, Bcl-6, or any combination thereof; or
   (b) said nuclease is an RNA-guided endonuclease, a CRISPR endonuclease, a type I CRISPR endonuclease, a type II CRISPR endonuclease, a type III CRISPR endonuclease, a type IV CRISPR endonuclease, a type V CRISPR endonuclease, a type VI CRISPR endonuclease, CRISPR associated protein 9 (Cas9), Cpf1, a zinc-finger nuclease (ZFNs), a Transcription activator-like effector nucleases (TALENs), a homing endonuclease, or a meganuclease.
18. The method of item 17, wherein said nuclease is Cas9 or Cpf1.
19. The method of item 18, wherein said nuclease further comprises a guide RNA.
20. The method of item 1, wherein said cell is stem cell, a primary cell, an immune cell, a T cell, or a dendritic cell.
21. A method for increasing the transduction efficiency of an independent polypeptide cargo to the cytosol of a target eukaryotic cell, said method comprising contacting said target eukaryotic cell with a synthetic peptide and said independent polypeptide cargo, wherein said synthetic peptide:
   (a) comprises an endosome leakage domain (ELD) operably linked to a cell penetrating domain (CPD), wherein said ELD is an endosomolytic peptide which is, or is derived from: a linear cationic alpha-helical antimicrobial peptide; a Cecropin-A/Melittin hybrid (CM series) peptide; pH-dependent membrane active peptide (PAMP); a peptide amphiphile; a peptide derived from the N terminus of the HA2 subunit of influenza hemagglutinin (HA); CM18; Diphtheria toxin T domain (DT); GALA; PEA; INF-7; LAH4; HGP; H5WYG; HA2; EB1; VSVG; *Pseudomonas* toxin; melittin; KALA; JST-1; C(LLKK)₃C; or G(LLKK)₃G;
   (b) is not covalently bound to said independent polypeptide cargo;
   (c) has an overall length of between 20 and 100 amino acid residues;
   (d) has a net charge of at least +6 at physiological pH; and
   (e) is soluble in aqueous solution at physiological pH,
   wherein said CPD enables intracellular delivery of said synthetic peptide, and said ELD enables escape of endosomally trapped independent polypeptide cargo to the cytosol of the target eukaryotic cell.
22. The method of item 21, wherein said CPD is, or is derived from: a cell-penetrating peptide or the protein transduction domain from a cell-penetrating peptide; TAT; PTD4; Penetratin (Antennapedia); pVEC; M918; Pep-1; Pep-2; Xentry; arginine stretch; transportan; SynB1; SynB3; or any combination thereof.
23. The method of item 21, wherein said synthetic peptide further comprises a histidine-rich domain consisting of a stretch of at least 3 amino acids comprising at least 50%, at least 55%, at least 60%, at least 65%, at least 70%, at least 75%, at least 80%, at least 85%, or at least 90% histidine residues; and/or comprises at least 2, at least 3, at least 4, at least 5, or at least 6 consecutive histidine residues.
24. The method of item 21, wherein said ELD and CPD are operably linked via one or more linker domains.
25. The method of item 21, wherein said independent polypeptide cargo is a transcription factor, a nuclease, a cytokine, a hormone, a growth factor, or an antibody.
26. The method of item 25, wherein said transcription factor is: HOXB4, NUP98-HOXA9, Oct3/4, Sox2, Klf4, c-Myc, MyoD, Pdx1, Ngn3, MafA, Blimp-1, Eomes, T-bet, FOXO3A, NF-YA, SALL4, ISL1, FoxA1, Nanog, Esrrb, Lin28, HIF1-alpha, Hlf, Runx1t1, Pbx1, Lmo2, Zfp37, Prdm5, Bcl-6, or any combination thereof.
27. The method of item 25, wherein said nuclease is an RNA-guided endonuclease, a CRISPR endonuclease, a type I CRISPR endonuclease, a type II CRISPR endonuclease, a type III CRISPR endonuclease, a type IV CRISPR endonuclease, a type V CRISPR endonuclease, a type VI CRISPR endonuclease, CRISPR associated protein 9 (Cas9), Cpf1, a zinc-finger nuclease (ZFNs), a Transcription activator-like effector nucleases (TALENs), a homing endonuclease, or a meganuclease.
28. The method of item 25, wherein said nuclease is Cas9 or Cpf1.
29. A method for increasing the transduction efficiency of an independent polypeptide cargo to the cytosol of a target eukaryotic cell, said method comprising contacting said target eukaryotic cell with a synthetic peptide and said independent polypeptide cargo which is not covalently bound to said synthetic peptide, wherein said synthetic peptide comprises an endosome leakage domain (ELD) operably linked to a cell penetrating domain, or an ELD operably linked to a CPD and a histidine-rich domain, wherein:
   (a) said ELD comprises the amino acid sequence of any one of **SEQ ID NOs: 1-15, 63, or 64;**
   (b) said CPD comprises the amino acid sequence of any one of **SEQ ID NOs: 16-27 or 65;** and
   (c) said histidine-rich domain comprises at least two consecutive histidine residues.
30. A method for delivering a CRISPR associated protein 9 (Cas9) to the nucleus of a target eukaryotic cell, said method comprising contacting said eukaryotic cell with a Cas9 recombinant protein comprising a nuclear localization signal, and a separate synthetic peptide shuttle agent less than 100 residues in length and comprising an endosome leakage domain (ELD) operably linked to a cell penetrating domain, or an ELD operably linked to a CPD and a histidine-rich domain, wherein:
   (a) said ELD comprises the amino acid sequence of any one of **SEQ ID NOs: 1-15, 63, or 64;**
   (b) said CPD comprises the amino acid sequence of any one of **SEQ ID NOs: 16-27 or 65;** and
   (c) said histidine-rich domain comprises at least two consecutive histidine residues.

### General Definitions

Headings, and other identifiers, e.g., (a), (b), (i), (ii), etc., are presented merely for ease of reading the specification and claims. The use of headings or other identifiers in the specification or claims does not necessarily require the steps orelements be performed in alphabetical or numerical order or the order in which they are presented.

The use of the word **"a"** or **"an"** when used in conjunction with the term "comprising" in the claims and/or the specification may mean "one" but it is also consistent with the meaning of "one or more", "at least one", and "one or more than one".

The term **"about"** is used to indicate that a value includes the standard deviation of error for the device or method being employed to determine the value. In general, the terminology "about" is meant to designate a possible variation of up to 10%. Therefore, a variation of 1, 2, 3, 4, 5, 6, 7, 8, 9 and 10% of a value is included in the term "about". Unless indicated otherwise, use of the term "about" before a range applies to both ends of the range.

As used in this specification and claim(s), the words **"comprising"** (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, un-recited elements or method steps.

As used herein, **"protein"** or **"polypeptide"** means any peptide-linked chain of amino acids, which may or may not comprise any type of modification (e.g., post-translational modifications such as acetylation, phosphorylation, glycosylation, sulfatation, sumoylation, prenylation, ubiquitination, etc).

As used herein, the expression **"is or is from"** or **"is from"** comprises functional variants of a given protein domain (CPD or ELD), such as conservative amino acid substitutions, deletions, modifications, as well as variants or function derivatives, which do not abrogate the activity of the protein domain. Other objects, advantages and features of the present description will become more apparent upon reading of the following non-restrictive description of specific embodiments thereof, given by way of example only with reference to the accompanying drawings.

### BRIEF DESCRIPTION OF THE DRAWINGS

In the appended drawings:

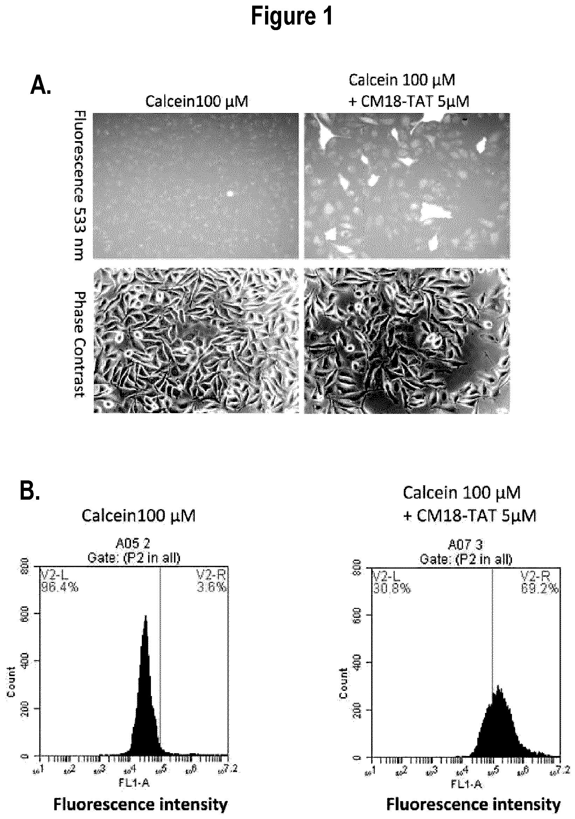

**Figure 1** shows a typical result of a calcein endosomal escape assay in which HEK293A cells were loaded with the fluorescent dye calcein ("100 µM calcein"), and were then treated (or not) with a shuttle agent that facilitates endosomal escape of the calcein ("100 µM calcein +CM18-TAT 5 µM"). Panel **A** shows the results of a fluorescence microscopy experiment, while panel **B** shows the results of a flow cytometry experiment.

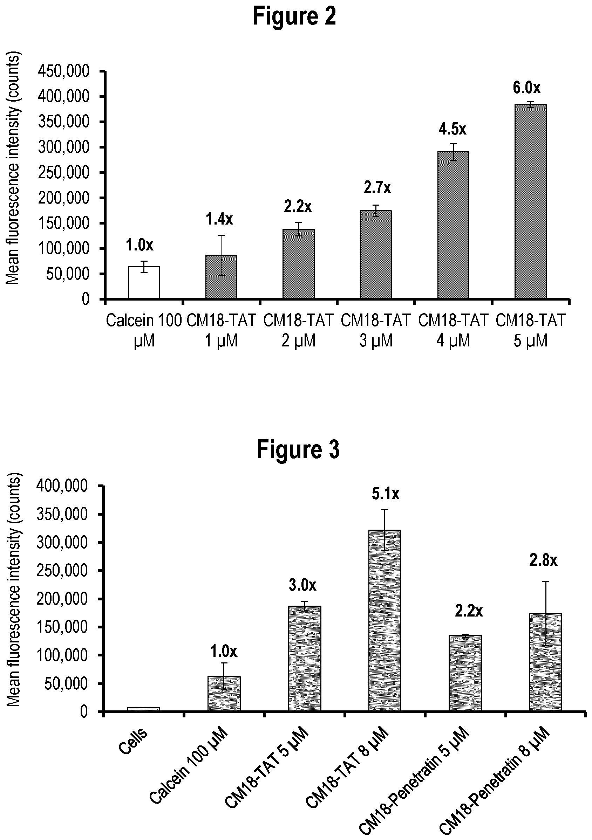

**Figure 2** shows the results of a calcein endosomal escape flow cytometry assay in which HeLa cells were loaded with calcein ("calcein 100 µM"), and were then treated with increasing concentrations of the shuttle agent CM18-TAT-Cys (labeled "CM18-TAT").

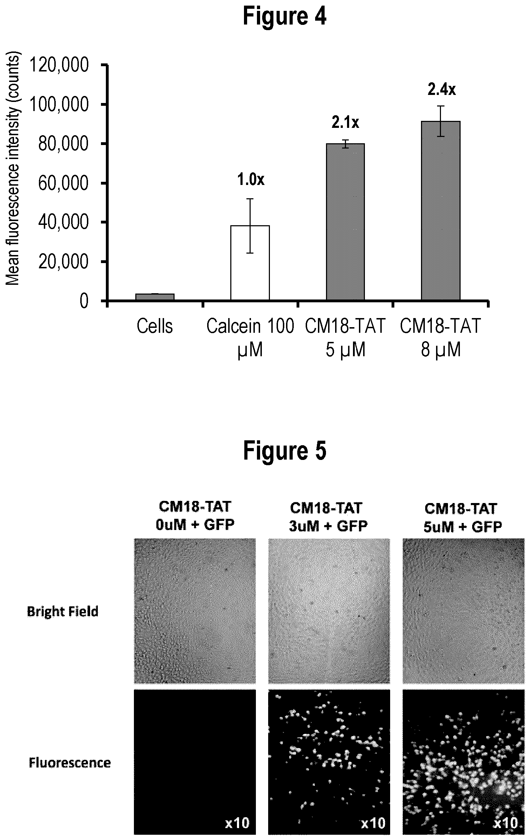

**Figures 3** **and** **4** show the results of calcein endosomal escape flow cytometry assays in which HeLa cells (Figure 3) or primary myoblasts (Figure 4) were loaded with calcein ("calcein 100 µM"), and were then treated with 5 µM or 8 µM of the shuttle agents CM18-TAT-Cys or CM18-Penetratin-Cys (labeled "CM18-TAT" and "CM18-Penetratin", respectively).
**Figure 5** shows the results of a GFP transduction experiment visualized by fluorescence microscopy in which a GFP cargo protein was co-incubated with 0, 3 or 5 µM of CM18-TAT-Cys (labeled "CM18-TAT"), and then exposed to HeLa cells. The cells were observed by bright field (upper panels) and fluorescence microscopy (lower panels).

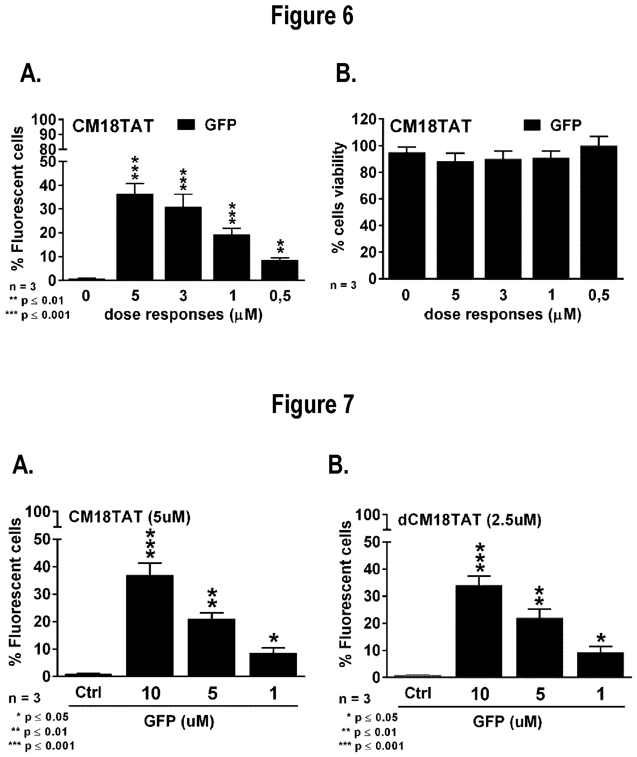

**Figure 6** shows the results of a GFP transduction efficiency experiment in which GFP cargo protein (10 µM) was co-incubated with different concentrations of CM18-TAT-Cys (labeled "CM18-TAT"), prior to being exposed to HeLa cells. Cells were evaluated by flow cytometry and the percentage of fluorescent (GFP-positive) cell is shown in panel **A,** and corresponding cell toxicity data is shown in panel **B.**
**Figure 7** shows the results of a GFP transduction efficiency experiment in which different concentrations of GFP cargo protein (10,5 or 1 µM) were co-incubated with either 5 µM of CM18-TAT-Cys (panel A, labeled "CM18TAT"), or 2.5 µM of dCM18-TAT-Cys (panel B, labeled "dCM18TAT"), prior to being exposed to HeLa cells. Cells were evaluated by flow cytometry and the percentages of fluorescent (GFP-positive) cells are shown.

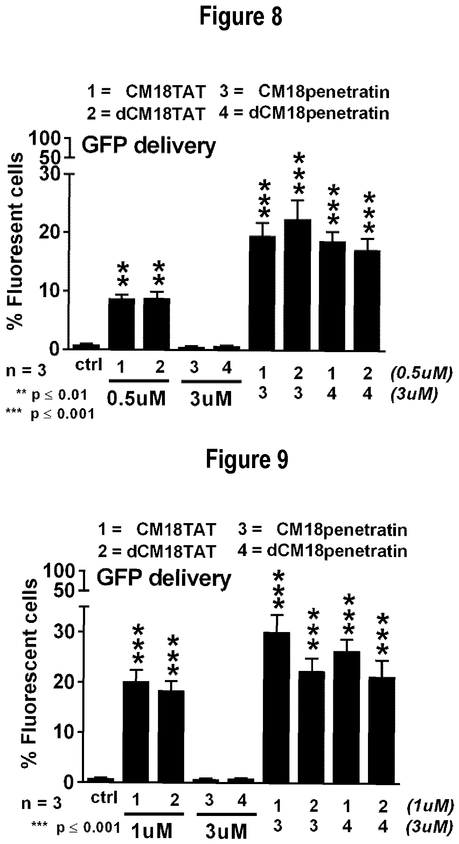

**Figures 8 and 9** show the results of GFP transduction efficiency experiments in which GFP cargo protein (10 µM) was co-incubated with different concentrations and combinations of CM18-TAT-Cys (labeled "CM18TAT"), CM18-Penetratin-Cys (labeled "CM18penetratin"), and dimers of each (dCM18-TAT-Cys (labeled "dCM18TAT"), dCM18-Penetratin-Cys (labeled "dCM18penetratin"), prior to being exposed to HeLa cells. Cells were evaluated by flow cytometry and the percentages of fluorescent (GFP-positive) cells are shown.

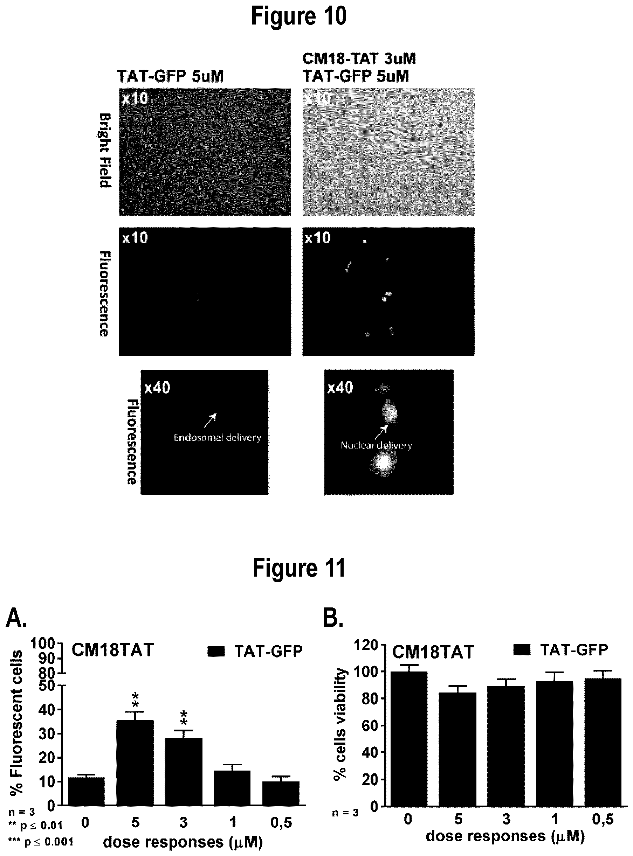

**Figure 10** shows typical results of a TAT-GFP transduction experiment in which TAT-GFP cargo protein (5 µM) was co-incubated with 3 µM of CM18-TAT-Cys (labeled "CM18-TAT"), prior to being exposed to HeLa cells. Cells and GFP fluorescence were visualized by bright field and fluorescence microscopy at 10x and 40x magnifications. Arrows indicate the endosome delivery of TAT-GFP in the absence of CM18-TAT-Cys, as well as its nuclear delivery in the presence of CM18-TAT-Cys.
**Figure 11** shows the results of a TAT-GFP transduction efficiency experiment in which TAT-GFP cargo protein (5 µM) was co-incubated with different concentrations of CM18-TAT-Cys (labeled "CM18TAT"), prior to being exposed to HeLa cells. Cells were evaluated by flow cytometry and the percentage of fluorescent (GFP-positive) cell is shown in panel **A,** and corresponding cell toxicity data is shown in panel **B.**

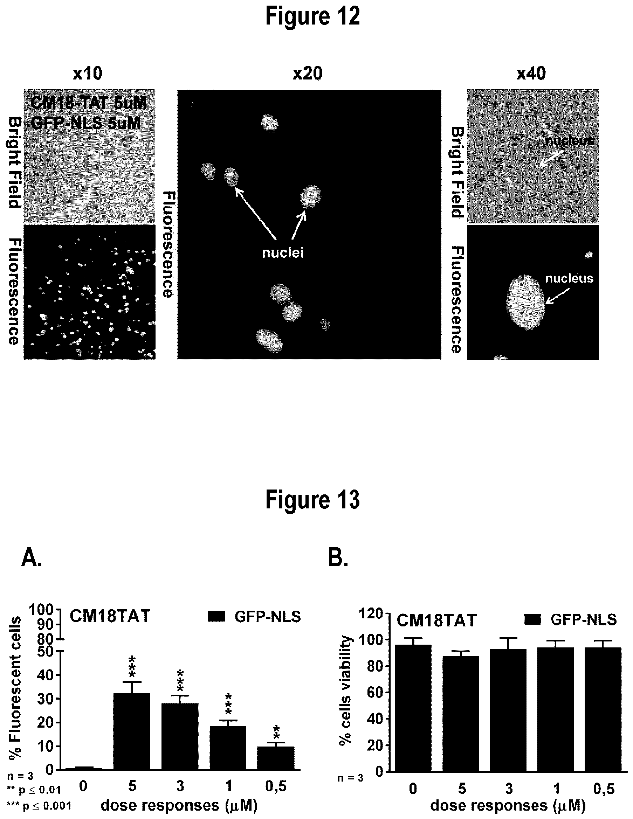

**Figure 12** shows typical results of a GFP-NLS transduction experiment in which GFP-NLS cargo protein (5 µM) was co-incubated with 5 µM of CM18-TAT-Cys (labeled "CM18-TAT"), prior to being exposed to HeLa cells for 5 minutes. Cells and GFP fluorescence were visualized by bright field and fluorescence microscopy at 10x, 20x, and 40x magnifications. Arrows indicate areas of nuclear delivery of GFP-NLS.
**Figure 13** shows the results of a GFP-NLS transduction efficiency experiment in which GFP-NLS cargo protein (5 µM) was co-incubated with different concentrations of CM18-TAT-Cys (labeled "CM18TAT"), prior to being exposed to HeLa cells. Cells were evaluated by flow cytometry and the percentage of fluorescent (GFP-positive) cell is shown in panel **A,** and corresponding cell toxicity data is shown in panel **B.**

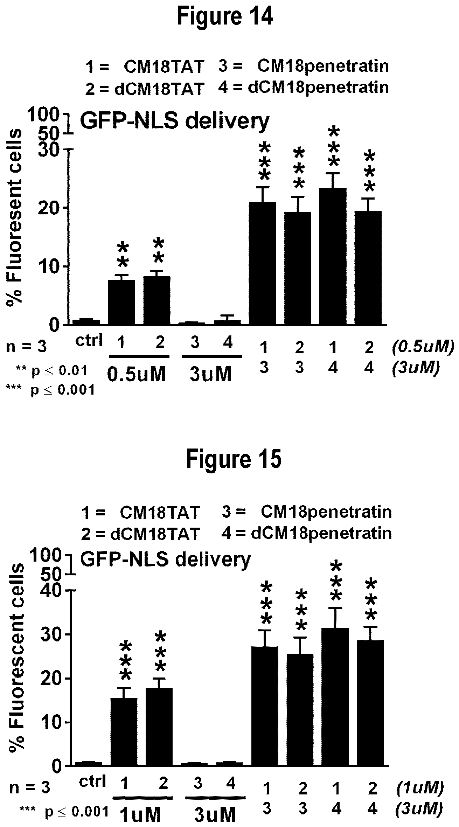

**Figures 14 and 15** show the results of GFP-NLS transduction efficiency experiments in which GFP-NLS cargo protein (5 µM) was co-incubated with different concentrations and combinations of CM18-TAT (labeled "CM18TAT"), CM18-Penetratin (labeled "CM18penetratin"), and dimers of each (dCM18-TAT-Cys, dCM18-Penetratin-Cys; labeled "dCM18TAT" and "dCM18penetratin", respectively), prior to being exposed to HeLa cells. Cells were evaluated by flow cytometry and the percentages of fluorescent (GFP-positive) cells are shown.

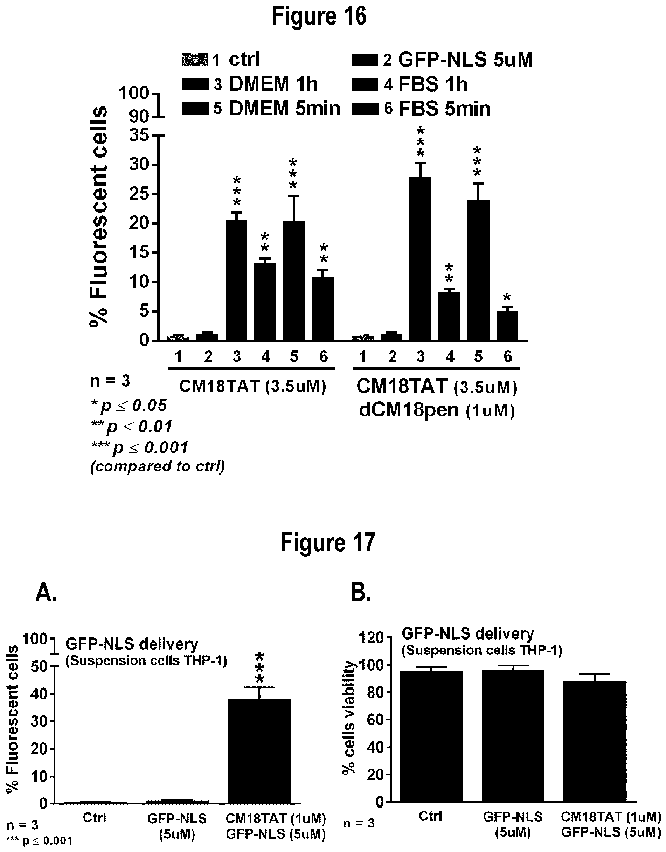

**Figure 16** shows the results of a GFP-NLS transduction efficiency experiment in which GFP-NLS cargo protein (5 µM) was co-incubated with either CM18-TAT-Cys (3.5 µM, labeled "CM18TAT") alone or with dCM18-Penetratin-Cys (1 µM, labeled "dCM18pen") for 5 minutes or 1 hour in plain DMEM media ("DMEM") or DMEM media containing 10% FBS ("FBS"), before being subjected to flow cytometry analysis. The percentages of fluorescent (GFP-positive) cells are shown. Cells that were not treated with shuttle agent or GFP-NLS ("ctrl"), and cells that were treated with GFP-NLS without shuttle agent ("GFP-NLS 5 µM") were used as controls.
**Figure 17** shows the results of a GFP-NLS transduction efficiency experiment in which GFP-NLS cargo protein (5 µM) was co-incubated with or without 1 µM CM18-TAT-Cys (labeled "CM18TAT"), prior to being exposed to THP-1 cells. Cells were evaluated by flow cytometry and the percentage of fluorescent (GFP-positive) cells is shown in panel **A,** and corresponding cell toxicity data is shown in panel **B.**

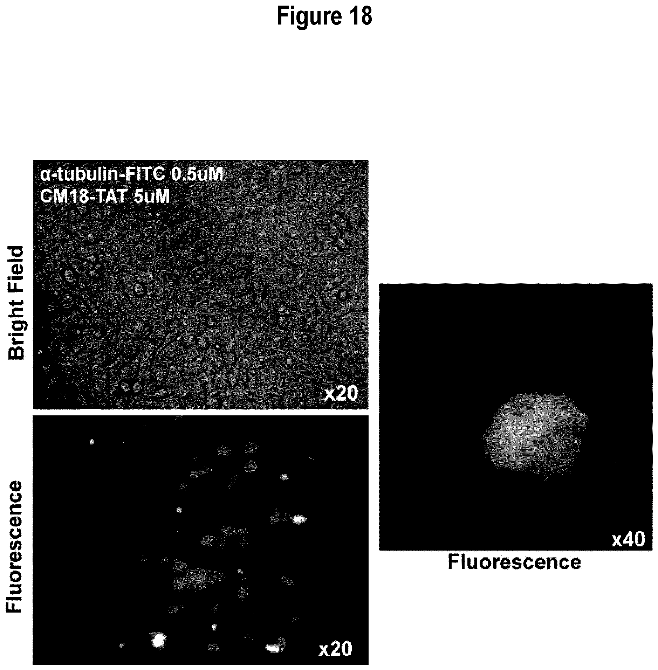

**Figure 18** shows the results of a transduction efficiency experiment in which the cargo protein, FITC-labeled anti-tubulin antibody (0.5 µM), was co-incubated with 5 µM of CM18-TAT-Cys (labeled "CM18-TAT"), prior to being exposed to HeLa cells. Functional antibody delivery was visualized by bright field (20x) and fluorescence microscopy (20x and 40x), in which fluorescent tubulin fibers in the cytoplasm were visualized.

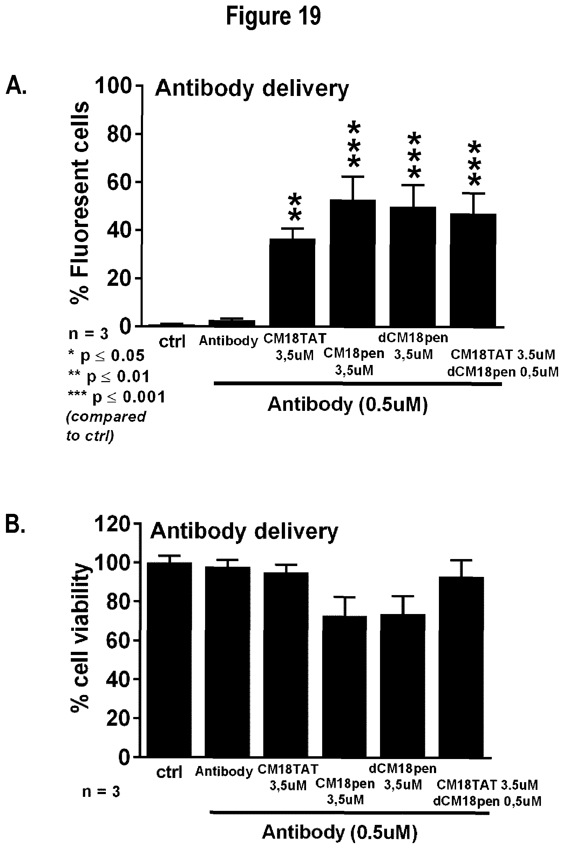

**Figure 19** shows the results of an FITC-labeled anti-tubulin antibody transduction efficiency experiment in which the antibody cargo protein (0.5 µM) was co-incubated with 3.5 µM of CM18-TAT-Cys (labeled "CM18TAT"), CM18-Penetratin-Cys (labeled "CM18pen")or dCM18-Penetratin-Cys (labeled "dCM18pen"), or a combination of 3.5 µM of CM18-TAT-Cys and 0.5 µM of dCM18-Penetratin-Cys, prior to being exposed to HeLa cells. Cells were evaluated by flow cytometry and the percentage of fluorescent (FITC-positive) cell is shown in panel **A,** and corresponding cell toxicity data is shown in panel **B.**

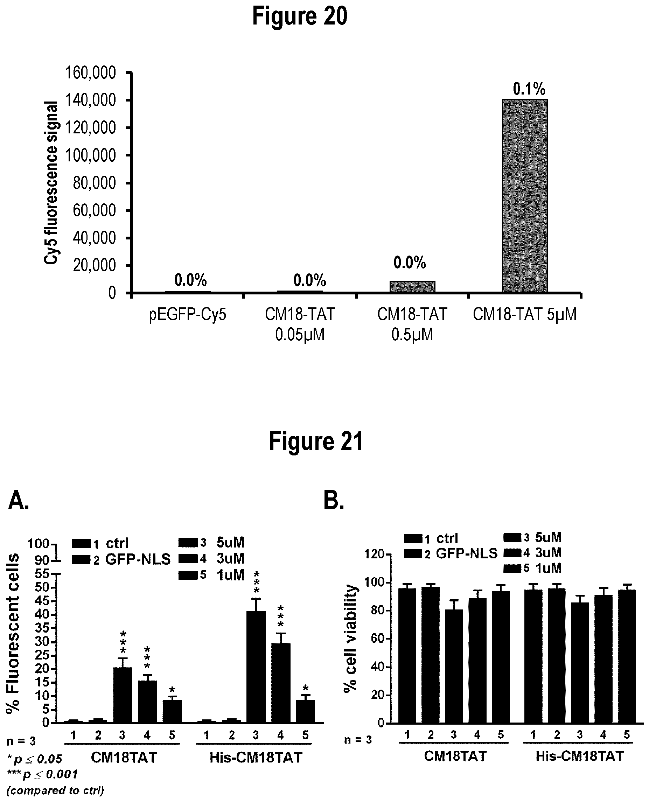

**Figure 20** shows the results of DNA transfection efficiency experiment in which plasmid DNA (pEGFP) was labeled with a Cy5™ dye was co-incubated with 0, 0.05, 0.5, or 5 µM of CM18-TAT-Cys (labeled "CM18-TAT"), prior to being exposed to HEK293A cells. Flow cytometry analysis allowed quantification of Cy5™ emission (corresponding to DNA intracellular delivery; y-axis) and GFP emission (corresponding to successful nuclear delivery of DNA; percentage indicated above each bar).
**Figure 21** shows the results of a GFP-NLS transduction efficiency experiment in which the GFP-NLS cargo protein (5 µM) was co-incubated with 1, 3, or 5 µM of CM18-TAT-Cys (labeled "CM18TAT"), of His-CM18-TAT (labeled "His-CM18TAT"), prior to being exposed to HeLa cells. Cells were evaluated by flow cytometry and the percentage of fluorescent (GFP-positive) cell is shown in panel **A,** and corresponding cell toxicity data is shown in panel **B.**

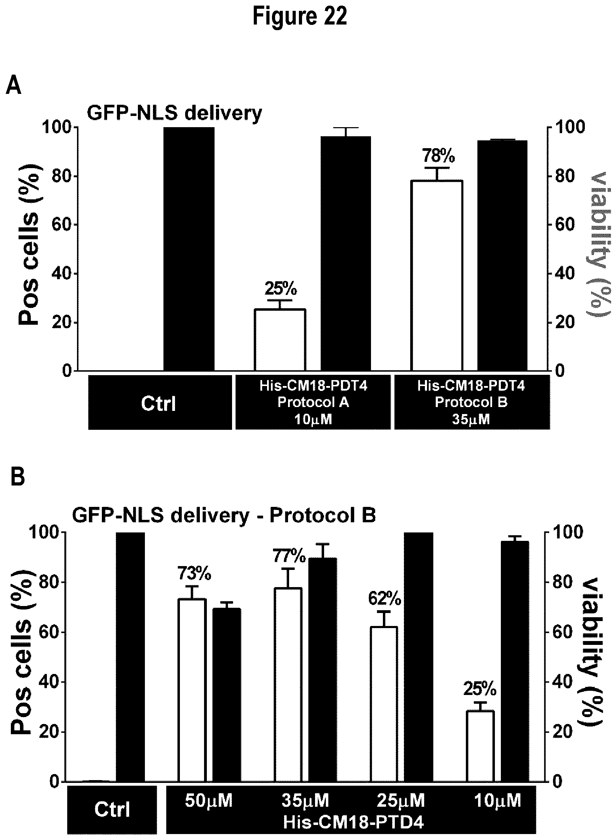

**Figure 22** shows the results of a transduction efficiency experiment in which GFP-NLS cargo protein was intracellularly delivered using the shuttle His-CM18-PTD4 in HeLa cells. GFP-NLS transduction efficiency was evaluated by flow cytometry and the percentage of GFP fluorescent cells ("Pos cells (%)"), as well as corresponding cell viability data ("viability (%)") are shown. Panel **A** shows a comparison of GFP-NLS transduction efficiencies using different transduction protocols (Protocol A vs. B). Panel **B** shows the effect of using different concentrations of the shuttle His-CM18-PTD4 when using Protocol B.

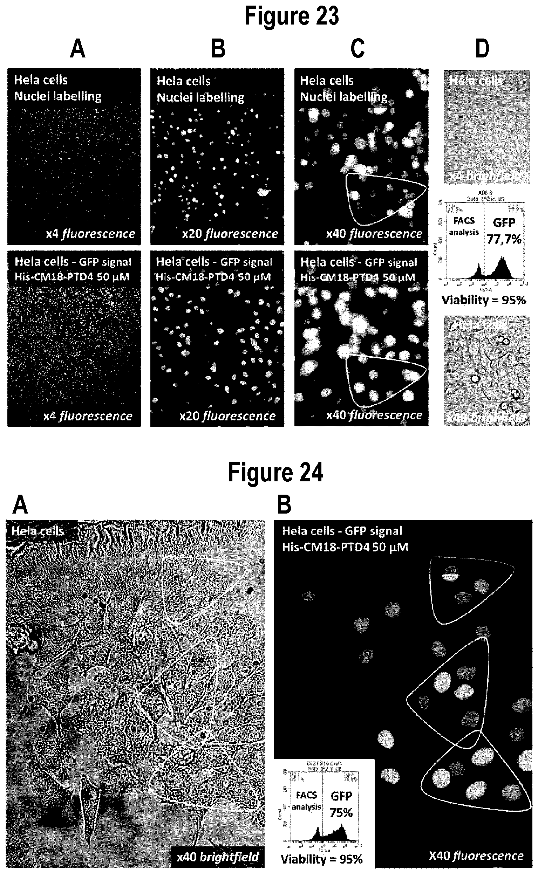

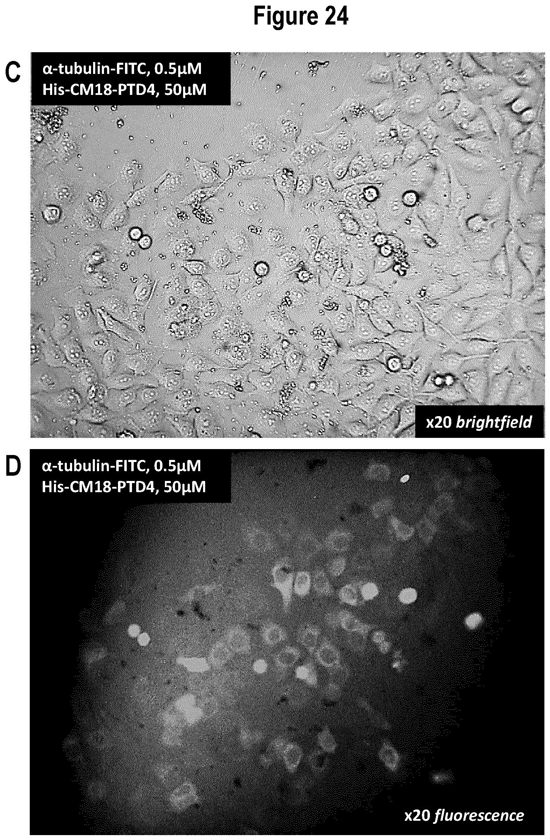

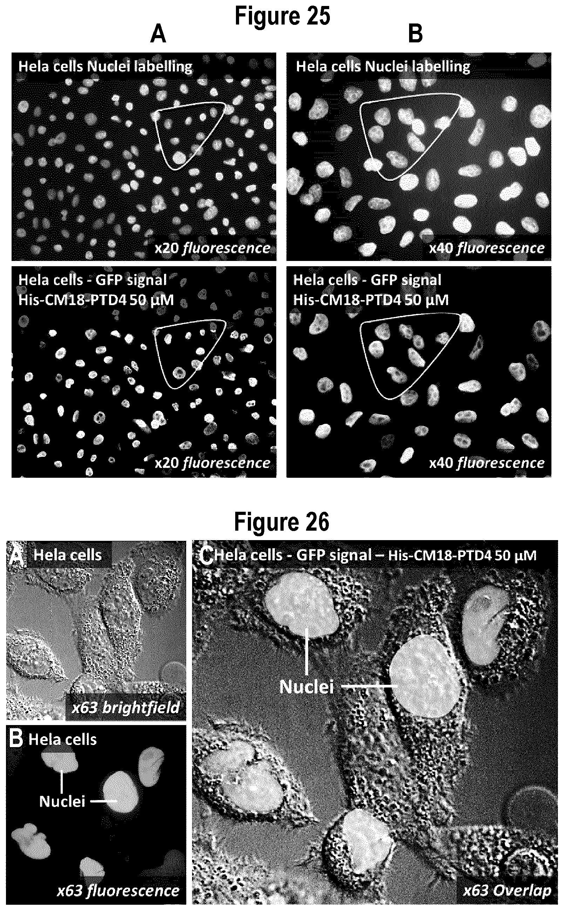

**Figures 23-26** are microscopy images showing the results of transduction experiments in which GFP-NLS **(****Figures 23, 24A, 24B****,** **25 and 26****)** or FITC-labeled anti-tubulin antibody **(****Figure 24C and 24D****)** cargo protein was intracellularly delivered with the shuttle His-CM18-PTD4 in HeLa cells. The bright field and fluorescence images of living cells are shown in **Figures 23****,** **24** **and** **26****.** In **Figure 25****,** the cells were fixed, permeabilized and subjected to immuno-labelling with an anti-GFP antibody and a fluorescent secondary antibody. White triangle windows indicate examples of areas of co-labelling between nuclei (DAPI) and GFP-NLS signals. **Figure 26** shows images captured by confocal microscopy.

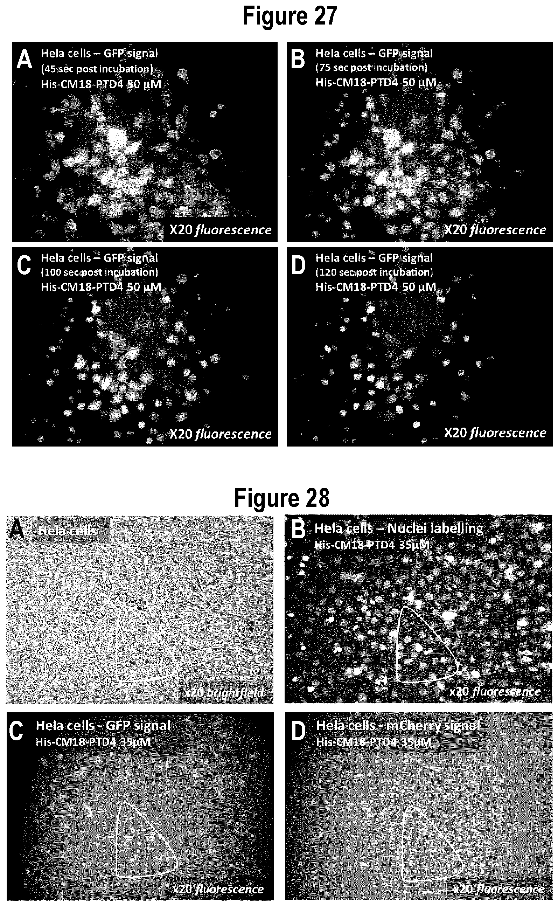

**Figure 27** shows microscopy images of a kinetic (time-course) transduction experiment in HeLa cells, where the fluorescence of GFP-NLS cargo protein was tracked after 45, 75, 100, and 120 seconds following intracellular delivery with the shuttle His-CM18-PTD4. The diffuse cytoplasmic fluorescence pattern observed after 45 seconds (panel **A**) gradually becomes a more concentrated nuclear pattern at 120 seconds (panel **D**).
**Figure 28** shows microscopy images of co-delivery transduction experiment in which two cargo proteins (GFP-NLS and mCherry™-NLS) are simultaneously delivered intracellularly by the shuttle His-CM18-PTD4 in HeLa cells. Cells and fluorescent signals were visualized by **(A)** bright field and **(B-D)** fluorescence microscopy. White triangle windows indicate examples of areas of co-labelling between nuclei (DAPI) and GFP-NLS or mCherry™.

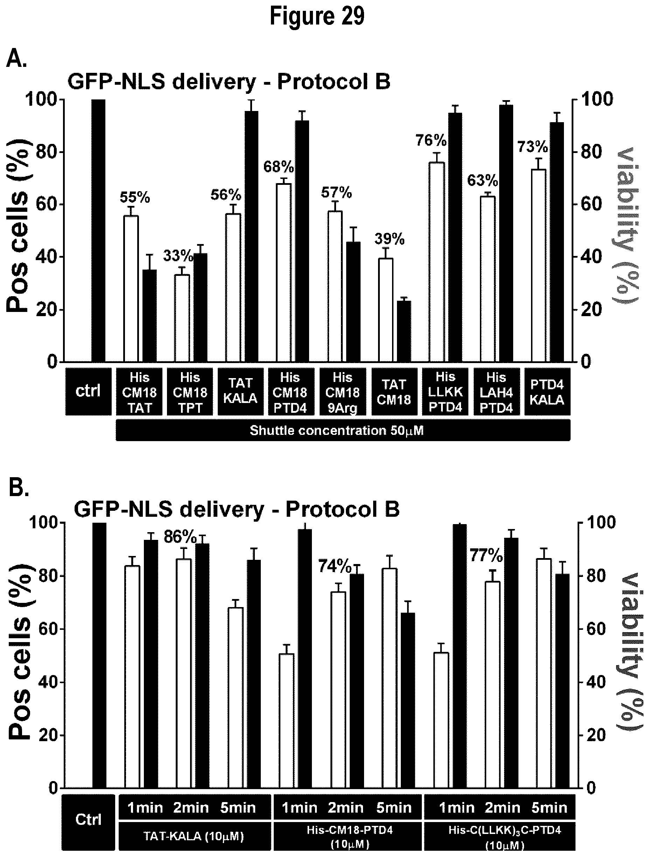

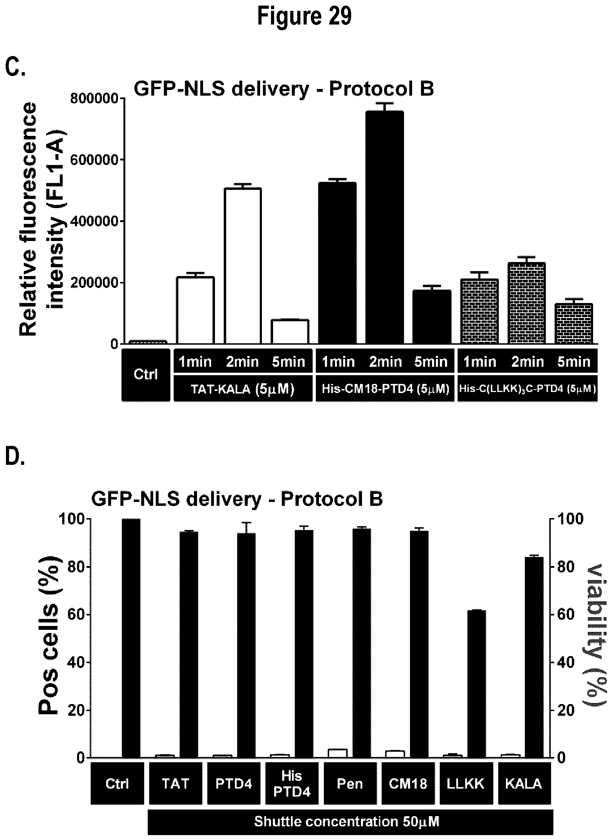

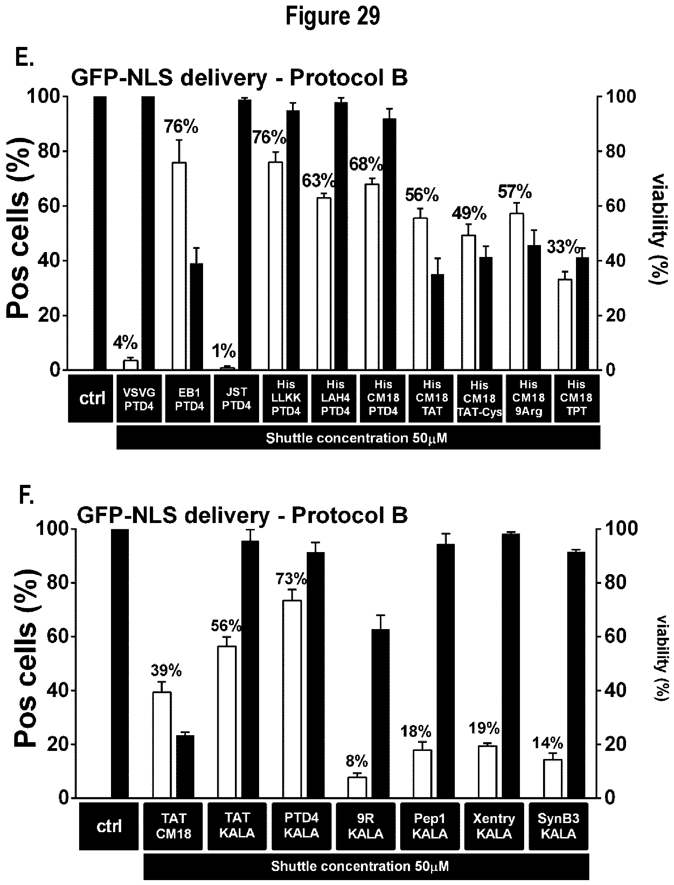

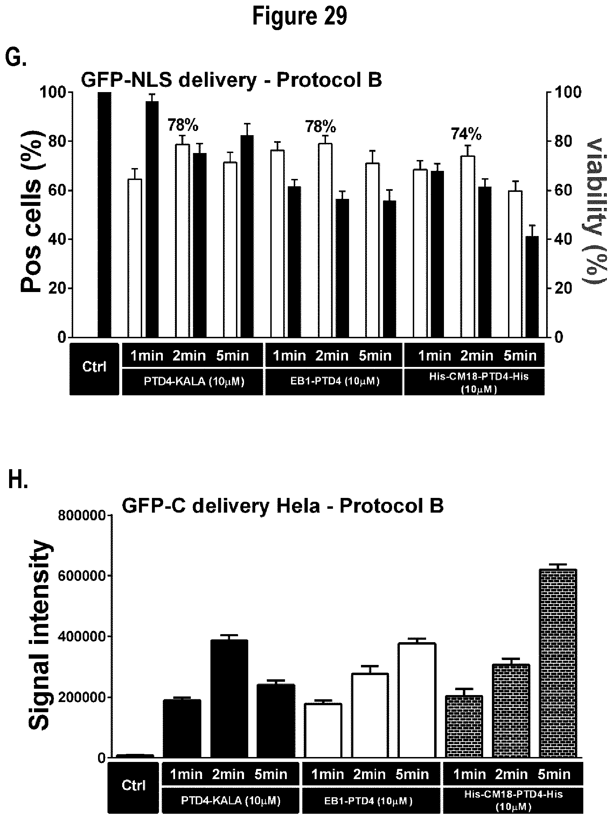

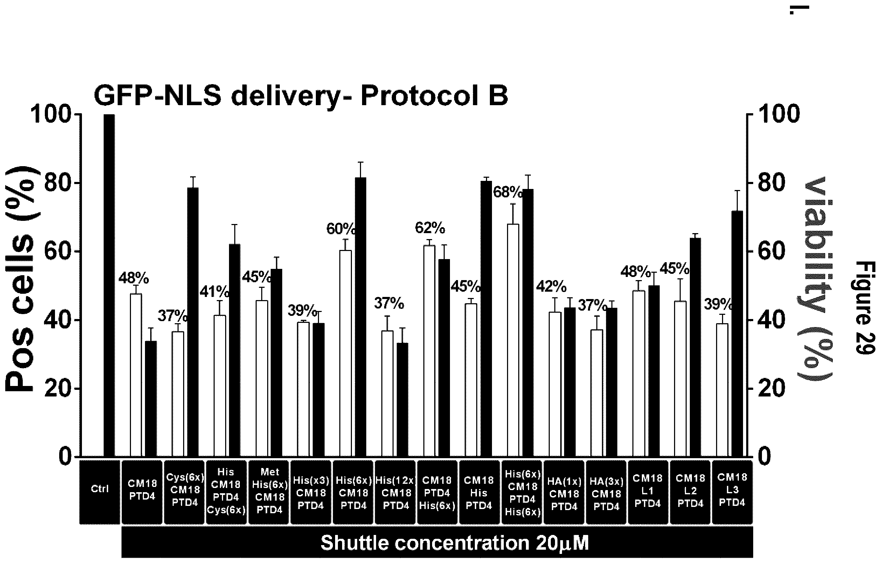

**Figure 29** shows the results of GFP-NLS transduction efficiency experiments in HeLa cells using different shuttle agents or single-domain/control peptides. GFP-NLS transduction efficiency was evaluated by flow cytometry and the percentage of GFP fluorescent cells ("Pos cells (%)"), as well as corresponding cell viability data ("viability (%)") are shown in panels A, B, D-G and I. In panels A and D-F, cells were exposed to the cargo/shuttle agent for 10 seconds. In panel I, cells were exposed to the cargo/shuttle agent for 1 minute. In panels B, C, G and H, cells were exposed to the cargo/shuttle agent for 1, 2, or 5 min. "Relative fluorescence intensity (FL1-A)" or "Signal intensity" corresponds to the mean of all fluorescence intensities from each cell with a GFP fluorescent signal after GFP-NLS fluorescent protein delivery with the shuttle agent. Panel D shows the results of a control experiment in which only single-domain peptides (ELD or CDP) or the peptide His-PTD4 (His-CPD) were used for the GFP-NLS transduction, instead of the multi-domain shuttle agents.

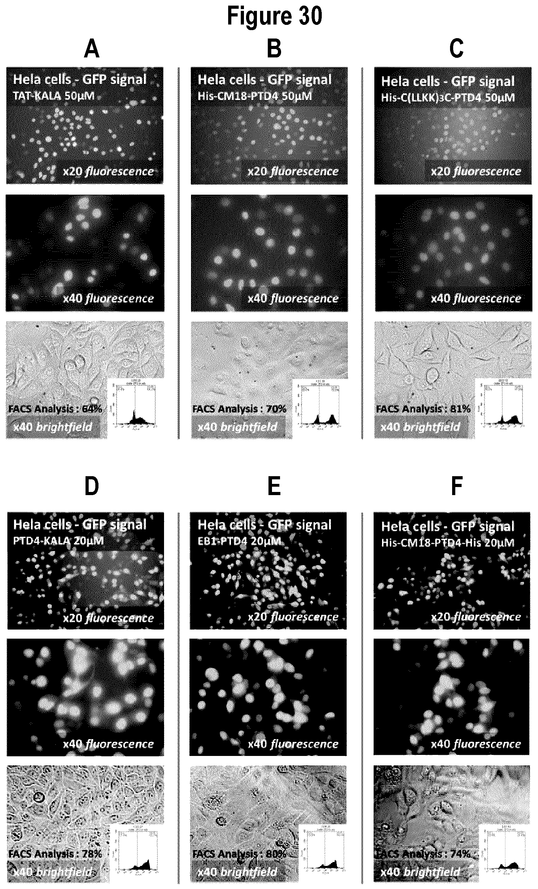

**Figure 30** shows microscopy images of HeLa cells transduced with GFP-NLS using the shuttle agent **(A)** TAT-KALA, **(B)** His-CM18-PTD4, **(C)** His-C(LLKK)₃C-PTD4, **(D)** PTD4-KALA, **(E)** EB1-PTD4, and **(F)** His-CM18-PTD4-His. The insets in the bottom row panels show the results of corresponding flow cytometry analyses, indicating the percentage of cells exhibiting GFP fluorescence.

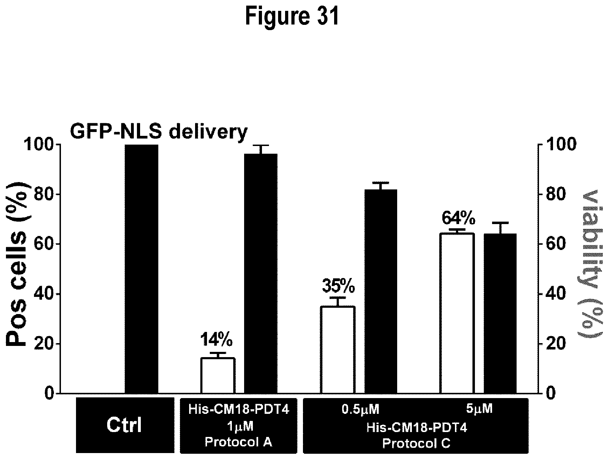

**Figure 31** shows the results of a transduction efficiency experiment in which GFP-NLS cargo protein was intracellularly delivered using the shuttle His-CM18-PTD4 in THP-1 cells using different Protocols (Protocol A vs C). GFP-NLS transduction efficiency was evaluated by flow cytometry and the percentage of GFP fluorescent cells ("Pos cells (%)"), as well as corresponding cell viability data ("viability (%)") are shown. "Ctrl" corresponds to THP-1 cells exposed to GFP-NLS cargo protein in the absence of a shuttle agent.

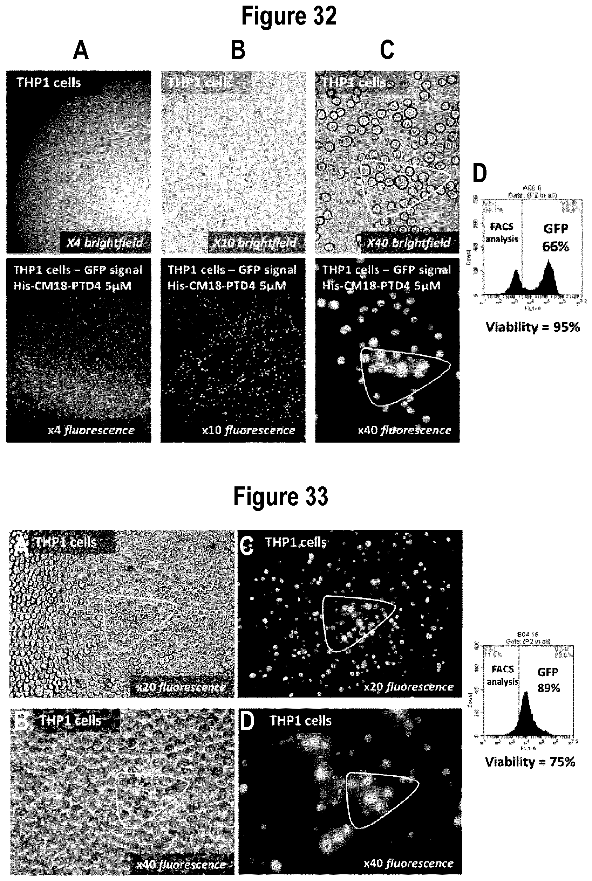

**Figure 32** shows microscopy images of THP-1 cells transduced with GFP-NLS cargo protein using the shuttle His-CM18-PTD4. Images captured under at 4x, 10x and 40x magnifications are shown in panels **A-C,** respectively. White triangle windows in panel **C** indicate examples of areas of co-labelling between cells (bright field) and GFP-NLS fluorescence. Panel **D** shows the results of corresponding flow cytometry analyses, indicating the percentage of cells exhibiting GFP fluorescence.
**Figure 33** shows microscopy images of THP-1 cells transduced with GFP-NLS cargo protein using the shuttle His-CM18-PTD4. White triangle windows indicate examples of areas of co-labelling between cells (bright field; panels **A** and **B**), and GFP-NLS fluorescence (panels **C** and **D**).

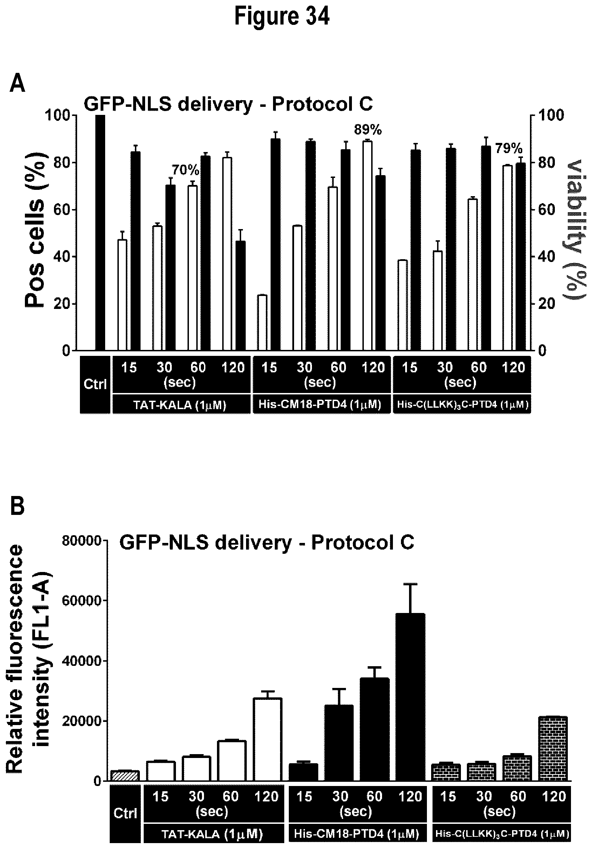

**Figure 34** shows the results of GFP-NLS transduction efficiency experiments in THP-1 cells using the shuttle TAT-KALA, His-CM18-PTD4, or His-C(LLKK)₃C-PTD4. The cargo protein/shuttle agents were exposed to the THP-1 cells for 15, 30, 60 or 120 seconds. GFP-NLS transduction efficiency was evaluated by flow cytometry and the percentage of GFP fluorescent cells ("Pos cells (%)"), as well as corresponding cell viability data ("viability (%)") are shown in panel **A.** In panel **B,** "Relative fluorescence intensity (FL1-A)" corresponds to the mean of all fluorescence intensities from each cell with a GFP fluorescent signal after GFP-NLS fluorescent protein delivery with the shuttle agent.

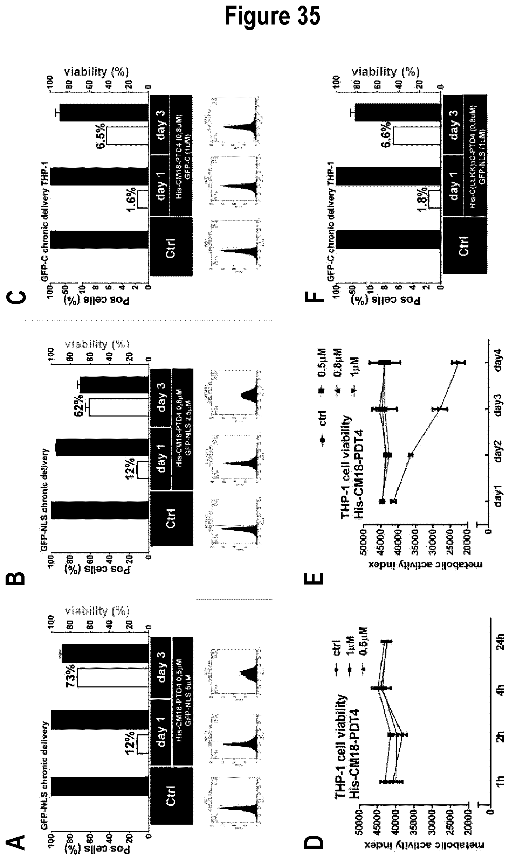

**Figure 35** shows the results of transduction efficiency experiments in which THP-1 cells were exposed daily to GFP-NLS cargo in the presence of a shuttle agent for 2.5 hours. His-CM18-PTD4 was used in panels **A-E,** and His-C(LLKK)₃C-PTD4 was used in panel **F.** GFP-NLS transduction efficiency was determined by flow cytometry at Day 1 or Day 3, and the results are expressed as the percentage of GFP fluorescent cells ("Pos cells (%)"), as well as corresponding cell viability data ("viability (%)") in panels **A-C** and **F.** Panel **D** shows the metabolic activity index of the THP-1 cells after 1, 2, 4, and 24h, and panel **E** shows the metabolic activity index of the THP-1 cells after 1 to 4 days, for cells exposed to the His-CM18-PTD4 shuttle.

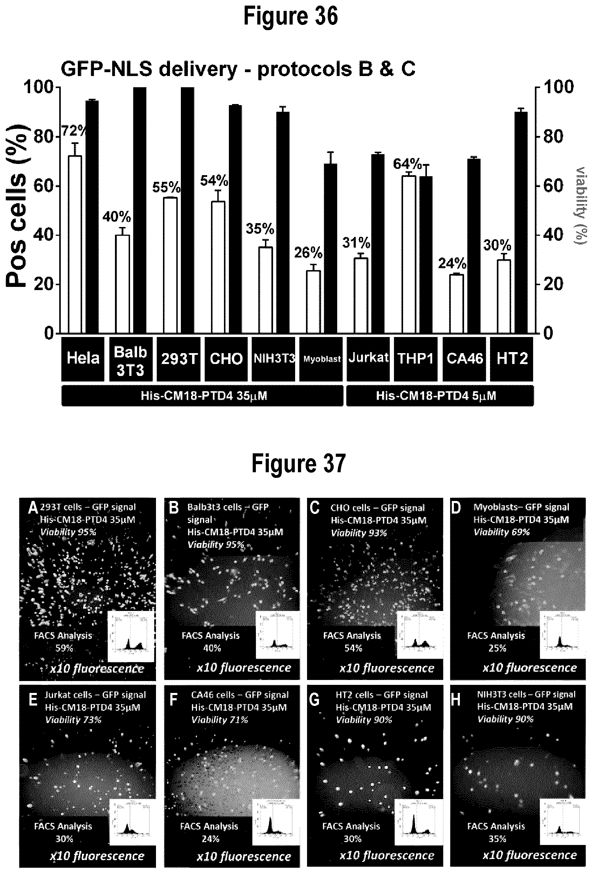

**Figure 36** shows a comparison of the GFP-NLS transduction efficiencies in a plurality of different types of cells (e.g., adherent and suspension, as well as cell lines and primary cells) using the shuttle His-CM18-PTD4, as measured by flow cytometry. The results are expressed as the percentage of GFP fluorescent cells ("Pos cells (%)"), as well as corresponding cell viability data ("viability (%)").
**Figure 37** shows fluorescence microscopy images of different types of cells **(A-H)** transduced with GFP-NLS cargo using the shuttle His-CM18-PTD4. GFP fluorescence was visualized by fluorescence microscopy at a 10x magnification. The results of parallel flow cytometry experiments are also provided in the insets (viability and percentage of GFP-fluorescing cells).

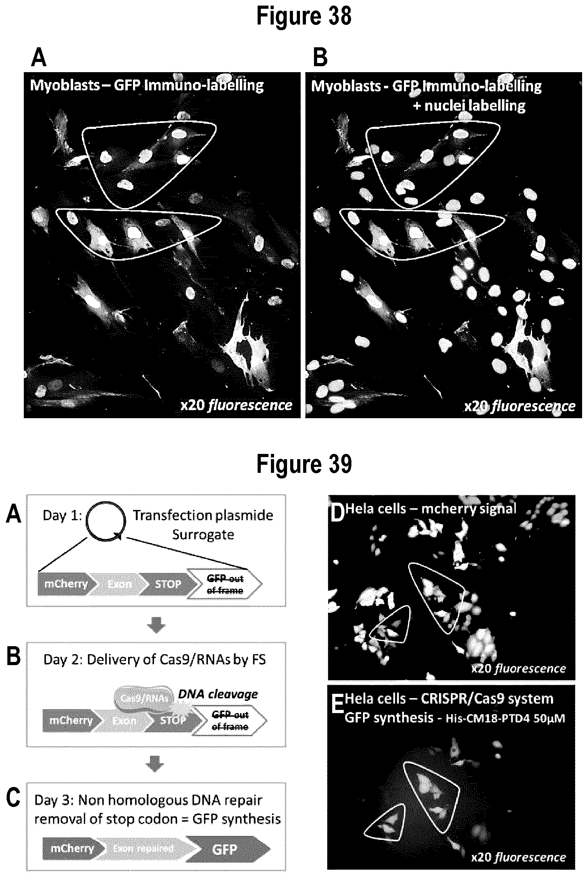

**Figure 38** shows fluorescence microscopy images of primary human myoblasts transduced with GFP-NLS using the shuttle His-CM18-PTD4. Cells were fixed and permeabilized prior to immuno-labelling GFP-NLS with an anti-GFP antibody and a fluorescent secondary antibody. Immuno-labelled GFP is shown in panel **A,** and this image is overlaid with nuclei (DAPI) labelling in panel **B.**
**Figure 39** shows a schematic layout (**A, B** and **C**) and sample fluorescence images (**D** and **E**) of a transfection plasmid surrogate assay used to evaluate the activity of intracellularly delivered CRISPR/Cas9-NLS complex. (**A**) At Day 1, cells are transfected with an expression plasmid encoding the fluorescent proteins mCherry™ and GFP, with a STOP codon separating their two open reading frames. Transfection of the cells with the expression plasmid results in only mCherry™ expression (**D**). A CRISPR/Cas9-NLS complex, which has been designed/programmed to cleave the plasmid DNA at the STOP codon, is then delivered intracellularly to the transfected cells expressing mCherry™, resulting double-stranded cleavage of the plasmid DNA at the STOP codon **(B).** In a fraction of the cells, random non-homologous DNA repair of the cleaved plasmid occurs and results in removal of the STOP codon **(C),** and thus GFP expression and fluorescence **(E).** White triangle windows indicate examples of areas of co-labelling of mCherry™ and GFP fluorescence.

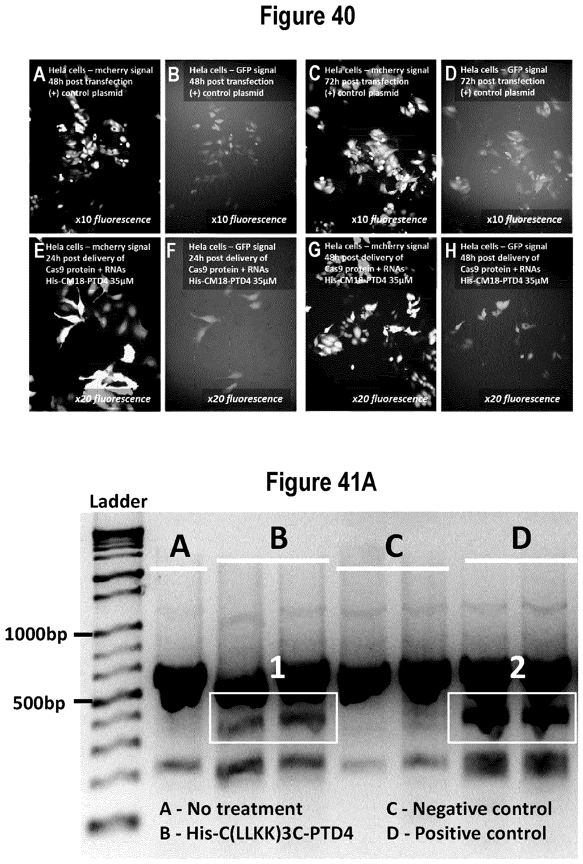

**Figure 40** shows fluorescence microscopy images of HeLa cells expressing mCherry™ and GFP, indicating CRISPR/Cas9-NLS-mediated cleavage of plasmid surrogate DNA. In panels **A-D,** HeLa cells were co-transfected with three plasmids: the plasmid surrogate as described in the brief description of **Figure 39****,** and two other expression plasmids encoding the Cas9-NLS protein and crRNA/tracrRNAs, respectively. CRISPR/Cas9-mediated cleavage of the plasmid surrogate at the STOP codon, and subsequent DNA repair by the cell, enables expression of GFP (panels **B** and **D**) in addition to mCherry™ (panels **A** and **C**). In panels **E-H,** HeLa cells were transfected with the plasmid surrogate and then transduced with an active CRISPR/Cas9-NLS complex using the shuttle His-CM18-PTD4. CRISPR/Cas9-NLS-mediated cleavage of the plasmid surrogate at the STOP codon, and subsequent DNA repair by the cell, enables expression of GFP (panels **F** and **H**) in addition to mCherry™ (panels **E** and **G**).
**Figure 41A** shows the products of a DNA cleavage assay (T7E1 assay) separated by agarose gel electrophoresis, which is used to measure CRISPR/Cas9-mediated cleavage of cellular genomic DNA. HeLa cells were transduced with a CRISPR-Cas9-NLS complex programmed to cleave the PPIB gene. The presence of the cleavage product framed in white boxes 1 and 2, indicates cleavage of the PPIB gene by the CRISPR-Cas9-NLS complex, which was delivered intracellularly using the shuttle His-C(LLKK)₃C-PTD4 (lane **B**) or with a lipidic transfection agent used as a positive control (lane **D**). This cleavage product is absent in negative controls (lanes **A** and **C**).

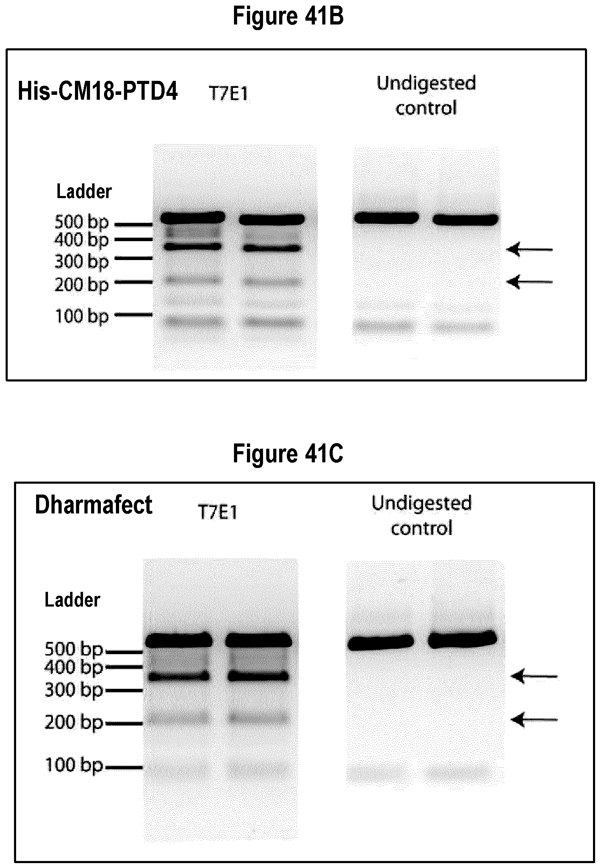

**Figure 41B** shows the products of a DNA cleavage assay (T7E1 assay) separated by agarose gel electrophoresis, which is used to measure CRISPR/Cas9-mediated cleavage of cellular genomic DNA (PPIB DNA sequences). The left panel shows the cleavage product of the amplified PPIB DNA sequence by the CRIPR/Cas9 complex after the delivery of the complex with the shuttle agent His-CM18-PTD4 in HeLa cells. The right panel shows amplified DNA sequence before the T7E1 digestion procedure as a negative control.

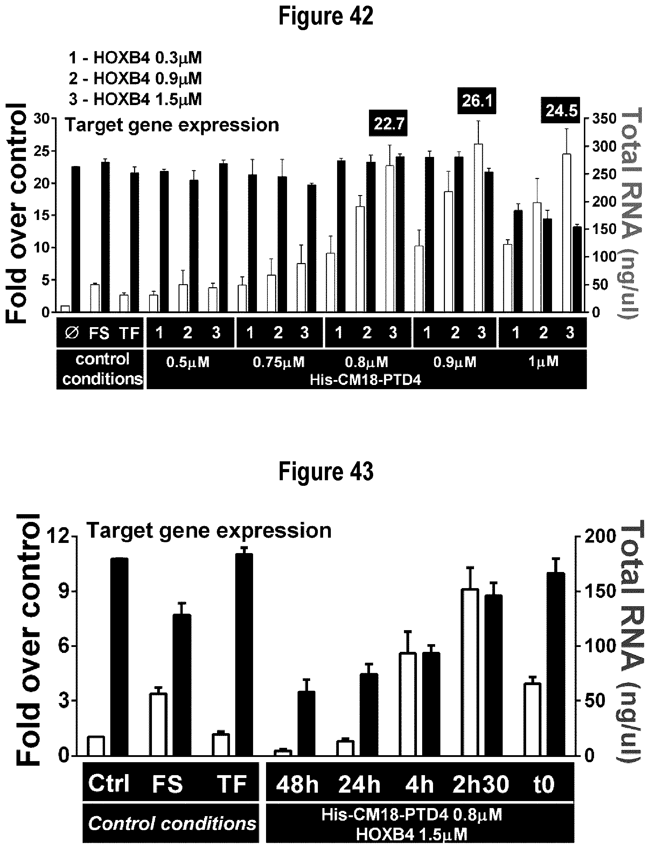

**Figure 41C** shows the products of a DNA cleavage assay (T7E1 assay) separated by agarose gel electrophoresis, which is used to measure CRISPR/Cas9-mediated cleavage of cellular genomic DNA (PPIB DNA sequences). The left panel shows the amplified PPIB DNA sequence after incubation of the HeLa cells with the Cas9/RNAs complex in presence of a lipidic transfection agent (DharmaFect™ transfection reagent # T-20XX-01) (positive control). The right panel shows amplified DNA sequence before the T7E1 digestion procedure as a negative control. **Figures 42-44** show the transcriptional activity of THP-1 cells that have been transduced with the transcription factor HOXB4 using different concentrations of the shuttle His-CM18-PTD4 and different cargo/shuttle exposure times. Successful intra-nuclear delivery of HOXB4 was determined by monitoring mRNA levels of a target gene by real-time PCR, and the results are normalized against those in the negative control (HOXB4 without shuttle agent) and expressed as "Fold over control" (left bars). Total cellular RNA (ng/µL) was quantified and used a marker for cell viability (right bars). "Ø" or "Ctrl" means "no treatment"; "TF" means "Transcription Factor alone"; "FS" means "shuttle alone".

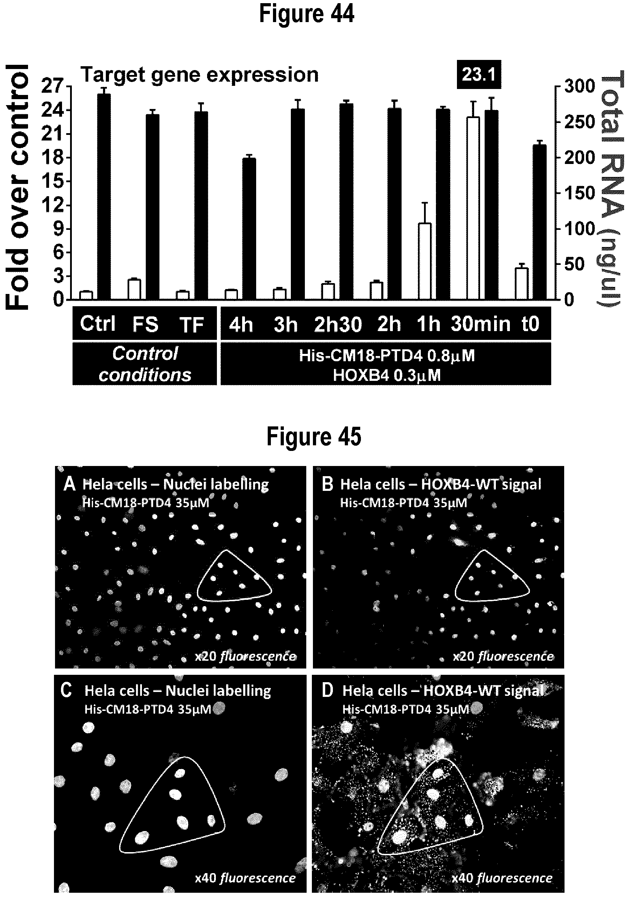

**Figure 45** shows fluorescence microscopy images of HeLa cells transduced with wild-type HOXB4 cargo using the shuttle His-CM18-PTD4. After a 30-minute incubation to allow transduced HOXB4-WT to accumulate in the nucleus, the cells were fixed, permeabilized and HOXB4-WT was labelled using a primary anti-HOXB4 monoclonal antibody and a fluorescent secondary antibody (panels **B** and **D**). Nuclei were labelled with DAPI (panels **A** and **C**). White triangle windows indicate examples of areas of co-labelling between nuclei and HOXB4 - compare **A** vs **B** (x20 magnification), and **C** vs **D** (x40 magnification).

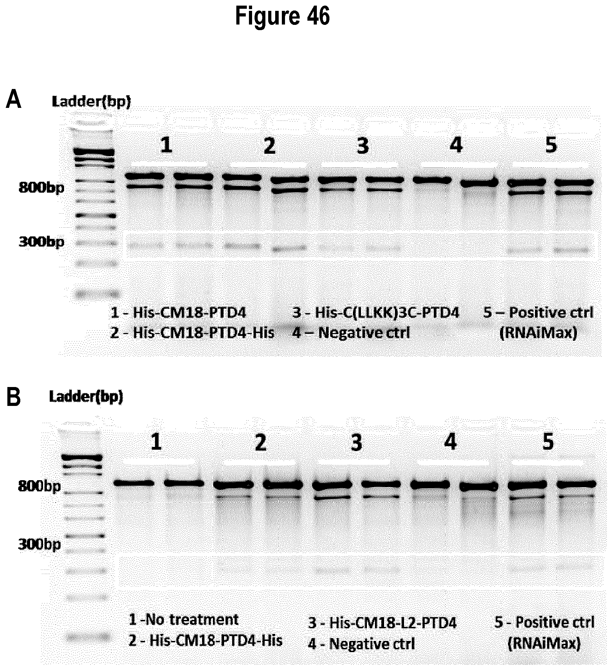

**Figure 46** shows the products of a DNA cleavage assay separated by agarose gel electrophoresis, which is used to measure CRISPR/Cas9-mediated cleavage of cellular genomic DNA (HPTR sequence) after intracellular delivery of the complex with different shuttle agents. Panel **A** shows the results with the shuttle agents: His-CM18-PTD4, His-CM18-PTD4-His, and His-C(LLKK)3C-PTD4 in HeLa cells. Panel **B** shows the results with His-CM18-PTD4-His and His-CM18-L2-PTD4 in Jurkat cells. Negative controls (lane 4 in panels A and B) show amplified HPTR DNA sequence after incubation of the cells with the CRISPR/Cas9 complex without the presence of the shuttle agent. Positive controls (lane 5 in panels A and B) show the amplified HPTR DNA sequence after incubation of the cells with the Cas9/RNAs complex in presence of a commercial lipidic transfection agent.

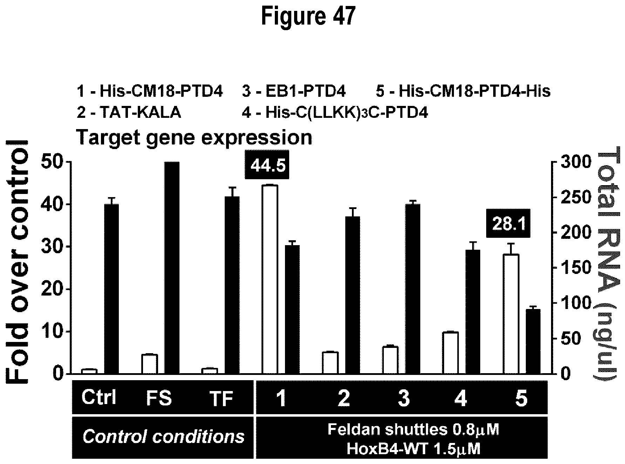

**Figure 47** shows the transcriptional activity of THP-1 cells that have been transduced with the transcription factor HOXB4 using the shuttle agents His-CM18-PTD4, TAT-KALA, EB1-PTD4, His-C(LLKK)3C-PTD4 and His-CM18-PTD4-His. Successful intra-nuclear delivery of HOXB4 was determined by monitoring mRNA levels of a target gene by real-time PCR, and the results were normalized against those in the negative control (HOXB4 without shuttle agent) and expressed as "Fold over control" (left bars). Total cellular RNA (ng/µL) was quantified and used a marker for cell viability (right bars). "Ø" or "Ctrl" means "no treatment"; "TF" means "Transcription Factor alone"; "FS" means "shuttle alone".

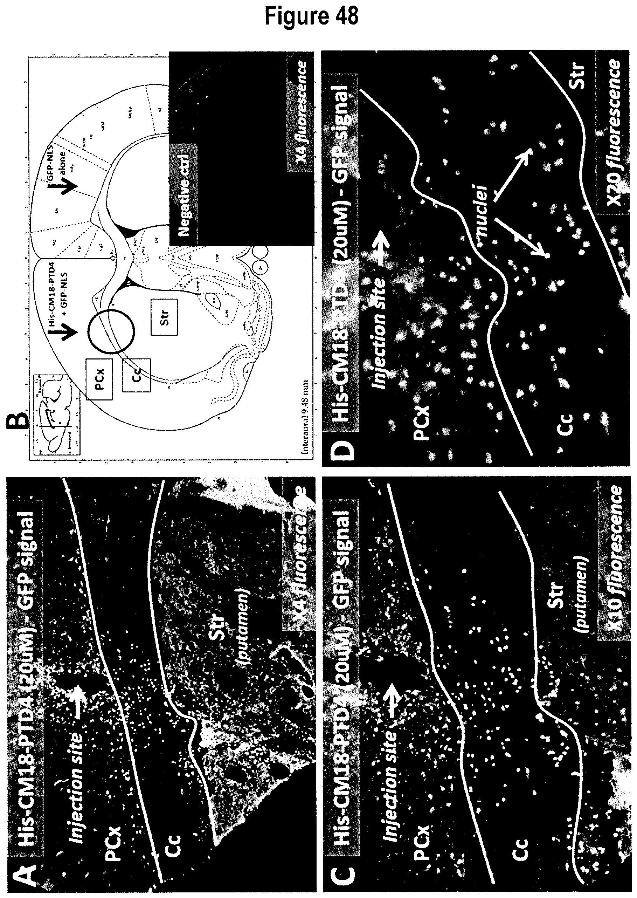

**Figure 48** shows *in vivo* GFP-NLS delivery in rat parietal cortex by His-CM18-PTD4. Briefly, GFP-NLS (20 µM) was injected in the parietal cortex of rat in presence of the shuttle agent His-CM18-PTD4 (20 µM) for 10 min. Dorso-ventral rat brain slices were collected and analysed by fluorescence microscopy at **(A)** 4x, **(C)** 10x and **(D)** 20x magnifications. The injection site is located in the deepest layers of the parietal cortex (PCx). In presence of the His-CM18-PTD4 shuttle agent, the GFP-NLS diffused in cell nuclei of the PCx, of the Corpus Callus (Cc) and of the striatum (Str) (white curves mark limitations between brains structures). Panel **B** shows the stereotaxic coordinates of the injection site (black arrows) from the rat brain atlas of Franklin and Paxinos. The injection of GFP-NLS in presence of His-CM18-PTD4 was performed on the left part of the brain, and the negative control (injection of GFP-NLS alone), was done on the contralateral site. The black circle and connected black lines in panel **B** show the areas observed in the fluorescent pictures (A, C and D).

### SEQUENCE LISTING

This application contains a Sequence Listing in computer readable form entitled Sequence_Listing.txt, created April 3, 2016 having a size of about 57 kb. The computer readable form is incorporated herein by reference.

| **SEQ ID NO:** | **Description** | **SEQ ID NO:** | **Description** |
|---|---|---|---|
| **1** | CM18 | **53** | Mitochondrial signal sequence from 18S rRNA |
| **2** | Diphtheria toxin T domain (DT) | **54** | Peroxisome signal sequence - PTS1 |
| **3** | GALA | **55** | Nucleolar signal sequence from BIRC5 |
| **4** | PEA | **56** | Nucleolar signal sequence from RECQL4 |
| **5** | INF-7 | **57** | CM18-TAT |
| **6** | LAH4 | **58** | CM18-Penetratin |
| **7** | HGP | **59** | His-CM18-TAT |
| **8** | H5WYG | **60** | GFP |
| **9** | HA2 | **61** | TAT-GFP |
| **10** | EB1 | **62** | GFP-NLS |
| **11** | VSVG | **63** | C(LLKK)₃C |
| **12** | Pseudomonas toxin | **64** | G(LLKK)₃G |
| **13** | Melittin | **65** | PTD4 |
| **14** | KALA | **66** | TAT-CM18 |
| **15** | JST-1 | **67** | TAT-KALA |
| **16** | SP | **68** | His-CM 18-PTD4 |
| **17** | TAT | **69** | His-CM18-9Arg |
| **18** | Penetratin (Antennapedia) | **70** | His-CM 18-Transportan |
| **19** | pVEC | **71** | His-LAH4-PTD4 |
| **20** | M918 | **72** | His-C(LLKK)₃C-PTD4 |
| **21** | Pep-1 | **73** | mCherry™-NLS |
| **22** | Pep-2 | **74** | Cas9-NLS |
| **23** | Xentry | **75** | crRNA (Example 13.3) |
| **24** | Arginine stretch | **76** | tracrRNA (Example 13.3) |
| **25** | Transportan | **77** | Feldan tracrRNA (Example 13.5, 13.6) |
| **26** | SynB1 | **78** | PPIB crRNA (Example 13.5) |
| **27** | SynB3 | **79** | Dharmacon tracrRNA (Example 13.5) |
| **28** | E1a | **80** | HOXB4-WT |
| **29** | SV40 T-Ag | **81** | His-PTD4 |
| **30** | c-myc | **82** | PTD4-KALA |
| **31** | Op-T-NLS | **83** | 9Arg-KALA |
| **32** | Vp3 | **84** | Pep1-KALA |
| **33** | Nucleoplasmin | **85** | Xentry-KALA |
| **34** | Histone 2B NLS | **86** | SynB3-KALA |
| **35** | Xenopus N1 | **87** | VSVG-PTD4 |
| **36** | PARP | **88** | EB1-PTD4 |
| **37** | PDX-1 | **89** | JST-PTD4 |
| **38** | QKI-5 | **90** | CM18-PTD4 |
| **39** | HCDA | **91** | 6Cys-CM18-PTD4 |
| **40** | H2B | **92** | CM18-L1-PTD4 |
| **41** | v-Rel | **93** | CM18-L2-PTD4 |
| **42** | Amida | **94** | CM18-L3-PTD4 |
| **43** | RanBP3 | **95** | His-CM18-TAT |
| **44** | Pho4p | **96** | His-C M 18-PTD4-6Cys |
| **45** | LEF-1 | **97** | 3His-CM18-PTD4 |
| **46** | TCF-1 | **98** | 12His-CM18-PTD4 |
| **47** | BDV-P | **99** | HA-CM18-PTD4 |
| **48** | TR2 | **100** | 3HA-CM18-PTD4 |
| **49** | SOX9 | **101** | CM18-His-PTD4 |
| **50** | Max | **102** | His-CM 18-PTD4-His |
| **51** | Mitochondrial signal sequence from Tim9 | **103** | HPRT crRNA (Example 13.6) |
| **52** | Mitochondrial signal sequence from Yeast cytochrome c oxidase subunit IV | **104-106** | Examples of serine/glycine-rich linkers |

### DETAILED DESCRIPTION

The present invention stems from the surprising discovery that multi-domain synthetic peptides comprising an endosome leakage domain (ELD) operably linked to a cell penetrating domain (CPD) can significantly increase the transduction efficiency of an independent polypeptide cargo to the cytosol of eukaryotic target cells. In contrast, this increase in transduction efficiency was not found using independent single-domain peptides containing only an ELD, or only a CPD used alone or together (i.e., in a mixture of separate single-domain peptides). Accordingly, in some aspects the present invention relates to a polypeptide-based shuttle agent comprising an endosome leakage domain (ELD) operably linked to a cell penetrating domain (CPD), or an ELD operably linked to a histidine-rich domain and a CPD, for use in a clinical or therapeutic *in vivo* method for increasing the transduction efficiency of an independent polypeptide cargo to the cytosol of a target eukaryotic cell, wherein the cargo is a biologically active polypeptide, and wherein the polypeptide based shuttle agent has a predicted net charge of at least +6 at a physiological pH and is used at a concentration sufficient to increase the transduction efficiency of the independent polypeptide cargo at the cytosol of target eukaryotic cell.

### Synthetic peptides and polypeptide-based shuttle agents

As used herein, the term **"synthetic"** used in expressions such as "synthetic peptide" or"synthetic polypeptide" is intended to refer to non-naturally occurring molecules that can be produced *in vitro* (e.g., synthesized chemically and/or produced using recombinant DNA technology). The purities of various synthetic preparations may be assessed by for example high-performance liquid chromatography analysis and mass spectroscopy. Chemical synthesis approaches may be advantageous over cellular expression systems (e.g., yeast or bacteria protein expression systems), as they may preclude the need for extensive recombinant protein purification steps (e.g., required for clinical use). In contrast, longer synthetic polypeptides may be more complicated and/or costly to produce via chemical synthesis approaches and such polypeptides may be more advantageously produced using cellular expression systems. In some embodiments, the peptides or shuttle agent of the present invention may be chemically synthesized (e.g., solid- or liquid phase peptide synthesis), as opposed to expressed from a recombinant host cell. In some embodiments, the peptides or shuttle agent of the present description may lack an N-terminal methionine residue. A person of skill in the art may adapt a synthetic peptide or shuttle agent of the present invention by using one or more modified amino acids (e.g., non-naturally-occurring amino acids), or by chemically modifying the synthetic peptide or shuttle agent of the present description, to suit particular needs of stability or other needs.

The expression **"polypeptide-based"** when used here in the context of a shuttle agent of the present invention, is intended to distinguish the presently described shuttle agents from non-polypeptide or non-protein-based shuttle agents such as lipid- or cationic polymer-based transduction agents, which are often associated with increased cellular toxicity and may not be suitable for use in human therapy.

As used herein, the expression **"increasing transduction efficiency"** refers to the ability of a shuttle agent (e.g., a polypeptide-based shuttle agent of the present description) to improve the percentage or proportion of a population of target cells into which a cargo of interest (e.g., a polypeptide cargo) is delivered intracellularly across the plasma membrane. Immunofluorescence microscopy, flow cytometry, and other suitable methods may be used to assess cargo transduction efficiency. In some embodiments, a shuttle agent of the present invention may enable a transduction efficiency of at least 25%, 30%, 35%, 40%, 45%, 50%, 55%, 60%, 65%, 70%, 75%, 80%, or 85%, for example as measure by immunofluorescence microscopy, flow cytometry, FACS, and other suitable methods. In some embodiments, a shuttle agent of the present invention may enable one of the aforementioned transduction efficiencies together wish a cell viability of at least 25%, 30%, 35%, 40%, 45%, 50%, 55%, 60%, 65%, 70%, 75%, 80%, 85%, 90%, or 95%, for example as measure by the assay described in **Example 3.3a,** or by another suitable assay known in the art.

As used herein, the term **"independent"** is generally intended refer to molecules or agents which are not covalently bound to one another. For example, the expression **"independent polypeptide cargo"** is intended to refer to a polypeptide cargo to be delivered intracellularly that is not covalently bound (e.g., not fused) to a shuttle agent of the present description. In some aspects, having shuttle agents that are independent of (not fused to) a polypeptide cargo may be advantageous by providing increased shuttle agent versatility -- e.g., not being required to re-engineer a new fusion protein for different polypeptide cargoes, and/or being able to readily vary the ratio of shuttle agent to cargo (as opposed to being limited to a 1:1 ratio in the case of a fusion protein).

In addition to increasing target cell transduction efficiency, shuttle agents of the present invention may facilitate the delivery of a cargo of interest (e.g., a polypeptide cargo) to the cytosol of target cells. In this regard, efficiently delivering an extracellular cargo to the cytosol of a target cell using approaches based on cell penetrating peptides can be challenging, as the cargo often becomes trapped in intracellular endosomes after crossing the plasma membrane, which may limit its intracellular availability and may result in its eventual metabolic degradation. For example, use of the protein transduction domain from the HIV-1 Tat protein has been reported to result in massive sequestration of the cargo into intracellular vesicles. In some aspects, shuttle agents of the present description may facilitate the ability of endosomally-trapped cargo to escape from the endosome and gain access to the cytoplasmic compartment. In this regard, the expression **"to the cytosol"** in the phrase "increasing the transduction efficiency of an independent polypeptide cargo to the cytosol," is intended to refer to the ability of shuttle agents of the present description to allow an intracellularly delivered cargo of interest to escape endosomal entrapment and gain access to the cytoplasmic compartment. After a cargo of interest has gained access to the cytosol, it may be subsequently targeted to various subcellular compartments (e.g., nucleus, nucleolus, mitochondria, peroxisome). In some embodiments, the expression "to the cytosol" is thus intended to encompass not only cytosolic delivery, but also delivery to other subcellular compartments that first require the cargo to gain access to the cytoplasmic compartment.

As used herein, a **"domain"** or "protein domain" generally refers to a part of a protein having a particular functionality or function. Some domains conserve their function when separated from the rest of the protein, and thus can be used in a modular fashion. By combining such domains from different proteins of viral, bacterial, or eukaryotic origin, it becomes possible in accordance with the present invention to not only design multi-domain polypeptide-based shuttle agents that are able to deliver a cargo intracellularly, but also enable the cargo to escape endosomes and reach the cytoplasmic compartment.

The modular characteristic of many protein domains can provide flexibility in terms of their placement within the shuttle agents of the present description. However, some domains may perform better when engineered at certain positions of the shuttle agent (e.g., at the N- or C-terminal region, or therebetween). The position of the domain within its endogenous protein is sometimes an indicator of where the domain should be engineered within the shuttle agent, and of what type/length of linker should be used. Standard recombinant DNA techniques can be used by the skilled person to manipulate the placement and/or number of the domains within the shuttle agents of the present description in view of the present disclosure. Furthermore, assays disclosed herein, as well as others known in the art, can be used to assess the functionality of each of the domains within the context of the shuttle agents (e.g., their ability to facilitate cell penetration across the plasma membrane, endosome escape, and/or access to the cytosol). Standard methods can also be used to assess whether the domains of the shuttle agent affect the activity of the cargo to be delivered intracellularly. In this regard, the expression **"operably linked"** as used herein refers to the ability of the domains to carry out their intended function(s) (e.g., cell penetration, endosome escape, and/or subcellular targeting) within the context of the shuttle agents of the present description. For greater clarity, the expression "operably linked" is meant to define a functional connection between two or more domains without being limited to a particular order or distance between same.

In some embodiments, synthetic peptide or polypeptide-based shuttle agent of the present invention may comprise a minimum length of 20, 21, 22, 23, 24, 25, 26, 27, 28, 29, or 30 amino acid residues and a maximum length of 35, 40, 45, 50, 55, 60, 65, 70, 75, 80, 85, 90, 95, 100, 105, 110, 115, 120, 125, 130, 135, 140, 145, or 150 amino acid residues. In some embodiments, shorter synthetic peptide or polypeptide-based shuttle agents are particularly advantageous because they may be more easily synthesized and purified by chemical synthesis approaches, which may be more suitable for clinical use (as opposed to recombinant proteins that must be purified from cellular expression systems). While numbers and ranges in the present invention are often listed as multiples of 5, the present description should not be so limited. For example, the maximum length described herein should be understood as also encompassing a length of 36, 37, 38...51, 62, etc., in the present invention, and that their non-listing herein is only for the sake of brevity. The same reasoning applies to the % of identities listed herein (e.g., 86%, 87%...93%...), the percentages of histidine residues, etc.

The synthetic peptide or polypeptide-based shuttle agent of the present invention comprises a predicted net charge of at least +6, +7, at least +8, at least +9, at least +10, at least +11, at least +12, at least +13, at least +14, or at least +15 at physiological pH. These positive charges are generally conferred by the greater presence of positively-charged lysine and/or arginine residues, as opposed to negatively charged aspartate and/or glutamate residues.

In some embodiments, synthetic peptide or polypeptide-based shuttle agent of the present invention may be soluble in aqueous solution (e.g., at physiological pH), which facilitates their use in for example cell culture media to delivery cargoes intracellularly to live cells.

In some embodiments, synthetic peptide or polypeptide-based shuttle agent of the present invention may comprise or consist of the amino acid sequence of any one of **SEQ ID NOs: 57-59, 66-72, or 82-102,** or a functional variant thereof having at least 65%, 66%, 67%, 68%, 69%, 70%, 71%, 72%, 73%, 74%, 75%, 76%, 77%, 78%, 79%, 80%, 81% 82%, 83%, 84%, 85%, 86%, 87%, 88%, 89%, 90%, 91%, 92%, 93%, 94%, or 95% identity to any one of **SEQ ID NOs: 57-59, 66-72, or 82-102.**

In some embodiments, synthetic peptide or polypeptide-based shuttle agents of the present invention may comprise oligomers (e.g., dimers, trimers, etc.) of a synthetic peptide or polypeptide-based shuttle agent as defined herein. Such oligomers may be constructed by covalently binding the same or different types of shuttle agent monomers (e.g., using disulfide bridges to link cysteine residues introduced into the monomer sequences).

In some embodiments, the synthetic peptide or polypeptide-based shuttle agent of the present invention may comprise an N-terminal and/or a C-terminal cysteine residue.

### Endosome leakage domains (ELDs)

A synthetic peptide or polypeptide-based shuttle agent of the present invention comprises an endosome leakage domain (ELD) for facilitating endosome escape and access to the cytoplasmic compartment. As used herein, the expression **"endosome leakage domain"** refers to a sequence of amino acids which confers the ability of endosomally-trapped macromolecules to gain access to the cytoplasmic compartment. Without being bound by theory, endosome leakage domains are short sequences (often derived from viral or bacterial peptides), which are believed to induce destabilization of the endosomal membrane and liberation of the endosome contents into the cytoplasm. As used herein, the expression **"endosomolytic peptide"** is intended to refer to this general class of peptides having endosomal membrane-destabilizing properties. Accordingly, in some embodiments, synthetic peptide or polypeptide-based shuttle agents of the present invention may comprise an ELD which is an endosomolytic peptide. The activity of such peptides may be assessed for example using the calcein endosome escape assays described in **Example 2.**

In some embodiments, the ELD may be a peptide that disrupts membranes at acidic pH, such as pH-dependent membrane active peptide (PMAP) or a pH-dependent lytic peptide. For example, the peptides GALA and INF-7 are amphiphilic peptides that form alpha helixes when a drop in pH modifies the charge of the amino acids which they contain. More particularly, without being bound by theory, it is suggested that ELDs such as GALA induce endosomal leakage by forming pores and flip-flop of membrane lipids following conformational change due to a decrease in pH (Kakudo, Chaki et al., 2004, Li, Nicol et al., 2004). In contrast, it is suggested that ELDs such as INF-7 induce endosomal leakage by accumulating in and destabilizing the endosomal membrane (EI-Sayed, Futaki et al., 2009). Accordingly in the course of endosome maturation, the concomitant decline in pH causes a change in the conformation of the peptide and this destabilizes the endosome membrane leading to the liberation of the endosome contents. The same principle is thought to apply to the toxin A of *Pseudomonas* (Varkouhi, Scholte et al., 2011). Following a decline in pH, the conformation of the domain of translocation of the toxin changes, allowing its insertion into the endosome membrane where it forms pores (London 1992, O'Keefe 1992). This eventually favors endosome destabilization and translocation of the complex outside of the endosome. The above described ELDs are encompassed within the ELDs of the present invention, as well as other mechanisms of endosome leakage whose mechanisms of action may be less well defined.

In some embodiments, the ELD may be an antimicrobial peptide (AMP) such as a linear cationic alpha-helical antimicrobial peptide (AMP). These peptides play a key role in the innate immune response due to their ability to strongly interact with bacterial membranes. Without being bound by theory, these peptides are thought to assume a disordered state in aqueous solution, but adopt an alpha-helical secondary structure in hydrophobic environments. The latter conformation thought to contribute to their typical concentration-dependent membrane-disrupting properties. When accumulated in endosomes at a certain concentrations, some antimicrobial peptides may induce endosomal leakage.

In some embodiments, the ELD may be an antimicrobial peptide (AMP) such as Cecropin-A/Melittin hybrid (CM series) peptide. Such peptides are thought to be among the smallest and most effective AMP-derived peptides with membrane-disrupting ability. Cecropins are a family of antimicrobial peptides with membrane-perturbing abilities against both Gram-positive and Gram-negative bacteria. Cecropin A (CA), the first identified antibacterial peptide, is composed of 37 amino acids with a linear structure. Melittin (M), a peptide of 26 amino acids, is a cell membrane lytic factor found in bee venom. Cecropin-melittin hybrid peptides have been shown to produce short efficient antibiotic peptides without cytotoxicity for eukaryotic cells (i.e., non-hemolytic), a desirable property in any antibacterial agent. These chimeric peptides were constructed from various combinations of the hydrophilic N-terminal domain of Cecropin A with the hydrophobic N-terminal domain of Melittin, and have been tested on bacterial model systems. Two 26-mers, CA(1-13)M(1-13) and CA(1-8) M(1-18) (Boman et al., 1989), have been shown to demonstrate a wider spectrum and improved potency of natural Cecropin A without the cytotoxic effects of melittin.

In an effort to produce shorter CM series peptides, the authors of Andreu et al., 1992 constructed hybrid peptides such as the 26-mer (CA(1-8)M(1-18)), and compared them with a 20-mer (CA(1-8)M(1-12)), a 18-mer (CA(1-8)M(1-10)) and six 15-mers ((CA(1-7)M(1-8), CA(1-7)M(2-9), CA(1-7)M(3-10), CA(1-7)M(4-11), CA(1-7)M(5-12), and CA(1-7)M(6-13)). The 20 and 18-mers maintained similar activity comparatively to CA(1-8)M(1-18). Among the six 15-mers, CA(1-7)M(1-8) showed low antibacterial activity, but the other five showed similar antibiotic potency compared to the 26-mer without hemolytic effect. Accordingly, in some embodiments, synthetic peptide or polypeptide-based shuttle agents of the present invention may comprise an ELD which is or is from CM series peptide variants, such as those described above.

In some embodiments, the ELD may be the CM series peptide CM18 composed of residues 1-7 of Cecropin-A (KWKLFKKIGAVLKVLTTG) fused to residues 2-12 of Melittin (YGRKKRRQRRR), [C(1-7)M(2-12)]. When fused to the cell penetrating peptide TAT, CM18 was shown to independently cross the plasma membrane and destabilize the endosomal membrane, allowing some endosomally-trapped cargos to be released to the cytosol (Salomone et al., 2012). However, the use of a CM18-TAT11 peptide fused to a fluorophore (atto-633) in some of the author's experiments, raises uncertainty as to the contribution of the peptide versus the fluorophore, as the use of fluorophores themselves have been shown to contribute to endosomolysis -- e.g., via photochemical disruption of the endosomal membrane (Erazo-Oliveras et al., 2014).

In some embodiments, the ELD may be CM18 having the amino acid sequence of **SEQ ID NO: 1,** or a variant thereof having at least 70%, 71%, 72%, 73%, 74%, 75%, 76%, 77%, 78%, 79%, 80%, 81%, 82%, 83%, 84%, 85%, 86%, 87%, 88%, 89%, 85%, 90%, 91%, 92%, 93%, 94%, or 95% identity to **SEQ ID NO: 1** and having endosomolytic activity.

In some embodiments, the ELD may be a peptide derived from the N terminus of the HA2 subunit of influenza hemagglutinin (HA), which may also cause endosomal membrane destabilization when accumulated in the endosome.

In some embodiments, synthetic peptide or polypeptide-based shuttle agents of the present invention may comprise an ELD which is or is from an ELD set forth in **Table A,** or a variant thereof having endosome escape activity and/or pH-dependent membrane disrupting activity.

**Table A: Examples of endosome leakage domains**

| **Name** | **Amino acid sequence** | **SEQ ID NO:** | **Reference(s)** |
|---|---|---|---|
| CM18 | KWKLFKKIGAVLKVLTTG | 1 | (Salomone, Cardarelli et al., 2012) |
| Diphtheria toxin T domain (DT) | VGSSLSCINLDWDVIRDKTKTKIESLKEHGPIK NKMSESPNKTVSEEKAKQYLEEFHQTALEHP ELSELKTVTGTNPVFAGANYAAWAVNVAQVID SETADNLEKTTAALSILPGIGSVMGIADGAVHH NTEEIVAQSIALSSLMVAQAIPLVGELVDIGFAA YNFVESIINLFQVVHNSYNRPAYSPG | 2 | (Uherek, Fominaya et al., 1998, Glover, Ng et al., 2009) |
| GALA | WEAALAEALAEALAEHLAEALAEALEALAA | 3 | (Parente, Nir et al., 1990) (Li, Nicol et al., 2004) |
| PEA | VLAGNPAKHDLDIKPTVISHRLHFPEGGSLAAL TAHQACHLPLETFTRHRQPRGWEQLEQCGYP VQRLVALYLAARLSWNQVDQVIRNALASPGS GGDLGEAIREQPEQARLALT | 4 | (Fominaya and Wels 1996) |
| INF-7 | GLFEAIEGFIENGWEGMIDGWYGC | 5 | (EI-Sayed, Futaki et al., 2009) |
| LAH4 | KKALLALALHHLAHLALHLALALKKA | 6 | (Kichler, Mason et al., 2006) Kichler et al., 2003 |
| HGP | LLGRRGWEVLKYWWNLLQYWSQEL | 7 | (Zhang, Cui et al., 2006) |
| H5WYG | GLFHAIAHFIHGGWHGLIHGWYG | 8 | (Midoux, Kichler et al., 1998) |
| HA2 | GLFGAIAGFIENGWEGMIDGWYG | 9 | (Lorieau, Louis et al., 2010) |
| EB1 | LIRLWSHLIHIWFQNRRLKWKKK | 10 | (Amand, Norden et al., 2012) |
| VSVG | KFTIVFPHNQKGNWKNVPSNYHYCP | 11 | (Schuster, Wu et al., 1999) |
| *Pseudomonas* toxin | EGGSLAALTAHQACHLPLETFTRHRQPRGWE QLEQCGYPVQRLVALYLAARLSWNQVDQVIR NALASPGSGGDLGEAIREQPEQARLALTLAAA ESERFVRQGTGNDEAGAANAD | 12 | (Fominaya, Uherek et al., 1998) |
| Melittin | GIGAVLKVLTTGLPALISWIKRKRQQ | 13 | (Tan, Chen et al., 2012) |
| KALA | WEAKLAKALAKALAKHLAKALAKALKACEA | 14 | (Wyman, Nicol et al., 1997) |
| JST-1 | GLFEALLELLESLWELLLEA | 15 | (Gottschalk, Sparrow et al., 1996) |
| C(LLKK)₃C | CLLKKLLKKLLKKC | 63 | (Luan et al., 2014) |
| G(LLKK)₃G | GLLKKLLKKLLKKG | 64 | (Luan et al., 2014) |

The shuttle agents of the present invention comprise one or more ELD or type of ELD. More particularly, they can comprise at least 2, at least 3, at least 4, at least 5, or more ELDs. In some embodiments, the shuttle agents can comprise between 1 and 10 ELDs, between 1 and 9 ELDs, between 1 and 8 ELDs, between 1 and 7 ELDs, between 1 and 6 ELDs, between 1 and 5 ELDs, between 1 and 4 ELDs, between 1 and 3 ELDs, etc.

In some embodiments, the order or placement of the ELD relative to the other domains (CPD, histidine-rich domains) within the shuttle agents of the present invention may be varied provided the shuttling ability of the shuttle agent is retained.

In some embodiments, the ELD may be a variant or fragment of any one those listed in **Table A,** and having endosomolytic activity. In some embodiments, the ELD may comprise or consist of the amino acid sequence of any one of **SEQ ID NOs: 1-15, 63, or 64,** or a sequence which is at least 70%, 71%, 72%, 73%, 74%, 75%, 76%, 77%, 78%, 79%, 80%, 81%, 82%, 83%, 84%, 85%, 86%, 87%, 88%, 89%, 85%, 90%, 91%, 92%, 93%, 94%, or 95% identical to any one of **SEQ ID NOs: 1-15, 63, or 64,** and having endosomolytic activity.

### Cell penetration domains (CPDs)

As used herein, the expression **"cell penetration domain"** refers to a sequence of amino acids which confers the ability of a macromolecule (e.g., peptide or protein) containing the CPD to be transduced into a cell.

In some embodiments, the CPD may be (or may be from) a cell-penetrating peptide or the protein transduction domain of a cell-penetrating peptide. Cell-penetrating peptides can serve as carriers to successfully deliver a variety of cargos intracellularly (e.g., polynucleotides, polypeptides, small molecule compounds or other macromolecules/compounds that are otherwise membrane-impermeable). Cell-penetrating peptides often include short peptides rich in basic amino acids that, once fused (or otherwise operably linked) to a macromolecule, mediate its internalization inside cells (Shaw, Catchpole et al., 2008). The first cell-penetrating peptide was identified by analyzing the cell penetration ability of the HIV-1 trans-activator of transcription (Tat) protein (Green and Loewenstein 1988, Vives, Brodin et al., 1997). This protein contains a short hydrophilic amino acid sequence, named "TAT", which promotes its insertion within the plasma membrane and the formation of pores. Since this discovery, many other cell-penetrating peptides have been described. In this regard, in some embodiments, the CPD can be a cell-penetrating peptide as listed in **Table B,** or a variant thereof having cell-penetrating activity.

**Table B: Examples of cell-penetrating peptides**

| **Name** | **Amino acid sequence** | **SEQ ID NO:** | **Reference(s)** |
|---|---|---|---|
| SP | AAVALLPAVLLALLAP | 16 | (Mahlum, Mandal et al., 2007) |
| TAT | YGRKKRRQRRR | 17 | (Green and Loewenstein 1988, Fawell, Seery et al., 1994, Vives, Brodin et al., 1997) |
| Penetratin (Antennapedia) | RQIKIWFQNRRMKWKK | 18 | (Perez, Joliot et al., 1992) |
| pVEC | LLIILRRRIRKQAHAHSK | 19 | (Elmquist, Lindgren et al., 2001) |
| M918 | MVTVLFRRLRIRRACGPPRVRV | 20 | (EI-Andaloussi, Johansson et al., 2007) |
| Pep-1 | KETWWETWWTEWSQPKKKRKV | 21 | (Morris, Depollier et al., 2001) |
| Pep-2 | KETWFETWFTEWSQPKKKRKV | 22 | (Morris, Chaloin et al., 2004) |
| Xentrv | LCLRPVG | 23 | (Montrose, Yang et al., 2013) |
| Arginine stretch | RRRRRRRRR | 24 | (Zhou, Wu et al., 2009) |
| Transportan | WTLNSAGYLLGKINLKALAALAKKIL | 25 | (Hallbrink, Floren et al., 2001) |
| SynB1 | RGGRLSYSRRRFSTSTGR | 26 | (Drin, Cottin et al., 2003) |
| SynB3 | RRLSYSRRRF | 27 | (Drin, Cottin et al., 2003) |
| PTD4 | YARAAARQARA | 65 | (Ho et al, 2001) |

Without being bound by theory, cell-penetrating peptides are thought to interact with the cell plasma membrane before crossing by pinocytosis or endocytosis. In the case of the TAT peptide, its hydrophilic nature and charge are thought to promote its insertion within the plasma membrane and the formation of a pore (Herce and Garcia 2007). Alpha helix motifs within hydrophobic peptides (such as SP) are also thought to form pores within plasma membranes (Veach, Liu et al., 2004).

Shuttle agents of the present invention comprise one or more CPD or type of CPD. More particularly, they may comprise at least 2, at least 3, at least 4, or at least 5 or more CPDs. In some embodiments, the shuttle agents can comprise between 1 and 10 CPDs, between 1 and 6 CPDs, between 1 and 5 CPDs, between 1 and 4 CPDs, between 1 and 3 CPDs, etc.

In some embodiments, the CPD may be TAT having the amino acid sequence of **SEQ ID NO: 17,** or a variant thereof having at least 70%, 71%, 72%, 73%, 74%, 75%, 76%, 77%, 78%, 79%, 80%, 81% 82%, 83%, 84%, 85%, 86%, 87%, 88%, 89%, 90%, 91%, 92%, 93%, 94%, or 95% identity to **SEQ ID NO: 17** and having cell penetrating activity; or Penetratin having the amino acid sequence of **SEQ ID NO: 18,** or a variant thereof having at least 70%, 71%, 72%, 73%, 74%, 75%, 76%, 77%, 78%, 79%, 80%, 81% 82%, 83%, 84%, 85%, 86%, 87%, 88%, 89%, 85%, 86%, 87%, 88%, 89%, 90%, 91%, 92%, 93%, 94%, or 95% identity to **SEQ ID NO: 18** and having cell penetrating activity.

In some embodiments, the CPD may be PTD4 having the amino acid sequence of **SEQ ID NO: 65,** or a variant thereof having at least 70%, 71%, 72%, 73%, 74%, 75%, 76%, 77%, 78%, 79%, 80%, 81% 82%, 83%, 84%, 85%, 86%, 87%, 88%, 89%, 90%, 91%, 92%, 93%, 94%, or 95% identity to **SEQ ID NO: 65.**

In some embodiments, the order or placement of the CPD relative to the other domains (ELD, histidine-rich domains) within the shuttle agents of the present invention may be varied provided the shuttling ability of the shuttle agent is retained.

In some embodiments, the CPD may be a variant or fragment of any one those listed in **Table B,** and having cell penetrating activity. In some embodiments, the CPD may comprise or consist of the amino acid sequence of any one of **SEQ ID NOs: 16-27 or 65,** or a sequence which is at least 70%, 71%, 72%, 73%, 74%, 75%, 76%, 77%, 78%, 79%, 80%, 81%, 82%, 83%, 84%, 85%, 86%, 87%, 88%, 89%, 85%, 90%, 91%, 92%, 93%, 94%, or 95% identical to any one of **SEQ ID NOs: 16-27 or 65,** and having cell penetrating activity.

### Histidine-rich domains

In some aspects, the shuttle agents of the present invention may comprise a histidine-rich domain. In other embodiments, the shuttle agents of the present invention may be combined/used together with a further independent synthetic peptide comprising or consisting essentially of a histidine-rich domain and a CPD (e.g., but lacking an ELD). This latter approach may provide the added advantage of allowing the concentration of the histidine-rich domain to be varied or controlled independently from the concentration of the ELD and the CPD contained in the shuttle agent. Without being bound by theory, the histidine-rich domain may act as a proton sponge in the endosome, providing another mechanism of endosomal membrane destabilization.

In some embodiments, the histidine-rich domain may be a stretch of at least 2, at least 3, at least 4, at least 5, or at least 6 amino acids comprising at least 30%, at least 35%, at least 40%, at least 45%, at least 50%, at least 55%, at least 60%, at least 65%, at least 70%, at least 75%, at least 80%, at least 85%, or at least 90% histidine residues. In some embodiments, the histidine-rich domain may comprise at least 2, at least 3, at least 4 at least 5, at least 6, at least 7, at least 8, or at least 9 consecutive histidine residues. Without being bound by theory, the histidine-rich domain in the shuttle agent may act as a proton sponge in the endosome through protonation of their imidazole groups under acidic conditions of the endosomes, providing another mechanism of endosomal membrane destabilization and thus further facilitating the ability of endosomally-trapped cargos to gain access to the cytosol. In some embodiments, the histidine-rich domain may be located at the N or C terminus of the synthetic peptide or shuttle agent. In some embodiments, the histidine-rich domain may be located N-terminal or C terminal to the CPD and/or ELD.

In some embodiments, the order or placement of the histidine-rich domain relative to the other domains (CPD, ELD) within the shuttle agents of the present invention may be varied provided the shuttling ability of the shuttle agent is retained. In some embodiments, the shuttle agents of the present invention may comprise more than one histidine-rich domain (e.g., histidine-rich domains at the amino and carboxyl termini).

### Linkers

In some embodiments, **suitable linkers** (e.g., flexible polypeptide linkers) can be used to operably connect the domains (CPDs, ELDs, or histidine-rich domains) to one another within the context of synthetic peptides and shuttle agents of the present invention. In some embodiments, linkers may be formed by adding sequences of small hydrophobic amino acids without rotatory potential (such as glycine) and polar serine residues that confer stability and flexibility. Linkers may be soft and allow the domains of the shuttle agents to move. In some embodiments, prolines may be avoided since they can add significant conformational rigidity. In some embodiments, the linkers may be serine/glycine-rich linkers (e.g., GGS (**SEQ ID NO: 104**), GGSGGGS (**SEQ ID NO: 105**), GGSGGGSGGGS (**SEQ ID NO: 106**), or the like). In some embodiments, the use shuttle agents comprising a suitable linker may be advantageous for delivering an independent polypeptide cargo to suspension cells, rather than to adherent cells.

### Cargos

The synthetic peptide or polypeptide-based shuttle agent of the present invention is provided for use in a clinical or therapeutic *in vivo* method for increasing transduction efficiency of an independent polypeptide cargo to the cytosol of a target eukaryotic cell. Also provided is an *in vitro* method of increasing transduction efficiency of an independent polypeptide cargo to the cytosol of a target eukaryotic cell as described herein. In some embodiments, the polypeptide cargo may be fused to one or more CPDs to further facilitate intracellular delivery. In some embodiments, the CPD fused to the polypeptide cargo may be the same or different from the CPD of the shuttle agent of the present description. Such fusion proteins may be constructed using standard recombinant technology. In some embodiments, the independent polypeptide cargo may be fused, complexed with, or covalently bound to a second biologically active cargo (e.g., a biologically active polypeptide or compound). Alternatively or simultaneously, the polypeptide cargo may comprise a subcellular targeting domain.

In some embodiments, the polypeptide cargo must be delivered to the nucleus for it to carry out its intended biological effect. One such example is when the cargo is a polypeptide intended for nuclear delivery (e.g., a transcription factor). In this regard, studies on the mechanisms of translocation of viral DNA have led to the identification of nuclear localization signals (NLSs). The NLS sequences are recognized by proteins (importins α and β), which act as transporters and mediators of translocation across the nuclear envelope. NLSs are generally enriched in charged amino acids such as arginine, histidine, and lysine, conferring a positive charge which is partially responsible for their recognition by importins. Accordingly, in some embodiments, the polypeptide cargo may comprise an NLS for facilitating nuclear delivery, such as one or more of the NLSs as listed in **Table C,** or a variant thereof having nuclear targeting activity. Of course, it is understood that, in certain embodiments, the polypeptide cargo may comprise its natural NLS.

**Table C: Nuclear localization signals**

| **Name** | **Amino acid sequence** | **SEQ ID NO:** | **Reference(s)** |
|---|---|---|---|
| E1a | KRPRP | 28 | (Kohler, Gorlich et al., 2001) |
| SV40 T-Ag | PKKKRKV | 29 | (Lanford, Kanda et al., 1986) |
| c-myc | PAAKRVKLD | 30 | (Makkerh, Dingwall et al., 1996) |
| Op-T-NLS | SSDDEATADSQHAAPPKKKRKV | 31 | (Chan and Jans 1999) |
| Vp3 | KKKRK | 32 | (Nakanishi, Shum et al., 2002) |
| Nucleoplasmin | KRPAATKKAGQAKKKK | 33 | (Fanara, Hodel et al., 2000) |
| Histone 2B NLS | DGKKRKRSRK | 34 | (Moreland, Langevin et al., 1987) |
| Xenopus N1 | VRKKRKTEEESPLKDKDAKKSKQE | 35 | (Kleinschmidt and Seiter 1988) |
| PARP | KRKGDEVDGVDECAKKSKK | 36 | (Schreiber, Molinete et al., 1992) |
| PDX-1 | RRMKWKK | 37 | (Moede, Leibiger et al., 1999) |
| QKI-5 | RVHPYQR | 38 | (Wu, Zhou et al., 1999) |
| HCDA | KRPACTLKPECVQQLLVCSQEAKK | 39 | (Somasekaram, Jarmuz et al., 1999) |
| H2B | GKKRSKA | 40 | (Moreland, Langevin et al., 1987) |
| v-Rel | KAKRQR | 41 | (Gilmore and Temin 1988) |
| Amida | RKRRR | 42 | (Irie, Yamagata et al., 2000) |
| RanBP3 | PPVKRERTS | 43 | (Welch, Franke et al., 1999) |
| Pho4p | PYLNKRKGKP | 44 | (Welch, Franke et al., 1999) |
| LEF-1 | KKKKRKREK | 45 | (Prieve and Waterman 1999) |
| TCF-1 | KKKRRSREK | 46 | (Prieve and Waterman 1999) |
| BDV-P | PRPRKIPR | 47 | (Shoya, Kobayashi et al., 1998) |
| TR2 | KDCVINKHHRNRCQYCRLQR | 48 | (Yu, Lee et al., 1998) |
| SOX9 | PRRRK | 49 | (Sudbeck and Scherer 1997) |
| Max | PQSRKKLR | 50 | (Kato, Lee et al., 1992) |

Once delivered to the cytoplasm, **recombinant** proteins are exposed to protein trafficking system of eukaryotic cells. Indeed, all proteins are synthetized in the cell's cytoplasm and are then redistributed to their final subcellular localization by a system of transport based on small amino acid sequences recognized by shuttle proteins (Karniely and Pines 2005, Stojanovski, Bohnert et al., 2012). In addition to NLSs, other localization sequences can mediate subcellular targeting to various organelles following intracellular delivery of the polypeptide cargos of the present description. Accordingly, in some embodiments, polypeptide cargos of the present invention may comprise a subcellular localization signal for facilitating delivery of the shuttle agent and cargo to specific organelles, such as one or more of the sequences as listed in **Table D,** or a variant thereof having corresponding subcellular targeting activity.

**Table D: Subcellular localization signals**

| **Name** | **Amino acid sequence** | **SEQ ID NO:** | **Reference(s)** |
|---|---|---|---|
| Mitochondrial signal sequence from Tim9 | NLVERCFTD | 51 | (Milenkovic, Ramming et al., 2009) |
| Mitochondrial signal sequence from Yeast cytochrome c oxidase subunit IV | MLSLRQSIRFFK | 52 | (Hurt, Pesold-Hurt et al., 1985) |
| Mitochondrial signal sequence from 18S rRNA | MLISRCKWSRFPGNQR | 53 | (Bejarano and Gonzalez 1999) |
| Peroxisome signal sequence - PTS1 | SKL | 54 | (Gould, Keller et al., 1989) |
| Nucleolar signal sequence from BIRC5 | MQRKPTIRRKNLRLRRK | 55 | (Scott, Boisvert et al., 2010) |
| Nucleolar signal sequence from RECQL4 | KQAWKQKWRKK | 56 | (Scott, Boisvert et al., 2010) |

In some embodiments, the cargo can be a biologically active compound such as a biologically active (recombinant) polypeptide (e.g., a transcription factor, a cytokine, or a nuclease) intended for intracellular delivery. As used herein, the expression **"biologically active"** refers to the ability of a compound to mediate a structural, regulatory, and/or biochemical function when introduced in a target cell.

In some embodiments, the cargo may be a recombinant polypeptide intended for nuclear delivery, such as a transcription factor. In some embodiments, the transcription factor can be HOXB4 (Lu, Feng et al., 2007), NUP98-HOXA9 (Takeda, Goolsby et al., 2006), Oct3/4, Sox2, Sox9, Klf4, c-Myc (Takahashi and Yamanaka 2006), MyoD (Sung, Mun et al., 2013), Pdx1, Ngn3 and MafA (Akinci, Banga et al., 2012), Blimp-1 (Lin, Chou et al., 2013), Eomes, T-bet (Gordon, Chaix et al., 2012), FOXO3A (Warr, Binnewies et al., 2013), NF-YA (Dolfini, Minuzzo et al., 2012), SALL4 (Aguila, Liao et al., 2011), ISL1 (Fonoudi, Yeganeh et al., 2013), FoxA1 (Tan, Xie et al., 2010), Nanog, Esrrb, Lin28 (Buganim et al., 2014), HIF1-alpha (Lord-Dufour et al., 2009), Hlf, Runx1t1, Pbx1, Lmo2, Zfp37, Prdm5 (Riddell et al., 2014), or Bcl-6 (Ichii, Sakamoto et al., 2004).

In some embodiments, the cargo may be a recombinant polypeptide intended for nuclear delivery, such as a nuclease useful for genome editing technologies. In some embodiments, the nuclease may be an RNA-guided endonuclease, a CRISPR endonuclease, a type I CRISPR endonuclease, a type II CRISPR endonuclease, a type III CRISPR endonuclease, a type IV CRISPR endonuclease, a type V CRISPR endonuclease, a type VI CRISPR endonuclease, CRISPR associated protein 9 (Cas9), Cpf1 (Zetsche et al., 2015), a zinc-finger nuclease (ZFN), a Transcription activator-like effector nuclease (TALEN) (Cox et al., 2015), a homing endonuclease, a meganuclease, or any combination thereof. Other nucleases not explicitly mentioned here may nevertheless be encompassed in the present description. In some embodiments, the nuclease may be fused to a nuclear localization signal (e.g., Cas9-NLS; Cpf1-NLS; ZFN-NLS; TALEN-NLS). In some embodiments, the nuclease may be complexed with a nucleic acid (e.g., one or more guide RNAs, a crRNA, a tracrRNAs, or both a crRNA and a tracrRNA). In some embodiments, the nuclease may possess DNA or RNA-binding activity, but may lack the ability to cleave DNA.

In some embodiments, the shuttle agents of the present invention may be used for intracellular delivery (e.g., nuclear delivery) of one or more CRISPR endonucleases, for example one or more of the CRISPR endonucleases described below.

Type I and its subtypes A, B, C, D, E, F and I, including their respective Cas1, Cas2, Cas3, Cas4, Cas6, Cas7 and Cas8 proteins, and the signature homologs and subunits of these Cas proteins including Cse1, Cse2, Cas7, Cas5, and Cas6e subunits in *E. coli* (type I-E) and Csy1, Csy2, Csy3, and Cas6f in *Pseudomonas aeruginosa* (type I-F) (Wiedenheft et al., 2011; Makarova et al, 2011). Type II and its subtypes A, B, C, including their respective Cas1, Cas2 and Cas9 proteins, and the signature homologs and subunits of these Cas proteins including Csn complexes (Makarova et al, 2011). Type III and its subtypes A, B and MTH326-like module, including their respective Cas1, Cas2, Cas6 and Cas10 proteins, and the signature homologs and subunits of these Cas proteins including Csm and CMR complexes (Makarova et al, 2011). Type IV represents the Csf3 family of Cas proteins. Members of this family show up near CRISPR repeats in *Acidithiobacillus ferrooxidans* ATCC 23270, *Azoarcus* sp. (strain *EbN1*), and *Rhodoferax ferrireducens* (strain DSM 15236/ATCC BAA-621/T118). In the latter two species, the CRISPR/Cas locus is found on a plasmid. Type V and it subtypes have only recently been discovered and include Cpf1, C2c1, and C2c3. Type VI includes the enzyme C2c2, which reported shares little homology to known sequences.

In some embodiments, the shuttle agents of the present invention may be used in conjunction with one or more of the nucleases, endonucleases, RNA-guided endonuclease, CRISPR endonuclease described above, for a variety of applications, such as those described herein. CRISPR systems interact with their respective nucleic acids, such as DNA binding, RNA binding, helicase, and nuclease motifs (Makarova et al, 2011; Barrangou & Marraffini, 2014). CRISPR systems may be used for different genome editing applications including:
- a Cas-mediated genome editing method conducting to non-homologous end-joining (NHEJ) and/or Homologous-directed recombination (HDR) (Cong et al, 2013);
- a catalytically dead Cas (dCas) that can repress and /or activate transcription initiation when bound to promoter sequences, to one or several gRNA(s) and to a RNA polymerase with or without a complex formation with others protein partners (Bikard et al, 2013);
- a catalytically dead Cas (dCas) that can also be fused to different functional proteins domains as a method to bring enzymatic activities at specific sites of the genome including transcription repression, transcription activation, chromatin remodeling, fluorescent reporter, histone modification, recombinase system acetylation, methylation, ubiquitylation, phosphorylation, sumoylation, ribosylation and citrullination (Gilbert et al, 2013).

The person of ordinary skill in the art will understand that the present shuttle agents, although exemplified with Cas9 in the present examples, may be used with other nucleases as described herein. Thus, nucleases such as Cpf1, Cas9, and variants of such nucleases or others, are encompassed by the present description. It should be understood that, in one aspect, the present invention may broadly cover any cargo having nuclease activity, such an RNA-guided endonuclease, or variants thereof (e.g., those that can bind to DNA or RNA, but have lost their nuclease activity; or those that have been fused to a transcription factor).

In some embodiments, the polypeptide cargo may be a cytokine such as a chemokine, an interferon, an interleukin, a lymphokine, or a tumour necrosis factor. In some embodiments, the polypeptide cargo may be a hormone or growth factor. In some embodiments, the cargo may be an antibody (e.g., a labelled antibody). In some embodiments, the cargo can be a detectable label (fluorescent polypeptide or reporter enzyme) that is intended for intracellular delivery, for example, for research and/or diagnostic purposes.

In some embodiments, the cargo may be a globular protein or a fibrous protein. In some embodiments, the cargo may have a molecule weight of any one of about 5, 10, 15, 20, 25, 30, 35, 40, 45, to 50 to about 150, 200, 250, 300, 350, 400, 450, 500 kDa or more. In some embodiments, the cargo may have a molecule weight of between about 20 to 200 kDa.

### Non-toxic, metabolizable synthetic peptides and shuttle agents

In some embodiments, synthetic peptides and shuttle agents of the present invention may be non-toxic to the intended target eukaryotic cells at concentrations up to 50 µM, 45 µM, 40 µM, 35 µM, 30 µM, 25 µM, 20 µM, 15 µM, 10 µM, 9 µM, 8 µM, 7 µM, 6 µM, 5 µM, 4 µM, 3 µM, 2 µM, 1 µM, 0.5 µMm 0.1 µM, or 0.05 µM. Cellular toxicity of shuttle agents of the present description may be measured using any suitable method. Furthermore, transduction protocols may be adapted (e.g., concentrations of shuttle and/or cargo used, shuttle/cargo exposure times, exposure in the presence or absence of serum), to reduce or minimize toxicity of the shuttle agents, and/or to improve/maximize transduction efficiency.

In some embodiments, synthetic peptides and shuttle agents of the present invention may be readily metabolizable by intended target eukaryotic cells. For example, the synthetic peptides and shuttle agents may consist entirely or essentially of peptides or polypeptides, for which the target eukaryotic cells possess the cellular machinery to metabolize/degrade. Indeed, the intracellular half-life of the synthetic peptides and polypeptide-based shuttle agents of the present description is expected to be much lower than the half-life of foreign organic compounds such as fluorophores. However, fluorophores can be toxic and must be investigated before they can be safely used clinically (Alford et al., 2009). The synthetic peptides and shuttle agents of the present invention are suitable for clinical use or therapeutic use. In some embodiments, the synthetic peptides and shuttle agents of the present invention may avoid the use of domains or compounds for which toxicity is uncertain or has not been ruled out.

### Cocktails

Described herein is a composition comprising a cocktail of at least 2, at least 3, at least 4, or at least 5 different types of the synthetic peptides or polypeptide-based shuttle agents as defined herein. Combining different types of synthetic peptides or polypeptide-based shuttle agents (e.g., different shuttle agents comprising different types of CPDs) may provide increased versatility for delivering different polypeptide cargos intracellularly. Furthermore, without being bound by theory, combining lower concentrations of different types of shuttle agents may help reduce cellular toxicity associated with using a single type of shuttle agent (e.g., at higher concentrations).

### Methods

In some embodiments, the present invention relates to an *in vitro* method for increasing the transduction efficiency of a polypeptide cargo to the cytosol of a target eukaryotic cell, the method comprising contacting the target eukaryotic cells with a polypeptide based shuttle agent comprising an endosome leakage domain (ELD) operably linked to a cell penetrating domain (CPD), or an ELD operably linked to a histidine rich domain and a CPD, wherein the polypeptide based shuttle agent has a predicted net charge of at least +6 at physiological pH and is used at a concentration sufficient to increase the transduction efficiency of the independent polypeptide cargo to the cytosol of the target eukaryotic cell. The present invention also relates to a polypeptide-based shuttle agent for use in a clinical or therapeutic *in vivo* method as described herein. The method may comprise contacting the target eukaryotic cell with the synthetic peptide, polypeptide-based shuttle agent, or composition as defined herein, and the polypeptide cargo. In some embodiments, the synthetic peptide or polypeptide-based shuttle agent may be pre-incubated with the polypeptide cargo to form a mixture, prior to exposing the target eukaryotic cell to that mixture. In some embodiments, the type of CPD may be selected based on the amino acid sequence of the polypeptide cargo to be delivered intracellularly. In other embodiments, the type of CPD and ELD may be selected to take into account the amino acid sequence of the polypeptide cargo to be delivered intracellularly, the type of cell, the type of tissue, etc.

In some embodiments, the method may comprise multiple treatments of the target cells with the synthetic peptide or polypeptide-based shuttle agent (e.g., 1, 2, 3, 4 or more times per day, and/or on a pre-determined schedule). In such cases, lower concentrations of the synthetic peptide or polypeptide-based shuttle agent may be advisable (e.g., for reduced toxicity). In some embodiments, the cells may be suspension cells or adherent cells. In some embodiments, the person of skill in the art will be able to adapt the teachings of the present description using different combinations of shuttles, domains, uses and methods to suit particular needs of delivering a polypeptide cargo to particular cells with a desired viability.

In some embodiments, the methods of the present description may apply to methods of delivering a polypeptide cargo intracellularly to a cell *in vivo.* Such methods may be accomplished by parenteral administration or direct injection into a tissue, organ, or system.

In some embodiments, the synthetic peptide or polypeptide-based shuttle agent and the polypeptide cargo may be exposed to the target cell in the presence or absence of serum. In some embodiments, the method may be suitable for clinical or therapeutic use.

Described herein is a kit for increasing the transduction efficiency of a polypeptide cargo to the cytosol of a target eukaryotic cell. The kit may comprise the synthetic peptide or polypeptide-based shuttle agent, as defined herein, and a suitable container.

In some embodiments, the target eukaryotic cells may be an animal cell, a mammalian cell, or a human cell. In some embodiments, the target eukaryotic cells may be a stem cell (e.g., embryonic stem cells, pluripotent stem cells, induced pluripotent stem cells, neural stem cells, mesenchymal stem cells, hematopoietic stem cells, peripheral blood stem cells), primary cells (e.g., myoblasts, fibroblasts), or an immune cell (e.g., T cells, dendritic cells, antigen presenting cells). In some embodiments, the present description relates to an isolated cell comprising a synthetic peptide or polypeptide-based shuttle agent as defined herein. In some embodiments, the cell may be a protein-induced pluripotent stem cell. It will be understood that cells that are often resistant or not amenable to protein transduction may be interesting candidates for the synthetic peptides or polypeptide-based shuttle agents of the present invention.

Other objects, advantages and features of the present description will become more apparent upon reading of the following non-restrictive description of specific embodiments thereof, given by way of example only with reference to the accompanying drawings.

### EXAMPLES

### Example 1:

### Materials and Methods

### 1.1 Materials

All chemicals were purchased from Sigma-Aldrich (St. Louis, MO, USA or Oakville, ON, Canada) or equivalent grade from BioShop Canada Inc. (Mississauga, ON, Canada) or VWR (Ville Mont-Royal, QC, Canada), unless otherwise noted.

### 1.2 Reagents

**Table 1.1: Reagents**

| **Material** | **Company** | **City, Province-State, Country** |
|---|---|---|
| **RPMI 1640 media** | Sigma-Aldrich | Oakville, ON, Canada |
| DMEM | Sigma-Aldrich | Oakville, ON, Canada |
| **Fetal bovine serum (FBS)** | NorthBio | Toronto, ON, Canada |
| **L-glutamine-Penicillin-Streptomycin** | Sigma-Aldrich | Oakville, ON, Canada |
| **Trypsin-EDTA solution** | Sigma-Aldrich | Oakville, ON, Canada |
| **pEGFP-C1** | CLONTECH Laboratories | Palo Alto, CA, USA |
| **FITC-Antibody α-tubulin** | Abcam ab64503 | Cambridge, MA, USA |
| **ITS** | Invitrogen/41400-045 | Burlington, ON, Canada |
| **FGF 2** | Feldan Bio/1D-07-017 | Quebec, QC, Canada |
| **Dexamethasone** | Sigma-Aldrich/D8893 | Oakville, ON, Canada |
| **Bovine serum albumin (BSA)** | Sigma-Aldrich/A-1933 | Oakville, ON, Canada |
| **MB1 media** | GE Healthcare HyClone | Logan, Utah, USA |
| **Calcein** | Sigma-Aldrich/ C0875 | Oakville, ON, Canada |
| **HisTrap™ FF column** | GE Healthcare | Baie d'Urfe, QC, Canada |
| **Q Sepharose™** | GE Healthcare | Baie d'Urfe, QC, Canada |
| **SP Sepharose™** | GE Healthcare | Baie d'Urfe, QC, Canada |
| **Amicon Ultra centrifugal filters** | EMD Millipore | Etobicoke, ON Canada |
| ***Label* IT°Cy°5 kit** | Mirus Bio LLC | Madison, WI, USA |
| **Calf serum** | NorthBio | Toronto, ON, Canada |
| **beta-mercaptoethanol** | Sigma-Aldrich | Oakville, ON, Canada |
| **IL-2** | Feldan Bio/ rhlL-2 Research | Quebec, QC, Canada |
| **Rezazurine sodium salt** | Sigma-Aldrich/R7017-1G | Oakville, ON, Canada |
| **Anti-HOXB4 monoclonal antibody** | Novus Bio #NBP2-37257 | Oakville, ON, Canada |
| **Alexa™-594 Anti-Mouse** | Abcam #150116 | Toronto, ON, Canada |
| **Fluoroshield™ with DAPI** | Sigma #F6057 | Oakville, ON, Canada |
| **GFP Monoclonal antibody** | Feldan Bio #A017 | Quebec, QC, Canada |
| **Phusion™ High-Fidelity DNA polymerase** | (NEB #M0530S) | Whitby, ON, Canada |
| **Edit-R™ Synthetic crRNA Positive Controls** | (Dharmacon #U-007000-05) | Ottawa, ON, Canada |
| **T7 Endonuclease I** | (NEB, Cat #M0302S) | Whitbv, ON, Canada |
| **FastFect™ transfection reagent** | (Feldan Bio # 9K-01 0-0001) | Quebec, QC, Canada |

### 1.3 Cell lines

HeLa, HEK293A, HEK293T, THP-1, CHO, NIH3T3, CA46, Balb3T3 and HT2 cells were obtained from American Type Culture Collection (Manassas, VA, USA) and cultured following the manufacturer's instructions. Myoblasts are primary human cells kindly provided by Professor J.P. Tremblay (Université Laval, Quebec, Canada).

**Table 1.2: Cell lines and culture conditions**

| **Cell lines** | **Description** | **ATCC/others** | **Culture media** | **Serum** | **Additives** |
|---|---|---|---|---|---|
| **HeLa (adherent cells)** | Human cervical carcinoma cells | ATCC™ CCL-2 | DMEM | 10% FBS | L-glutamine 2 mM Penicillin 100 units Streptomycin 100µg/mL |
| **HEK 293A (adherent cells)** | Human embryonic Epithelial kidney cells | ATCC™ CRL-1573 | DMEM | 10% FBS | L-glutamine 2 mM Penicillin 100 units Streptomycin 100µg/mL |
| **HEK 293T (adherent cells)** | Human embryonic Epithelial kidney cells | ATCC™ CRL-3216 | DMEM | 10% FBS | L-glutamine 2 mM Penicillin 100 units Streptomycin 100µg/mL |
| **THP-1 (suspension cells)** | Acute monocytic leukemia | ATCC™ TIB202 | RPMI 1640 | 10% FBS | 2- mercaptoethanol 0.05 mM L-glutamine 2 mM Penicillin 100 units Streptomycin 100µg/mL |
| **Myoblasts (primary adherent cells)** | Human (13 months) myoblasts | Kindly provided by Professor JP Tremblay | MB1 | 15% FBS | ITS 1x, FGF 2 10 ng/mL, Dexamethasone 0.39µg/mL, BSA 0.5mg/mL, MB1 85% |
| **CHO (adherent cells)** | Chinese hamster ovary cells | ATCC™ CCL-61 | DMEM | 10% FBS | L-glutamine 2 mM Penicillin 100 units Streptomycin 100 µg/mL |
| **NIH3T3 (adherent cells)** | Fibroblasts | ATCC™ CRL-1658 | DMEM | 10% Calf serum | L-glutamine 2 mM Penicillin 100 units Streptomycin 100 µg/mL |
| **HT2 (suspension cells)** | T lymphocytes | ATCC™ CRL-1841 | RPMI 1640 | 10% FBS | 200 IU/mL IL-2 β-mercaptoethanol 0.05 mM L-glutamine 2 mM Penicillin 100 units Streptomycin 100 µg/mL |
| **CA46 (suspension cells)** | Homo sapiens Burkitt's lymphoma | ATCC™ CRL-1648 | RPMI 1640 | 20% FBS | L-glutamine 2 mM Penicillin 100 units Streptomycin 100 µg/mL |
| **Balb3T3 (adherent cells)** | Fibroblasts | ATCC™ CCL-163 | DMEM | 10% Calf serum | L-glutamine 2 mM Penicillin 100 units Streptomycin 100 µg/mL |
| **Jurkat (suspension cells)** | Human T cells | ATCC™ TIB-152 | RPMI 1640 | 10% FBS | L-glutamine 2 mM Penicillin 100 units Streptomycin 100 µg/mL |

| | | | | | |
|---|---|---|---|---|---|
| **FBS:** Fetal bovine serum | | | | | |

### 1.4 Protein purification

Fusion proteins were expressed in bacteria (*E. coli* BL21DE3) under standard conditions using an isopropyl β-D-1-thiogalactopyranoside (IPTG) inducible vector containing a T5 promoter. Culture media contained 24 g yeast extract, 12 g tryptone, 4 mL glycerol, 2.3 g KH₂PO₄, and 12.5 g K₂HPO₄ per liter. Bacterial broth was incubated at 37°C under agitation with appropriate antibiotic (e.g., ampicillin). Expression was induced at optical density (600 nm) between 0.5 and 0.6 with a final concentration of 1 mM IPTG for 3 hours at 30°C. Bacteria were recuperated following centrifugation at 5000 RPM and bacterial pellets were stored at -20°C.

Bacterial pellets were resuspended in Tris buffer (Tris 25 mM pH 7.5, NaCl 100mM, imidazole 5 mM) with phenylmethylsulfonyl fluoride (PMSF) 1 mM, and lysed by passing 3 times through the homogenizer Panda 2K™ at 1000 bar. The solution was centrifuged at 15000 RPM, 4°C for 30 minutes. Supernatants were collected and filtered with a 0.22 µM filtration device.

Solubilized proteins were loaded, using a FPLC (AKTA Explorer 100R), on HisTrap™ FF column previously equilibrated with 5 column volumes (CV) of Tris buffer. The column was washed with 30 column volumes (CV) of Tris buffer supplemented with 0.1% Triton™ X-114 followed with 30 CV of Tris buffer with imidazole 40 mM. Proteins were eluted with 5 CV of Tris buffer with 350 mM Imidazole and collected. Collected fractions corresponding to specific proteins were determined by standard denaturing SDS-PAGE.

Purified proteins were diluted in Tris 20 mM at the desired pH according to the protein's pl and loaded on an appropriate ion exchange column (Q Sepharose™ or SP Sepharose™) previously equilibrated with 5 CV of Tris 20 mM, NaCl 30 mM. The column was washed with 10 CV of Tris 20 mM, NaCl 30 mM and proteins were eluted with a NaCl gradient until 1 M on 15 CV. Collected fractions corresponding to specific proteins were determined by standard denaturing SDS-PAGE. Purified proteins were then washed and concentrated in PBS 1X on Amicon Ultra™ centrifugal filters 10,000 MWCO. Protein concentration was evaluated using a standard Bradford assay.

### 1.5 Synthetic peptides and shuttle agents

All peptides used in this study were purchased from GLBiochem (Shanghai, China) and their purities were confirmed by high-performance liquid chromatography analysis and mass spectroscopy. In some cases, chimeric peptides were synthesized to contain a C-terminal cysteine residue to allow the preparation of peptide dimers. These dimeric peptides were directly synthetized with a disulfide bridge between the C-terminal cysteines of two monomers. The amino acid sequences and characteristics of each of the synthetic peptides and shuttle agents tested in the present examples are summarized in **Table 1.3.**

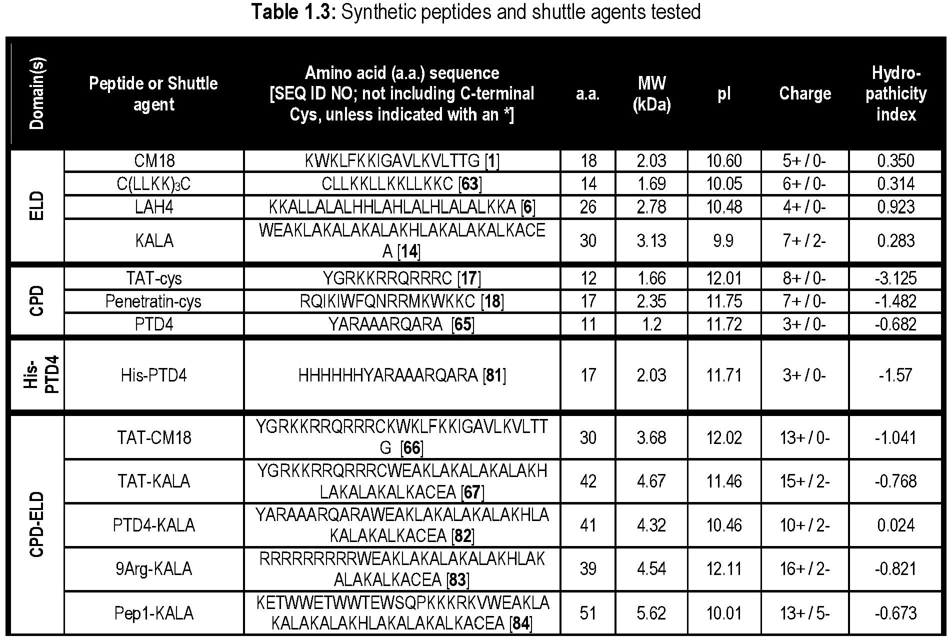

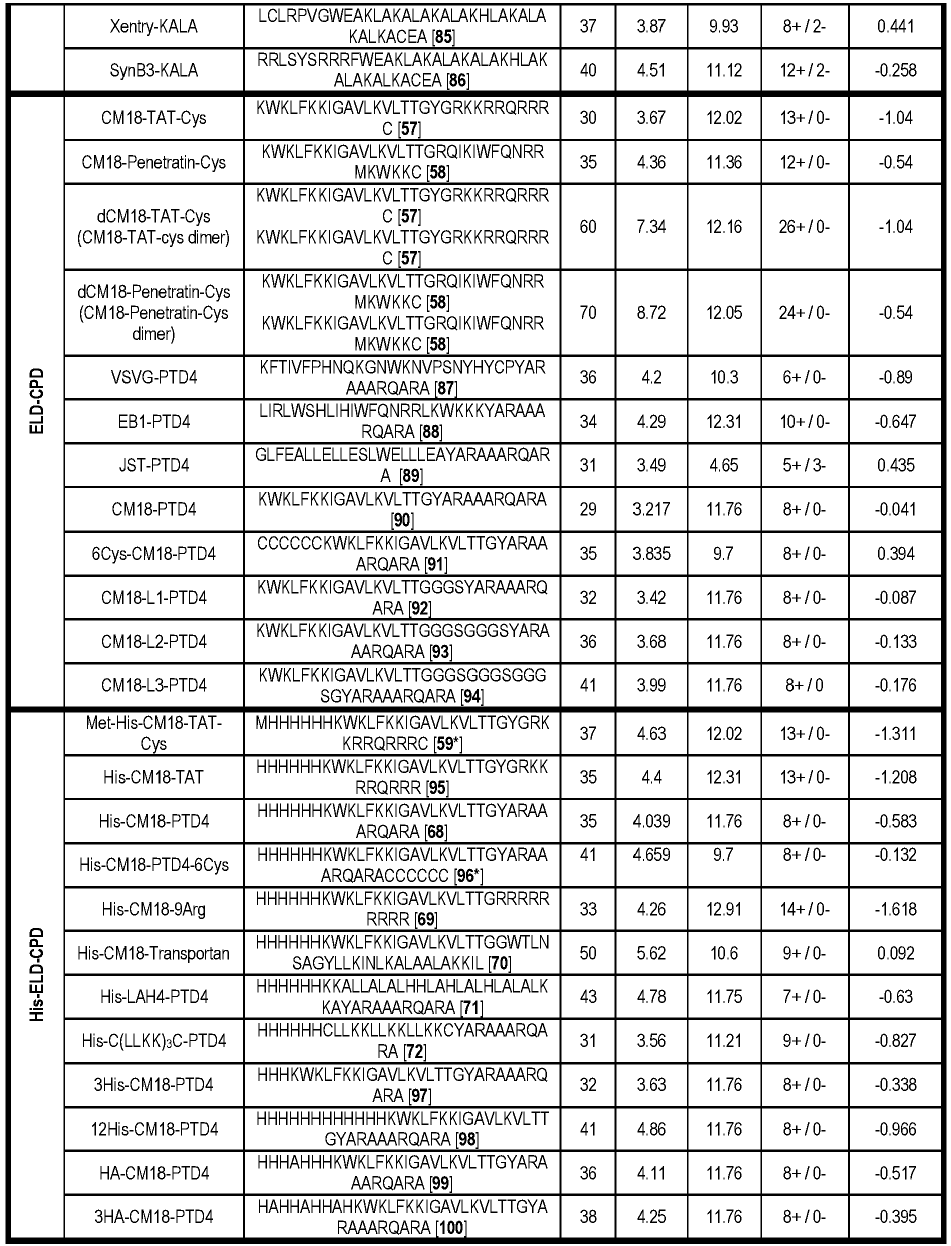

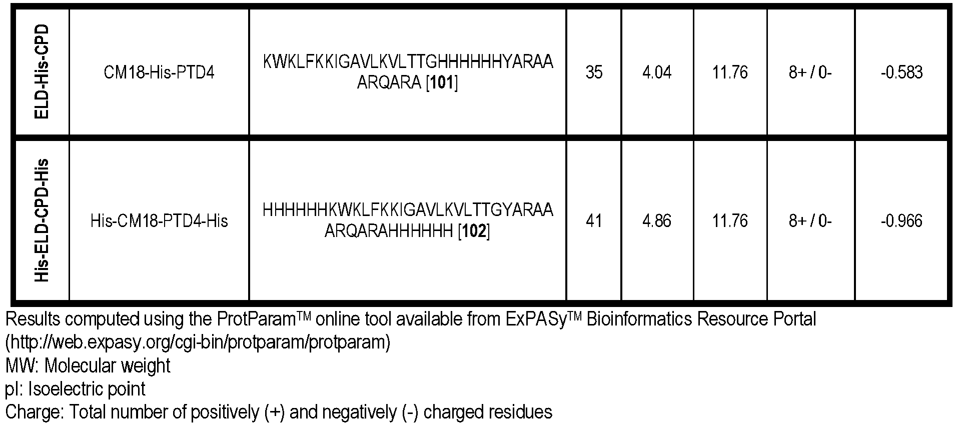

### Example 2:

### Peptide shuttle agents facilitate escape of endosomally-trapped calcein

### 2.1 Endosome escape assays

Microscopy-based and flow cytometry-based fluorescence assays were developed to study endosome leakage and to determine whether the addition of the shuttle agents facilitates endosome leakage of the polypeptide cargo.

### 2.1.1 Endosomal leakage visualization by microscopy

Calcein is a membrane-impermeable fluorescent molecule that is readily internalized by cells when administered to the extracellular medium. Its fluorescence is pH-dependent and calcein self-quenches at higher concentrations. Once internalized, calcein becomes sequestered at high concentrations in cell endosomes and can be visualized by fluorescence microscopy as a punctate pattern. Following endosomal leakage, calcein is released to the cell cytoplasm and this release can be visualized by fluorescence microscopy as a diffuse pattern.

One day before the calcein assay was performed, mammalian cells (e.g., HeLa, HEK293A, or myoblasts) in exponential growth phase were harvested and plated in a 24-well plate (80,000 cells per well). The cells were allowed to attach by incubating overnight in appropriate growth media, as described in **Example 1.** The next day, the media was removed and replaced with 300 µL of fresh media without FBS containing 62.5 µg/mL (100 µM) of calcein, except for HEK293A (250 µg/mL, 400 µM). At the same time, the shuttle agent(s) to be tested was added at a predetermined concentration. The plate was incubated at 37°C for 30 minutes. The cells were washed with 1x PBS (37°C) and fresh media containing FBS was added. The plate was incubated at 37°C for 2.5 hours. The cells were washed three times and were visualized by phase contrast and fluorescence microscopy (IX81™, Olympus).

A typical result is shown in **Figure 1A****,** in which untreated HEK293A cells loaded with calcein ("100 µM calcein") show a low intensity, punctate fluorescent pattern when visualized by fluorescence microscopy (upper left panel). In contrast, HeLa cells treated with a shuttle agent that facilitates endosomal escape of calcein ("100 µM calcein + CM18-TAT 5 µM") show a higher intensity, more diffuse fluorescence pattern in a greater proportion of cells (upper right panel).

### 2.1.2 Endosomal leakage quantification by flow cytometry

In addition to microscopy, flow cytometry allows a more quantitative analysis of the endosomal leakage as the fluorescence intensity signal increases once the calcein is released in the cytoplasm. Calcein fluorescence is optimal at physiological pH (e.g., in the cytosol), as compared to the acidic environment of the endosome.

One day before the calcein assay was performed, mammalian cells (e.g., HeLa, HEK293, or myoblasts) in exponential growth phase were harvested and plated in a 96-well plate (20,000 cells per well). The cells were allowed to attach by incubating overnight in appropriate growth media, as described in **Example 1.** The next day, the media in wells was removed and replaced with 50 µL of fresh media without serum containing 62.5 µg/mL (100 µM) of calcein, except for HEK293A (250 µg/mL, 400 µM). At the same time, the shuttle agent(s) to be tested was added at a predetermined concentration. The plate was incubated at 37°C for 30 minutes. The cells were washed with 1x PBS (37°C) and fresh media containing 5-10% serum was added. The plate was incubated at 37°C for 2.5 hours. The cells were washed with 1x PBS and detached using trypsinization. Trypsinization was stopped by addition of appropriate growth media, and calcein fluorescence was quantified using flow cytometry (Accuri C6, Becton, Dickinson and Company (BD)).

Untreated calcein-loaded cells were used as a control to distinguish cells having a baseline of fluorescence due to endosomally-trapped calcein from cells having increased fluorescence due to release of calcein from endosomes. Fluorescence signal means ("mean counts") were analyzed for endosomal escape quantification. In some cases, the "Mean Factor" was calculated, which corresponds to the fold-increase of the mean counts relative to control (untreated calcein-loaded cells). Also, the events scanned by flow cytometry corresponding to cells (size and granularity) were analyzed. The cellular mortality was monitored with the percentage of cells in the total events scanned. When it became lower than the control, it was considered that the number of cellular debris was increasing due to toxicity and the assay was discarded.

A typical result is shown in **Figure 1B****,** in which an increase in fluorescence intensity (right-shift) is observed for calcein-loaded HeLa cells treated with a shuttle agent that facilitates endosomal escape ("Calcein 100 µM + CM18-TAT 5 µM", right panel), as compared to untreated calcein-loaded HeLa cells ("Calcein 100 µM", left panel). The increase in calcein fluorescence is caused by the increase in pH associated with the release of calcein from the endosome (acidic) to the cytoplasm (physiological).

### 2.2 Results from endosome escape assays

### 2.2.1 HeLa cells

HeLa cells were cultured and tested in the endosomal escape assays as described in **Example 2.1.** The results of flow cytometry analyses are summarized below. In each case, the flow cytometry results were also confirmed by fluorescence microscopy (data not shown).

**Table 2.1: CM18-Penetratin-Cys v. Controls in HeLa cells**

| **Domains** | **Peptide** | **Cells** | **Concetration (µM)** | **Mean Counts (± St. Dev.; n=3)** | **Mean Factor** |
|---|---|---|---|---|---|
| - | **No peptide** | HeLa | 0 | 55 359 ± 6844 | 1.0 |
| ELD | **CM18** | HeLa | 5 | 46 564 ± 9618 | 0.8 |
| CPD | **TAT-Cys** | HeLa | 5 | 74 961 ± 9337 | 1.3 |
| | **Penetratin-Cys** | HeLa | 5 | 59 551 ± 7119 | 1.1 |
| ELD + CPD | **CM18 + TAT-Cys** | HeLa | 5 + 5 | 64 333 ± 6198 | 1.2 |
| | **CM18 + Penetratin-Cys** | HeLa | 5 + 5 | 40 976 ± 8167 | 0.7 |
| ELD-CPD | **CM18-Penetratin-Cys** | HeLa | 5 | 262 066 ± 28 146 | **4.7** |

**Table 2.2: CM18-TAT-Cys v. Control in HeLa cells**

| **Domains** | **Peptide** | **Cells** | **Concentration (µM)** | **Mean counts (n=3)** | **Stand. dev.** | **Mean Factor** |
|---|---|---|---|---|---|---|
| - | **No peptide** | HeLa | 0 | 53 369 | 4192 | 1.0 |
| ELD-CPD | **CM18-TAT-Cys** | HeLa | 5 | 306 572 | 46 564 | **5.7** |

The results in **Tables 2.1 and 2.2** show that treating calcein-loaded HeLa cells with the shuttle agents CM18-Penetratin-Cys and CM18-TAT-Cys (having the domain structure ELD-CPD) results in increased mean cellular calcein fluorescence intensity, as compared to untreated control cells or cells treated with single-domain peptides used alone (CM18, TAT-Cys, Penetratin-Cys) or together (CM18 + TAT-Cys, CM18 + Penetratin-Cys). These results suggest that CM18-Penetratin-Cys and CM18-TAT-Cys facilitate escape of endosomally-trapped calcein, but that single domain peptides (used alone or together) do not.

**Table 2.3: Dose response of CM18-TAT-Cys in HeLa cells, data from Figure 2**

| **Domains** | **Peptide** | **Cells** | **Concentration (µM)** | **Mean counts (n=3)** | **Stand. dev.** | **Mean Factor** |
|---|---|---|---|---|---|---|
| - | **No peptide** ("calcein 100 µM") | HeLa | 0 | 63 872 | 11 587 | 1.0 |
| ELD-CPD | **CM18-TAT-Cys** | HeLa | 1 | 86 919 | 39165 | **1.4** |
| | **CM18-TAT-Cys** | HeLa | 2 | 137 887 | 13119 | **2.2** |
| | **CM18-TAT-Cys** | HeLa | 3 | 174 327 | 11 519 | **2.7** |
| | **CM18-TAT-Cys** | HeLa | 4 | 290 548 | 16 593 | **4.5** |
| | **CM18-TAT-Cys** | HeLa | 5 | 383 685 | 5578 | **6.0** |

**Table 2.4: Dose response of CM18-TAT-Cys in HeLa cells**

| **Domains** | **Peptide** | **Cells** | **Concentration (µM)** | **Mean counts (n=3)** | **Stand. dev.** | **Mean Factor** |
|---|---|---|---|---|---|---|
| - | **No peptide** | HeLa | 0 | 81 013 | 14 213 | 1.0 |
| ELD-CPD | **CM18-TAT-Cys** | HeLa | 3 | 170 652 | 63 848 | **2.1** |
| | **CM18-TAT-Cys** | HeLa | 4 | 251 799 | 33 880 | **3.1** |
| | **CM18-TAT-Cys** | HeLa | 5 | 335 324 | 10 651 | **4.1** |

**Table 2.5: Dose response of CM18-TAT-Cys and CM18-Penetratin-Cys in HeLa cells, data from Figure 3**

| **Domains** | **Peptide** | **Cells** | **Concentration (µM)** | **Mean counts (n=3)** | **Stand. dev.** | **Mean Factor** |
|---|---|---|---|---|---|---|
| - | **No peptide** | HeLa | 0 | 62 503 | 23 752 | 1.0 |
| ELD-CPD | **CM18-TAT-Cys** | HeLa | 5 | 187180 | 8593 | **3.0** |
| | **CM18-TAT-Cys** | HeLa | 8 | 321 873 | 36 512 | **5.1** |
| | **CM18-Penetratin-Cys** | HeLa | 5 | 134 506 | 2992 | **2.2** |
| | **CM18-Penetratin-Cys** | HeLa | 8 | 174 233 | 56 922 | **2.8** |

The results in Tables 2.3 (**Figure 2****), 2.4, and 2.5 (****Figure 3****)** suggest that CM18-TAT-Cys and CM18-Penetratin-Cys facilitate escape of endosomally-trapped calcein in HeLa cells in a dose-dependent manner. In some cases, concentrations of CM18-TAT-Cys or CM18-Penetratin-Cys above 10 µM were associated with an increase in cell toxicity in HeLa cells.

**Table 2.6: Dimers v. monomers of CM18-TAT-Cys and CM18-Penetratin-Cys in HeLa cells**

| **Domains** | **Peptide** | **Cells** | **Concentration (µM)** | **Mean counts (n=4)** | **Stand. dev.** | **Mean Factor** |
|---|---|---|---|---|---|---|
| - | **No peptide** | HeLa | 0 | 60 239 | 9860 | 1.0 |
| ELD-CPD | **CM18-TAT-Cys** | HeLa | 4 | 128 461 | 25 742 | **2.1** |
| | **CM18-Penetratin-Cys** | HeLa | 4 | 116 873 | 3543 | **1.9** |
| ELD-CPD dimer | **dCM18-TAT-Cys** | HeLa | 2 | 79 380 | 4297 | **1.3** |
| | **dCM18-Penetratin-Cys** | HeLa | 2 | 128 363 | 8754 | **2.1** |

**Table 2.7: Monomers v. dimers of CM18-TAT-Cys and CM18-Penetratin-Cys in HeLa cells**

| **Domains** | **Peptide** | **Cells** | **Concentration (µM)** | **Mean counts (n=3)** | **Stand. dev.** | **Mean Factor** |
|---|---|---|---|---|---|---|
| - | **No peptide** | HeLa | 0 | 55 834 | 1336 | 1.0 |
| ELD-CPD | CM18-TAT-Cys | HeLa | 4 | 159 042 | 16 867 | **2.8** |
| ELD-CPD dimer | dCM18-TAT-Cys | HeLa | 2 | 174 274 | 9 553 | **3.1** |

The results in **Table 2.6 and 2.7** suggest that shuttle peptide dimers (which are molecules comprising more than one ELD and CPD) are able to facilitate calcein endosomal escape levels that are comparable to the corresponding monomers.

### 2.2.3 HEK293A cells

To examine the effects of the shuttle agents on a different cell line, HEK293A cells were cultured and tested in the endosomal escape assays as described in **Example 2.1.** The results of flow cytometry analyses are summarized below in **Table 2.8** and in **Figure 1B****.**

**Table 2.8: CM18-TAT-Cys in HEK293A cells**

| **Domains** | **Peptide** | **Cells** | **Concentration (µM)** | **Mean counts (n=2)** | **Stand. dev.** | **Mean Factor** |
|---|---|---|---|---|---|---|
| - | **No Peptide** | HEK293A | 0 | 165 819 | 7693 | 1.0 |
| ELD-CPD | **CM18-TAT**-Cys | HEK293A | 0.5 | 196182 | 17 224 | **1.2** |
| | **CM18-TAT**-Cys | HEK293A | 5 | 629 783 | 1424 | **3.8** |

The results in **Table 2.8 and in** **Figure 1B** show that treating calcein-loaded HEK293A cells with the shuttle agent CM18-TAT-Cys results in increased mean cellular calcein fluorescence intensity, as compared to untreated control cells.

### 2.2.2 Myoblasts

To examine the effects of the shuttle agents on primary cells, primary myoblast cells were cultured and tested in the endosomal escape assays as described in **Example 2.1.** The results of flow cytometry analyses are summarized below in **Tables 2.9 and 2.10,** and in **Figure 4****.** In each case, the flow cytometry results were also confirmed by fluorescence microscopy.

**Table 2.9: Dose response of CM18-TAT-Cys in primary myoblasts, data from Figure 4**

| **Domains** | **Peptide** | **Cells** | **Peptide Conc. (µM)** | **Mean counts (n=3)** | **Stand. dev.** | **Mean Factor** |
|---|---|---|---|---|---|---|
| - | **No peptide; no calcein** ("Cells") | Myoblasts | 0 | 863 | 61 | n/a |
| - | **No peptide** ("Calcein 100 µM") | Myoblasts | 0 | 38111 | 13715 | 1.0 |
| ELD-CPD | **CM18-TAT**-Cys | Myoblasts | 5 | 79 826 | 12 050 | **2.1** |
| | **CM18-TAT-**Cys | Myoblasts | 8 | 91 421 | 10 846 | **2.4** |

**Table 2.10: Dose response of CM18-TAT-Cys in primary myoblasts**

| **Domains** | **Peptide** | **Cells** | **Peptide Conc. (µM)** | **Mean counts (n=3)** | **Stand. dev.** | **Mean Factor** |
|---|---|---|---|---|---|---|
| - | **No peptide** | Myoblasts | 0 | 31 071 | 21 075 | 1.0 |
| ELD-CPD | **CM18-TAT**-Cys | Myoblasts | 5 | 91 618 | 10 535 | **2.9** |
| | **CM18-TAT**-Cys | Myoblasts | 7.5 | 95 289 | 11 266 | **3.1** |

The results in **Table 2.9** (shown graphically in **Figure 4**) and **Table 2.10** suggest that CM18-TAT-Cys facilitates escape of endosomally-trapped calcein in a dose-dependent manner in primary myoblasts. Concentrations of CM18-TAT-Cys above 10 µM were associated with an increase in cell toxicity in myoblast cells, as for HeLa cells.

**Table 2.11: Monomers v. dimers CM18-TAT-Cys and CM18-Penetratin-Cys in primary myoblasts**

| **Domains** | **Peptide** | **Cells** | **Concentration (µM)** | **Mean counts** | **Stand. dev.** | **Mean Factor** |
|---|---|---|---|---|---|---|
| - | No peptide | Myoblasts | 0 | 30 175 | 4687 | 1.0 |
| ELD-CPD | CM18-TAT-Cys | Myoblasts | 5 | 88 686 | 19 481 | **2.9** |
| ELD-CPD dimer | dCM18-TAT-Cys | Myoblasts | 2.5 | 64 864 | 1264 | **2.1** |
| ELD-CPD | CM18-Penetratin-Cys | Myoblasts | 5 | 65 636 | 3288 | **2.2** |
| ELD-CPD dimer | dCM18-Penetratin-Cys | Myoblasts | 2.5 | 71 547 | 10 975 | **2.4** |

The results in **Table 2.11** suggest that shuttle peptide dimers are able to facilitate calcein endosomal escape levels that are comparable to the corresponding monomers in primary myoblasts.

### Example 3:

### Peptide shuttle agents increase GFP transduction efficiency

### 3.1 Protein transduction assay

One day before the transduction assay was performed, mammalian cells (e.g., HEK293, CHO, HeLa, THP-1, and myoblasts) in exponential growth phase were harvested and plated in a 96-well plate (20,000 cells per well). The cells were incubated overnight in appropriate growth media containing FBS (see **Example 1**). The next day, in separate sterile 1.5 mL tubes, cargo protein at 0.5 to 10 µM (GFP, TAT-GFP, GFP-NLS, or FITC-labeled anti-tubulin antibody) was pre-mixed (pre-incubated) for 10 min at 37°C with shuttle agents (0.5 to 5 µM) in 50 µL of fresh medium without serum (unless otherwise specified). GFP, GFP-NLS and TAT-GFP are recombinant proteins developed and produced by Feldan (see **Example 3.4** below). FITC-labeled anti-tubulin antibody was purchased from Abcam (ab64503). The media in wells was removed and the cells were washed three times with freshly prepared phosphate buffered saline (PBS) previously warmed at 37°C. The cells were incubated with the cargo protein/shuttle agent mixture at 37°C for 5 or 60 min. After the incubation, the cells were quickly washed three times with freshly prepared PBS and/or heparin (0.5 mg/mL) previously warmed at 37°C. The washes with heparin were required for human THP-1 blood cells to avoid undesired cell membrane-bound protein background in subsequent analyses (microscopy and flow cytometry). The cells were finally incubated in 50 µL of fresh medium with serum at 37°C before analysis.

### 3.2 Fluorescence microscopy analysis

The delivery of fluorescent protein cargo in cytosolic and nuclear cell compartments was observed with an Olympus IX70™ microscope (Japan) equipped with a fluorescence lamp (Model U-LH100HGAPO) and different filters. The Olympus filter U-MF2™ (C54942-Exc495/Em510) was used to observe GFP and FITC-labeled antibody fluorescent signals. The Olympus filter HQ-TR™ (V-N41004-Exc555-60/Em645-75) was used to observe mCherry™ and GFP antibody fluorescent signals. The Olympus filter U-MWU2™ (Exc330/Em385) was used to observe DAPI or Blue Hoechst fluorescent signals. The cells incubated in 50 µL of fresh medium were directly observed by microscopy (Bright-field and fluorescence) at different power fields (4x to 40x). The cells were observed using a CoolSNAP-PRO™ camera (Series A02D874021) and images were acquired using the Image-Proplus™ software.

### 3.2a Cell immuno-labelling

Adherent cells were plated on a sterile glass strip at 1.5x10⁵ cells per well in a 24-plate well and incubated overnight at 37°C. For fixation, cells were incubated in 500 µL per well of formaldehyde (3.7% v/v) for 15 minutes at room temperature, and washed 3 times for 5 minutes with PBS. For permeabilization, cells were incubated in 500 µL per well of Triton™ X-100 (0.2%) for 10 minutes at room temperature, and washed 3 times for 5 minutes with PBS. For blocking, cells were incubated in 500 µL per well of PBS containing 1% BSA (PBS/BSA) for 60 minutes at room temperature. Primary mouse monoclonal antibody was diluted PBS/BSA (1%). Cells were incubated in 30 µL of primary antibody overnight at 4°C. Cells were washed 3 times for 5 minutes with PBS. Secondary antibody was diluted in PBS/BSA (1%) and cells were incubated in 250 µL of secondary antibody 30 minutes at room temperature in the dark. Cells were washed 3 times for 5 minutes with PBS. Glass strips containing the cells were mounted on microscope glass slides with 10 µL of the mounting medium Fluoroshield™ with DAPI.

### 3.3 Flow cytometry analysis:

The fluorescence of GFP was quantified using flow cytometry (Accuri C6, Becton, Dickinson and Company (BD)). Untreated cells were used to establish a baseline in order to quantify the increased fluorescence due to the internalization of the fluorescent protein in treated cells. The percentage of cells with a fluorescence signal above the maximum fluorescence of untreated cells, "mean %" or "Pos cells (%)", is used to identify positive fluorescent cells. "Relative fluorescence intensity (FL1-A)" corresponds to the mean of all fluorescence intensities from each cell with a fluorescent signal after fluorescent protein delivery with the shuttle agent. Also, the events scanned by flow cytometry corresponding to cells (size and granularity) were analyzed. The cellular toxicity (% cell viability) was monitored comparing the percentage of cells in the total events scanned of treated cells comparatively to untreated cells.

### 3.3a Viability analysis

The viability of cells was assessed with a rezazurine test. Rezazurine is a sodium salt colorant that is converted from blue to pink by mitochondrial enzymes in metabolically active cells. This colorimetric conversion, which only occurs in viable cells, can be measured by spectroscopy analysis in order to quantify the percentage of viable cells. The stock solution of rezazurine was prepared in water at 1 mg/100 mL and stored at 4°C. 25 µL of the stock solution was added to each well of a 96-well plate, and cells were incubated at 37°C for one hour before spectrometry analysis. The incubation time used for the rezazurine enzymatic reaction depended on the quantity of cells and the volume of medium used in the wells.

### 3.4 Construction and amino acid sequence of GFP

The GFP-enooding gene was cloned in a T5 bacterial expression vector to express a GFP protein containing a 6x histidine tag and a serine/glycine rich linker in the N-terminal end, and a serine/glycine rich linker and a stop codon (-) at the C-terminal end. Recombinant GFP protein was purified as described in **Example 1.4.** The sequence of the GFP construct was: 
MHHHHHHGGGGSGGGGSGGASTGTGIRMVSKGEELFTGVVPILVELDGDVNGHKFSVSGEGEGDAT
YGKLTLKFICTTGKLPVPWPTLVTTLTYGVQCFSRYPDHMKQHDFFKSAMPEGYVQERTIFFKDDG
NYKTRAEVKFEGDTLVNRIELKGIDFKEDGNILGHKLEYNYNSHNVYIMADKQKNGIKVNFKIRHN
IEDGSVQLADHYQQNTPIGDGPVLLPDNHYLSTQSALSKDPNEKRDHMVLLEFVTAAGITLGMDEL
YKGGSGGGSGGGSGWIRASSGGREIS- [SEQ ID NO: 60]
 (MW= 31.46 kDa; pl=6.19)
Serine/glycine rich linkers are in bold
GFP sequence is underlined

### 3.5 GFP transduction by CM18-TAT-Cys in HeLa cells: Fluorescence microscopy

HeLa cells were cultured and tested in the protein transduction assay described in **Example 3.1.** Briefly, GFP recombinant protein was co-incubated with 0, 3 or 5 µM of CM18-TAT, and then exposed to HeLa cells for 1 hour. The cells were observed by bright field and fluorescence microscopy as described in **Example 3.2.** The results presented in **Figure 5** show that GFP was delivered intracellularly to HeLa cells in the presence of the shuttle agent CM18-TAT.

### 3.6 GFP transduction by shuttle agents in HeLa cells: Dose responses (CM18-TAT-Cys, dCM18-TAT-Cys, GFP) and cell viability

HeLa cells were cultured and tested in the protein transduction assay described in **Examples 3.1-3.3.** Briefly, GFP recombinant protein was co-incubated with different concentrations of CM18-TAT-Cys ordimerized CM18-TAT-Cys (dCM18-TAT-Cys), and then exposed to HeLa cells for 1 hour. The results are shown in **Table 3.1** and **Figure 6****.**

**Table 3.1: Dose response (CM18-TAT) and cell viability, data from Figure 6A and 6B**

| **Shuttle** | **Cells** | **Concentration (µM)** | **Figure 6A** | | **Figure 6B** |
|---|---|---|---|---|---|
| | | | **Mean (%) (n=3)** | **Standard deviation** | **Cell (%) viability (± St. Dev.; n=3)** |
| **CM18-TAT-**Cys | HeLa | 0 | 0.69 | 0.12 | 95 ± 4 |
| | HeLa | 0.5 | **8.67** | 0.96 | 88.4 ± 6 |
| | HeLa | 1 | **20.03** | 2.55 | 90 ± 6 |
| | HeLa | 3 | **31.06** | 5.28 | 91 ± 5 |
| | HeLa | 5 | **36.91** | 4.33 | 90 ± 7 |

**Table 3.1** and **Figure 6A** show the results of flow cytometry analysis of the fluorescence intensity of HeLa cells transduced with GFP (5 µM) without or with 5, 3, 1, and 0.5 µM of CM18-TAT-Cys. Corresponding cellular toxicity data are presented in **Table 3.1** and in **Figure 6B****.** These results suggest that the shuttle agent CM18-TAT-Cys increases the transduction efficiency of GFP in a dose-dependent manner.

**Table 3.2: Dose response (GFP), data from Figures 7A and 7B**

| **Shuttle** | **Cells** | **Conc. of shuttle agent (µM)** | **Conc. of GFP (µM)** | **Mean (%) (n-3)** | **Standard deviation** |
|---|---|---|---|---|---|
| **Control** | HeLa | 0 | 10 | 0.93 | 0.08 |
| **CM18-TAT**-Cys | HeLa | 5 | 10 | **37.1** | 4.29 |
| | HeLa | 5 | 5 | **21.1** | 2.19 |
| | HeLa | 5 | 1 | **8.56** | 1.91 |
| | | | | | |
| **Control** | HeLa | 0 | 10 | **0.91** | 0.09 |
| **dCM18-TAT-**Cys | HeLa | 2.5 | 10 | **34.2** | 3.42 |
| | HeLa | 2.5 | 5 | **22.2** | 3.17 |
| | HeLa | 2.5 | 1 | **9.38** | 2.11 |

**Table 3.2** and **Figure 7** show the results of flow cytometry analysis of the fluorescence intensity of HeLa cells transduced with different concentrations of GFP (1 to 10 µM) without or with 5 µM of CM18-TAT-Cys **(****Figure 7A**) or 2.5 µM dCM18-TAT-Cys **(****Figure 7B****).**

### 3.7 GFP transduction in HeLa cells: Dose responses of CM18-TAT-Cys and CM18-Penetratin-Cys, and dimers thereof

HeLa cells were cultured and tested in the protein transduction assay described in **Example 3.1.** Briefly, GFP recombinant protein (5 µM) was co-incubated with different concentrations and combinations of CM18-TAT-Cys, CM18-Penetratin-Cys, and dimers of each (dCM18-TAT-Cys, dCM18-Penetratin-Cys), and then exposed to HeLa cells for 1 hour. The cells were subjected to flow cytometry analysis as described in **Example 3.3.** The results are shown in **Table 3.3** and **Figure 8****,** as well as in **Table 3.4** and **Figure 9****.**

**Table 3.3: Data in Figure 8**

| **No. in** **Figure 8** | **Shuttle agent** | **Cells** | **Concentration (µM)** | **Mean (%) (n=3)** | **Standard deviation** |
|---|---|---|---|---|---|
| Control ("ctrl") | No shuttle | HeLa | 0 | **0.43** | 0.08 |
| 1 | CM18-TAT-Cys | HeLa | 0.5 | **8.75** | 0.63 |
| 2 | dCM18-TAT-Cys | HeLa | 0.5 | **8.86** | 1.03 |
| 3 | CM18-Penetratin-Cys | HeLa | 3 | **0.59** | 0.11 |
| 4 | dCM 18-Penetratin-Cvs | HeLa | 3 | **0.73** | 0.08 |
| 1 + 3 | CM18-TAT-Cys + CM18-Penetratin-Cys | HeLa | 0.5 3 | **19.52** | 2.18 |
| 2+3 | dCM18-TAT-Cys + CM18-Penetratin-Cys | HeLa | 0.5 3 | **22.44** | 3.29 |
| 1 +4 | CM18-TAT-Cys + dCM 18-Penetratin-Cys | HeLa | 0.5 3 | **18.73** | 1.55 |
| 2+4 | dCM18-TAT-Cys + dCM18-Penetratin-Cys | HeLa | 0.5 3 | **17.19** | 1.93 |

The results in **Table 3.3** and **Figure 8** show that the transduction efficiency of GFP is increased in HeLa cells using the shuttle agents CM18-TAT-Cys and dCM18-TAT-Cys (see bars "1" and "2" in **Figure 8**). Although no GFP intracellular delivery was observed using CM18-Penetratin-Cys or dCM18-Penetratin-Cys alone (see bars "3" or "4" in **Figure 8**), combination of CM18-TAT-Cys with CM18-Penetratin-Cys (monomer or dimer) improved GFP protein delivery (see four right-most bars in **Figure 8**).

**Table 3.4: Data in Figure 9**

| **Figure 9** **No. in** | **Shuttle** | **Cells** | **Concentration (µM)** | **Mean (%) (n=3)** | **Standard deviation** |
|---|---|---|---|---|---|
| Control ("ctrl") | No shuttle | HeLa | 0 | **0.51** | 0.07 |
| 1 | CM18-TAT-Cys | HeLa | 1 | **20.19** | 2.19 |
| 2 | dCM18-TAT-Cys | HeLa | 1 | **18.43** | 1.89 |
| 3 | CM18-Penetratin-Cys | HeLa | 3 | **0.81** | 0.07 |
| 4 | dCM 18-Penetratin-Cys | HeLa | 3 | **0.92** | 0.08 |
| 1 +3 | CM18-TAT-Cys + CM18-Penetratin-Cys | HeLa | 1 | **30.19** | 3.44 |
| | | | 3 | | |
| 2+3 | dCM18-TAT-Cys + CM18-Penetratin-Cys | HeLa | 1 | **22.36** | 2.46 |
| | | | 3 | | |
| 1 +4 | CM18-TAT-Cys + dCM18-Penetratin-Cys | HeLa | 1 | **26.47** | 2.25 |
| | | | 3 | | |
| 2+4 | dCM18-TAT-Cys + dCM18-Penetratin-Cys | HeLa | 1 | **21.44** | 3.11 |
| | | | 3 | | |

The results in **Table 3.4** and **Figure 9** show that the transduction efficiency of GFP is increased in HeLa cells using the shuttle agents CM18-TAT-Cys and dCM18-TAT-Cys (see bars "1" and "2" in **Figure 9**). Although no GFP intracellular delivery was observed using CM18-Penetratin-Cys or dCM18-Penetratin-Cys alone (see bars "3" or "4" in **Figure 9**), combination of CM18-TAT-Cys with CM18-Penetratin-Cys (monomer or dimer) improved GFP protein delivery (see four right-most bars in **Figure 9**).

### 3.8 GFP transduction by shuttle agents in HeLa cells: Controls

HeLa cells were cultured and tested in the protein transduction assay described in **Example 3.1.** Briefly, GFP recombinant protein (5 µM) was co-incubated with 5 µM of each of the following peptide(s): TAT-Cys; CM18; Penetratin-Cys; TAT-Cys + CM18; Penetratin-Cys + CM18; and CM18-TAT-Cys, and then exposed to HeLa cells for 1 hour. GFP fluorescence was visualized by bright field and fluorescence microscopy. The microscopy results (data not shown) showed that GFP was successfully delivered intracellularly using CM18-TAT-Cys. However, GFP was not successfully delivered intracellularly using single-domain peptides used alone (CM18, TAT-Cys, Penetratin-Cys) or together (CM18 +TAT-Cys, CM18 + Penetratin-Cys). These results are consistent with those presented in **Tables 2.1 and 2.2** with respect to the calcein endosome escape assays.

### Example 4:

### Peptide shuttle agents increase TAT-GFP transduction efficiency

The experiments in **Example 3** showed the ability of shuttle agents to deliver GFP intracellularly. The experiments presented in this example show that the shuttle agents can also increase the intracellular delivery of a GFP cargo protein that is fused to a CPD (TAT-GFP).

### 4.1 Construction and amino acid sequence of TAT-GFP

Construction was performed as described in **Example 3.4,** except that a TAT sequence was cloned between the 6x histidine tag and the GFP sequences. The 6x histidine tag, TAT, GFP and a stop codon (-) are separated by serine/glycine rich linkers. The recombinant TAT-GFP protein was purified as described in **Example 1.4.** The sequence of the TAT-GFP construct was: 
MHHHHHHGGGGSGGGGSGGASTGTGRKKRRQRRRPPQGGGGSGGGGSGGGTGIRMVSKGEELFTGV
VPILVELDGDVNGHKFSVSGEGEGDATYGKLTLKFICTTGKLPVPWPTLVTTLTYGVQCFSRYPDH
MKQHDFFKSAMPEGYVQERTIFFKDDGNYKTRAEVKFEGDTLVNRIELKGIDFKEDGNILGHKLEY
NYNSHNVYIMADKQKNGIKVNFKIRHNIEDGSVQLADHYQQNTPIGDGPVLLPDNHYLSTQSALSK
DPNEKRDHMVLLEFVTAAGITLGMDELYKGGSGGGSGGGSGWIRASSGGREIS-
[SEQ ID NO: 61]
 (MW= 34.06 kDa ; pl=8.36)
TAT sequence is underlined
Serine/glycine rich linkers are in bold

### 4.2 TAT-GFP transduction by CM18-TAT-Cys in HeLa cells: Visualisation by fluorescence microscopy

HeLa cells were cultured and tested in the protein transduction assay described in **Example 3.1.** Briefly, TAT-GFP recombinant protein (5 µM) was co-incubated with 3 µM of CM18-TAT-Cys and then exposed to HeLa cells for 1 hour. Cells and GFP fluorescence were visualized by bright field and fluorescence microscopy (as described in **Example 3.2**) at 10x and 40x magnifications, and sample results are shown in **Figure 10****.** The microscopy results revealed that in the absence of CM18-TAT-Cys, TAT-GFP shows a low intensity, endosomal distribution as reported in the literature. In contrast, TAT-GFP is delivered to the cytoplasm and to the nucleus in the presence of the shuttle agent CM18-TAT-Cys. Without being bound by theory, the TAT peptide itself may act as a nuclear localization signal (NLS), explaining the nuclear localization of TAT-GFP. These results show that CM18-TAT-Cys is able to increase TAT-GFP transduction efficiency and allow endosomally-trapped TAT-GFP to gain access to the cytoplasmic and nuclear compartments.

### 4.3 TAT-GFP transduction by CM18-TAT-Cys in HeLa cells: Dose responses and viability of cells transduced

HeLa cells were cultured and tested in the protein transduction assay described in **Example 3.1.** Briefly, TAT-GFP-Cys recombinant protein (5 µM) was co-incubated with different concentrations of CM18-TAT-Cys (0, 0.5, 1, 3, or 5 µM) and then exposed to HeLa cells for 1 hour. The cells were subjected to flow cytometry analysis as described in **Example 3.3.** Results are shown in **Table 4.3** and **Figure 11A****.** Corresponding cellular toxicity data are presented in **Figure 11B****.**

**Table 4.3: Data from Figure 11A and 11B**

| **Shuttle agent** | **Cells** | **Concentration (µM)** | **Figure 11A** | | **Figure 11B** |
|---|---|---|---|---|---|
| | | | **Mean (%)** | **Standard deviation** | **Cell viability (%) St. n= 3)** |
| **CM18-TAT**-Cys | HeLa | 0 | **11.79¹** | 1.16 | 100 |
| | HeLa | 0.5 | **10.19** | 1.94 | 84.36 ± 5 |
| | HeLa | 1 | **14.46** | 2.59 | 89.26 ± 5.26 |
| | HeLa | 3 | **28.12** | 3.27 | 93.18 ± 6.28 |
| | HeLa | 5 | **35.5²** | 3.59 | 95.14 ± 5.28 |

| | | | | | |
|---|---|---|---|---|---|
| ¹ The fluorescence was mostly endosomal, as confirmed by fluorescence microscopy. ² Fluorescence was more diffuse and also nuclear, as confirmed by fluorescence microscopy. | | | | | |

### Example 5:

### Peptide shuttle agents increase GFP-NLS transduction efficiency and nuclear localization

The experiments in **Examples 3 and 4** showed the ability of shuttle agents to deliver GFP and TAT-GFP intracellularly. The experiments presented in this example show that the shuttle agents can facilitate nuclear delivery of a GFP protein cargo fused to a nuclear localization signal (NLS).

### 5.1 Construction and amino acid sequence of GFP-NLS

Construction was performed as described in **Example 3.4,** except that an optimized NLS sequence was cloned between the GFP sequence and the stop codon (-). The NLS sequence is separated from the GFP sequence and the stop codon by two serine/glycine rich linkers. The recombinant GFP-NLS protein was purified as described in **Example 1.4.** The sequence of the GFP-NLS construct was: 
MHHHHHHGGGGSGGGGSGGASTGIRMVSKGEELFTGVVPILVELDGDVNGHKFSVSGEGEGDATY
GKLTLKFICTTGKLPVPWPTLVTTLTYGVQCFSRYPDHMKQHDFFKSAMPEGYVQERTIFFKDDG
NYKTRAEVKFEGDTLVNRIELKGIDFKEDGNILGHKLEYNYNSHNVYIMADKQKNGIKVNFKIRH
NIEDGSVQLADHYQQNTPIGDGPVLLPDNHYLSTQSALSKDPNEKRDHMVLLEFVTAAGITLGMD
ELYKGGSGGGSGGGSGWIRASSGGRSSDDEATADSQHAAPPKKKRKVGGSGGGSGGGSGGGRGTE
IS- [SEQ ID NO: 62]
 (MW = 34.85 kDa; pl = 6.46)
NLS sequence is underlined
Serine/glycine rich linkers are in bold

### 5.2 Nuclear delivery of GFP-NLS by CM18-TAT-Cys in HeLa cells in 5 minutes: Visualisation by fluorescence microscopy

HeLa cells were cultured and tested in the protein transduction assay described in **Example 3.1.** Briefly, GFP-NLS recombinant protein (5 µM) was co-incubated with 5 µM of CM18-TAT-Cys, and then exposed to HeLa cells. GFP fluorescence was visualized by bright field and fluorescence microscopy after 5 minutes (as described in **Example 3.2**) at 10x, 20x and 40x magnifications, and sample results are shown in **Figure 12****.** The microscopy results revealed that GFP-NLS is efficiently delivered to the nucleus in the presence of the shuttle agent CM18-TAT-Cys, after only 5 minutes of incubation.

### 5.3 GFP-NLS transduction by CM18-TAT-Cys in HeLa cells: Dose responses and viability of cells transduced

HeLa cells were cultured and tested in the protein transduction assay described in **Example 3.1.** GFP-NLS recombinant protein (5 µM) was co-incubated with 0, 0.5, 1, 3, or 5 µM of CM18-TAT-Cys, and then exposed to HeLa cells for 1 hour. The cells were subjected to flow cytometry analysis as described in **Example 3.3.** Results are shown in **Table 5.1** and **Figure 13A****.** Corresponding cellular toxicity data are presented in **Figure 13B****.**

**Table 5.1: Data from Figure 13A and 13B**

| **Shuttle agent** | **Cells** | **Concentration (µM)** | **Figure 13A** | | **Figure 13B** |
|---|---|---|---|---|---|
| | | | **Mean (%)** | **Standard deviation** | **Cell viability (%) Dev.; (± St. n= 3)** |
| **CM18-TAT**-Cys | HeLa | 0 | **0.90** | 0.12 | 100 |
| | HeLa | 0.5 | **9.81** | 1.63 | 87.6 ± 4 |
| | HeLa | 1 | **18.42** | 2.47 | 93 ± 8 |
| | HeLa | 3 | **28.09** | 3.24 | 94 ± 5 |
| | HeLa | 5 | **32.26** | 4.79 | 93 ± 4 |

These results show that CM18-TAT-Cys is able to increase GFP-NLS transduction efficiency in HeLa cells in a dose-dependent manner.

### 5.4 GFP-NLS transduction by CM18-TAT-Cys, CM18-Penetratin-Cys, and dimers thereof in HeLa cells

HeLa cells were cultured and tested in the protein transduction assay described in **Example 3.1.** GFP-NLS recombinant protein (5 µM) was co-incubated with different concentrations and combinations of CM18-TAT-Cys, CM18-Penetratin-Cys, and dimers of each (dCM18-TAT-Cys, dCM18-Penetratin-Cys), and then exposed to HeLa cells for 1 hour. The cells were subjected to flow cytometry analysis as described in **Example 3.3.** The results are shown in **Tables 5.2 and 5.3,** and in **Figures 14 and 15****.**

**Table 5.2: Data in Figure 14**

| **No. in** **Figure 14** | **Shuttle agent** | **Cells** | **Concentration (µM)** | **Mean (%) (n=3)** | **Standard deviation** |
|---|---|---|---|---|---|
| ctrl | No shuttle | HeLa | 0 | **0.41** | 0.10 |
| 1 | CM18-TAT-Cys | HeLa | 0.5 | **7.64** | 0.85 |
| 2 | dCM18-TAT-Cys | HeLa | 0.5 | **8.29** | 0.91 |
| 3 | CM18-Penetratin-Cys | HeLa | 3 | **0.43** | 0.08 |
| 4 | dCM 18-Penetratin-Cys | HeLa | 3 | **0.85** | 0.07 |
| 1 +3 | CM18-TAT-Cys + CM18-Penetratin-Cys | HeLa | 0.5 | **21.1** | 2.47 |
| | | | 3 | | |
| 2+3 | dCM18-TAT-Cys + CM18-Penetratin-Cys | HeLa | 0.5 | **19.22** | 2.73 |
| | | | 3 | | |
| 1 +4 | CM18-TAT-Cys + dCM 18-Penetratin-Cys | HeLa | 0.5 | **23.44** | 2.51 |
| | | | 3 | | |
| 2+4 | dCM18-TAT-Cys + dCM 18-Penetratin-Cys | HeLa | 0.5 | **19.47** | 2.16 |
| | | | 3 | | |

**Table 5.3: Data in Figure 15**

| **No. in** **Figure 15** | **Shuttle agent** | **Cells** | **Concentration (µM)** | **Mean (%) (n=3)** | **Standard deviation** |
|---|---|---|---|---|---|
| ctrl | No shuttle | HeLa | 0 | **0.44** | 0.12 |
| 1 | CM18-TAT-Cys | HeLa | 1 | **15.56** | 2.24 |
| 2 | dCM18-TAT-Cys | HeLa | 1 | **17.83** | 2.13 |
| 3 | CM18-Penetratin-Cys | HeLa | 3 | **0.68** | 0.05 |
| 4 | dCM 18-Penetratin-Cys | HeLa | 3 | **0.84** | 0.07 |
| 1 +3 | CM18-TAT-Cys + CM18-Penetratin-Cys | HeLa | 1 | **27.26** | 3.61 |
| | | | 3 | | |
| 2+3 | dCM18-TAT-Cys + CM18-Penetratin-Cys | HeLa | 1 | **25.47** | 3.77 |
| | | | 3 | | |
| 1 +4 | CM18-TAT-Cys dCM18-Penetratin-Cys | HeLa | 1 | **31.47** | 4.59 |
| | | | 3 | | |
| 2+4 | dCM18-TAT-Cys + dCM 18-Penetratin-Cys | HeLa | 1 | **28.74** | 2.93 |
| | | | 3 | | |

The results in **Tables 5.2 and 5.3** and **Figures 14 and 15** show that the transduction efficiency of GFP-NLS is increased in HeLa cells using the shuttle agents CM18-TAT-Cys and dCM18-TAT-Cys (see bars "1" and "2" in **Figures 14 and 15**). Although no GFP-NLS intracellular delivery was observed using CM18-Penetratin-Cys or dCM18-Penetratin-Cys alone (see bars "3" and "4" in **Figures 14 and 15**), combination of CM18-TAT-Cys with CM18-Penetratin-Cys (monomer or dimer) improved GFP-NLS intracellular delivery (see four right-most bars in **Figures 14 and 15**).

### 5.5 GFP-NLS transduction by shuttle agents in HeLa cells: 5 min v. 1 h incubation; with or without FBS

HeLa cells were cultured and tested in the protein transduction assay described in **Example 3.1.** GFP-NLS recombinant protein (5 µM) was co-incubated with either CM18-TAT-Cys (3.5 µM) alone or with dCM18-Penetratin-Cys (1 µM). Cells were incubated for 5 minutes or 1 hour in plain DMEM media ("DMEM") or DMEM media containing 10% FBS ("FBS"), before being subjected to flow cytometry analysis as described in **Example 3.3.** The results are shown in **Table 5.4,** and in **Figure 16****.** Cells that were not treated with shuttle agent or GFP-NLS ("ctrl"), and cells that were treated with GFP-NLS without shuttle agent ("GFP-NLS 5 µM") were used as controls.

**Table 5.4: Data in Figure 16**

| **Shuttle** | **No. in** **Fig. 16** | **Cells** | **Medium** | **Incubation time** | **Shuttle Conc. (µM)** | **Mean (%) (n=3)** | **Standard deviation** |
|---|---|---|---|---|---|---|---|
| No shuttle (Ctrl) | 1 | HeLa | DMEM | 1h | 0 | **0.59** | 0.09 |
| GFP-NLS alone | 2 | HeLa | DMEM | 1h | 0 | **1.19** | 0.31 |
| CM18-TAT-Cys | 3 | HeLa | DMEM | 1 h | 3.5 | **20.69** | 1.19 |
| | 4 | HeLa | FBS | 1 h | 3.5 | **13.20** | 0.82 |
| CM18-TAT-Cys | 5 | HeLa | DMEM | 5 min | 3.5 | **20.45** | 4.26 |
| | 6 | HeLa | FBS | 5 min | 3.5 | **10.83** | 1.25 |
| | | | | | | | |
| No shuttle (Ctrl) | 1 | HeLa | DMEM | 1h | 0 | **0.53** | 0.11 |
| GFP-NLS alone | 2 | HeLa | DMEM | 1h | 0 | **1.25** | 0.40 |
| CM18-TAT-Cys + dCM 18-Penetratin-Cys | 3 | HeLa | DMEM | 1 h | 3.5 1 | **27.90** | 2.42 |
| | 4 | HeLa | FBS | 1 h | 3.5 1 | **8.35** | 0.46 |
| CM18-TAT-Cys + dCM 18-Penetratin-Cys | 5 | HeLa | DMEM | 5 min | 3.5 1 | **24.10** | 2.76 |
| | 6 | HeLa | FBS | 5 min | 3.5 1 | **5.02** | 0.72 |

The results in **Table 5.4** and **Figure 16** show that the addition of even a relatively low amount of the dimer dCM18-Penetratin-Cys (1 µM; "dCM18pen") to the CM18-TAT-Cys monomer improved GFP-NLS transduction efficiency. Interestingly, intracellular GFP-NLS delivery was achieved in as little as 5 minutes of incubation, and delivery was still achievable (although reduced) in the presence of FBS.

### 5.6 GFP-NLS transduction by shuttle agents in THP-1 suspension cells

The ability of the shuttle agents to deliver GFP-NLS intracellularly was tested in THP-1 cells, which is an acute monocytic leukemia cell line that grows in suspension. THP-1 cells were cultured (see **Example 1**) and tested in the protein transduction assay described in **Example 3.1.** GFP-NLS recombinant protein (5 µM) was co-incubated with or without 1 µM CM18-TAT-Cys, and exposed to the THP-1 cells for 5 minutes, before being subjected to flow cytometry analysis as described in **Example 3.3.** The results are shown in **Table 5.5** and in **Figure 17A****.** Corresponding cellular toxicity data are presented in **Figure 17B****.**

**Table 5.5: Data in Figure 17A and 17B**

| **Shuttle** | **Cells** | **Shuttle Conc. (µM)** | **Figure 17A** | | **Figure 17B** |
|---|---|---|---|---|---|
| | | | **Mean (%) (n=3)** | **Standard deviation** | **Cell (%) viability (± St. Dev.; n= 3)** |
| No shuttle (Ctrl) | THP-1 | 0 | **1.23** | 0.16 | 95 ± 4 |
| GFP-NLS alone | | 0 | **2.49** | 0.37 | 96 ± 3 |
| CM18-TAT-Cys | | 1 | **38.1** | 4.16 | 85 ± 6 |

The results in **Table 5.5** and **Figure 17** demonstrate the ability of the shuttle agents to deliver protein cargo intracellularly to a human monocytic cell line grown in suspension.

### Example 6:

### Peptide shuttle agents increase transduction efficiency of an FITC-labeled anti-tubulin antibody

The experiments in **Examples 3-5** showed the ability of shuttle agents to increase the transduction efficiency of GFP, TAT-GFP, and GFP-NLS. The experiments presented in this example show that the shuttle agents can also deliver a larger protein cargo: an FITC-labeled anti-tubulin antibody. The FITC-labeled anti-tubulin antibody was purchased from (Abcam, ab64503) and has an estimated molecular weight of 150 KDa. The delivery and microscopy protocols are described in **Example 3.**

### 6.1 Transduction of a functional antibody by CM18-TAT-Cys in HeLa cells: Visualization by microscopy

FITC-labeled anti-tubulin antibody (0.5 µM) was co-incubated with 5 µM of CM18-TAT-Cys and exposed to HeLa cells for 1 hour. Antibody delivery was visualized by bright field (20x) and fluorescence microscopy (20x and 40x). As shown in **Figure 18****,** fluorescent tubulin fibers in the cytoplasm were visualized, demonstrating the functionality of the antibody inside the cell.

### 6.2 Transduction of a functional antibody by CM18-TAT-Cys, CM18-Penetratin-Cys, and dimers in HeLa cells: Flow cytometry

HeLa cells were cultured and tested in the protein transduction assay described in **Example 3.1.** FITC-labeled anti-tubulin antibody (0.5 µM) was co-incubated with 3.5 µM of CM18-TAT-Cys, CM18-Penetratin-Cys or dCM18-Penetratin-Cys, or a combination of 3.5 µM of CM18-TAT-Cys and 0.5 µM of dCM18-Penetratin-Cys, and exposed to HeLa cells for 1 hour. The cells were subjected to flow cytometry analysis as described in **Example 3.3.** Results are shown in **Table 6.1** and **Figure 19A****.** Corresponding cellular toxicity data are presented in **Figure 19B****.**

**Table 6.1: Data from Figure 19A and 19B**

| **Domains** | **Shuttle agent** | **Cells** | **Shuttle Conc. (µM)** | **Figure** | | **Figure 19B** |
|---|---|---|---|---|---|---|
| | | | | **Mean (%) (n=3)** | **19A Standard deviation** | **Cell viability (%) (± St. Dev.; n**= **3)** |
| - | **No shuttle** ("Ctrl") | HeLa | 0 | **0.9** | 0.06 | 98 ± 1.0 |
| - | **Antibody alone** ("antibody") | HeLa | 0 | **2.66** | 0.61 | 96 ± 3.4 |
| ELD-CPD | **CM18-TAT**-Cys | HeLa | 3.5 | **36.56** | 4.06 | 95 ± 4.06 |
| | **CM18-Penetratin-**Cys | HeLa | 3.5 | **53.05** | 9.5 | 73 ± 9.5 |
| ELD-CPD dimer | **dCM18-Penetratin-**Cys | HeLa | 3.5 | **50.23** | 9.12 | 74 ± 9.0 |
| ELD-CPD + ELD-CPD dimer | **CM18-TAT-Cys** + **dCM18-Penetratin-**Cys | HeLa | 3.5 0.5 | **47.19** | 8.5 | 93 ± 8.5 |

The results in **Table 6.1** and **Figures 18** **and** **19** show that both CM18-TAT-Cys and CM18-Penetratin-Cys facilitate intracellular delivery of an FITC-labeled anti-tubulin antibody. In contrast to the results with GFP, TAT-GFP, and GFP-NLS in **Examples 3-5,** CM18-Penetratin-Cys was able to deliver the antibody cargo intracellularly when used alone (without CM18-TAT-Cys). However, combination of CM18-TAT-Cys and dCM18-Penetratin-Cys allowed for higher intracellular delivery as compared with CM18-TAT-Cys alone, and with less cell toxicity as compared to CM18-Penetratin-Cys and dCM18-Penetratin-Cys (see **Figure 19A and 19B**).

### Example 7:

### CM18-TAT-Cys enables intracellular plasmid DNA delivery but poor plasmid expression

The ability of the CM18-TAT-Cys shuttle agent to deliver plasmid DNA intracellularly was tested in this example on HEK293A cells using a plasmid encoding GFP.

### 7.1 Transfection assay in HEK293A cells

One day before the transfection assay was performed, mammalian cells (HEK293A) in exponential growth phase were harvested and plated in a 24-well plate (50,000 cells per well). The cells were incubated overnight in appropriate growth media containing FBS. The next day, in separate sterile 1.5 mL tubes, pEGFP labeled with a Cy5™ fluorochrome was mixed for 10 min at 37°C with CM18-TAT-Cys (0.05, 0.5, or 5 µM) in fresh PBS at a final 100 µL volume. The media in wells was removed and the cells were quickly washed three times with PBS and 500 µL of warm media without FBS was added. The pEGFP and CM18-TAT-Cys solution was added to the cells and incubated at 37°C for 4 hours. After the incubation, cells were washed with PBS and fresh media containing FBS was added. Cells were incubated at 37°C before being subjected to flow cytometry analysis as described in **Example 3.**

### 7.2 Plasmid DNA delivery with CM18-TAT-Cys

Plasmid DNA (pEGFP) was labeled with a Cy5™ dye following the manufacturer's instructions (Mirus Bio LLC). Cy5™ Moiety did not influence transfection efficiency when compared to unlabelled plasmid using standard transfection protoool (data not shown). Flow cytometry analysis allowed quantification of Cy5™ emission, corresponding to DNA intracellular delivery, and GFP emission, corresponding to successful nuclear delivery, DNA transcription and protein expression. The results are shown in **Table 7.1** and in **Figure 20****.**

**Table 7.1: Data from Figure 20**

| **Sample** | **DNA (ng)** | **Cy5™ fluorescence** | | **GFP expression** | |
|---|---|---|---|---|---|
| | | **Mean Cy5™ signal (n=3)** | **Standard deviation** | **Mean (% of cells with GFP signal; n=3)** | **Standard deviation** |
| **pEGFP-Cy5 alone** | **500** | **914** | **0** | **0.0%** | n/a |
| **CM18-TAT-**Cys**, 0.05 µM** | **500** | **1450** | 120 | **0.0%** | n/a |
| **CM18-TAT-**Cys, **0.5 µM** | **500** | **8362** | 294 | **0.0%** | n/a |
| **CM18-TAT-**Cys, **5 µM** | **500** | **140 497** | 3977 | **0.1%** | n/a |

The results shown in **Table 7.1** and in **Figure 20** show that CM18-TAT-Cys was able to increase the intracellular delivery the plasmid DNA when used at 0.05, 0.5 and 5 µM concentrations, as compared to cell incubated with DNA alone ("pEGFP-Cy5"). However, no expression of GFP was detected in the cells, which suggests that very little of the plasmid DNA gained access to the cytoplasmic compartment, allowing nuclear localization. Without being bound by theory, it is possible that the plasmid DNA was massively sequestered in endosomes, preventing escape to the cytoplasmic compartment. Salomone et al., 2013 reported the use of a CM18-TAT11 hybrid peptide to deliver plasmid DNA intracellularly. They used the luciferase enzyme reporter assay to assess transfection efficiency, which may not be ideal for quantifying the efficiency of cytoplasmic/nuclear delivery, as the proportion of plasmid DNA that is successfully released from endosomes and delivered to the nucleus may be overestimated due to the potent activity of the luciferase enzyme. In this regard, the authors of Salomone et al., 2013 even noted that the expression of luciferase occurs together with a massive entrapment of (naked) DNA molecules into vesicles, which is consistent with the results shown in **Table 7.1** and in **Figure 20****.**

### Example 8:

### Addition of a histidine-rich domain to shuttle agents further improves GFP-NLS transduction efficiency

### 8.1 GFP-NLS transduction by His-CM18-TAT-Cys in HeLa cells: Visualization by microscopy

GFP-NLS (5 µM; see **Example 5**) was co-incubated with 5 µM of CM18-TAT-Cys or His-CM18-TAT and exposed to HeLa cells for 1 hour. Nuclear fluorescence of intracellularly delivered GFP-NLS was confirmed by fluorescence microscopy (data not shown), indicating successful delivery of GFP-NLS to the nucleus.

### 8.2 GFP-NLS transduction by His-CM18-TAT in HeLa cells: Flow cytometry

HeLa cells were cultured and tested in the protein transduction assay described in **Example 3.1.** GFP-NLS (5 µM) was co-incubated with 0, 1, 3, or 5 µM of CM18-TAT-Cys or His-CM18-TAT, and exposed to HeLa cells for 1 hour. The cells were subjected to flow cytometry analysis as described in **Example 3.3.** Results are shown in **Table 8.1** and **Figure 21A****.** Corresponding cellular toxicity data are presented in **Figure 21B****.**

**Table 8.1: Data from Figure 21A and 21B**

| **Shuttle agent** | **Cells** | **Shuttle Conc. (µM)** | **Figure 21A** | | **Figure 21B** |
|---|---|---|---|---|---|
| | | | **Mean (%) cell with GFP signal (n=3)** | **Standard deviation** | **Cell viability (%) (± St. Dev.; n= 3)** |
| **Ctrl** (no shuttle, no GFP-NLS) | HeLa | 0 | **0.63** | 0.10 | 96 ± 3.17 |
| **GFP-NLS alone** | | 0 | **0.93** | 0.26 | 97 ± 2.05 |
| **CM18-TAT**-Cys | | 5 | **20.54** | 3.51 | 81 ± 6.34 |
| | | 3 | **15.66** | 2.18 | 89 ± 5.37 |
| | | 1 | **8.64** | 1.11 | 94 ± 4.28 |
| | | | | | |
| **Ctrl** (no shuttle, no GFP-NLS) | HeLa | 0 | **0.51** | 0.28 | 95 ± 4.19 |
| **GFP-NLS alone** | | 0 | **1.07** | 0.42 | 96 ± 3.16 |
| **His-CM18-TAT** | | 5 | **41.38** | 4.59 | 86 ± 4.59 |
| | | 3 | **29.58** | 3.61 | 91 ± 5.18 |
| | | 1 | **8.45** | 1.83 | 95 ± 3.55 |

Strikingly, the results in **Table 8.1** and in **Figure 21** show that His-CM18-TAT was able to increase GFP-NLS protein transduction efficiency by about 2-fold at 3 µM and 5 µM concentrations, as compared to CM18-TAT-Cys. These results suggest that adding a histidine-rich domain to a shuttle agent comprising an ELD and CPD, may significantly increase its polypeptide cargo transduction efficiency. Alternatively or in parallel, combining the shuttle agents with a further independent synthetic peptide containing a histidine-rich domain fused to a CPD (but lacking an ELD) may provide a similar advantage for protein transduction, with the added advantage of allowing the concentration of the histidine-rich domain to be varied orcontrolled independently from the concentration of the shuttle agent. Without being bound by theory, the histidine-rich domain may act as a proton sponge in the endosome, providing another mechanism of endosomal membrane destabilization.

### Example 9:

### His-CM18-PTD4 increases transduction efficiency and nuclear delivery of GFP-NLS, mCherry™-NLS and FITC-labeled anti-tubulin antibody

### 9.1 Protein transduction protocols

### Protocol A: Protein transduction assay for delivery in cell culture medium

One day before the transduction assay was performed, cells in exponential growth phase were harvested and plated in a 96-well plate (20,000 cells per well). The cells were incubated overnight in appropriate growth media containing FBS (see **Example 1**). The next day, in separate sterile 1.5-mL tubes, cargo protein at the desired concentration was pre-mixed (pre-incubated) for 10 min at 37°C with the desired concentration of shuttle agents in 50 µL of fresh serum-free medium (unless otherwise specified). The media in wells was removed and the cells were washed one to three times (depending on the type of cells used) with PBS previously warmed at 37°C. The cells were incubated with the cargo protein/shuttle agent mixture at 37°C for the desired length of time. After the incubation, the cells were washed three times with PBS and/or heparin (0.5 mg/mL) previously warmed at 37°C. The washes with heparin were used for human THP-1 blood cells to avoid undesired cell membrane-bound protein background in subsequent analyses (microscopy and flow cytometry). The cells were finally incubated in 50 µL of fresh medium with serum at 37°C before analysis.

### Protocol B: Protein transduction assay for adherent cells in PBS

One day before the transduction assay was performed, cells in exponential growth phase were harvested and plated in a 96-well plate (20,000 cells per well). The cells were incubated overnight in appropriate growth media containing serum (see **Example 1**). The next day, in separate sterile 1.5-mL tubes, shuttle agents were diluted in sterile distilled water at room temperature (if the cargo is or comprised a nucleic acid, nuclease-free water was used). Cargo protein(s) were then added to the shuttle agents and, if necessary, sterile PBS was added to obtain the desired concentrations of shuttle agent and cargo in a sufficient final volume to cover the cells (e.g., 10 to 100 µL per well for a 96-well plate). The shuttle agent/cargo mixture was then immediately used for experiments. At least three controls were included for each experiment, including: (1) shuttle agent alone (e.g., at highest concentration tested); (2) cargo alone; and (3) without any cargo or shuttle agent. The media in wells was removed, cells were washed once with PBS previously warmed at 37°C, and the shuttle agent/cargo mixture was then added to cover all cells for the desired length of time. The shuttle agent/cargo mixture in wells was removed, the cells were washed once with PBS, and fresh complete medium was added. Before analysis, the cells were washed once with PBS and fresh complete medium was added.

### Protocol C: Protein transduction assay for suspension cells in PBS

One day before the transduction assay was performed, suspension cells in exponential growth phase were harvested and plated in a 96-well plate (20,000 cells perwell). The cells were incubated overnight in appropriate growth media containing serum (see **Example 1**). The next day, in separate sterile 1.5-mL tubes, shuttle agents were diluted in sterile distilled water at room temperature (if the cargo is or comprised a nucleic acid, nuclease-free water was used). Cargo protein(s) were then added to the shuttle agents and, if necessary, sterile PBS or cell culture medium (serum-free) was added to obtain the desired concentrations of shuttle agent and cargo in a sufficient final volume to resuspend the cells (e.g., 10 to 100 µL per well in a 96-well plate). The shuttle agent/cargo mixture was then immediately used for experiments. At least three controls were included for each experiment, including: (1) shuttle agent alone (e.g., at highest concentration tested); (2) cargo alone; and (3) without any cargo or shuttle agent. The cells were centrifuged for 2 minutes at 400g, the medium was then removed and the cells were resuspended in PBS previously warmed at 37°C. The cells were centrifuged again 2 minutes at 400g, the PBS removed, and the cells were resuspended in the shuttle agent/cargo mixture. After the desired incubation time, 100 µL of complete medium was added directly on the cells. Cells were centrifuged for 2 minutes at 400g and the medium was removed. The pellet was resuspended and washed in 200 µL of PBS previously warmed at 37°C. After another centrifugation, the PBS was removed and the cells were resuspended in 100 µL of complete medium. The last two steps were repeated one time before analysis.

### 9.2 GFP-NLS transduction by His-CM18-PTD4 in HeLa cells using Protocol A or B: Flow cytometry

To compare the effects of different protocols on shuttle agent transduction efficiency, HeLa cells were cultured and tested in the protein transduction assays using Protocol A or B as described in **Example 9.1.** Briefly, GFP-NLS recombinant protein (5 µM; see **Example 5.1**) was co-incubated with 10 µM of His-CM18-PTD4 and exposed to HeLa cells for 1 hour using Protocol A, or was co-incubated with 35 µM of His-CM18-PTD4 and exposed to HeLa cells for 10 seconds using Protocol B. The cells were subjected to flow cytometry analysis as described in **Example 3.3.** Results are shown in **Table 9.1** and **Figure 22A****.** ("Pos cells (%)" is the percentage of cells emanating a GFP signal).

**Table 9.1: Comparison of Protein Transduction Protocols A and B: Data from Figure 22A**

| **Protocol** | **Shuttle** | **Cells** | **Conc. of shuttle (µM)** | **Conc. of GFP-NLS (µM)** | **Mean % cells with GFP signal (± St. Dev.; n=3)** | **Cell viability (%) (± St. Dev.: n=3)** |
|---|---|---|---|---|---|---|
| **B** | **None ("Ctrl")** | HeLa | 0 | 5 | **0.53** ± 0.07 | **100** |
| **A** | **His-CM18-PTD4** | HeLa | 10 | 5 | **25.4** ± 3.6 | **96.4** ± 2.7 |
| **B** | **His-CM18-PTD4** | HeLa | 35 | 5 | **78.3** ± 5.3 | **94.6** ±0.4 |

The above results show that higher protein transduction efficiency for the cargo GFP-NLS using the shuttle agent His-CM18-PTD4 was obtained using Protocol B, as compared to Protocol A.

### 9.3 GFP-NLS transduction by His-CM18-PTD4 in HeLa cells using Protocol B: Flow cytometry

A dose response experiment was performed to evaluate the effect of His-CM18-PTD4 concentration on protein transduction efficiency. HeLa cells were cultured and tested in the protein transduction assay described in Protocol B of **Example 9.1.** Briefly, GFP-NLS recombinant protein (5 µM; see **Example 5.1**) was co-incubated with 0, 50, 35, 25, or 10 µM of His-CM18-PTD4, and then exposed to HeLa cells for 10 seconds. The cells were subjected to flow cytometry analysis as described in **Example 3.3.** Results are shown in **Table 9.2** and **Figure 22B****.**

**Table 9.2: Dose response of shuttle agent using Protocol B: Data from Figure 22B**

| **Protocol** | **Shuttle** | **Cells** | **Conc. of shuttle (µM)** | **Conc. of GFP-NLS (µM)** | **Mean % cells with GFP signal (± St. Dev.; n=3)** | **Cell viability (%) (± St. Dev.; n=3)** |
|---|---|---|---|---|---|---|
| **B** | **None ("Ctrl")** | | 0 | 5 | **0.13** ± 0.1 | **100** ± 0 |
| | **His-CM18-PTD4** | HeLa | 50 | 5 | **73.2** ± 5.2 | **69.2** ± 2.7 |
| | | | 35 | 5 | **77.7** ± 7.8 | **79.6** ± 5.9 |
| | | | 25 | 5 | **62.1** ± 6.1 | **95.3** ± 3.7 |
| | | | 10 | 5 | **25.3** ± 3.6 | **96.3** ± 2.3 |

The above results show that His-CM18-PTD4 is able to increase GFP-NLS transduction efficiency in HeLa cells in a dose-dependent manner.

### 9.4 GFP-NLS transduction by His-CM18-PTD4 in HeLa cells using Protocol B: Visualization by microscopy

GFP-NLS recombinant protein (5 µM; see **Example 5.1**) was co-incubated with 35 µM of His-CM18-PTD4, and then exposed to HeLa cells for 10 seconds using Protocol B as described in **Example 9.1.** The cells were then subjected to fluorescence microscopy analysis as described in **Examples 3.2 and 3.2a.**

For the sample results shown in **Figures 23** **and** **24****,** GFP fluorescence of the HeLa cells was immediately visualized by bright field and fluorescence microscopy at 4x, 20x and 40x magnifications after the final washing step.

In **Figure 23****,** the upper panels in A, B and C show nuclei labelling (DAPI) at 4x, 20x and 40x magnifications, respectively, while the lower panels show corresponding GFP-NLS fluorescence. In panel C, white triangle windows indicate examples of areas of co-labelling between nuclei (DAPI) and GFP-NLS signals. In panel D, the upper and bottom panels show sample bright field images of the HeLa cells, and the middle panel shows the results of a corresponding FACS analysis (performed as described in **Example 3.3**), which indicates the percentage of cells in a 96-plate with a GFP signal. No significant GFP fluorescence was observed in negative control samples (i.e., cells exposed to GFP-NLS without any shuttle agent; data not shown).

**Figure 24** shows bright field (panel A) and fluorescent images (panel B). The inset in panel B shows the results of a corresponding FACS analysis (performed as described in **Example 3.3**), which indicates the percentage of cells in a 96-plate well with a GFP signal. No significant GFP fluorescence was observed in negative control samples (i.e., cells exposed to GFP-NLS without any shuttle agent; data not shown).

For the sample results shown in **Figure 25****,** the HeLa cells were fixed, permeabilized and subjected to immuno-labelling as described in **Example 3.2a** before visualization by fluorescence microscopy as described in **Example 3.2.** GFP-NLS was labelled using a primary mouse monoclonal anti-GFP antibody (Feldan, #A017) and a secondary goat anti-mouse Alexa™-594 antibody (Abcam #150116). The upper panels in **Figure 25** show nuclei labelling (DAPI), and the lower panels show corresponding labelling for GFP-NLS. Panels A and B show sample images at 20x and 40x magnifications, respectively. White triangle windows indicate examples of areas of co-labelling between nuclei and GFP-NLS. No significant GFP-NLS labelling was observed in negative control samples (i.e., cells exposed to GFP-NLS without any shuttle agent; data not shown).

**Figure 26** shows sample images captured with confocal microscopy at 63x magnification of living cells. Panel A of **Figure 26** shows a bright field image, while panel B shows the corresponding fluorescent GFP-NLS. Panel C is an overlay between the images in panels A and B. No significant GFP-NLS fluorescence was observed in negative control samples (i.e., cells exposed to GFP-NLS without any shuttle agent; data not shown).

### 9.4a FTIC-labeled anti-tubulin antibody transduction by His-CM18-PTD4 in HeLa cells using Protocol B: Visualization by microscopy

FITC-labeled anti-tubulin antibody (0.5 µM; Abcam, ab64503) was co-incubated with 50 µM of His-CM18-PTD4, and then exposed to HeLa cells for 10 seconds using Protocol B as described in **Example 9.1.** The cells were then subjected to fluorescence microscopy analysis as described in **Examples 3.2 and 3.2a,** wherein the FITC fluorescence of the anti-tubulin antibody in the HeLa cells was immediately visualized by bright field and fluorescence microscopy at 20x magnification after the final washing step. Sample results are shown in **Figure 24C and 24D****.** No significant FITC fluorescence was observed in negative control samples (i.e., cells exposed to the FITC-labeled anti-tubulin antibody without any shuttle agent; data not shown).

Overall, the results in **Examples 9.4 and 9.4a** show that GFP-NLS and FITC-labeled anti-tubulin antibody cargos are successfully transduced and delivered to the nucleus and/or the cytosol of HeLa cells in the presence of the shuttle agent His-CM18-PTD4.

### 9.5 GFP-NLS kinetic transduction by His-CM18-PTD4 in HeLa cells: Visualization by microscopy

GFP-NLS recombinant protein (5 µM; see **Example 5.1**) was co-incubated with 50 µM of His-CM18-PTD4, and then exposed to HeLa cells for 10 seconds using Protocol B as described in **Example 9.1.** After a washing step, the GFP fluorescence of the HeLa cells was immediately visualized by fluorescence microscopy **(Example 3.2)** at 20x magnification after different intervals of time. Typical results are shown in **Figure 27****,** in which fluorescence microscopy images were captured after 45, 75, 100, and 120 seconds (see panels A, B, C and D, respectively).

As shown in **Figure 27A****,** diffuse cellular GFP fluorescence was generally observed after 45 seconds, with areas of lower GFP fluorescence in the nucleus in many cells. These results suggest predominantly cytoplasmic and low nuclear distribution of the GPF-NLS delivered intracellularly via the shuttle agent after 45 seconds. **Figures 27B-****27D** show the gradual redistribution of GFP fluorescence to the cell nuclei at 75 seconds (panel B), 100 seconds (panel C), and 120 seconds (panel D) following exposure to the His-CM18-PTD4 shuttle agent and GFP-NLS cargo. No significant cellular GFP fluorescence was observed in negative control samples (i.e., cells exposed to GFP-NLS without any shuttle agent; data not shown).

The results in **Example 9.5** show that GFP-NLS is successfully delivered to the nucleus of HeLa cells in the presence of the shuttle agent His-CM18-PTD4 by 2 minutes.

### 9.6 GFP-NLS and mCherry™-NLS co-transduction by His-CM18-PTD4 in HeLa cells: Visualization by microscopy

mCherry™-NLS recombinant protein was constructed, expressed and purified from a bacterial expression system as described in **Example 1.4.** The sequence of the mCherry™-NLS recombinant protein was: 
MHHHHHHGGGGSGGGGSGGASTGIRMVSKCEEDNMAIIKEFMRFKVHMEGSVNGHEFEIEGEG
EGRPYEGTQTAKLKVTKGGPLPFAWDILSPQFMYGSKAYVKHPADIPDYLKLSFPEGFKWERV
MNFEDGGVVTVTQDSSLQDGEFIYKVKLRGTNFPSDGQVMQKKTMGWEASSERMYPEDGALKG
EIKQRLKLKDGGHYDAEVKTTYKAKKPVQLPGAYNVNIKLDITSHNEDYTIVEQYERAEGRHS
TGGMDELYKGGSGGGSGGGSGWIRASSGGRSSDDEATADSQHAAPPKKKRKVGGSGGGSGGGS
GGGRGTEIS [SEQ ID NO: 73]
 (MW = 34.71 kDa; pl = 6.68)
NLS sequence is underlined
Serine/glycine rich linkers are in bold

GFP-NLS recombinant protein (5 µM; see **Example 5.1**) and mCherry™-NLS recombinant protein (5 µM) were co-incubated together with 35 µM of His-CM18-PTD4, and then exposed to HeLa cells for 10 seconds using Protocol B as described in **Example 9.1.** After washing steps, the cells were immediately visualized by bright field and fluorescence microscopy at 20x magnifications as described in **Example 3.2.** Sample results are shown in **Figure 28****,** in which corresponding images showing bright field (panel A), DAPI fluorescence (panel B), GFP-NLS fluorescence (panel C), and mCherry™-NLS fluorescence (panel D) are shown. White triangle windows indicate examples of areas of co-labelling between GFP-NLS and mCherry™ fluorescence signals in cell nuclei. No significant cellular GFP or mCherry™ fluorescence was observed in negative control samples (i.e., cells exposed to GFP-NLS or mCherry™ without any shuttle agent; data not shown).

These results show that GFP-NLS and mCherry™-NLS are successfully delivered together to the nucleus in HeLa cells in the presence of the shuttle agent His-CM18-PTD4.

### 9.7 GFP-NLS transduction by His-CM18-PTD4 in THP-1 suspension cells: Flow cytometry

The ability of the His-CM18-PTD4 to deliver GFP-NLS in the nuclei of suspension cells was tested using THP-1 cells. THP-1 cells were cultured and tested in the protein transduction assays using Protocols A and C as described in **Example 9.1.** GFP-NLS (5 µM; see **Example 5.1**) was co-incubated with 1 µM of His-CM18-PTD4 and exposed to THP-1 cells for 1 hour (Protocol A), or was co-incubated with 5 µM of His-CM18-PTD4 and exposed to THP-1 cells for 15 seconds (Protocol C). The cells were subjected to flow cytometry analysis as described in **Example 3.3.** Results are shown in **Table 9.3** and in **Figure 31****.**

**Table 9.3: Data from Figure 31**

| **Protocol** | **Shuttle** | **Cells** | **Conc. of shuttle (µM)** | **Conc. of GFP-NLS** | **Mean % cells with GFP signal (± St. Dev.; n=3)** | **Cell viability (%) (± St. Dev.; n=3)** |
|---|---|---|---|---|---|---|
| **C** | **No shuttle ("Ctrl")** | THP-1 | 0 | 5 | **0.2** ± 0.03 | **99.1** ± 0.7 |
| **A** | **His-CM18-PTD4** | | 1 | 5 | **14.2** ± 2.2 | **96.9** ± 3.6 |
| **C** | **His-CM18-PTD4** | | 0.5 | 5 | **34.9** ± 3.8 | **82.1** ± 2.7 |
| | | | 5 | 5 | **64.1** ± 1.6 | **64.0** ± 4.1 |

### 9.8 GFP-NLS transduction by His-CM18-PTD4 in THP-1 cells: Visualization by microscopy

GFP-NLS recombinant protein (5 µM; see **Example 5.1**) was co-incubated with 5 µM of His-CM18-PTD4, and then exposed to THP-1 cells for 15 seconds using Protocol C as described in **Example 9.1.** The cells were subjected to microscopy visualization as described in **Example 3.2.**

For the sample results shown in **Figure 32****,** GFP fluorescence of the HeLa cells was immediately visualized by bright field (upper panels) and fluorescence (lower panels) microscopy at 4x, 10x and 40x magnifications (panels A-C, respectively) after the final washing step. White triangle windows in panel C indicate examples of areas of co-labelling between brightfield and fluorescence images. Panel D shows typical results of a corresponding FACS analysis (performed as described in **Example 3.3**), which indicates the percentage of cells in a 96-plate well with a GFP signal. Additional results are shown in **Figure 33****,** in which panels A and B show bright field images, and panels C and D show corresponding fluorescence images. White triangle windows indicate examples of areas of co-labelling between panels A and C, as well as panels B and D. The right-most panel shows typical results of a corresponding FACS analysis (performed as described in **Example 3.3**), which indicates the percentage of cells in a 96-plate well with a GFP signal.

No significant cellular GFP fluorescence was observed in negative control samples (i.e., cells exposed to GFP-NLS without any shuttle agent; data not shown).

The results in this example show that GFP-NLS is successfully delivered intracellularly in THP-1 cells in the presence of the shuttle agent His-CM18-PTD4.

### Example 10:

### Different multi-domain shuttle agents, but not single-domain peptides, successfully transduce GFP-NLS in HeLa and THP-1 cells

### 10.1 GFP-NLS transduction by different shuttle agents in HeLa cells: Flow cytometry

HeLa cells were cultured and tested in the protein transduction assays using Protocol B as described in **Example 9.1.** Briefly, GFP-NLS recombinant protein (5 µM; see **Example 5.1**) was co-incubated with 50 µM of different shuttle agents and exposed to the HeLa cells for 10 seconds. The cells were subjected to flow cytometry analysis as described in **Example 3.3.** Results are shown in **Table 10.1** and **Figure 29A****.** "Pos cells (%)" is the mean percentages of all cells that emanate a GFP signal. The negative control ("Ctrl") corresponds to cells that were incubated with GFP-NLS recombinant protein (5 µM) without any shuttle agent.

**Table 10.1: Data from Figure 29A**

| **Protocol** | **Shuttle agent** | **Cells** | **Conc. of shuttle (µM)** | **Conc. of GFP-NLS (µM)** | **Mean % cells with GFP signal (± St. Dev.; n=3)** | **Cell viability (%) (± St. Dev.; n=3)** |
|---|---|---|---|---|---|---|
| **B** | **No shuttle "ctrl"** | HeLa | 0 | 5 | **0** | **100** |
| | **His-CM18-TAT** | HeLa | 50 | | **55.5** ± 3.6 | **35.2** ± 5.7 |
| | **His-CM18-Transportan (TPT)** | HeLa | | | **33.2** ± 2.8 | **41.3** ± 3.3 |
| | **TAT-KALA** | HeLa | | | **56.3** ± 3.6 | **95.6** ± 4.3 |
| | **His-CM18-PTD4** | HeLa | | | **68** ± 2.2 | **92** ± 3.6 |
| | **His-CM18-9Arg** | HeLa | | | **57.2** ± 3.9 | **45.8** ± 5.4 |
| | **TAT-CM18** | HeLa | | | **39.4** ± 3.9 | **23.5** ± 1.1 |
| | **His-C(LLKK)₃C-PTD4** | HeLa | | | **76** ± 3.8 | **95** ± 2.7 |
| | **His-LAH4-PTD4** | HeLa | | | **63** ± 1.64 | **98** ± 1.5 |
| | **PTD4-KALA** | HeLa | | | **73.4** ± 4.12 | **91.4 ±** 3.67 |

### 10.2 GFP-NLS transduction by different shuttle agents with varying incubation times in HeLa cells: Flow cytometry

HeLa cells were cultured and tested in the protein transduction assays using Protocol B as described in **Example 9.1.** Briefly, GFP-NLS recombinant protein (5 µM; see **Example 5.1**) was co-incubated with 10 µM of TAT-KALA, His-CM18-PTD4, or His-C(LLKK)3C-PTD4 for 1, 2, or 5 minutes. After the final washing step, the cells were subjected to flow cytometry analysis as described in **Example 3.3.** Results are shown in **Table 10.2** and **Figure 29B****.** "Pos cells (%)" is the mean percentages of all cells that emanate a GFP signal. The negative control ("Ctrl") corresponds to cells that were incubated with GFP-NLS recombinant protein (5 µM) without any shuttle agent.

**Table 10.2: Data from Figure 29B**

| **Protocol** | **Shuttle agent** | **Cells** | **Conc. of shuttle (µM)** | **Incubation time** | **Mean % cells with GFP signal (± St. Dev.; n=3)** | **Cell viability (%) (± St. Dev.; n=3)** |
|---|---|---|---|---|---|---|
| - | **No shuttle ("Ctrl")** | HeLa | 0 | 5 min. | **0** ± n/a | **97.5** ± 1.7 |
| B | **TAT-KALA** | HeLa | 10 | 1 min. | **83.7** ± 3.5 | **93.5** ± 2.7 |
| | | | | 2 min. | **86.2** ± 4.3 | **92.1** ± 3.1 |
| | | | | 5 min. | **68.1** ± 3.0 | **86** ± 4.4 |
| | **His-CM18-PTD4** | HeLa | 10 | 1 min. | **50.6** ± 3.5 | **97.6** ± 2.7 |
| | | | | 2 min. | **74 ±** 3.3 | **80.9** ± 3.2 |
| | | | | 5 min. | **82.7** ± 5.0 | **66.2** ± 4.4 |
| | **His-C(LLKK)₃C-PTD4** | HeLa | 10 | 1 min. | **51.1** ± 3.5 | **99.5** ± 2.7 |
| | | | | 2 min. | **77.8** ± 4.3 | **94.3** ± 3.2 |
| | | | | 5 min. | **86.4** ± 4.0 | **80.8** ± 4.4 |

### 10.3 GFP-NLS transduction by TAT-KALA, His-CM18-PTD4 and His-C(LLKK)₃C-PTD4 with varying incubation times in HeLa cells: Flow cytometry

HeLa cells were cultured and tested in the protein transduction assays using Protocol C as described in **Example 9.1.** Briefly, GFP-NLS recombinant protein (5 µM; see **Example 5.1**) was co-incubated with 5 µM of TAT-KALA, His-CM18-PTD4, or His-C(LLKK)3C-PTD4 for 1, 2, or 5 minutes. After the final washing step, the cells were subjected to flow cytometry analysis as described in **Example 3.3.** Results are shown in **Table 10.3** and **Figure 29C****.** The negative control ("Ctrl") corresponds to cells that were incubated with GFP-NLS recombinant protein (5 µM) without any shuttle agent.

**Table 10.3: Data from Figure 29C**

| **Protocol** | **Shuttle agent** | **Cells** | **Conc. of shuttle (µM)** | **Incubation time** | **Relative fluorescence intensity (FL1-A) (n=3)** | **St. Dev.** |
|---|---|---|---|---|---|---|
| | **No shuttle ("Ctrl")** | | 0 | 5 min. | 8903 | 501 |
| **C** | **TAT-KALA** | HeLa | 10 | 1 min. | 216 367 | 13 863.48 |
| | | | | 2 min. | 506 158 | 14 536.28 |
| | | | | 5 min. | 78 010 | 2 463.96 |
| | **His-CM18-PTD4** | HeLa | 10 | 1 min. | 524151 | 12 366.48 |
| | | | | 2 min. | 755 624 | 26 933.16 |
| | | | | 5 min. | 173 930 | 15 567.33 |
| | **His-C(LLKK)₃C-PTD4** | HeLa | 10 | 1 min. | 208 968 | 23 669.19 |
| | | | | 2 min. | 262 411.5 | 19 836.84 |
| | | | | 5 min. | 129 890 | 16 693.29 |

### 10.4 GFP-NLS transduction by different shuttle agents in HeLa cells: Flow cytometry

HeLa cells were cultured and tested in the protein transduction assays using Protocol B as described in **Example 9.1.** Briefly, GFP-NLS recombinant protein (5 µM; see **Example 5.1**) was co-incubated with 50 µM of different shuttle agents (see **Table 1.3** for amino acid sequences and properties) and exposed to the HeLa cells for 10 seconds. The cells were subjected to flow cytometry analysis as described in **Example 3.3.** Results are shown in **Tables 10.3a & 10.3b** and **Figure 29E & 29F****.** "Pos cells (%)" is the mean percentages of all cells that emanate a GFP signal. The negative control ("Ctrl") corresponds to cells that were incubated with GFP-NLS recombinant protein (5 µM) without any shuttle agent.

**Table 10.3a: Data from Figure 29E**

| **Domain structure** | **Shuttle agent** | **Conc. of shuttle (µM)** | **Conc. of GFP-NLS (µM)** | **Mean % cells with GFP signal (± St. Dev.; n=3)** | **Cell viability (%) (± St. Dev.; n=3)** |
|---|---|---|---|---|---|
| - | **No shuttle ("Ctrl")** | 0 | 5 | **0** | **100** |
| **ELD-CPD** | **VSVG-PTD4** | 50 | 5 | **3.5** ± 1.1 | **100** |
| | **EB1-PTD4** | | | **75.8** ± 8.26 | **39** ± 5.6 |
| | **JST-PTD4** | | | **0.84** ± 0.69 | **98.9** ± 0.57 |
| **His-ELD-CPD** | **His-C(LLKK)₃C-PTD4** | 50 | 5 | **76** ± 3.8 | **95** ± 2.7 |
| | **His-LAH4-PTD4** | | | **63 ±** 1.64 | **98** ± 1.5 |
| | **His-CM18-PTD4** | | | **68** ± 2.2 | **92** ± 3.6 |
| | **His-CM18-TAT** | | | **55.5** ± 3.6 | **35.2** ± 5.7 |
| | **His-CM18-TAT-Cys*** | | | **49.3** ± 4.1 | **41.4** ± 3.91 |
| | **His-CM18-9Arg** | | | **57.2** ± 3.93 | **45.8** ± 3.53 |
| | **His-CM18-Transportan (TPT)** | | | **33.2** ± 2.82 | **41.3** ± 3.29 |

| | | | | | |
|---|---|---|---|---|---|
| Not shown in Figure 29E | | | | | |

**Table 10.3b: Data from Figure 29F**

| **Domain structure** | **Shuttle agent** | **Conc. of shuttle (µM)** | **Conc. of GFP-NLS (µM)** | **Mean % cells with GFP signal (± St. Dev.; n=3)** | **Cell viability (%) (± St. Dev.; n=3)** |
|---|---|---|---|---|---|
| - | **No shuttle ("Ctrl")** | 0 | 5 | 0 | 100 |
| **CPD-ELD** | **TAT-CM18** | 50 | 5 | **39.4** ± 3.9 | **23.5** ± 1.1 |
| | **TAT-KALA** | | | **56.3** ± 3.6 | **95.6** ± 4.3 |
| | **PTD4-KALA** | | | **73.4** ± 4.12 | **91.4** ± 3.67 |
| | **9Arg-KALA** | | | **7.8** ± 1.53 | **62.8** ± 5.11 |
| | **Pep1-KALA** | | | **17.2** ± 3.07 | **94.7** ± 3.77 |
| | **Xentry-KALA** | | | **19.4** ± 1.01 | **98.3** ± 0.64 |
| | **SynB3-KALA** | | | **14.3** ± 2.37 | **91.1** ± 0.82 |

HeLa cells were cultured and tested in the protein transduction assays using Protocol B as described in **Example 9.1.** Briefly, GFP-NLS recombinant protein (5 µM; see **Example 5.1**) was co-incubated with 10 µM of TAT-KALA, His-CM18-PTD4, or His-C(LLKK)3C-PTD4 for 1, 2, or 5 minutes. After the final washing step, the cells were subjected to flow cytometry analysis as described in **Example 3.3.** Results are shown in **Tables 10.3c & 10.3b** and **Figure 29G and 29H****.** "Pos cells (%)" is the mean percentages of all cells that emanate a GFP signal. The negative control ("Ctrl") corresponds to cells that were incubated with GFP-NLS recombinant protein (5 µM) without any shuttle agent.

**Table 10.3c: Data from Figure 29G**

| **Domain structure** | **Shuttle agent** | **Conc. of shuttle (µM)** | **Conc. of GFP-NLS (µM)** | **Incubation time (min)** | **Mean % cells with GFP signal (± St. Dev.; n=3)** | **Cell viability (%) (± St. Dev.; n=3)** |
|---|---|---|---|---|---|---|
| - | **No shuttle ("Ctrl")** | 0 | 5 | 5 | 0 ± n/a | **98.3** ± 0.9 |
| **CPD-ELD** | **PTD4-KALA** | | | 1 | **64.6** ± 4.3 | **96.2** ± 3.0 |
| | | 10 | 5 | 2 | **78.8** ± 3.6 | **75.3** ± 3.8 |
| | | | | 5 | **71.4** ± 4.2 | **82.4** ± 4.7 |
| **ELD-CPD** | **EB1-PTD4** | | | 1 | **76.3** ± 3.5 | **61.7** ± 2.7 |
| | | 10 | 5 | 2 | **79.0** ± 3.3 | **56.6** ± 3.2 |
| | | | | 5 | **71.1** ± 5.0 | **55.8** ± 4.4 |
| **His-ELD-CPD-His** | **His-CM18-PTD4-His** | | | 1 | **68.6** ± 3.5 | **68.1** ± 2.7 |
| | | 10 | 5 | 2 | **74.1** ± 4.3 | **61.6** ± 3.2 |
| | | | | 5 | **59.8** ± 4.0 | **41.2** ± 4.4 |

**Table 10.3d: Data from Figure 29H**

| **Domain structure** | **Shuttle agent** | **Conc. of shuttle (µM)** | **Conc. of GFP-NLS (µM)** | **Incubation time (min)** | **Relative Fluorescence Intensity (FL1-A) (± St. Dev.; n=3)** |
|---|---|---|---|---|---|
| - | **No shuttle ("Ctrl")** | 0 | 5 | 5 | **8903** ± 501.37 |
| **CPD-ELD** | **PTD4-KALA** | 10 | 5 | 1 | **190 287** ± 9445 |
| | | | | 2 | **386 480** ± 17 229 |
| | | | | 5 | **241 230** ± 14 229 |
| **ELD-CPD** | **EB1-PTD4** | 10 | 5 | 1 | **178 000** ± 11 934 |
| | | | | 2 | **277 476** ± 25 319 |
| | | | | 5 | **376 555** ± 16 075 |
| **His-ELD-CPD-His** | **His-CM18-PTD4-His** | 10 | 5 | 1 | **204 338** ± 22 673 |
| | | | | 2 | **307 329** ± 19 618 |
| | | | | 5 | **619 964** ± 17 411 |

The shuttle agent CM18-PTD4 was used as a model to demonstrate the modular nature of the individual protein domains, as well as their ability to be modified. More particularly, the presence or absence of: an N-terminal cysteine residue ("Cys"); different flexible linkers between the ELD and CPD domains ("L1": GGS; "L2": GGSGGGS; and "L3": GGSGGGSGGGS) and different lengths, positions, and variants to histidine-rich domains; were studied.

HeLa cells were cultured and tested in the protein transduction assays using Protocol B as described in **Example 9.1.** Briefly, GFP-NLS recombinant protein (5 µM; see **Example 5.1)** was co-incubated with 20 µM of different shuttle peptide variants (see **Table 1.3** for amino acid sequences and properties) of the shuttle agent His-CM18-PTD4 for 1 minute. After the final washing step, the cells were subjected to flow cytometry analysis as described in **Example 3.3.** Results are shown in **Table 10.3e** and **Figure 29I****.** "Pos cells (%)" is the mean percentages of all cells that emanate a GFP signal. The negative control ("Ctrl") corresponds to cells that were incubated with GFP-NLS recombinant protein (5 µM) without any shuttle agent.

**Table 10.3e: Data from Figure 29I**

| **structure** | **Shuttle agent** | **Conc. of shuttle (µm)** | **Conc. of GFP-NLS (µm)** | **Mean % cells with GFP signal (± St. Dev.; n=3)** | **Cell viability (%) (± St. Dev.; n=3)** |
|---|---|---|---|---|---|
| - | **No shuttle ("Ctrl")** | 0 | 5 | 0 | **99.6** ±0.12 |
| **ELD-CPD** | **CM18-PTD4** | 20 | 5 | **47.6** ± 2.6 | **33.9** ± 3.7 |
| | **Cys-CM18-PTD4** | | | **36.6** ± 2.3 | **78.7** ± 3.1 |
| | **CM18-L1-PTD4** | | | **48.5** ± 3.0 | **50.1** ± 3.8 |
| | **CM18-L2-PTD4** | | | **45.5** ± 6.5 | **64.0** ± 1.3 |
| | **CM18-L3-PTD4** | | | **39.0** ± 2.7 | **71.9 ±** 6.0 |
| **His-ELD-CPD** | **His-CM18-PTD4** | 20 | 5 | **60.3** ± 3.2 | **81.6 ±** 4.5 |
| | **His-CM 18-PTD4-6Cys** | | | **41.3 ±** 4.28 | **62** ± 5.76 |
| | **Met-His-CM18-PTD4-Cys** | | | **45.6** ± 3.88 | **54.9** ± 3.45 |
| | **3His-CM18-PTD4** | | | **39.4** ± 0.5 | **39.2** ± 3.3 |
| | **12His-CM18-PTD4** | | | **36.9** ± 4.3 | **33.4** ± 4.3 |
| | **HA-CM18-PTD4** | | | **42.3** ± 4.2 | **68.3** ± 4.1 |
| | **3HA-CM18-PTD4** | | | **37.2** ± 3.9 | **43.6** ± 2.8 |
| **ELD-His-CPD** | **CM18-His-PTD4** | 20 | 5 | **61.7** ± 1.8 | **57.7** ± 4.2 |
| **His-ELD-CPD-His** | **His-CM18-PTD4-His** | 20 | 5 | **68.0** ± 6.0 | **78.6** ± 1.1 |

These results show that variations in a given shuttle (e.g., CM18-PTD4) may be used to modulate the degree of transduction efficiency and cell viability of the given shuttle. More particularly, the addition of an N-terminal cysteine residue to CM18-PTD4 (see Cys-CM18-PTD4), decreased GFP-NLS transduction efficiency by 11% (from 47.6% to 36.6%), but increased cell viability from 33.9% to 78.7%. Introduction of flexible linker domains (L1, L2, and L3) of different lengths between the CM18 and PTD4 domains did not result in a dramatic loss of transduction efficiency, but increased cell viability (see CM18-L1-PTD4, CM18-L2-PTD4, and CM18-L3-PTD4). Finally, variations to the amino acid sequences and/or positions of the histidine-rich domain(s) did not result in a complete loss of transduction efficiency and cell viability of His-CM18-PTD4 (see 3His-CM18-PTD4, 12His-CM18-PTD4, HA-CM18-PTD4, 3HA-CM18-PTD4, CM18-His-PTD4, and His-CM18-PTD4-His). Of note, adding a second histidine-rich domain at the C terminus of His-CM18-PTD4 (i.e., His-CM18-PTD4-His) increased transduction efficiency from 60% to 68% with similar cell viability.

### 10.5 Lack of GFP-NLS transduction by single-domain peptides or a His-CPD peptide in HeLa cells: Flow cytometry

HeLa cells were cultured and tested in the protein transduction assays using Protocol B as described in **Example 9.1.** Briefly, GFP-NLS recombinant protein (5 µM; see **Example 5.1)** was co-incubated with 50 µM of different single-domain peptides (TAT; PTD4; Penetratin; CM18; C(LLKK)₃C; KALA) or the two-domain peptide His-PTD4 (lacking an ELD), and exposed to the HeLa cells for 10 seconds. After the final washing step, the cells were subjected to flow cytometry analysis as described in **Example 3.3.** Results are shown in **Table 10.4** and **Figure 29D****.** "Pos cells (%)" is the mean percentages of all cells that emanate a GFP signal. The negative control ("Ctrl") corresponds to cells that were incubated with GFP-NLS recombinant protein (5 µM) without any single-domain peptide or shuttle agent.

**Table 10.4: Data from Figure 29D**

| **Protocol** | **Domain** | **Single-domain peptide** | **Cells** | **Conc. of shuttle (µM)** | **Conc. of GFP-NLS (µM)** | **Mean % cells with GFP signal (± St. Dev.; n=3)** | **Cell viability (%) (± St. Dev.; n=3)** |
|---|---|---|---|---|---|---|---|
| **B** | - | **No peptide ("Ctrl")** | HeLa | 0 | 5 | **0.1** ± 0.02 | **98.3** ± 0.59 |
| | **CPD** | **TAT** | HeLa | 50 | 5 | **1.1** ± 0.27 | **94.6** ± 0.44 |
| | | **PTD4** | | | | **1.1** ± 0.06 | **94** ± 4.5 |
| | | **Penetratin (Pen)** | | | | **3.6** ± 0.1 | **96** ± 0.6 |
| | **ELD** | **CM18** | HeLa | 50 | 5 | **2.9** ± 0.2 | **95** ± 1.2 |
| | | **C(LLKK)₃C** | | | | **1.1** ± 0.57 | **61.8** ± 0.1 |
| | | **KALA** | | | | **1.4** ± 0.13 | **84** ± 0.7 |
| | **His-CPD** | **His-PTD4** | HeLa | 50 | 5 | **1.04** ± 0.12 | **96.5** ± 0.28 |

These results show that the single-domain peptides TAT, PTD4, Penetratin, CM18, C(LLKK)₃C, KALA, or the two-domain peptide His-PTD4 (lacking an ELD), are not able to successfully transduce GFP-NLS in HeLa cells.

### 10.6 GFP-NLS transduction by TAT-KALA, His-CM18-PTD4, His-C(LLKK)₃C-PTD4, PTD4-KALA, EB1-PTD4, and His-CM18-PTD4-His in HeLa cells: Visualization by microscopy

GFP-NLS recombinant protein (5 µM; see **Example 5.1)** was co-incubated with 50 µM of shuttle agent, and then exposed to HeLa cells for 10 seconds using Protocol B as described in **Example 9.1.** The cells were visualized by microscopy as described in **Example 3.2,** after an incubation time of 2 minutes.

For the sample results shown in **Figure 30****,** GFP fluorescence of the HeLa cells was immediately visualized by bright field (bottom row panels) and fluorescence (upper and middle row panels) microscopy at 20x or 40x magnifications after the final washing step. The results with the shuttle agents TAT-KALA, His-CM18-PTD4, and His-C(LLKK)₃C-PTD4 are shown in panels A, B and C, respectively. The results with the shuttle agents PTD4-KALA, EB1-PTD4, and His-CM18-PTD4-His are shown in panels D, E and F, respectively. The insets in the bottom row panels show the results of corresponding FACS analyses (performed as described in **Example 3.3),** which indicates the percentage of cells in a 96-plate well with a GFP signal. No significant cellular GFP fluorescence was observed in negative control samples (i.e., cells exposed to GFP-NLS without any shuttle agent; data not shown).

### 10.7 GFP-NLS transduction by TAT-KALA, His-CM18-PTD4 and His-C(LLKK)₃C-PTD4 with varying incubation times in THP-1 cells: Flow cytometry

THP-1 cells were cultured and tested in the protein transduction assays using Protocol C as described in **Example 9.1.** Briefly, GFP-NLS recombinant protein (5 µM; see **Example 5.1)** was co-incubated with 1 µM of TAT-KALA, His-CM18-PTD4, or His-C(LLKK)3C-PTD4 for 15, 30, 60, or 120 seconds. After the final washing step, the cells were subjected to flow cytometry analysis as described in **Example 3.3.** The mean percentages of cells emanating a GFP signal ("Pos cells (%)") are shown in **Table 10.4a** and in **Figure 34A****.** The mean fluorescence intensity is shown in **Table 10.5** and **Figure 34B****.** The negative control ("Ctrl") corresponds to cells that were incubated with GFP-NLS recombinant protein (5 µM) without any shuttle agent.

**Table 10.4a: Data from Figure 34A**

| **Protocol** | **Shuttle agent** | **Cells** | **Conc. of shuttle (µM)** | **(µM)** | **Incubation time (sec.)** | **Mean % cells with GFP signal (± St. Dev.; n=3)** | **Cell viability (%) (± St. Dev.; n=3)** |
|---|---|---|---|---|---|---|---|
| **C** | **No shuttle ("Ctrl")** | THP-1 | 0 | 5 | 120 | **1.12** ± 0.27 | **97.3** ± 1.55 |
| | **TAT-KALA** | THP-1 | 1 | 5 | 15 | **47** ± 3.5 | **84.6** ± 2.7 |
| | | | | | 30 | **52.9** ± 1.3 | **70.3** ± 3.2 |
| | | | | | 60 | **70.1** ± 2.0 | **82.7** ± 1.4 |
| | | | | | 120 | **82.1** ± 2.5 | **46.3** ± 4.9 |
| | **His-CM18-PTD4** | THP-1 | 1 | 5 | 15 | **23.7** ± 0.2 | **90** ± 3.0 |
| | | | | | 30 | **53** ± 0.3 | **89** ± 1.1 |
| | | | | | 60 | **69.6** ± 4.2 | **85.3** ± 3.6 |
| | | | | | 120 | **89** ± 0.8 | **74.3** ± 3.2 |
| | **His-C(LLKK)₃C-PTD4** | THP-1 | 1 | 5 | 15 | **38.4** ± 0.3 | **85.2** ± 2.8 |
| | | | | | 30 | **42.3** ± 4.2 | **86** ± 2.0 |
| | | | | | 60 | **64.5** ± 1.0 | **86.9** ± 3.8 |
| | | | | | 120 | **78.7** ± 0.3 | **79.6** ± 2.8 |

**Table 10.5: Data from Figure 34B**

| **Protocol** | **Shuttle agent** | **Cells** | **Conc. of shuttle (µM)** | **Incubation time (sec.)** | **Relative fluorescence intensity (FL1-A) (n = 3)** | **Standard Deviation** |
|---|---|---|---|---|---|---|
| **C** | **No shuttle ("Ctrl")** | THP-1 | 0 | 120 | 217 | 23.09 |
| | **TAT-KALA** | THP-1 | 1 | 15 | 6 455.12 | 333.48 |
| | | | | 30 | 8 106.81 | 436.28 |
| | | | | 60 | 13 286.2 | 463.96 |
| | | | | 120 | 27 464.92 | 2 366.48 |
| | **His-CM18-PTD4** | THP-1 | 1 | 15 | 5 605.45 | 933.16 |
| | | | | 30 | 25 076.41 | 5 567.33 |
| | | | | 60 | 34 046.94 | 3 669.19 |
| | | | | 120 | 55 613.48 | 9 836.84 |
| | **His-C(LLKK)₃C-PTD4** | THP-1 | 1 | 15 | 5 475.12 | 693.29 |
| | | | | 30 | 5 755.8 | 635.18 |
| | | | | 60 | 8 267.38 | 733.29 |
| | | | | 120 | 21 165.06 | 209.37 |

### Example 11:

### Repeated daily treatments with low concentrations of shuttle agent in the presence of serum results in GFP-NLS transduction in THP-1 cells

### 11.1 GFP-NLS transduction with His-CM18-PTD4 or His-C(LLKK)3C-PTD4 in THP-1 cells: Flow cytometry

THP-1 cells were cultured and tested in the protein transduction assay using Protocol A as described in **Example 9.1,** but with the following modifications. GFP-NLS recombinant protein (5, 2.5, or 1 µM; see **Example 5.1)** was co-incubated with 0.5 or 0.8 µM of His-CM18-PTD4, or with 0.8 µM of His-C(LLKK)₃C-PTD4, and then exposed to THP-1 cells each day for 150 min in the presence of cell culture medium containing serum. Cells were washed and subjected to flow cytometry analysis as described in **Example 3.3** after 1 or 3 days of repeated exposure to the shuttle agent/cargo. The results are shown in **Table 11.1** and in **Figure 35A****, B, C and F.** The negative control ("Ctrl") corresponds to cells that were incubated with GFP-NLS recombinant protein (5 µM) without any shuttle agent.

**Table 11.1: Data from Figure 35A, B, C and F**

| Figure | Shuttle agent | Cells | Conc. of shuttle (µM) | Conc. of GFP-NLS (µM) | Exposure to shuttle/cargo (days) | Mean % cells with GFP signal Dev.; n=3) | Cell viability (%) (± St. Dev.; n=3) |
|---|---|---|---|---|---|---|---|
| **35A** | **No shuttle (Ctrl)** | THP-1 | 0 | 5 | 0 | **0.15 ±** 0.04 | **98.7** ± 0.1 |
| | **His-CM18-PTD4** | | 0.5 | 5 | 1 | **12.1** ± 1.5 | **98.2** ± 2.4 |
| | | | | | 3 | **73.4** ± 1.1 | **84.3** ± 3.8 |
| **35B** | **No shuttle (Ctrl)** | THP-1 | 0 | 5 | 0 | **0.36** ± 0.09 | 97.1 ± 1.2 |
| | **His-CM18-PTD4** | | 0.8 | 2.5 | 1 | **12.2** ± 0.9 | **92.3** ± 1.9 |
| | | | | | 3 | **62.4** ± 3.5 | **68.5** ± 2.2 |
| **35C** | **No shuttle (Ctrl)** | THP-1 | 0 | 5 | 0 | **0.28** ± 0.05 | **96.4** ± 2.0 |
| | **His-CM18-PTD4** | | 0.8 | 1 | 1 | **1.6** ± 0.2 | **98.4** ± 6.4 |
| | | | | | 3 | **6.5** ± 0.9 | **80.6** ± 4.6 |
| **35F** | **No shuttle (Ctrl)** | THP-1 | 0 | 5 | 0 | **0.62** ± 0.11 | **96.3** ± 1.4 |
| | **His-C(LLKK)₃C-** | | 0.8 | 1 | 1 | **1.8** ± 0.2 | **97.2** ± 2.2 |
| | **PTD4** | | | | 3 | **6.6** ± 0.8 | **76.6** ± 3.4 |

The viability of THP-1 cells repeatedly exposed to His-CM18-PTD4 and GFP-NLS was determined as described in **Example 3.3a.** The results are shown in **Tables 11.2 and 11.3** and in **Figure 35D and 35E****.** The results in **Table 11.2** and **Figure 35D** show the metabolic activity index of the THP-1 cells after 1, 2, 4, and 24h, and the results in **Table 11.3** and **Figure 35E** show the metabolic activity index of the THP-1 cells after 1 to 4 days.

**Table 11.2: Data from Figure 35D**

| Shuttle agent | Cells | Conc. of shuttle (µM) | Conc. of GFP-NLS (µM) | Mean metabolic activity index (± St. Dev.; n=3) (Exposure to shuttle/cargo) | | | |
|---|---|---|---|---|---|---|---|
| | | | | 1h | 2h | 4h | 24h |
| **No shuttle (Ctrl)** | THP-1 | 0 | 5 | **40810** ± 757.39 | **38223** ± 238.66 | **44058** ± 320.23 | **42362** ± 333.80 |
| **His-CM18-PTD4** | THP-1 | 0.5 | 5 | **9974** ± 1749.85 | **9707** ± 1259.82 | **3619** ± 2247.54 | **2559** ± 528.50 |
| | | 1 | 5 | **42915** ± 259.67 | **41386** ± 670.66 | **44806** ± 824.71 | **43112** ± 634.56 |

**Table 11.3: Data from Figure 35E**

| Shuttle agent | Cells | Conc. of shuttle (µM) | Conc. of GFP-NLS (µM) | Mean metabolic activity index (± St. Dev.; n=3) (Exposure to shuttle/cargo) | | | |
|---|---|---|---|---|---|---|---|
| | | | | 1 day | 2 days | 3 days | 4 days |
| **No shuttle (Ctrl)** | THP-1 | 0 | 5 | **44684** ± 283.27 | **43389** ± 642.47 | **45312** ± 963.40 | **43697** ± 1233 |
| **His-CM18-PTD4** | THP-1 | 0.5 | 5 | **44665** ± 310.3 | **42664** ± 398.46 | **43927** ± 3511.54 | **43919** ± 4452.25 |
| | | 0.8 | 5 | **44531** ± 176.66 | **43667** ± 421.66 | **44586** ± 383.68 | **44122** ± 239.98 |
| | | 1 | 5 | **41386** ± 670.66 | **36422** ± 495.01 | **27965** ± 165.33 | **22564** ± 931.28 |

The results in **Example 11** show that repeated daily (or chronic) treatments with relatively low concentrations of His-CM18-PTD4 or His-C(LLKK)₃C-PTD4 in the presence of serum result in intracellular delivery of GFP-NLS in THP-1 cells. The results also suggest that the dosages of the shuttle agents and the cargo can be independently adjusted to improve cargo transduction efficiency and/or cell viability.

### Example 12:

### His-CM18-PTD4 increases transduction efficiency and nuclear delivery of GFP-NLS in a plurality of cell lines

### 12.1 GFP-NLS transduction with His-CM18-PTD4 in different adherent & suspension cells: Flow cytometry

The ability of the shuttle agent His-CM18-PTD4 to deliver GFP-NLS to the nuclei of different adherent and suspension cells using Protocols B (adherent cells) or C (suspension cells) as described in **Example 9.1** was examined. The cell lines tested included: HeLa, Balb3T3, HEK 293T, CHO, NIH3T3, Myoblasts, Jurkat, THP-1, CA46, and HT2 cells, which were cultured as described in **Example 1.** GFP-NLS (5 µM; see **Example 5.1)** was co-incubated with 35 µM of His-CM18-PTD4 and exposed to adherent cells for 10 seconds (Protocol B), or was co-incubated with 5 µM of His-CM18-PTD4 and exposed to suspension cells for 15 seconds (Protocol C). Cells were washed and subjected to flow cytometry analysis as described in **Example 3.3.** Results are shown in **Table 12.1** and **Figure 36****.** "Pos cells (%)" is the mean percentages of all cells that emanate a GFP signal.

**Table 12.1: Data from Figure 36**

| **Shuttle agent** | **Protocol** | **Conc. of shuttle (µM)** | **Conc. of GFP-NLS (µM)** | **Cells** | **Mean % cells with GFP signal (± St. Dev.; n=3)** | **Cell viability (%) St. Dev.; n=3)** |
|---|---|---|---|---|---|---|
| **His-CM18-PTD4** | B | 35 | 5 | HeLa | **72.3** ± 5.3 | **94.6** ± 0.4 |
| | | | | Balb3T3 | **40.2** ± 3.1 | **98.4** ± 0.6 |
| | | | | HEK 293T | **55.3** ± 0.2 | **95.3** ± 1.2 |
| | | | | CHO | **53.7** ± 4.6 | **92.8** ± 0.1 |
| | | | | NIH3T3 | **35.4** ± 3.9 | **3.3** ± 5.4 |
| | | | | Myoblasts | **25.6** ± 2.6 | **23.5** ± 1.1 |
| | C | 5 | 5 | Ju rkat | **30.7** ± 2.2 | **73.6** ± 0.7 |
| | | | | THP-1 | **64.1** ± 1.6 | **64.1** ± 4.5 |
| | | | | CA46 | **24.4** ± 0.6 | **71.6** ± 1.0 |
| | | | | HT2 | **30.5** ± 2.5 | **90.6** ± 1.5 |

### 12.2 GFP-NLS transduction with His-CM18-PTD4 in several adherent and suspension cells: visualization by microscopy

GFP-NLS recombinant protein (5 µM; see **Example 5.1)** was co-incubated with 35 µM of His-CM18-PTD4 and exposed to adherent cells for 10 seconds using Protocol A, or was co-incubated with 5 µM of His-CM18-PTD4 and exposed to suspension cells for 15 seconds using Protocol B, as described in **Example 9.1.** After washing the cells, GFP fluorescence was visualized by bright field and fluorescence microscopy. Sample images captured at 10x magnifications showing GFP fluorescence are shown in **Figure 37** for (A) 293T, (B) Balb3T3, (C) CHO, (D) Myoblasts, (E) Jurkat, (F) CA46, (G) HT2, and (H) NIH3T3 cells. The insets show corresponding flow cytometry results performed as described in **Example 3.3,** indicating the percentage of GFP-NLS-positive cells. No significant cellular GFP fluorescence was observed in negative control samples (i.e., cells exposed to GFP-NLS without any shuttle agent; data not shown).

Nuclear localization of the GFP-NLS was further confirmed in fixed and permeabilized myoblasts using cell immuno-labelling as described in **Example 3.2a.** GFP-NLS was labeled using a primary mouse monoclonal anti-GFP antibody (Feldan, #A017) and a secondary goat anti-mouse Alexa™-594 antibody (Abcam #150116). Nuclei were labelled with DAPI. Sample results for primary human myoblast cells are shown in **Figure 38****,** in which GFP immuno-labelling is shown in panel A, and an overlay of the GFP immuno-labelling and DAPI labelling is shown in panel B. No significant cellular GFP labelling was observed in negative control samples (i.e., cells exposed to GFP-NLS without any shuttle agent; data not shown).

The microscopy results revealed that GFP-NLS is successfully delivered to the nucleus of all the tested cells using the shuttle agent His-CM18-PTD4.

### Example 13:

### His-CM18-PTD4 enables transduction of a CRISPR/Cas9-NLS system and genome editing in Hela cells

### 13.1 Cas9-NLS recombinant protein

Cas9-NLS recombinant protein was constructed, expressed and purified from a bacterial expression system as described in **Example 1.4.** The sequence of the Cas9-NLS recombinant protein produced was: 
MDKKYSIGLDIGTNSVGWAVITDEYKVPSKKFKVLGNTDRHSIKKNLIGALLFDSGETAEATRLKR
TARRRYTRRKNRICYLQEIFSNEMAKVDDSFFHRLEESFLVEEDKKHERHPIFGNIVDEVAYHEKY
PTIYHLRKKLVDSTDKADLRLIYLALAHMIKFRGHFLIEGDLNPDNSDVDKLFIQLVQTYNQLFEE
NPINASGVDAKAILSARLSKSRRLENLIAQLPGEKKNGLFGNLIALSLGLTPNFKSNFDLAEDAKL
QLSKDTYDDDLDNLLAQIGDQYADLFLAAKNLSDAILLSDILRVNTEITKAPLSASMIKRYDEHHQ
DLTLLKALVRQQLPEKYKEIFFDQSKNGYAGYIDGGASQEEFYKFIKPILEKMDGTEELLVKLNRE
DLLRKQRTFDNGSIPHQIHLGELHAILRRQEDFYPFLKDNREKIEKILTFRIPYYVGPLARGNSRF
AWMTRKSEETITPWNFEEVVDKGASAQSFIERMTNFDKNLPNEKVLPKHSLLYEYFTVYNELTKVK
YVTEGMRKPAFLSGEQKKAIVDLLFKTNRKVTVKQLKEDYFKKIECFDSVEISGVEDRFNASLGTY
HDLLKIIKDKDFLDNEENEDILEDIVLTLTLFEDREMIEERLKTYAHLFDDKVMKQLKRRRYTGWG
RLSRKLINGIRDKQSGKTILDFLKSDGFANRNFMQLIHDDSLTFKEDIQKAQVSGQGDSLHEHIAN
LAGSPAIKKGILQTVKVVDELVKVMGRHKPENIVIEMARENQTTQKGQKNSRERMKRIEEGIKELG
SQILKEHPVENTQLQNEKLYLYYLQNGRDMYVDQELDINRLSDYDVDHIVPQSFLKDDSIDNKVLT
RSDKNRGKSDNVPSEEVVKKMKNYWRQLLNAKLITQRKFDNLTKAERGGLSELDKAGFIKRQLVET
RQITKHVAQILDSRMNTKYDENDKLIREVKVITLKSKLVSDFRKDFQFYKVREINNYHHAHDAYLN
AVVGTALIKKYPKLESEFVYGDYKVYDVRKMIAKSEQEIGKATAKYFFYSNIMNFFKTEITLANGE
IRKRPLIETNGETGEIVWDKGRDFATVRKVLSMPQVNIVKKTEVQTGGFSKESILPKRNSDKLIAR
KKDWDPKKYGGFDSPTVAYSVLVVAKVEKGKSKKLKSVKELLGITIMERSSFEKNPIDFLEAKGYK
EVKKDLIIKLPKYSLFELENGRKRMLASAGELQKGNELALPSKYVNFLYLASHYEKLKGSPEDNEQ
KQLFVEQHKHYLDEIIEQISEFSKRVILADANLDKVLSAYNKHRDKPIREQAENIIHLFTLTNLGA
PAAFKYFDTTIDRKRYTSTKEVLDATLIHQSITGLYETRIDLSQLGGDGGRSSDDEATADSQHAAP
PKKKRKVGGSGGGSGGGSGGGRHHHHHH [SEQ ID NO: 74]
 (MW = 162.9 kDa; pl = 9.05)
NLS sequence is underlined
Serine/glycine rich linkers are in bold

### 13.2 Transfection plasmid surrogate assay

This assay enables one to visually identify cells that have been successfully delivered an active CRISPR/Cas9 complex. As shown in **Figure 39A****,** the assay involves transfecting cells with an expression plasmid DNA encoding the fluorescent proteins mCherry™ and GFP, with a STOP codon separating their two open reading frames. Transfection of the cells with the expression plasmid results in mCherry™ expression, but no GFP expression **(****Figure 39B****).** A CRISPR/Cas9 complex, which has been designed/programmed to cleave the plasmid DNA at the STOP codon, is then delivered intracellularly to the transfected cells expressing mCherry™ **(****Figure 39D****).** Successful transduction of an active CRISPR/Cas9 complex results in the CRISPR/Cas9 complex cleaving the plasmid DNA at the STOP codon **(****Figure 39C****).** In a fraction of the cells, random non-homologous DNA repair of the cleaved plasmid occurs and results in removal of the STOP codon, and thus GFP expression and fluorescence **(****Figure 39E****).**

On Day 1 of the transfection plasmid surrogate assay, DNA plasmids for different experimental conditions (250 ng) are diluted in DMEM (50 µL) in separate sterile 1.5-mL tubes, vortexed and briefly centrifuged. In separate sterile 1.5-mL tubes, Fastfect™ transfection reagent was diluted in DMEM (50 µL) with no serum and no antibiotics at a ratio of 3:1 (3 µL of Fastfect™ transfection reagent for 1 µg of DNA) and then quickly vortexed and briefly centrifuged. The Fastfect™/DMEM mixture was then added to the DNA mix and quickly vortexed and briefly centrifuged. The Fastfect™/DMEM/DNA mixture is then incubated for 15-20 min at room temperature, before being added to the cells (100 µL per well). The cells are then incubated at 37°C and 5% CO₂ for 5h. The media is then changed for complete medium (with serum) and further incubated at 37°C and 5% CO₂ for 24-48h. The cells are then visualized under fluorescent microscopy to view the mCherry™ signal.

### 13.3 His-CM18-PTD4 - mediated CRISPR/Cas9-NLS system delivery and cleavage of plasmid DNA

RNAs (crRNA & tracrRNA) were designed to target a nucleotide sequence of the EMX1 gene, containing a STOP codon between the mCherry™ and GFP coding sequences in the plasmid of **Example 13.2.** The sequences of the crRNA and tracrRNA used were as follows:

| |
|---|
| - **crRNA [SEQ ID NO: 75]:** |
| 5'-GAGUCCGAGCAGAAGAAGAAGUUUUAGAGCUAUGCUGUUUUG-3' |
| - **tracrRNA [SEQ ID NO: 76]:** |
| 5'-AAACAGCAUAGCAAGUUAAAAUAAGGCUAGUCCGUUAUCAACUUGAAAAAGUGGCACCGAGUCGGUGCU-3' |

HeLa cells were cultured and subjected to the transfection plasmid surrogate assay as described in **Example 13.2).** On Day 1, the HeLa cells were transfected with a plasmid surrogate encoding the mCherry™ protein as shown in **Figure 39A****.** On Day 2, a mix of Cas9-NLS recombinant protein (2 µM; see **Example 13.1)** and RNAs (crRNA & tracrRNA; 2 µM; see above) were co-incubated with 50 µM of His-CM18-PTD4, and the mixture (CRISPR/Cas9 complex) was exposed to HeLa cells for 10 seconds using Protocol B as described in **Example 9.1.** Double-stranded plasmid DNA cleavage by the CRISPR/Cas9 complex at the STOP codon between the mCherry™ and GFP coding sequences **(****Figure 39B****),** and subsequent non-homologous repair by the cell in some cases results in removal of the STOP codon **(****Figure 39C****),** thereby allowing expression of both the mCherry™ and GFP fluorescent proteins in the same cell on Day 3 **(****Figure 39D-E****).** White triangle windows in panels D and E indicate examples of areas of co-labelling between mCherry™ and GFP.

As a positive control for the CRISPR/Cas9-NLS system, HeLa cells were cultured and ∞-transfected with three plasmids: the plasmid surrogate (as described in **Example 13.2)** and other expression plasmids encoding the Cas9-NLS protein **(Example 13.1)** and the crRNA/tracrRNAs **(Example 13.3).** Typical fluorescence microscopy results are shown in **Figure 40A-D****.** Panels A and B show cells 24 hours post-transfection, while panels C and D show cells 72 hours post-transfection.

**Figure 40E-H** shows the results of a parallel transfection plasmid surrogate assay performed using 35 µM of the shuttle His-CM18-PTD4, as described for **Figure 39****.** Panels E and F show cells 24 hours post-transduction, while panels G and H show cells 48 hours post-transduction. Panels E and G show mCherry™ fluorescence, and panels F and H show GFP fluorescence, the latter resulting from removal of the STOP codon by the transduced CRISPR/Cas9-NLS complex and subsequent non-homologous repair by the cell. No significant cellular GFP fluorescence was observed in negative control samples (i.e., cells exposed to CRISPR/Cas9-NLS complex without any shuttle agent; data not shown).

### 13.4 T7E1 assay

The T7E1 assay was performed with the Edit-R™ Synthetic crRNA Positive Controls (Dharmacon #U-007000-05) and the T7 Endonuclease I (NEB, Cat#M0302S). After the delivery of the CRISPR/Cas9 complex, cells were lysed in 100 µL of Phusion™ High-Fidelity DNA polymerase (NEB #M0530S) laboratory with additives. The cells were incubated for 15-30 minutes at 56°C, followed by deactivation for 5 minutes at 96°C. The plate was briefly centrifuged to collect the liquid at bottom of the wells. 50-µL PCR samples were set up for each sample to be analyzed. The PCR samples were heated to 95°C for 10 minutes and then slowly (>15 minutes) cooled to room temperature. PCR product (∼5 µL) was then separated on an agarose gel (2%) to confirm amplification. 15 µL of each reaction was incubated with T7E1 nuclease for 25 minutes at 37 °C. Immediately, the entire reaction volume was run with the appropriate gel loading buffer on an agarose gel (2%).

### 13.5 His-CM18-PTD4 and His-C(LLKK)₃C-PTD4 -mediated CRISPR/Cas9-NLS system delivery and cleavage of genomic PPIB sequence

A mix composed of a Cas9-NLS recombinant protein (25 nM; **Example 13.1**) and crRNA/tracrRNA (50 nM; see below) targeting a nucleotide sequence of the PPIB gene were co-incubated with 10 µM of His-CM18-PTD4 or His-C(LLKK)₃C-PTD4, and incubated with HeLa cells for 16h in medium without serum using Protocol A as described in **Example 9.1.**

The sequences of the crRNA and tracrRNAs constructed and their targets were:

| |
|---|
| - **Feldan tracrRNA [SEQ ID NO: 77]:** |
| 5'-AAACAGCAUAGCAAGUUAAAAUAAGGCUAGUCCGUUAUCAACUUGAAAAAGUGGCACCGAGUCGGUGCU-3' |
| - **PPIB crRNA [SEQ ID NO: 78]:** |
| 5'-GUGUAUUUUGACCUACGAAUGUUUUAGAGCUAUGCUGUUUUG-3' |
| - **Dharmacon tracrRNA [SEQ ID NO: 79]:** |
| 5'-AACAGCAUAGCAAGUUAAAAUAAGGCUAGUCCGUUAUCAACUUGAAAAAGUGGCACCGAGUCGGUGCUUUUUUU-3' |

After 16h, HeLa cells were washed with PBS and incubated in medium with serum for 48h. HeLa cells were harvested to proceed with the T7E1 protocol assay as described in **Example 13.4.**

**Figure 41A** shows an agarose gel with the PPIB DNA sequences after PCR amplification. Lane **A** shows the amplified PPIB DNA sequence in HeLa cells without any treatment (i.e., no shuttle or Cas9/RNAs complex). Lanes **B:** The two bands framed in white box #1 are the cleavage product of the PPIB DNA sequence by the CRIPR/Cas9 complex after the delivery of the complex with the shuttle His-C(LLKK)₃C-PTD4. Lane **C:** These bands show the amplified PPIB DNA sequence after incubation of the HeLa cells with the Cas9/RNAs complex without shuttle (negative control). Lane **D:** The bands framed in white box #2 show the amplified PPIB DNA sequence after incubation of the HeLa cells with the Cas9/RNAs complex in presence of a lipidic transfection agent (DharmaFect™ tranfection reagent #T-20XX-01) (positive control). Similar results were obtained using the shuttle His-CM18-PTD4 (data not shown).

**Figure 41B** shows an agarose gel with the PPIB DNA sequences after PCR amplification. The left panel shows the cleavage product of the amplified PPIB DNA sequence by the CRIPR/Cas9 complex after the delivery of the complex with the shuttle agent His-CM18-PTD4 in HeLa cells. The right panel shows amplified DNA sequence before the T7E1 digestion procedure as a negative control.

**Figure 41C** shows an agarose gel with the PPIB DNA sequences after PCR amplification. The left panel shows the amplified PPIB DNA sequence after incubation of the HeLa cells with the Cas9/RNAs complex in presence of a lipidic transfection agent (DharmaFect™ transfection reagent # T-20XX-01) (positive control). The right panel shows amplified DNA sequence before the T7E1 digestion procedure as a negative control.

These results show that the shuttle agents His-CM18-PTD4 and His-C(LLKK)₃C-PTD4 successfully deliver a functional CRISPR/Cas9 complex to the nucleus of HeLa cells, and that this delivery results in CRISPR/Cas9-mediated cleavage of genomic DNA.

### 13.6 CRISPR/Cas9-NLS system delivery by different shuttle agents, and cleavage of genomic HPTR sequence in HeLa and Jurkat cells

A mix composed of a Cas9-NLS recombinant protein (2.5 µM; **Example 13.1)** and crRNA/tracrRNA (2 µM; see below) targeting a nucleotide sequence of the HPTR gene were co-incubated with 35 µM of His-CM18-PTD4, His-CM18-PTD4-His, His-C(LLKK)3C-PTD4, or EB1-PTD4, and incubated with HeLa or Jurkat cells for 2 minutes in PBS using Protocol B as described in **Example 9.1.**

The sequences of the crRNA and tracrRNAs constructed and their targets were:

| |
|---|
| - **Feldan tracrRNA [SEQ ID NO: 77]:** |
| 5'-AAACAGCAUAGCAAGUUAAAAUAAGGCUAGUCCGUUAUCAACUUGAAAAAGUGGCACCGAGUCGGUGCU-3' |
| - **HPRT crRNA [SEQ ID NO: 103]:** |
| 5'-AAUUAUGGGGAUUACUAGGAGUUUUAGAGCUAUGCU-3' |

After 2 minutes, cells were washed with PBS and incubated in medium with serum for 48h. Cells were harvested to proceed with the T7E1 protocol assay as described in **Example 13.4.** **Figure 46** shows an agarose gel with the HPTR DNA sequences after PCR amplification and the cleavage product of the amplified HPTR DNA sequence by the CRISPR/Cas9 complex after the delivery of the complex with the different shuttle agents. Panel **A** shows the results with the shuttle agents: His-CM18-PTD4, His-CM18-PTD4-His, and His-C(LLKK)3C-PTD4 in HeLa cells. Panel **B** shows the results with His-CM18-PTD4 and His-CM18-L2-PTD4 in Jurkat cells. Negative controls (lanes 4) show amplified HPTR DNA sequence after incubation of the cells with the CRISPR/Cas9 complex without the presence of the shuttle agent. Positive controls (lane 5 in panels A and B) show the amplified HPTR DNA sequence after incubation of the cells with the Cas9/RNAs complex in presence of a lipidic transfection agent (Lipofectamine® RNAiMAX™ Transfection Reagent ThermoFisher Product No. 13778100).
These results show that different polypeptide shuttle agents of the present description may successfully deliver a functional CRISPR/Cas9 complex to the nucleus of HeLa and Jurkat cells, and that this delivery results in CRISPR/Cas9-mediated cleavage of genomic DNA.

### Example 14:

### His-CM18-PTD4 enables transduction of the transcription factor HOXB4 in THP-1 cells

### 14.1 HOXB4-WT recombinant protein

Human HOXB4 recombinant protein was constructed, expressed and purified from a bacterial expression system as described in **Example 1.4.** The sequence of the HOXB4-WT recombinant protein produced was: 
MHHHHHHMAMSSFLINSNYVDPKFPPCEEYSQSDYLPSDHSPGYYAGGQRRESSFQPEAGFGRRAA
CTVQRYPPPPPPPPPPPGLSPRAPAPPPAGALLPEPGQRCEAVSSSPPPPPCAQNPLHPSPSHSACK
EPVVYPWMRKVHVSTVNPNYAGGEPKRSRTAYTRQQVLELEKEFHYNRYLTRRRRVEIAHALCLSE
RQIKIWFQNRRMKWKKDHKLPNTKIRSGGAAGSAGGPPGRPNGGPRAL [SEQ ID NO: 80]
 (MW = 28.54 kDa; pl =9.89)

The initiator methionine and the 6x Histidine tag are shown in bold.

### 14.2 Real-Time Polymerase Chain Reaction (rt-PCR)

Control and treated cells are transferred to separate sterile 1.5-mL tubes and centrifuged for 5 minutes at 300g. The cell pellets are resuspended in appropriate buffer to lyse the cells. RNAase-free 70% ethanol is then added followed by mixing by pipetting. The lysates are transferred to an RNeasy™ Mini spin column and centrifuged 30 seconds at 13000 RPM. After several washes with appropriate buffers and centrifugation steps, the eluates are collected in sterile 1.5-mL tubes on ice, and the RNA quantity in each tube is then quantified with a spectrophotometer. For DNase treatment, 2 µg of RNA is diluted in 15 µL of RNase-free water. 1.75 µL of 10X DNase buffer and 0.75 µL of DNase is then added, followed by incubation at 37°C for 15 minutes. For reverse transcriptase treatment, 0.88 µL of EDTA (50 nM) is added, followed by incubation at 75°C for 5 minutes. In a PCR tube, 0.5 µg of DNase-treated RNA is mixed with 4 µL of iScript™ Reverse transcription Supermix (5X) and 20 µL of nuclease-free water. The mix is incubated in a PCR machine with the following program: 5 min at 25°C, 30 min at 42°C and 5 min at 85°C. Newly synthesized cDNA is transferred in sterile 1.5-mL tubes and diluted in 2 µL of nuclease-free water. 18 µL per well of a qPCR machine (CFX-96™) mix is then added in a PCR plate for analysis.

### 14.3 HOXB4-WT transduction by His-CM18-PTD4 in THP-1 cells: Dose responses and viability

THP-1 cells were cultured and tested in the protein transduction assay using Protocol A as described in **Example 9.1.** Briefly, THP-1 cells were plated at 30 000 cells/well one day before transduction. HOXB4-WT recombinant protein (0.3, 0.9, or 1.5 µM; **Example 14.1)** was co-incubated with different concentrations of His-CM18-PTD4 (0, 0.5, 7.5, 0.8 or 1 µM) and then exposed to THP-1 cells for 2.5 hours in the presence of serum. The cells were subjected to real time-PCR analysis as described in **Example 14.2** to measure the mRNA levels of a target gene as a marker for HOXB4 activity, which was then normalized to the target gene mRNA levels detected in the negative control cells (no treatment), to obtain a "Fold over control" value. Total RNA levels (ng/µL) were also measured as a marker for cell viability. Results are shown in **Table 14.1** and **Figure 42****.**

**Table 14.1: Data from Figure 42**

| **Cargo / shuttle agent (****Fig. 41****)** | **Cells** | **Conc. of shuttle (µM)** | **Conc. of HOXB4-WT (µM)** | **Fold over control (mean ± St. Dev)** | **Total RNA in ng/µL (mean ± St. Dev)** |
|---|---|---|---|---|---|
| **No treatment ("0̸")** | THP-1 | 0 | 0 | **1** ± 0.1 | **263** ± 0.4 |
| **HOXB4-WT alone ("TF")** | THP-1 | 0 | 1.5 | **4.3** ± 0.1 | **271** ± 6.0 |
| **His-CM18-PTD4 alone ("FS")** | THP-1 | 1 | 0 | **2.7** ± 0.3 | **252 ±** 10.7 |
| **His-CM18-PTD4 + HOXB4-WT** | THP-1 | 0.5 | 0.3 | **2.7** ± 0.6 | **255 ±** 3.9 |
| | | | 0.9 | **4.3 ±** 2.1 | **239** ± 17.5 |
| | | | 1.5 | **3.8** ± 0.7 | **269** ± 6.4 |
| **His-CM18-PTD4 + HOXB4-WT** | THP-1 | 0.75 | 0.3 | **4.2** ± 1.2 | **248** ± 28 |
| | | | 0.9 | **5.7** ± 2.5 | **245 ±** 31 |
| | | | 1.5 | **7.5** ± 2.8 | **230** ± 3.3 |
| **His-CM18-PTD4 + HOXB4-WT** | THP-1 | 0.8 | 0.3 | **9.1** ± 2.7 | **274 ±** 4.4 |
| | | | 0.9 | **16.4** ± 1.7 | **272** ± 12.5 |
| | | | 1.5 | **22.7** ± 3.2 | **282** ± 4.7 |
| **His-CM18-PTD4 + HOXB4-WT** | THP-1 | 0.9 | 0.3 | **10.2** ± 2.5 | **280** ± 11.3 |
| | | | 0.9 | **18.7** ± 3.1 | **281** ± 9.2 |
| | | | 1.5 | **26.1** ± 3.5 | **253** ± 7.1 |
| **His-CM18-PTD4 + HOXB4-WT** | THP-1 | 1 | 0.3 | **10.5** ± 0.7 | **184** ± 12.3 |
| | | | 0.9 | **17** ± 3.7 | 168 ± 16.2 |
| | | | 1.5 | **24.5** ± 3.9 | **154** ± 4.7 |

These results show that exposing THP-1 cells to a mixture of the shuttle agent His-CM18-PTD4 and the transcription factor HOXB4-WT for 2.5 hours in the presence of serum results in a dose-dependent increase in mRNA transcription of the target gene. These results suggest that HOXB4-WT is successfully delivered in an active form to the nucleus of THP-1 cells, where it can mediate transcriptional activation.

### 14.4 HOXB4-WT transduction by His-CM18-PTD4 in THP-1 cells: Time course and viability (0 to 48 hours)

THP-1 cells were cultured and tested in the protein transduction assay using Protocol A as described in **Example 9.1.** Briefly, THP-1 cells were plated at 30 000 cells/well one day before the first time course experiment. HOXB4-WT recombinant protein (1.5 µM; **Example 14.1)** was co-incubated with His-CM18-PTD4 (0.8 µM) and then exposed to THP-1 cells for 0, 2.5, 4, 24 or 48 hours in presence of serum. The cells were subjected to real time-PCR analysis as described in **Example 14.2** to measure mRNA levels of a target gene as a marker for HOXB4 activity, which was then normalized to the target gene mRNA levels detected in the negative control cells (no treatment), to obtain a "Fold over control" value. Total RNA levels (ng/µL) were also measured as a marker for cell viability. Results are shown in **Table 14.2** and **Figure 43****.**

**Table 14.2: Data from Figure 43**

| **Cargo shuttle agent (****Fig. 43****)** | **Cells** | **Conc. of shuttle (µM)** | **Conc. of HOXB4-WT (µM)** | **Exposure time (hours)** | **Fold over control (mean ± St. Dev)** | **Total RNA in ng/µL (mean ± St. Dev)** |
|---|---|---|---|---|---|---|
| **No treatment ("Ctrl")** | THP-1 | 0 | 0 | - | **1** ± 0.1 | **180** ± 0.4 |
| **HOXB4-WT alone ("TF")** | THP-1 | 0 | 1.5 | 2.5 h | **3.4** ± 0.3 | **129** ± 10.7 |
| **His-CM18-PTD4 alone ("FS")** | THP-1 | 0.8 | 0 | 2.5 h | **1.2** ±0.14 | **184** ± 6.0 |
| **His-CM18-PTD4 + HOXB4-WT** | THP-1 | 0.8 | 1.5 | 48 h | **0.27** ± 0.1 | **58** ± 11.2 |
| | | | | 24 h | **0.8** ±0.14 | **74** ± 9.2 |
| | | | | 4 h | **5.6** ± 1.2 | **94** ± 7.1 |
| | | | | 2.5 h | **9.1** ± 1.2 | **146** ± 11.6 |
| | | | | 0 | **3.9** ± 0.4 | **167** ± 13 |

### 14.5 HOXB4-WT transduction by His-CM18-PTD4 in THP-1 cells: Time course and viability (0 to 4 hours)

THP-1 cells were cultured and tested in the protein transduction assay using Protocol A as described in **Example 9.1.** Briefly, THP-1 cells were plated at 30 000 cells/well one day before the first time course experiment. HOXB4-WT recombinant protein (0.3 µM; **Example 14.1)** was co-incubated with His-CM18-PTD4 (0.8 µM) and then exposed to THP-1 cells for 0, 0.5, 1, 2, 2.5, 3 or 4 hours in presence of serum. The cells were subjected to real time-PCR analysis as described in **Example 14.2** to measure mRNA levels of a target gene as a marker for HOXB4 activity, which was then normalized to target gene mRNA levels detected in the negative control cells (no treatment), to obtain a "Fold over control" value. Total RNA levels (ng/µL) were also measured as a marker for cell viability. Results are shown in **Table 14.3** and **Figure 44****.**

**Table 14.3: Data from Figure 44**

| **Cargo / shuttle agent (****Fig. 42****)** | **Cells** | **Conc. of shuttle (µM)** | **Conc of HOXB4-WT (µM)** | **Exposure time (hours)** | **Fold over control (mean ± St. Dev)** | **Total RNA ng/µL (mean ± St. Dev)** |
|---|---|---|---|---|---|---|
| **No treatment ("Ctrl")** | THP-1 | 0 | 0 | - | **1** ± 0.1 | **289** ± 9.2 |
| **His-CM18-PTD4 alone ("FS")** | THP-1 | 0 | 0.3 | 2.5 h | **2.5** ± 0.2 | **260** ± 7.1 |
| **HOXB4-WT alone ("TF")** | THP-1 | 0.8 | 0 | 2.5 h | **1** ± 0.14 | **264** ± 12.3 |
| **His-CM18-PTD4 + HOXB4-WT** | THP-1 | 0.8 | 0.3 | 4 h | **1.2** ± 0.1 | **198** ± 6.0 |
| | | | | 3 h | **1.3** ± 0.21 | **268** ± 12.5 |
| | | | | 2.5 h | **2** ± 0.3 | **275** ± 4.7 |
| | | | | 2 h | **2.2** ± 0.2 | **269** ± 12.5 |
| | | | | 1 | **9.7** ± 2.6 | **268** ± 3.9 |
| | | | | 0.5 | **23.1** ± 2.0 | **266** ± 17.5 |
| | | | | 0 | **4** ± 0.5 | **217** ± 6.4 |

### 14.6 HOXB4-WT transduction by His-CM18-PTD4 in HeLa cells: immuno-labelling and visualization by microscopy

Recombinant HOXB4-WT transcription factor (25 µM; **Example 14.1)** was co-incubated with 35 µM of His-CM18-PTD4 and exposed to HeLa cells for 10 seconds using Protocol B as described in **Example 9.1.** After a 30-minute incubation to allow transduced HOXB4-WT to accumulate in the nucleus, the cells were fixed, permeabilized and immuno-labelled as described in **Example 3.2a.** HOXB4-WT was labelled using a primary mouse anti-HOXB4 monoclonal antibody (Novus Bio #NBP2-37257) diluted 1/500, and a secondary anti-mouse antibody Alexa™-594 (Abcam #150116) diluted 1/1000. Nuclei were labelled with DAPI. The cells were visualized by bright field and fluorescence microscopy at 20x and 40x magnifications as described in **Example 3.2,** and sample results are shown in **Figure 45****.** Co-localization was observed between nuclei labelling (panels A and C) and HOXB4-WT labelling (panels B and D), indicating that HOXB4-WT was successfully delivered to the nucleus after 30 min in the presence of the shuttle agent His-CM18-PTD4. White triangle windows show examples of areas of co-localization between the nuclei (DAPI) and HOXB4-WT immuno-labels.

### 14.7 HOXB4-WT transduction by different shuttle agents in THP-1 cells: Dose responses and viability

THP-1 cells were cultured and tested in the protein transduction assay using Protocol A as described in **Example 9.1.** Briefly, THP-1 cells were plated at 30 000 cells/well one day before the first time course experiment. HOXB4-WT recombinant protein (1.5 µM; **Example 14.1)** co-incubated with the shuttle agents His-CM18-PTD4, TAT-KALA, EB1-PTD4, His-C(LLKK)₃C-PTD4 and His-CM18-PTD4-His at 0.8 µM, and then exposed to THP-1 cells for2.5 hours in presence of serum. The cells were subjected to real time-PCR analysis as described in **Example 14.2** to measure mRNA levels of a target gene as a marker for HOXB4 activity, which was then normalized to target gene mRNA levels detected in the negative control cells (no treatment), to obtain a "Fold over control" value. Total RNA levels (ng/µL) were also measured as a marker for cell viability. Results are shown in **Table 14.4** and **Figure 47****.**

**Table 14.4: Data from Figure 47**

| **Cargo / shuttle agent** | **Shuttle conc. (µM)** | **HOXB4-WT Conc. (µM)** | **Exposure time** | **Fold over control (mean ± St. Dev)** | **Total RNA in ng/µL (mean ± St. Dev)** |
|---|---|---|---|---|---|
| No treatment ("Ctrl") | 0 | 0 | - | **1** ± 0.09 | **240.3** ± 8.9 |
| H is-CM 18-PTD4 alone ("FS") | 0 | 1.5 | 2.5 h | **2.5** ± 0.3 | **303.9** ± 7.6 |
| HOXB4-WT alone ("TF") | 0.8 | 0 | 2.5 h | **1** ± 0.11 | **251.9** ± 11.9 |
| **His-CM18-PTD4** + HOXB4-WT | 0.8 | 1.5 | 2.5 h | **44.5** ± 0.09 | **182 ±** 5.97 |
| **TAT-KALA** + HOXB4-WT | | | | **5.1** ± 0.21 | **222.4** ± 12.5 |
| **EB1-PTD4** + HOXB4-WT | | | | **6.4** ± 0.3 | **240.4** ± 4.71 |
| **His-C(LLKK)3C-PTD4** + HOXB4-WT | | | | **9.8** ± 0.19 | **175.3** ± 11.25 |
| **His-CM18-PTD4-His** + HOXB4-WT | | | | **28.1** ± 2.61 | **91.4** ± 3.92 |

### Example 15:

### In vivo GFP-NLS delivery in rat parietal cortex by His-CM18-PTD4

The ability of the shuttle agent His-CM18-PTD4 to deliver GFP-NLS *in vivo* in the nuclei of rat brain cells was tested.

In separate sterile 1.5-mL tubes, shuttle agent His-CM18-PTD4 was diluted in sterile distilled water at room temperature. GFP-NLS, used as cargo protein, was then added to the shuttle agent and, if necessary, sterile PBS was added to obtain the desired concentrations of shuttle agent and cargo in a sufficient final volume for injection in rat brain (e.g., 5 µL per each injection brain site). The shuttle agent/cargo mixture was then immediately used for experiments. One negative control was included for the experiment, which corresponds to the injection of the GFP-NLS alone.

Bilateral injections were performed in the parietal cortex of three rats. In the left parietal cortex (ipsilateral), a mix composed of the shuttle agent (20 µM) and the GFP-NLS (20 µM) was injected, and in the right parietal cortex (contralateral), only the GFP-NLS (20 µM) was injected as a negative control. For surgical procedures, mice were anesthetized with isoflurane. Then the animal was placed in a stereotaxic frame, and the skull surface was exposed. Two holes were drilled at the appropriate sites to allow bilateral infusion of the shuttle/cargo mix or GFP-NLS alone (20 µM) with 5-µL Hamilton syringe. Antero-posterior (AP), lateral (L), and dorso-ventral (DV) coordinates were taken relative to the bregma: (a) AP +0.48 mm, L ± 3 mm, V - 5 mm; (b) AP - 2 mm, L ± 1.3 mm, V -1.5 mm; (c) AP - 2.6 mm, L ± 1.5 mm, V - 1.5 mm. The infused volume of the shuttle/cargo mix or cargo alone was 5 µL per injection site and the injection was performed for 10 minutes. After that, experimenter waited 1 min before removing the needle from the brain. All measures were taken before, during, and after surgery to minimize animal pain and discomfort. Animals were sacrificed by perfusion with paraformaldehyde (4%) 2 h after surgery, and brain were collected and prepared for microcopy analysis. Experimental procedures were approved by the Animal Care Committee in line with guidelines from the Canadian Council on Animal Care.

Dorso-ventral rat brain slices were collected and analysed by fluorescence microscopy and results are shown in **Figure 48** at (A) 4x, (C) 10x and (D) 20x magnifications. The injection site is located in the deepest layers of the parietal cortex (PCx). In the presence of the His-CM18-PTD4 shuttle, the GFP-NLS diffused in cell nuclei of the PCx, of the Corpus Callus (Cc) and of the striatum (Str) (White curves mean limitations between brains structures). Panel B shows the stereotaxic coordinates of the injection site (black arrows) from the rat brain atlas of Franklin and Paxinos. The injection of GFP-NLS in presence of His-CM18-PTD4 was performed on the left part of the brain, and the negative control (an injection of GFP-NLS alone), was done on the contralateral site. The black circle and connected black lines in panel B show the areas observed in the fluorescent pictures (A, C and D).

This experiment demonstrated the cell delivery of the cargo GFP-NLS after its stereotaxic injection in the rat parietal cortex in the presence of the shuttle agent His-CM18-PTD4. Results show the delivery of the GFP-NLS in the nucleus of cells from the deeper layers of the parietal cortex (injection site) to the corpus callus and the dorsal level of the striatum (putamen). In contrast, the negative control in which GFP-NLS is only detectable locally around the injection site. This experiment shows that shuttle agent induced nuclear delivery of the cargo in the injection site (parietal cortex) and its diffusion through both neighboring brain areas (corpus callus and striatum rat brain).

### REFERENCES

Andreu, D., Ubach, J., Boman, A., Wahlin, B., Wade, D., Merrifield, R. B., and Boman, H. G. (1992) Shortened cecropin A-melittin hybrids. Significant size reduction retains potent antibiotic activity. FEBS letters 296, 190-194
Aguila, J. R., W. Liao, J. Yang, C. Avila, N. Hagag, L. Senzel and Y. Ma (2011). "SALL4 is a robust stimulator for the expansion of hematopoietic stem cells." Blood 118(3): 576-585.
Akinci, E., A. Banga, L. V. Greder, J. R. Dutton and J. M. Slack (2012). "Reprogramming of pancreatic exocrine cells towards a beta (beta) cell character using Pdx1, Ngn3 and MafA." Biochem J 442(3): 539-550.
Alford et al., (2009). "Toxicity of organic fluorophores used in molecular imaging: literature review." Mol Imaging. 8(6):341-54.
Amand, H. L., B. Norden and K. Fant (2012). "Functionalization with C-terminal cysteine enhances transfection efficiency of cell-penetrating peptides through dimer formation." Biochem Biophys Res Commun 418(3): 469-474.
Bejarano, L. A. and C. Gonzalez (1999). "Motif trap: a rapid method to clone motifs that can target proteins to defined subcellular localisations." J Cell Sci 112 (Pt 23): 4207-4211.
Bikard et al., Programmable repression and activation of bacterial gene expression using an engineered CRISPR-Cas system. Nucleic Acids Res. 41, 7429-7437.
Boman, H. G., Wade, D., Boman, I. A., Wahlin, B., and Merrifield, R. B. (1989) Antibacterial and antimalarial properties of peptides that are cecropin-melittin hybrids. FEBS letters 259, 103-106.
Buganim et al., (2014) "The Developmental Potential of iPSCs Is Greatly Influenced by Reprogramming Factor Selection". Cell stem cell. 15, 295-309.
Chan, C. K. and D. A. Jans (1999). "Enhancement of polylysine-mediated transferrinfection by nuclear localization sequences: polylysine does not function as a nuclear localization sequence." Hum Gene Ther 10(10): 1695-1702.
Chan, C. K. and D. A. Jans (2001). "Enhancement of MSH receptor- and GAL4-mediated gene transfer by switching the nuclear import pathway." Gene Ther 8(2): 166-171.
Cong et al., (2013). Multiplex genome engineering using CRISPR/Cas systems. Science 339, 819-823.
Cox et al. (2015). "Therapeutic genome editing: prospects and challenges". Nature medicine, 21: 121-131.
Dolfini, D., M. Minuzzo, G. Pavesi and R. Mantovani (2012). "The short isoform of NF-YA belongs to the embryonic stem cell transcription factor circuitry." Stem Cells 30(11): 2450-2459.
Drin, G., S. Cottin, E. Blanc, A. R. Rees and J. Temsamani (2003). "Studies on the internalization mechanism of cationic cell-penetrating peptides." J Biol Chem 278(33): 31192-31201.
El-Andaloussi, S., H. J. Johansson, T. Holm and U. Langel (2007). "A novel cell-penetrating peptide, M918, for efficient delivery of proteins and peptide nucleic acids." Mol Ther 15(10): 1820-1826.
El-Sayed, A., S. Futaki and H. Harashima (2009). "Delivery of macromolecules using arginine-rich cell-penetrating peptides: ways to overcome endosomal entrapment." AAPS J 11(1): 13-22.
Elmquist, A., M. Lindgren, T. Bartfai and U. Langel (2001). "VE-cadherin-derived cell-penetrating peptide, pVEC, with carrier functions." Exp Cell Res 269(2): 237-244.
Erazo-Oliveras et al., (2014) "Protein delivery into live cells by incubation with an endosomolytic agent." Nat Methods. (8):861-7.
Fanara, P., M. R. Hodel, A. H. Corbett and A. E. Hodel (2000). "Quantitative analysis of nuclear localization signal (NLS)-importin alpha interaction through fluorescence depolarization. Evidence for auto-inhibitory regulation of NLS binding." J Biol Chem 275(28): 21218-21223.
Fasoli A et al., (2014) "Mechanistic insight into CM18-Tat11 peptide membrane-perturbing action by whole-cell patch-clamp recording." Molecules. 19(7):9228-39.
Fawell, S., J. Seery, Y. Daikh, C. Moore, L. L. Chen, B. Pepinsky and J. Barsoum (1994). "Tat-mediated delivery of heterologous proteins into cells." Proc Natl Acad Sci U S A 91(2): 664-668.
Fominaya, J., C. Uherek and W. Wels (1998). "A chimeric fusion protein containing transforming growth factor-alpha mediates gene transfer via binding to the EGF receptor." Gene Ther 5(4): 521-530.
Fominaya, J. and W. Wels (1996). "Target cell-specific DNA transfer mediated by a chimeric multidomain protein. Novel non-viral gene delivery system." J Biol Chem 271(18): 10560-10568.
Fonoudi, H., M. Yeganeh, F. Fattahi, Z. Ghazizadeh, H. Rassouli, M. Alikhani, B. A. Mojarad, H. Baharvand, G. H. Salekdeh and N. Aghdami (2013). "ISL1 protein transduction promotes cardiomyocyte differentiation from human embryonic stem cells." PLoS One 8(1): e55577.
Gilbert et al., CRISPR-mediated modular RNA-guided regulation of transcription in eukaryotes. Cell 154, 442-451.
Gilmore, T. D. and H. M. Temin (1988). "v-rel oncoproteins in the nucleus and in the cytoplasm transform chicken spleen cells." J Virol 62(3): 703-714.
Glover, D. J., S. M. Ng, A. Mechler, L. L. Martin and D. A. Jans (2009). "Multifunctional protein nanocarriers for targeted nuclear gene delivery in nondividing cells." FASEB J 23(9): 2996-3006.
Gordon, S. M., J. Chaix, L. J. Rupp, J. Wu, S. Madera, J. C. Sun, T. Lindsten and S. L. Reiner (2012). "The transcription factors T-bet and Eomes control key checkpoints of natural killer cell maturation." Immunity 36(1): 55-67.
Gottschalk, S., J. T. Sparrow, J. Hauer, M. P. Mims, F. E. Leland, S. L. Woo and L. C. Smith (1996). "A novel DNA-peptide complex for efficient gene transfer and expression in mammalian cells." Gene Ther 3(5): 448-457.
Gould, S. J., G. A. Keller, N. Hosken, J. Wilkinson and S. Subramani (1989). "A conserved tripeptide sorts proteins to peroxisomes." J Cell Biol 108(5): 1657-1664.
Green, M. and P. M. Loewenstein (1988). "Autonomous functional domains of chemically synthesized human immunodeficiency virus tat trans-activator protein." Cell 55(6): 1179-1188.
Grimes, M. L., J. Zhou, E. C. Beattie, E. C. Yuen, D. E. Hall, J. S. Valletta, K. S. Topp, J. H. LaVail, N. W. Bunnett and W. C. Mobley (1996). "Endocytosis of activated TrkA: evidence that nerve growth factor induces formation of signaling endosomes." J Neurosci 16(24): 7950-7964.
Hallbrink, M., A. Floren, A. Elmquist, M. Pooga, T. Bartfai and U. Langel (2001). "Cargo delivery kinetics of cell-penetrating peptides." Biochim Biophys Acta 1515(2): 101-109.
Herce, H. D. and A. E. Garcia (2007). "Molecular dynamics simulations suggest a mechanism for translocation of the HIV-1 TAT peptide across lipid membranes." Proc Natl Acad Sci U S A 104(52): 20805-20810.
Ho et al., (2001). "Synthetic protein transduction domains: enhanced transduction potential in vivo." Cancer Research 61: 474-477.
Hurt, E. C., B. Pesold-Hurt, K. Suda, W. Oppliger and G. Schatz (1985). "The first twelve amino acids (less than half of the pre-sequence) of an imported mitochondrial protein can direct mouse cytosolic dihydrofolate reductase into the yeast mitochondrial matrix." EMBO J 4(8): 2061-2068.
Ichii, H., A. Sakamoto, Y. Kuroda and T. Tokuhisa (2004). "Bcl6 acts as an amplifier for the generation and proliferative capacity of central memory CD8+ T cells." J Immunol 173(2): 883-891.
Irie, Y., K. Yamagata, Y. Gan, K. Miyamoto, E. Do, C. H. Kuo, E. Taira and N. Miki (2000). "Molecular cloning and characterization of Amida, a novel protein which interacts with a neuron-specific immediate early gene product arc, contains novel nuclear localization signals, and causes cell death in cultured cells." J Biol Chem 275(4): 2647-2653.
Kakudo, T., S. Chaki, S. Futaki, I. Nakase, K. Akaji, T. Kawakami, K. Maruyama, H. Kamiya and H. Harashima (2004). "Transferrin-modified liposomes equipped with a pH-sensitive fusogenic peptide: an artificial viral-like delivery system." Biochemistry 43(19): 5618-5628.
Kamiely, S. and O. Pines (2005). "Single translation--dual destination: mechanisms of dual protein targeting in eukaryotes." EMBO Rep 6(5): 420-425.
Kato, G. J., W. M. Lee, L. L. Chen and C. V. Dang (1992). "Max: functional domains and interaction with c-Myc." Genes Dev 6(1): 81-92.
Kichler, A., A. J. Mason and B. Bechinger (2006). "Cationic amphipathic histidine-rich peptides for gene delivery." Biochim Biophys Acta 1758(3): 301-307.
Kichler et al., (2003). "Histidine-rich amphipathic peptide antibiotics promote efficient delivery of DNA into mammalian cells". Proc Natl Acad Sci U S A. 2003 Feb 18; 100(4): 1564-1568.
Kira et al., (2011). "Unification of Cas protein families and a simple scenario for the origin and evolution of CRISPR-Cas systems". Biol Direct. 2011; 6: 38.
Kleinschmidt, J. A. and A. Seiter (1988). "Identification of domains involved in nuclear uptake and histone binding of protein N1 of Xenopus laevis." EMBO J 7(6): 1605-1614.
Kohler, M., D. Gorlich, E. Hartmann and J. Franke (2001). "Adenoviral E1A protein nuclear import is preferentially mediated by importin alpha3 in vitro." Virology 289(2): 186-191.
Lanford, R. E., P. Kanda and R. C. Kennedy (1986). "Induction of nuclear transport with a synthetic peptide homologous to the SV40 T antigen transport signal." Cell 46(4): 575-582.
Lord-Dufour et al., (2009) "Evidence for transcriptional regulation of the glucose-6-phosphate transporter by HIF-1alpha: Targeting G6PT with mumbaistatin analogs in hypoxic mesenchymal stromal cells". Stem cells 27: 489-497.
Li, W., F. Nicol and F. C. Szoka, Jr. (2004). "GALA: a designed synthetic pH-responsive amphipathic peptide with applications in drug and gene delivery." Adv Drug Deliv Rev 56(7): 967-985.
Lin, M. H., F. C. Chou, L. T. Yeh, S. H. Fu, H. Y. Chiou, K. I. Lin, D. M. Chang and H. K. Sytwu (2013). "B lymphocyte-induced maturation protein 1 (BLIMP-1) attenuates autoimmune diabetes in NOD mice by suppressing Th1 and Th17 cells." Diabetologia 56(1): 136-146.
Liu, X., P. K. Tian, D. W. Ju, M. H. Zhang, M. Yao, X. T. Cao and J. R. Gu (2003). "Systemic genetic transfer of p21WAF-1 and GM-CSF utilizing of a novel oligopeptide-based EGF receptor targeting polyplex." Cancer Gene Ther 10(7): 529-539.
London, E. (1992). "Diphtheria toxin: membrane interaction and membrane translocation." Biochim Biophys Acta 1113(1): 25-51.
Lorieau, J. L., J. M. Louis and A. Bax (2010). "The complete influenza hemagglutinin fusion domain adopts a tight helical hairpin arrangement at the lipid:water interface." Proc Natl Acad Sci U S A 107(25): 11341-11346.
Lu, S. J., Q. Feng, Y. Ivanova, C. Luo, T. Li, F. Li, G. R. Honig and R. Lanza (2007). "Recombinant HoxB4 fusion proteins enhance hematopoietic differentiation of human embryonic stem cells." Stem Cells Dev 16(4): 547-559.
Luan et al., (2015). "Peptide amphiphiles with multifunctional fragments promoting cellular uptake and endosomal escape as efficient gene vectors." J. Mater. Chem. B, 3: 1068-1078.
Mack, M., B. Luckow, P. J. Nelson, J. Cihak, G. Simmons, P. R. Clapham, N. Signoret, M. Marsh, M. Stangassinger, F. Borlat, T. N. Wells, D. Schlondorff and A. E. Proudfoot (1998). "Aminooxypentane-RANTES induces CCR5 internalization but inhibits recycling: a novel inhibitory mechanism of HIV infectivity." J Exp Med 187(8): 1215-1224.
Maeng, C. H., J. H. Yi, J. Lee, J. Y. Hong, M. K. Choi, H. A. Jung, J. O. Park, S. H. Park, Y. S. Park, W. K. Kang and H. Y. Lim (2013). "Effects of single nucleotide polymorphisms on treatment outcomes and toxicity in patients treated with sunitinib." Anticancer Res 33(10): 4619-4626.
Mahlum, E., D. Mandal, C. Halder, A. Maran, M. J. Yaszemski, R. B. Jenkins, M. E. Bolander and G. Sarkar (2007). "Engineering a noncarrier to a highly efficient carrier peptide for noncovalently delivering biologically active proteins into human cells." Anal Biochem 365(2): 215-221.
Makarova et al., (2011). "Evolution and classification of the CRISPR-Cas systems." Nat Rev Microbiol. 9(6): 467-477.
Makkerh, J. P., C. Dingwall and R. A. Laskey (1996). "Comparative mutagenesis of nuclear localization signals reveals the importance of neutral and acidic amino acids." Curr Biol 6(8): 1025-1027.
Martinez-Fong, D., I. Navarro-Quiroga, I. Ochoa, I. Alvarez-Maya, M. A. Meraz, J. Luna and J. A. Arias-Montano (1999). "Neurotensin-SPDP-poly-L-lysine conjugate: a nonviral vector for targeted gene delivery to neural cells." Brain Res Mol Brain Res 69(2): 249-262.
Maurer, M. and E. von Stebut (2004). "Macrophage inflammatory protein-1." Int J Biochem Cell Biol 36(10): 1882-1886.
McKay, T., P. Reynolds, S. Jezzard, D. Curiel and C. Coutelle (2002). "Secretin-mediated gene delivery, a specific targeting mechanism with potential for treatment of biliary and pancreatic disease in cystic fibrosis." Mol Ther 5(4): 447-454.
Midoux, P., A. Kichler, V. Boutin, J. C. Maurizot and M. Monsigny (1998). "Membrane permeabilization and efficient gene transfer by a peptide containing several histidines." Bioconjug Chem 9(2): 260-267.
Milenkovic, D., T. Ramming, J. M. Muller, L. S. Wenz, N. Gebert, A. Schulze-Specking, D. Stojanovski, S. Rospert and A. Chacinska (2009). "Identification of the signal directing Tim9 and Tim10 into the intermembrane space of mitochondria." Mol Biol Cell 20(10): 2530-2539.
Miyoshi, I., N. Kasai and Y. Hayashizaki (1994). "[Structure and regulation of human thyroid-stimulating hormone (TSH) gene]." Nihon Rinsho 52(4): 940-947.
Moede, T., B. Leibiger, H. G. Pour, P. Berggren and I. B. Leibiger (1999). "Identification of a nuclear localization signal, RRMKWKK, in the homeodomain transcription factor PDX-1." FEBS Lett 461(3): 229-234.
Montrose, K., Y. Yang, X. Sun, S. Wiles and G. W. Krissansen (2013). "Xentry, a new class of cell-penetrating peptide uniquely equipped for delivery of drugs." Sci Rep 3: 1661.
Moreland, R. B., G. L. Langevin, R. H. Singer, R. L. Garcea and L. M. Hereford (1987). "Amino acid sequences that determine the nuclear localization of yeast histone 2B." Mol Cell Biol 7(11): 4048-4057.
Morris, M. C., L. Chaloin, M. Choob, J. Archdeacon, F. Heitz and G. Divita (2004). "Combination of a new generation of PNAs with a peptide-based carrier enables efficient targeting of cell cycle progression." Gene Ther 11(9): 757-764.
Morris, M. C., J. Depollier, J. Mery, F. Heitz and G. Divita (2001). "A peptide carrier for the delivery of biologically active proteins into mammalian cells." Nat Biotechnol 19(12): 1173-1176.
Nakanishi, A., D. Shum, H. Morioka, E. Otsuka and H. Kasamatsu (2002). "Interaction of the Vp3 nuclear localization signal with the importin alpha 2/beta heterodimer directs nuclear entry of infecting simian virus 40." J Virol 76(18): 9368-9377.
O'Keefe, D. O. (1992). "Characterization of a full-length, active-site mutant of diphtheria toxin." Arch Biochem Biophys 296(2): 678-684.
Parente, R. A., S. Nir and F. C. Szoka, Jr. (1990). "Mechanism of leakage of phospholipid vesicle contents induced by the peptide GALA." Biochemistry 29(37): 8720-8728.
Paul, R. W., K. E. Weisser, A. Loomis, D. L. Sloane, D. LaFoe, E. M. Atkinson and R. W. Overell (1997). "Gene transfer using a novel fusion protein, GAL4/invasin." Hum Gene Ther 8(10): 1253-1262.
Perez, F., A. Joliot, E. Bloch-Gallego, A. Zahraoui, A. Triller and A. Prochiantz (1992). "Antennapedia homeobox as a signal for the cellular internalization and nuclear addressing of a small exogenous peptide." J Cell Sci 102 (Pt 4): 717-722.
Pimenta, D. C., V. C. Chen, J. Chao, M. A. Juliano and L. Juliano (2000). "Alpha1-antichymotrypsin and kallistatin hydrolysis by human cathepsin D." J Protein Chem 19(5): 411-418.
Prieve, M. G. and M. L. Waterman (1999). "Nuclear localization and formation of beta-catenin-lymphoid enhancer factor 1 complexes are not sufficient for activation of gene expression." Mol Cell Biol 19(6): 4503-4515.
Rajagopalan, R., J. Xavier, N. Rangaraj, N. M. Rao and V. Gopal (2007). "Recombinant fusion proteins TAT-Mu, Mu and Mu-Mu mediate efficient non-viral gene delivery." J Gene Med 9(4): 275-286.
Riddell et al., (2014) "Reprogramming committed murine blood cells to induced hematopoietic stem cells with defined factors". Cell, 157: 549-564
Rodolphe Barrangou and Luciano A. Marraffini (2014). "CRISPR-Cas Systems: Prokaryotes Upgrade to Adaptive Immunity". Cell Volume 54, Issue 2, p234-244, 24 April 2014.
Salomone, F., F. Cardarelli, M. Di Luca, C. Boccardi, R. Nifosi, G. Bardi, L. Di Bari, M. Serresi and F. Beltram (2012). "A novel chimeric cell-penetrating peptide with membrane-disruptive properties for efficient endosomal escape." J Control Release 163(3): 293-303.
Salomone F., Cardarelli F, Signore G, Boccardi C, Beltram F. (2013) "In vitro efficient transfection by CM18-Tat11 hybrid peptide: a new tool for gene-delivery applications." PLoS One. 8(7):e70108.
Schneider, H., R. P. Harbottle, Y. Yokosaki, J. Kunde, D. Sheppard and C. Coutelle (1998). "A novel peptide, PLAEIDGIELTY, for the targeting of alpha9beta1-integrins." FEBS Lett 429(3): 269-273.
Schreiber, V., M. Molinete, H. Boeuf, G. de Murcia and J. Menissier-de Murcia (1992). "The human poly(ADP-ribose) polymerase nuclear localization signal is a bipartite element functionally separate from DNA binding and catalytic activity." EMBO J 11(9): 3263-3269.
Schuster, M. J., G. Y. Wu, C. M. Walton and C. H. Wu (1999). "Multicomponent DNA carrier with a vesicular stomatitis virus G-peptide greatly enhances liver-targeted gene expression in mice." Bioconjug Chem 10(6): 1075-1083.
Scott, M. S., F. M. Boisvert, M. D. McDowall, A. I. Lamond and G. J. Barton (2010). "Characterization and prediction of protein nucleolar localization sequences." Nucleic Acids Res 38(21): 7388-7399.
Shaw, P. A., I. R. Catchpole, C. A. Goddard and W. H. Colledge (2008). "Comparison of protein transduction domains in mediating cell delivery of a secreted CRE protein." Biochemistry 47(4): 1157-1166.
Shoya, Y., T. Kobayashi, T. Koda, K. Ikuta, M. Kakinuma and M. Kishi (1998). "Two proline-rich nuclear localization signals in the amino- and carboxyl-terminal regions of the Boma disease virus phosphoprotein." J Virol 72(12):9755-9762.
Somasekaram, A., A. Jarmuz, A. How, J. Scott and N. Navaratnam (1999). "Intracellular localization of human cytidine deaminase. Identification of a functional nuclear localization signal." J Biol Chem 274(40): 28405-28412.
Stojanovski, D., M. Bohnert, N. Pfanner and M. van der Laan (2012). "Mechanisms of protein sorting in mitochondria." Cold Spring Harb Perspect Biol 4(10).
Sudbeck, P. and G. Scherer (1997). "Two independent nuclear localization signals are present in the DNA-binding high-mobility group domains of SRY and SOX9." J Biol Chem 272(44): 27848-27852.
Sung, M. S., J. Y. Mun, O. Kwon, K. S. Kwon and D. B. Oh (2013). "Efficient myogenic differentiation of human adipose-derived stem cells by the transduction of engineered MyoD protein." Biochem Biophys Res Commun 437(1): 156-161.
Takahashi, K. and S. Yamanaka (2006). "Induction of pluripotent stem cells from mouse embryonic and adult fibroblast cultures by defined factors." Cell 126(4): 663-676.
Takeda, A., C. Goolsby and N. R. Yaseen (2006). "NUP98-HOXA9 induces long-term proliferation and blocks differentiation of primary human CD34+ hematopoietic cells." Cancer Res 66(13): 6628-6637.
Tan, Y., Z. Xie, M. Ding, Z. Wang, Q. Yu, L. Meng, H. Zhu, X. Huang, L. Yu, X. Meng and Y. Chen (2010). "Increased levels of FoxA1 transcription factor in pluripotent P19 embryonal carcinoma cells stimulate neural differentiation." Stem Cells Dev 19(9): 1365-1374.
Tan, Y. X., C. Chen, Y. L. Wang, S. Lin, Y. Wang, S. B. Li, X. P. Jin, H. W. Gao, F. S. Du, F. Gong and S. P. Ji (2012). "Truncated peptides from melittin and its analog with high lytic activity at endosomal pH enhance branched polyethylenimine-mediated gene transfection." J Gene Med 14(4): 241-250.
Uherek, C., J. Fominaya and W. Wels (1998). "A modular DNA carrier protein based on the structure of diphtheria toxin mediates target cell-specific gene delivery." J Biol Chem 273(15): 8835-8841.
Varkouhi, A. K., M. Scholte, G. Storm and H. J. Haisma (2011). "Endosomal escape pathways for delivery of biologicals." J Control Release 151(3): 220-228.
Veach, R. A., D. Liu, S. Yao, Y. Chen, X. Y. Liu, S. Downs and J. Hawiger (2004). "Receptor/transporter-independent targeting of functional peptides across the plasma membrane." J Biol Chem 279(12): 11425-11431.
Vives, E., P. Brodin and B. Lebleu (1997). "A truncated HIV-1 Tat protein basic domain rapidly translocates through the plasma membrane and accumulates in the cell nucleus." J Biol Chem 272(25): 16010-16017.
Wagstaff, K. M., D. J. Glover, D. J. Tremethick and D. A. Jans (2007). "Histone-mediated transduction as an efficient means for gene delivery." Mol Ther 15(4): 721-731.
Warr, M. R., M. Binnewies, J. Flach, D. Reynaud, T. Garg, R. Malhotra, J. Debnath and E. Passegue (2013). "FOXO3A directs a protective autophagy program in haematopoietic stem cells." Nature 494(7437): 323-327.
Welch, K., J. Franke, M. Kohler and I. G. Macara (1999). "RanBP3 contains an unusual nuclear localization signal that is imported preferentially by importin-alpha3." Mol Cell Biol 19(12): 8400-8411.
Wiedenheft et al., (2011). RNA-guided complex from a bacterial immune system enhances target recognition through seed sequence interactions. Proc. Natl. Acad. Sci. USA 108, 10092-10097.
Witzel, I., M. Graeser, T. Kam, M. Schmidt, R. Wirtz, D. Schutze, A. Rausch, F. Janicke, K. Milde-Langosch and V. Muller (2013). "Androgen receptor expression is a predictive marker in chemotherapy-treated patients with endocrine receptor-positive primary breast cancers." J Cancer Res Clin Oncol 139(5): 809-816.
Wu, J., L. Zhou, K. Tonissen, R. Tee and K. Artzt (1999). "The quaking I-5 protein (QKI-5) has a novel nuclear localization signal and shuttles between the nucleus and the cytoplasm." J Biol Chem 274(41): 29202-29210.
Wyman, T. B., F. Nicol, O. Zelphati, P. V. Scaria, C. Plank and F. C. Szoka, Jr. (1997). "Design, synthesis, and characterization of a cationic peptide that binds to nucleic acids and permeabilizes bilayers." Biochemistry 36(10): 3008-3017.
Yu, Z., C. H. Lee, C. Chinpaisal and L. N. Wei (1998). "A constitutive nuclear localization signal from the second zinc-finger of orphan nuclear receptor TR2." J Endocrinol 159(1): 53-60.
Zetsche et al., (2015). "Cpf1 Is a Single RNA-Guided Endonuclease of a Class 2 CRISPR-Cas System". Cell. 25. pii: S0092-8674(15)01200-3 [http://dx.doi.org/10.1016/j.cell.2015.09.038].
Zhang, J. K., J. Cui, Y. J. Lv and H. Yan (2006). "[Analysis of reasons for eye trauma in construction workers and visual acuity after treatment]." Zhonghua Lao Dong Wei Sheng Zhi Ye Bing Za Zhi 24(6): 371-372.
Zhou, H., S. Wu, J. Y. Joo, S. Zhu, D. W. Han, T. Lin, S. Trauger, G. Bien, S. Yao, Y. Zhu, G. Siuzdak, H. R. Scholer, L. Duan and S. Ding (2009). "Generation of induced pluripotent stem cells using recombinant proteins." Cell Stem Cell 4(5): 381-384.

## Claims

1. A polypeptide-based shuttle agent comprising an endosome leakage domain (ELD) operably linked to a cell penetrating domain (CPD), or an ELD operably linked to a histidine-rich domain and a CPD, for use in a clinical or therapeutic *in vivo* method for increasing the transduction efficiency of an independent polypeptide cargo to the cytosol of target eukaryotic cells, wherein the cargo is a biologically active polypeptide, and wherein the polypeptide-based shuttle agent has a predicted net charge of at least +6 at physiological pH and is used at a concentration sufficient to increase the transduction efficiency of the independent polypeptide cargo to the cytosol of target eukaryotic cell.

2. An *in vitro* method for increasing the transduction efficiency of an independent polypeptide cargo to the cytosol of target eukaryotic cells, the method comprising contacting the target eukaryotic cells with a polypeptide-based shuttle agent comprising an endosome leakage domain (ELD) operably linked to a cell penetrating domain (CPD), or an ELD operably linked to a histidine-rich domain and a CPD, wherein the polypeptide-based shuttle agent has a predicted net charge of at least +6 at physiological pH and is used at a concentration sufficient to increase the transduction efficiency of the independent polypeptide cargo to the cytosol of target eukaryotic cell.

3. The polypeptide-based shuttle agent for the use of claim 1, or the *in vitro* method of claim 2, wherein the polypeptide-based shuttle agent:
(a) comprises a minimum length of 20, 21, 22, 23, 24, 25, 26, 27, 28, 29, or 30 amino acid residues and a maximum length of 35, 40, 45, 50, 55, 65, 70, 75, 80, 85, 90, 95, 100, 105, 110, 115, 120, 125, 130, 135, 140, 145, or 150 amino acid residues;
(b) has a predicted net charge of at least +7, +8, +9, +10, +11, +12, +13, +14, or +15 at physiological pH;
(c) is soluble in aqueous solution; or
(d) any combination of (a) to (c).

4. The polypeptide-based shuttle agent for the use of claim 1 or 3, or the *in vitro* method of claim 2 or 3, wherein:
(a) said ELD is or is from: an endosomolytic peptide; an antimicrobial peptide (AMP); a linear cationic alpha-helical antimicrobial peptide; a Cecropin-A/Melittin hybrid (CM) peptide; pH-dependent membrane active peptide (PAMP); a peptide amphiphile; a peptide derived from the N terminus of the HA2 subunit of influenza hemagglutinin (HA); CM18; Diphtheria toxin T domain (DT); GALA; PEA; INF-7; LAH4; HGP; H5WYG; HA2; EB1; VSVG; Pseudomonas toxin; melittin; KALA; JST-1; C(LLKK)₃C; G(LLKK)₃G; or any combination thereof;
(b) said CPD is or is from: a cell-penetrating peptide or the protein transduction domain from a cell-penetrating peptide; TAT; PTD4; Penetratin (Antennapedia); pVEC; M918; Pep-1; Pep-2; Xentry; arginine stretch; transportan; SynB1; SynB3; or any combination thereof;
(c) said histidine-rich domain is a stretch of at least 3, at least 4, at least 5, or at least 6 amino acids comprising at least 50%, at least 55%, at least 60%, at least 65%, at least 70%, at least 75%, at least 80%, at least 85%, or at least 90% histidine residues; and/or comprises at least 2, at least 3, at least 4, at least 5, at least 6, at least 7, at least 8, or at least 9 consecutive histidine residues; or
(d) any combination of (a) to (c).

5. The polypeptide-based shuttle agent for the use of claim 1, 3 or 4, or the *in vitro* method of any one of claims 2 to 4, wherein the polypeptide-based shuttle agent comprises:
(a) an ELD comprising the amino acid sequence of any one of SEQ ID NOs: 1-15, 63, 64, or a variant or fragment thereof having endosomolytic activity;
(b) a CPD comprising the amino acid sequence of any one of SEQ ID NOs: 16-27 or 65, or a variant or fragment thereof having cell penetrating activity;
(c) a histidine-rich domain having at least 2, at least 3, at least 4, at least 5, or at least 6 consecutive histidine residues; or
(d) any combination of (a) to (c).

6. The polypeptide-based shuttle agent for the use of any one of claims 1 and 3 to 5, or the *in vitro* method of any one of claims 2 to 5, wherein said domains are operably linked via one or more linker domains.

7. The polypeptide-based shuttle agent for the use of any one of claims 1 and 3 to 6, or the *in vitro* method of any one of claims 2 to 6, wherein the polypeptide-based shuttle agent comprises:
(a) an ELD which is CM18, KALA, or C(LLKK)₃C having the amino acid sequence of SEQ ID NO: 1, 14, or 63, or a variant thereof having at least 85%, 90%, or 95% identity to SEQ ID NO: 1, 14, or 63, and having endosomolytic activity;
(b) a CPD which is TAT or PTD4 having the amino acid sequence of SEQ ID NO: 17 or 65, or a variant thereof having at least 85%, 90%, or 95% identity to SEQ ID NO: 17 or 65 and having cell penetrating activity; or Penetratin having the amino acid sequence of SEQ ID NO: 18, or a variant thereof having at least 85%, 90%, or 95% identity to SEQ ID NO: 18 and having cell penetrating activity;
(c) a histidine-rich domain comprising at least 6 consecutive histidine residues; or
(d) any combination of (a) to (c).

8. The polypeptide-based shuttle agent for the use of any one of claims 1 and 3 to 7, or the *in vitro* method of any one of claims 2 to 7, wherein the polypeptide-based shuttle agent comprises or consists of the amino acid sequence of any one of SEQ ID NOs: 57-59, 66-72, or 82-102, or a functional variant thereof having at least 85%, 90%, or 95% identity to any one of SEQ ID NOs: 10 57-59, 66-72, or 82-102.

9. The polypeptide-based shuttle agent for the use of any one of claims 1 and 3 to 8, or the *in vitro* method of any one of claims 2 to 8, wherein said independent polypeptide cargo:
(a) lacks a CPD or a CPD as defined in claim 4(b);
(b) is a recombinant protein;
(c) comprises a subcellular targeting domain;
(d) is complexed with a DNA and/or RNA molecule; or
(e) any combination of (a) to (d).

10. The polypeptide-based shuttle agent for the use of claim 9, or the *in vitro* method of claim 9, wherein said subcellular targeting domain is:
(a) a nuclear localization signal (NLS), optionally from: Ela, T-Ag, c-myc, T-Ag, op-T-NLS, Vp3, nucleoplasmin, histone 2B, Xenopus N1, PARP, PDX-1, QKI-5, HCDA, H2B, v-Rel, Amida, RanBP3, Pho4p, LEF-1, TCF-1, BDV-P, TR2, SOX9, or Max;
(b) a nucleolar signal sequence, optionally from BIRC5 or RECQL4;
(c) a mitochondrial signal sequence, optionally from Tim9 or Yeast cytochrome c oxidase subunit IV; or
(d) a peroxisome signal sequence, optionally from PTS1.

11. The polypeptide-based shuttle agent for the use of any one of claims 1 and 3 to 10, or the *in vitro* method of any one of claims 2 to 10, wherein said polypeptide cargo is a transcription factor, a nuclease, a cytokine, a hormone, a growth factor, or an antibody.

12. The polypeptide-based shuttle agent for the use of claim 11, or the *in vitro* method of claim 11, wherein:
(a) said transcription factor is: HOXB4, NUP98-HOXA9, Oct3/4, Sox2, Sox9, Klf4, c-Myc, MyoD, Pdxl, Ngn3, MafA, Blimp-1, Eomes, T-bet, FOXO3A, NF-YA, SALL4, ISL1, FoxA1, Nanog, Esrrb, Lin28, HIF1-alpha, Hlf, Runxltl, Pbx1, Lmo2, Zfp37, Prdm5, Bcl-6, or any combination thereof; and/or
(b) said nuclease is: an RNA-guided endonuclease, a CRISPR endonuclease, a type I CRISPR endonuclease, a type II CRISPR endonuclease, a type III CRISPR endonuclease, a type IV CRISPR endonuclease, a type V CRISPR endonuclease, a type VI CRISPR endonuclease, CRISPR associated protein 9 (Cas9), Cpfl, a zinc-finger nuclease (ZFNs), a Transcription activator-like effector nucleases (TALENs), a homing endonuclease, a meganuclease, or any combination thereof.

13. The polypeptide-based shuttle agent for the use of any one of claims 1 and 3 to 12, or the *in vitro* method of any one of claims 2 to 12, for increasing the transduction efficiency of a polypeptide cargo to the cytosol of target eukaryotic cells intended for use in cell therapy, genome editing, adoptive cell transfer, and/or regenerative medicine.

14. The polypeptide-based shuttle agent for the use of any one of claims 1 and 3 to 13, or the *in vitro* method of any one of claims 2 to 13, wherein said target eukaryotic cells are animal cells, mammalian cells, human cells, stem cells, primary cells, immune cells, T cells, or dendritic cells.

15. A synthetic peptide comprising an endosome leakage domain (ELD) operably linked to a cell penetrating domain (CPD), or an ELD operably linked to a histidine-rich domain and a CPD, wherein the synthetic peptide is the polypeptide-based shuttle agent is as defined in any one of claims 3 to 8, and wherein the CPD is or is from: PTD4; Penetratin (Antennapedia); pVEC; M918; Pep-1; Pep-2; Xentry; arginine stretch; transportan; SynB1; SynB3; or any combination thereof, and wherein the polypeptide-based shuttle agent has a predicted net charge of at least +6 at physiological pH.

## Patentansprüche

1. Shuttle-Mittel auf Polypeptidbasis, umfassend eine Endosomenleckdomäne (endosome leakage domain - ELD), die funktionsfähig mit einer zellpenetrierenden Domäne (cell penetrating domain - CPD) verbunden ist, oder eine ELD, die funktionsfähig mit einer histidinreichen Domäne und einer CPD verbunden ist, zur Verwendung in einem klinischen oder therapeutischen *In-vivo*-Verfahren zur Erhöhung der Transduktionseffizienz einer unabhängigen Polypeptidladung zum Cytosol von eukaryotischen Zielzellen, wobei die Ladung ein biologisch aktives Polypeptid ist und wobei das Shuttle-Mittel auf Polypeptidbasis eine vorhergesagte Nettoladung von mindestens +6 bei physiologischem pH aufweist und in einer Konzentration verwendet wird, die ausreicht, um die Transduktionseffizienz der unabhängigen Polypeptidladung zum Cytosol der eukaryotischen Zielzelle zu erhöhen.

2. *In-vitro*-Verfahren zur Erhöhung der Transduktionseffizienz einer unabhängigen Polypeptidladung zum Cytosol von eukaryotischen Zielzellen, wobei das Verfahren das Inkontaktbringen der eukaryotischen Zielzellen mit einem Shuttle-Mittel auf Polypeptidbasis umfasst, das eine Endosomenleckdomäne (ELD), die funktionsfähig mit einer zellpenetrierenden Domäne (CPD) verbunden ist, oder eine ELD, die funktionsfähig mit einer histidinreichen Domäne und einer CPD verbunden ist, umfasst, wobei das Shuttle-Mittel auf Polypeptidbasis eine vorhergesagte Nettoladung von mindestens +6 bei physiologischem pH aufweist und in einer Konzentration verwendet wird, die ausreicht, um die Transduktionseffizienz der unabhängigen Polypeptidladung zum Cytosol der eukaryotischen Zielzelle zu erhöhen.

3. Shuttle-Mittel auf Polypeptidbasis zur Verwendung nach Anspruch 1 oder *In-vitro-*Verfahren nach Anspruch 2, wobei das Shuttle-Mittel auf Polypeptidbasis:
(a) eine Mindestlänge von 20, 21, 22, 23, 24, 25, 26, 27, 28, 29 oder 30 Aminosäureresten und eine Höchstlänge von 35, 40, 45, 50, 55, 65, 70, 75 80, 85, 90, 95, 100, 105, 110, 115, 120, 125, 130, 135, 140, 145 oder 150 Aminosäureresten umfasst;
(b) eine vorhergesagte Nettoladung von mindestens +7, +8, +9, +10, +11, +12, +13, +14 oder +15 bei physiologischem pH aufweist;
(c) in wässriger Lösung löslich ist; oder
(d) eine beliebige Kombination von (a) bis (c) ist.

4. Shuttle-Mittel auf Polypeptidbasis zur Verwendung nach Anspruch 1 oder 3 oder *In-vitro-*Verfahren nach Anspruch 2 oder 3, wobei:
(a) die ELD Folgendes ist oder davon stammt: ein endosomolytisches Peptid; ein antimikrobielles Peptid (AMP); ein lineares kationisches alpha-helikales antimikrobielles Peptid; ein Cecropin-A/Melittin-Hybrid(CM)-Peptid; pH-abhängiges membranaktives Peptid (PAMP); ein Peptidamphiphil; ein von dem N-Terminus der HA2-Untereinheit von Influenza-Hämagglutinin (HA) abgeleitetes Peptid; CM18
Diphtherietoxin-T-Domäne (DT); GALA; PEA; INF-7; LAH4; HGP; H5WYG; HA2; EB1;
VSVG; Pseudomonas-Toxin; Melittin; KALA; JST-1; C(LLKK)₃C; G(LLKK)₃G; oder eine beliebige Kombination davon;
(b) die CPD Folgendes ist oder davon stamm: ein zellpenetrierendes Peptid oder die Proteintransduktionsdomäne von einem zellpenetrierenden Peptid; TAT; PTD4; Penetratin (Antennapedia); pVEC; M918 Pep-1 Pep-2; Xentry; Arginin-Abschnitt; Transportan; SynB1; SynB3; oder eine beliebige Kombination davon;
(c) die histidinreiche Domäne ein Abschnitt von mindestens 3, mindestens 4, mindestens 5 oder mindestens 6 Aminosäuren ist, der mindestens 50 %, mindestens 55 %, mindestens 60 %, mindestens 65 %, mindestens 70 %, mindestens 75 %, mindestens 80 %, mindestens 85 % oder mindestens 90 % Histidinreste umfasst; und/oder mindestens 2, mindestens 3, mindestens 4, mindestens 5, mindestens 6, mindestens 7, mindestens 8 oder mindestens 9 aufeinanderfolgende Histidinreste umfasst; oder
(d) eine beliebige Kombination von (a) bis (c).

5. Shuttle-Mittel auf Polypeptidbasis zur Verwendung nach Anspruch 1, 3 oder 4 oder *In-*vitro-Verfahren nach einem der Ansprüche 2 bis 4, wobei das Shuttle-Mittel auf Polypeptidbasis Folgendes umfasst:
(a) eine ELD, umfassend die Aminosäuresequenz einer beliebigen der SEQ ID NOs: 1-15, 63, 64 oder eine Variante oder ein Fragment davon mit endosomolytischer Aktivität;
(b) eine CPD, umfassend die Aminosäuresequenz einer beliebigen der SEQ ID NOs: 16-27 oder 65 oder eine Variante oder ein Fragment davon mit zellpenetrierender Aktivität;
(c) eine histidinreiche Domäne mit mindestens 2, mindestens 3, mindestens 4, mindestens 5 oder mindestens 6 aufeinanderfolgenden Histidinresten; oder
(d) eine beliebige Kombination von (a) bis (c).

6. Shuttle-Mittel auf Polypeptidbasis zur Verwendung nach einem der Ansprüche 1 und 3 bis 5 oder *In-vitro*-Verfahren nach einem der Ansprüche 2 bis 5, wobei die Domänen funktionsfähig über eine oder mehrere Linkerdomänen verbunden sind.

7. Shuttle-Mittel auf Polypeptidbasis zur Verwendung nach einem der Ansprüche 1 und 3 bis 6 oder *In-vitro*-Verfahren nach einem der Ansprüche 2 bis 6, wobei das Shuttle-Mittel auf Polypeptidbasis Folgendes umfasst:
(a) eine ELD, die CM18, KALA oder C (LLKK)₃C ist, mit der Aminosäuresequenz von SEQ ID NO: 1, 14 oder 63 oder eine Variante davon mit mindestens 85 %, 90 % oder 95 % Identität zu SEQ ID NO: 1, 14 oder 63 und mit endosomolytischer Aktivität;
(b) eine CPD, die TAT oder PTD4 ist, mit der Aminosäuresequenz von SEQ ID NO: 17 oder 65 oder eine Variante davon mit mindestens 85 %, 90 % oder 95 % Identität zu SEQ ID NO: 17 oder 65 und mit zellpenetrierender Aktivität; oder Penetratin mit der Aminosäuresequenz von SEQ ID NO: 18 oder eine Variante davon mit mindestens 85 %, 90 % oder 95 % Identität zu SEQ ID NO: 18 und mit zellpenetrierender Aktivität;
(c) eine histidinreiche Domäne, umfassend mindestens 6 aufeinanderfolgende Histidinreste; oder
(d) eine beliebige Kombination von (a) bis (c).

8. Shuttle-Mittel auf Polypeptidbasis zur Verwendung nach einem der Ansprüche 1 und 3 bis 7 oder *In-vitro*-Verfahren nach einem der Ansprüche 2 bis 7, wobei das Shuttle-Mittel auf Polypeptidbasis die Aminosäuresequenz einer beliebigen der SEQ ID NOs: 57-59, 66-72 oder 82-102 oder eine funktionelle Variante davon mit mindestens 85 %, 90 % oder 95 % Identität zu einer der SEQ ID NOs: 10 57-59, 66-72 oder 82-102 umfasst oder daraus besteht.

9. Shuttle-Mittel auf Polypeptidbasis zur Verwendung nach einem der Ansprüche 1 und 3 bis 8 oder *In-vitro*-Verfahren nach einem der Ansprüche 2 bis 8, wobei die unabhängige Polypeptidladung:
(a) keine CPD oder CPD aufweist, wie in Anspruch 4 (b) definiert;
(b) ein rekombinantes Protein ist;
(c) eine subzelluläre Targeting-Domäne umfasst;
(d) mit einem DNA- und/oder RNA-Molekül komplexiert ist; oder
(e) eine beliebige Kombination von (a) bis (d) ist.

10. Shuttle-Mittel auf Polypeptidbasis zur Verwendung nach Anspruch 9 oder *In-vitro-*Verfahren nach Anspruch 9, wobei die subzelluläre Targeting-Domäne Folgendes ist:
(a) ein Kernlokalisierungssignal (nuclear localization signal - NLS), optional von: Ela, T-Ag, c-myc, T-Ag, op-T-NLS, Vp3, Nucleoplasmin, Histon 2B, Xenopus N1, PARP, PDX-1, QKI-5, HCDA, H2B, v-Rel, Amida, RanBP3, Pho4p, LEF-1, TCF-1, BDV-P, TR2, SOX9 oder Max;
(b) eine nukleolare Signalsequenz, optional von BIRC5 oder RECQL4;
(c) eine mitochondriale Signalsequenz, gegebenenfalls aus der Tim9- oder Hefe-Cytochrom-c-Oxidase-Untereinheit IV; oder
(d) eine Peroxisomensignalsequenz, optional von PTS1.

11. Shuttle-Mittel auf Polypeptidbasis zur Verwendung nach einem der Ansprüche 1 und 3 bis 10 oder *In-vitro*-Verfahren nach einem der Ansprüche 2 bis 10, wobei die Polypeptidladung ein Transkriptionsfaktor, eine Nuklease, ein Cytokin, ein Hormon, ein Wachstumsfaktor oder ein Antikörper ist.

12. Shuttle-Mittel auf Polypeptidbasis zur Verwendung nach Anspruch 11 oder *In-vitro-*Verfahren nach Anspruch 11, wobei:
(a) der Transkriptionsfaktor Folgendes ist: HOXB4, NUP98-HOXA9, Oct3/4, Sox2, Sox9, Klf4, c-Myc, MyoD, Pdx1, Ngn3, MafA, Blimp-1, Eomes, T-bet, FOXO3A, NF-YA, SALL4, ISL1, FoxA1, Nanog, Esrrb, Lin28, HIF1-alpha, Hlf, Runx1t1, Pbx1, Lmo2, Zfp37, Prdm5, Bcl-6 oder eine beliebige Kombination davon; und/oder
(b) die Nuklease Folgendes ist: eine RNA-gesteuerte Endonuklease, eine CRISPR-Endonuklease, eine CRISPR-Endonuklease vom Typ I, eine CRISPR-Endonuklease vom Typ II, eine CRISPR-Endonuklease vom Typ III, eine CRISPR-Endonuklease vom Typ IV, eine CRISPR-Endonuklease vom Typ V, eine CRISPR-Endonuklease vom Typ VI, CRISPR-assoziiertes Protein 9 (Cas9), Cpfl, eine Zinkfinger-Nuklease (ZFNs), TALENs (Transcription Activator-like Effector Nucleases), eine Homing-Endonuklease, eine Meganuklease oder eine beliebige Kombination davon.

13. Shuttle-Mittel auf Polypeptidbasis zur Verwendung nach einem der Ansprüche 1 und 3 bis 12 oder *In-vitro*-Verfahren nach einem der Ansprüche 2 bis 12 zur Erhöhung der Transduktionseffizienz einer Polypeptidladung zum Cytosol von eukaryotischen Zielzellen, das zur Verwendung bei Zelltherapie, Genomeditierung, adoptivem Zelltransfer und/oder regenerativer Medizin bestimmt ist.

14. Shuttle-Mittel auf Polypeptidbasis zur Verwendung nach einem der Ansprüche 1 und 3 bis 13 oder *In-vitro*-Verfahren nach einem der Ansprüche 2 bis 13, wobei die eukaryotischen Zielzellen tierische Zellen, Säugetierzellen, menschliche Zellen, Stammzellen, Primärzellen, Immunzellen, T-Zellen oder dendritische Zellen sind.

15. Synthetisches Peptid, umfassend eine Endosomenleckdomäne (ELD), die funktionsfähig mit einer zellpenetrierenden Domäne (CPD) verbunden ist, oder eine ELD, die funktionsfähig mit einer histidinreichen Domäne und einer CPD verbunden ist, wobei das synthetische Peptid das auf Polypeptid basierende Shuttle-Mittel ist, wie in einem der Ansprüche 3 bis 8 definiert, wobei die CPD Folgendes ist oder davon stammt: PTD4; Penetratin (Antennapedia); pVEC; M918 Pep-1 Pep-2; Xentry; Arginin-Abschnitt; Transportan; SynB1; SynB3; oder eine beliebige Kombination davon, und wobei das Shuttle-Mittel auf Polypeptidbasis eine vorhergesagte Nettoladung von mindestens +6 bei physiologischem pH aufweist.

## Revendications

1. Agent navette à base de polypeptide comprenant un domaine de fuite d'endosome (ELD) lié de manière opérationnelle à un domaine de pénétration cellulaire (CPD), ou un ELD lié de manière opérationnelle à un domaine riche en histidine et à un CPD, destiné à être utilisé dans un procédé clinique ou thérapeutique in vivo pour augmenter l'efficacité de transduction d'une cargaison polypeptidique indépendante vers le cytosol de cellules eucaryotes cibles, dans lequel la cargaison est un polypeptide biologiquement actif, et dans lequel l'agent navette à base de polypeptide a une charge nette estimée d'au moins +6 au pH physiologique et est utilisé à une concentration suffisante pour augmenter l'efficacité de transduction de la cargaison polypeptidique indépendante vers le cytosol de la cellule eucaryote cible.

2. Procédé in vitro destiné à augmenter l'efficacité de transduction d'une cargaison polypeptidique indépendante vers le cytosol de cellules eucaryotes cibles, le procédé comprenant la mise en contact des cellules eucaryotes cibles avec un agent navette à base de polypeptide comprenant un domaine de fuite d'endosome (ELD) lié de manière opérationnelle à un domaine de pénétration cellulaire (CPD), ou un ELD lié de manière opérationnelle à un domaine riche en histidine et à un CPD, dans lequel l'agent navette à base de polypeptide a une charge nette estimée d'au moins +6 au pH physiologique et est utilisé à une concentration suffisante pour augmenter l'efficacité de transduction de la cargaison polypeptidique indépendante vers le cytosol de la cellule eucaryote cible.

3. Agent navette à base de polypeptide destiné à être utilisé selon la revendication 1, ou procédé in vitro selon la revendication 2, dans lequel l'agent navette à base de polypeptide :
(a) comprend une longueur minimale de 20, 21, 22, 23, 24, 25, 26, 27, 28, 29, ou 30 résidus d'acides aminés et une longueur maximale 35, 40, 45, 50, 55, 65, 70, 75, 80, 85, 90, 95, 100, 105, 110, 115, 120, 125, 130, 135, 140, 145, ou 150 résidus d'acides aminés ;
(b) a une charge nette estimée d'au moins +7, +8, +9, +10, +11, +12, +13, +14, ou +15 au pH physiologique ;
(c) est soluble en solution aqueuse ; ou
(d) toute combinaison de (a) à (c).

4. Agent navette à base de polypeptide destiné à être utilisé selon la revendication 1 ou la revendication 3, ou procédé in vitro selon la revendication 2 ou la revendication 3, dans lequel :
(a) ledit ELD est ou provient de : un peptide endosomolytique ; un peptide antimicrobien (AMP) ; un peptide antimicrobien alpha-hélicoïdal cationique linéaire ; un peptide hybride de Cécropine-A/Mélittine (CM) ; un peptide actif à membrane dépendante du pH (PAMP) ; un peptide amphiphile ; un peptide dérivé de l'extrémité N terminale de la sous-unité HA2 de l'hémagglutinine de la grippe (HA) ; CM18 ; le domaine T de la toxine diphtérique (DT) ; GALA; PEA; INF-7 ; LAH4 ; HGP ; H5WYG; HA2 ; EB1; VSVG ; une toxine de Pseudomonas ; la mélittine ; KALA ; JST-1 ; C(LLKK)₃C ; G(LLKK)₃G ; ou toute combinaison de ceux-ci ;
(b) ledit CPD est ou provient de : un peptide de pénétration cellulaire ou le domaine de transduction protéique provenant d'un peptide de pénétration cellulaire ; TAT ; PTD4 ; la Pénétratine (Antennapedia) ; pVEC ; M918 ; Pep-1 ; Pep- 2 ; Xentry ; une vergeture d'arginine ; le transportan ; SynB1 ; SynB3 ; ou toute combinaison de ceux-ci ;
(c) ledit domaine riche en histidine et une vergeture d'au moins 3, d'au moins 4, d'au moins 5, ou d'au moins 6 acides aminés comprenant au moins 50 %, au moins 55 %, au moins 60 %, au moins 65 %, au moins 70 %, au moins 75 %, au moins 80 %, au moins 85 %, ou au moins 90 % résidus d'histidine ; et/ou comprend au moins 2, au moins 3, au moins 4, au moins 5, au moins 6, au moins 7, au moins 8, ou au moins 9 résidus d'histidine consécutifs ; ou
(d) toute combinaison de (a) à (c).

5. Agent navette à base de polypeptide destiné à être utilisé selon la revendication 1, 3 ou 4, ou procédé in vitro selon l'une quelconque des revendications 2 à 4, dans lequel l'agent navette à base de polypeptide comprend :
(a) un ELD comprenant la séquence d'acides aminés selon l'une quelconque des SEQ ID No : 1-15, 63, 64, ou un variant ou un fragment de celle-ci ayant une activité endosomolytique ;
(b) un CPD comprenant la séquence d'acides aminés selon l'une quelconque des SEQ ID No : 16-27 ou 65, ou un variant ou un fragment de celle-ci ayant une activité de pénétration cellulaire ;
(c) un domaine riche en histidine ayant au moins 2, au moins 3, au moins 4, au moins 5, ou au moins 6 résidus d'histidine consécutifs ; ou
(d) toute combinaison de (a) à (c).

6. Agent navette à base de polypeptide destiné à être utilisé selon l'une quelconque des revendications 1 et 3 à 5, ou procédé in vitro selon l'une quelconque des revendications 2 à 5, dans lequel lesdits domaines sont liés de manière fonctionnelle par l'intermédiaire d'au moins un domaine de liaison.

7. Agent navette à base de polypeptide destiné à être utilisé selon l'une quelconque des revendications 1 et 3 à 6, ou procédé in vitro selon l'une quelconque des revendications 2 à 6, dans lequel l'agent navette à base de polypeptide comprend :
(a) un ELD qui est CM18, KALA, ou C(LLKK)₃C ayant la séquence d'acides aminés de SEQ ID No : 1, 14, ou 63, ou un variant de celle-ci ayant au moins 85 %, 90 %, ou 95 % d'identité avec SEQ ID No : 1, 14, ou 63, et ayant une activité endosomolytique ;
(b) un CPD qui est TAT ou PTD4 ayant la séquence d'acides aminés de SEQ ID No : 17 ou 65, ou un variant de celle-ci ayant au moins 85 %, 90 %, ou 95 % d'identité avec SEQ ID No : 17 ou 65 et ayant une activité de pénétration cellulaire ; ou la Pénétratine ayant la séquence d'acides aminés de SEQ ID No : 18, ou un variant de celle-ci ayant au moins 85 %, 90 %, ou 95 % d'identité avec SEQ ID No : 18 et ayant une activité de pénétration cellulaire ;
(c) un domaine riche en histidine comprenant au moins 6 résidus d'histidine consécutifs ; ou
(d) toute combinaison de (a) à (c).

8. Agent navette à base de polypeptide destiné à être utilisé selon l'une quelconque des revendications 1 et 3 à 7, ou procédé in vitro selon l'une quelconque des revendications 2 à 7, dans lequel l'agent navette à base de polypeptide comprend ou est constitué de la séquence d'acides aminés de l'une quelconque des SEQ ID No : 57-59, 66-72, ou 82-102, ou un variant fonctionnel de celui-ci ayant au moins 85 %, 90 %, ou 95 % d'identité avec l'une quelconque des SEQ ID No : 10 57-59, 66-72, ou 82-102.

9. Agent navette à base de polypeptide destiné à être utilisé selon l'une quelconque des revendications 1 et 3 à 8, ou procédé in vitro selon l'une quelconque des revendications 2 à 8, dans lequel ladite cargaison polypeptidique indépendante :
(a) ne dispose pas d'un CPD ou d'un CPD tel que défini dans la revendication 4(b) ;
(b) est une protéine recombinante ;
(c) comprend un domaine de ciblage subcellulaire ;
(d) est complexée à une molécule d'ADN et/ou d'ARN ; ou
(e) toute combinaison de (a) à (d).

10. Agent navette à base de polypeptide destiné à être utilisé selon la revendication 9, ou procédé in vitro selon la revendication 9, dans lequel ledit domaine de ciblage subcellulaire est :
(a) un signal de localisation nucléaire (NLS), provenant éventuellement de : Ela, T-Ag, c-myc, T-Ag, op-T-NLS, Vp3, la nucléoplasmine, histone 2B, Xenopus N1, PARP, PDX-1, QKI-5, HCDA, H2B, v-Rel, Amida, RanBP3, Pho4p, LEF-1, TCF-1, BDV-P, TR2, SOX9, ou Max ;
(b) une séquence de signaux nucléolaires, provenant éventuellement de BIRC5 ou RECQL4;
(c) une séquence de signaux mitochondriens, provenant éventuellement de Tim9 ou de la sous-unité IV oxydase cytochrome c de levure ; ou
(d) une séquence de signal de peroxysome, provenant éventuellement de PTS1.

11. Agent navette à base de polypeptide destiné à être utilisé selon l'une quelconque des revendications 1 et 3 à 10, ou procédé in vitro selon l'une quelconque des revendications 2 à 10, dans lequel ladite cargaison polypeptidique est un facteur de transcription, une nucléase, une cytokine, une hormone, un facteur de croissance, ou un anticorps.

12. Agent navette à base de polypeptide destiné à être utilisé selon la revendication 11, ou procédé in vitro selon la revendication 11, dans lequel :
(a) ledit facteur de transcription et : HOXB4, NUP98-HOXA9, Oct3/4, Sox2, Sox9, Klf4, c-Myc, MyoD, Pdx1, Ngn3, MafA, Blimp-1, Eomes, T-bet, FOXO3A, NF-YA, SALL4, ISL1, FoxA1, Nanog, Esrrb, Lin28, HIF1-alpha, Hlf, Runxltl, Pbx1, Lmo2, Zfp37, Prdm5, Bcl-6, ou toute combinaison de ceux-ci ; et/ou
(b) ladite nucléase est : une endonucléase guidée par l'ARN, une endonucléase CRISPR, une endonucléase CRISPR de type I, une endonucléase CRISPR de type II, une endonucléase CRISPR de type III, une endonucléase CRISPR de type IV, une endonucléase CRISPR de type V, une endonucléase CRISPR de type VI, la protéine 9 associée à CRISPR (Cas9), Cpf1, une nucléase à doigt de zinc (ZFN), une nucléase effectrice de type activateur de transcription (TALEN), une endonucléase homing, une méganucléase, ou toute combinaison de ceux-ci.

13. Agent navette à base de polypeptide destiné à être utilisé selon l'une quelconque des revendications 1 et 3 à 12, ou procédé in vitro selon l'une quelconque des revendications 2 à 12, pour augmenter l'efficacité de transduction d'une cargaison polypeptidique vers le cytosol de cellules eucaryotes cibles destinées à être utilisées en thérapie cellulaire, dans la modification du génome, dans le transfert cellulaire adoptif, et/ou en médecine régénérative.

14. Agent navette à base de polypeptide destiné à être utilisé selon l'une quelconque des revendications 1 et 3 à 13, ou procédé in vitro selon l'une quelconque des revendications 2 à 13, dans lequel lesdites cellules eucaryotes cibles sont des cellules animales, des cellules de mammifères, des cellules humaines, des cellules souches, des cellules primaires, des cellules immunitaires, des lymphocytes T, ou des cellules dendritiques.

15. Peptide synthétique comprenant un domaine de fuite d'endosome (ELD) lié de manière opérationnelle à un domaine de pénétration cellulaire (CPD), ou un ELD lié de manière opérationnelle à un domaine riche en histidine et un CPD, dans lequel le peptide synthétique est l'agent navette à base de polypeptide tel que défini dans l'une quelconque des revendications 3 à 8, et dans lequel le CPD est ou provient de : PTD4 ; la Pénétratine (Antennapedia) ; pVEC ; M918 ; Pep-1 ; Pep-2 ; Xentry ; une vergeture d'arginine ; le transportan ; SynB1 ; SynB3 ; ou toute combinaison de ceux-ci, et dans lequel l'agent navette à base de polypeptide a une charge nette estimée d'au moins +6 au pH physiologique.


### SEQUENCE LISTING

<110> FELDAN BIO INC.
   GUAY, David
   DEL'GUIDICE, Thomas
   LEPETIT-STOFFAES, Jean-Pascal
<120> POLYPEPTIDE-BASED SHUTTLE AGENTS FOR IMPROVING THE TRANSDUCTION EFFICIENCY OF POLYPEPTIDE CARGOS TO THE CYTOSOL OF TARGET EUKARYOTIC CELLS, USES THEREOF, METHODS AND KITS RELATING TO SAME
<130> 016995-0010
<140> Not yet assigned
   <141> 2016-04-08
<150> US 62/145,760
   <151> 2015-04-10
<150> US 62/246,892
   <151> 2015-10-27
<160> 103
<170> PatentIn version 3.5
<210> 1
   <211> 18
   <212> PRT
   <213> Artificial sequence
<220>
   <223> CM18
<400> 1 
Lys Trp Lys Leu Phe Lys Lys Ile Gly Ala Val Leu Lys Val Leu Thr
1               5                   10                  15

Thr Gly
<210> 2
   <211> 189
   <212> PRT
   <213> Artificial sequence
<220>
   <223> Diphtheria toxin T domain (DT)
<400> 2 
Val Gly Ser Ser Leu Ser Cys Ile Asn Leu Asp Trp Asp Val Ile Arg
1               5                   10                  15

Asp Lys Thr Lys Thr Lys Ile Glu Ser Leu Lys Glu His Gly Pro Ile
            20                  25                  30

Lys Asn Lys Met Ser Glu Ser Pro Asn Lys Thr Val Ser Glu Glu Lys
 
35                  40                  45

Ala Lys Gln Tyr Leu Glu Glu Phe His Gln Thr Ala Leu Glu His Pro
    50                  55                  60

Glu Leu Ser Glu Leu Lys Thr Val Thr Gly Thr Asn Pro Val Phe Ala
65                  70                  75                  80

Gly Ala Asn Tyr Ala Ala Trp Ala Val Asn Val Ala Gln Val Ile Asp
                85                  90                  95

Ser Glu Thr Ala Asp Asn Leu Glu Lys Thr Thr Ala Ala Leu Ser Ile
            100                 105                 110

Leu Pro Gly Ile Gly Ser Val Met Gly Ile Ala Asp Gly Ala Val His
        115                 120                 125

His Asn Thr Glu Glu Ile Val Ala Gln Ser Ile Ala Leu Ser Ser Leu
    130                 135                 140

Met Val Ala Gln Ala Ile Pro Leu Val Gly Glu Leu Val Asp Ile Gly
145                 150                 155                 160

Phe Ala Ala Tyr Asn Phe Val Glu Ser Ile Ile Asn Leu Phe Gln Val
                165                 170                 175

Val His Asn Ser Tyr Asn Arg Pro Ala Tyr Ser Pro Gly
            180                 185
<210> 3
   <211> 30
   <212> PRT
   <213> Artificial sequence
<220>
   <223> GALA
<400> 3 
Trp Glu Ala Ala Leu Ala Glu Ala Leu Ala Glu Ala Leu Ala Glu His
1               5                   10                  15

Leu Ala Glu Ala Leu Ala Glu Ala Leu Glu Ala Leu Ala Ala
            20                  25                  30
<210> 4
   <211> 115
   <212> PRT
   <213> Artificial sequence
<220>
   <223> PEA
<400> 4 
Val Leu Ala Gly Asn Pro Ala Lys His Asp Leu Asp Ile Lys Pro Thr
1               5                   10                  15

Val Ile Ser His Arg Leu His Phe Pro Glu Gly Gly Ser Leu Ala Ala
            20                  25                  30

Leu Thr Ala His Gln Ala Cys His Leu Pro Leu Glu Thr Phe Thr Arg
        35                  40                  45

His Arg Gln Pro Arg Gly Trp Glu Gln Leu Glu Gln Cys Gly Tyr Pro
    50                  55                  60

Val Gln Arg Leu Val Ala Leu Tyr Leu Ala Ala Arg Leu Ser Trp Asn
65                  70                  75                  80

Gln Val Asp Gln Val Ile Arg Asn Ala Leu Ala Ser Pro Gly Ser Gly
                85                  90                  95

Gly Asp Leu Gly Glu Ala Ile Arg Glu Gln Pro Glu Gln Ala Arg Leu
            100                 105                 110

Ala Leu Thr
        115
<210> 5
   <211> 24
   <212> PRT
   <213> Artificial sequence
<220>
   <223> INF-7
<400> 5 
Gly Leu Phe Glu Ala Ile Glu Gly Phe Ile Glu Asn Gly Trp Glu Gly
1               5                   10                  15

Met Ile Asp Gly Trp Tyr Gly Cys
            20
<210> 6
   <211> 26
   <212> PRT
   <213> Artificial sequence
<220>
   <223> LAH4
<400> 6 
Lys Lys Ala Leu Leu Ala Leu Ala Leu His His Leu Ala His Leu Ala
1               5                   10                  15

Leu His Leu Ala Leu Ala Leu Lys Lys Ala
            20                  25
<210> 7
   <211> 24
   <212> PRT
   <213> Artificial sequence
<220>
   <223> HGP
<400> 7 
Leu Leu Gly Arg Arg Gly Trp Glu Val Leu Lys Tyr Trp Trp Asn Leu
1               5                   10                  15

Leu Gln Tyr Trp Ser Gln Glu Leu
            20
<210> 8
   <211> 23
   <212> PRT
   <213> Artificial sequence
<220>
   <223> H5WYG
<400> 8 
Gly Leu Phe His Ala Ile Ala His Phe Ile His Gly Gly Trp His Gly
1               5                   10                  15

Leu Ile His Gly Trp Tyr Gly
            20
<210> 9
   <211> 23
   <212> PRT
   <213> Artificial sequence
<220>
   <223> HA2
<400> 9 
Gly Leu Phe Gly Ala Ile Ala Gly Phe Ile Glu Asn Gly Trp Glu Gly
1               5                   10                  15

Met Ile Asp Gly Trp Tyr Gly
            20
<210> 10
   <211> 23
   <212> PRT
   <213> Artificial sequence
<220>
   <223> EB1
<400> 10 
Leu Ile Arg Leu Trp Ser His Leu Ile His Ile Trp Phe Gln Asn Arg
1               5                   10                  15

Arg Leu Lys Trp Lys Lys Lys
            20
<210> 11
   <211> 25
   <212> PRT
   <213> Artificial sequence
<220>
   <223> VSVG
<400> 11 
Lys Phe Thr Ile Val Phe Pro His Asn Gln Lys Gly Asn Trp Lys Asn
1               5                   10                  15

Val Pro Ser Asn Tyr His Tyr Cys Pro
            20                  25
<210> 12
   <211> 115
   <212> PRT
   <213> Artificial sequence
<220>
   <223> Pseudomonas toxin
<400> 12 
Glu Gly Gly Ser Leu Ala Ala Leu Thr Ala His Gln Ala Cys His Leu
1               5                   10                  15

Pro Leu Glu Thr Phe Thr Arg His Arg Gln Pro Arg Gly Trp Glu Gln
            20                  25                  30

Leu Glu Gln Cys Gly Tyr Pro Val Gln Arg Leu Val Ala Leu Tyr Leu
        35                  40                  45

Ala Ala Arg Leu Ser Trp Asn Gln Val Asp Gln Val Ile Arg Asn Ala
    50                  55                  60

Leu Ala Ser Pro Gly Ser Gly Gly Asp Leu Gly Glu Ala Ile Arg Glu
 
65                  70                  75                  80

Gln Pro Glu Gln Ala Arg Leu Ala Leu Thr Leu Ala Ala Ala Glu Ser
                85                  90                  95

Glu Arg Phe Val Arg Gln Gly Thr Gly Asn Asp Glu Ala Gly Ala Ala
            100                 105                 110

Asn Ala Asp
        115
<210> 13
   <211> 26
   <212> PRT
   <213> Artificial sequence
<220>
   <223> Melittin
<400> 13 
Gly Ile Gly Ala Val Leu Lys Val Leu Thr Thr Gly Leu Pro Ala Leu
1               5                   10                  15

Ile Ser Trp Ile Lys Arg Lys Arg Gln Gln
            20                  25
<210> 14
   <211> 30
   <212> PRT
   <213> Artificial sequence
<220>
   <223> KALA
<400> 14 
Trp Glu Ala Lys Leu Ala Lys Ala Leu Ala Lys Ala Leu Ala Lys His
1               5                   10                  15

Leu Ala Lys Ala Leu Ala Lys Ala Leu Lys Ala Cys Glu Ala
            20                  25                  30
<210> 15
   <211> 20
   <212> PRT
   <213> Artificial sequence
<220>
   <223> JST-1
<400> 15 
Gly Leu Phe Glu Ala Leu Leu Glu Leu Leu Glu Ser Leu Trp Glu Leu
1               5                   10                  15
 
Leu Leu Glu Ala
            20
<210> 16
   <211> 16
   <212> PRT
   <213> Artificial sequence
<220>
   <223> SP
<400> 16 
Ala Ala Val Ala Leu Leu Pro Ala Val Leu Leu Ala Leu Leu Ala Pro
1               5                   10                  15
<210> 17
   <211> 11
   <212> PRT
   <213> Artificial sequence
<220>
   <223> TAT
<400> 17 
Tyr Gly Arg Lys Lys Arg Arg Gln Arg Arg Arg
1               5                   10
<210> 18
   <211> 16
   <212> PRT
   <213> Artificial sequence
<220>
   <223> Penetratin (Antennapedia)
<400> 18 
Arg Gln Ile Lys Ile Trp Phe Gln Asn Arg Arg Met Lys Trp Lys Lys
1               5                   10                  15
<210> 19
   <211> 18
   <212> PRT
   <213> Artificial sequence
<220>
   <223> pVEC
<400> 19 
Leu Leu Ile Ile Leu Arg Arg Arg Ile Arg Lys Gln Ala His Ala His
1               5                   10                  15

Ser Lys
<210> 20
   <211> 22
   <212> PRT
   <213> Artificial sequence
<220>
   <223> M918
<400> 20 
Met Val Thr Val Leu Phe Arg Arg Leu Arg Ile Arg Arg Ala Cys Gly
1               5                   10                  15

Pro Pro Arg Val Arg Val
            20
<210> 21
   <211> 21
   <212> PRT
   <213> Artificial sequence
<220>
   <223> Pep-1
<400> 21 
Lys Glu Thr Trp Trp Glu Thr Trp Trp Thr Glu Trp Ser Gln Pro Lys
1               5                   10                  15

Lys Lys Arg Lys Val
            20
<210> 22
   <211> 21
   <212> PRT
   <213> Artificial sequence
<220>
   <223> Pep-2
<400> 22 
Lys Glu Thr Trp Phe Glu Thr Trp Phe Thr Glu Trp Ser Gln Pro Lys
1               5                   10                  15

Lys Lys Arg Lys Val
            20
<210> 23
   <211> 7
   <212> PRT
   <213> Artificial sequence
<220>
   <223> Xentry
<400> 23 
Leu Cys Leu Arg Pro Val Gly
1               5
<210> 24
   <211> 9
   <212> PRT
   <213> Artificial sequence
<220>
   <223> Arginine stretch
<400> 24 
Arg Arg Arg Arg Arg Arg Arg Arg Arg
1               5
<210> 25
   <211> 26
   <212> PRT
   <213> Artificial sequence
<220>
   <223> Transportan
<400> 25 
Trp Thr Leu Asn Ser Ala Gly Tyr Leu Leu Gly Lys Ile Asn Leu Lys
1               5                   10                  15

Ala Leu Ala Ala Leu Ala Lys Lys Ile Leu
            20                  25
<210> 26
   <211> 18
   <212> PRT
   <213> Artificial sequence
<220>
   <223> SynB1
<400> 26 
Arg Gly Gly Arg Leu Ser Tyr Ser Arg Arg Arg Phe Ser Thr Ser Thr
1               5                   10                  15

Gly Arg
<210> 27
   <211> 10
   <212> PRT
   <213> Artificial sequence
<220>
   <223> SynB3
<400> 27 
Arg Arg Leu Ser Tyr Ser Arg Arg Arg Phe
1               5                   10
<210> 28
   <211> 5
   <212> PRT
   <213> Artificial sequence
<220>
   <223> E1a
<400> 28 
Lys Arg Pro Arg Pro
1               5
<210> 29
   <211> 7
   <212> PRT
   <213> Artificial sequence
<220>
   <223> SV40 T-Ag
<400> 29 
Pro Lys Lys Lys Arg Lys Val
1               5
<210> 30
   <211> 9
   <212> PRT
   <213> Artificial sequence
<220>
   <223> c-myc
<400> 30 
Pro Ala Ala Lys Arg Val Lys Leu Asp
1               5
<210> 31
   <211> 22
   <212> PRT
   <213> Artificial sequence
<220>
   <223> Op-T-NLS
<400> 31 
Ser Ser Asp Asp Glu Ala Thr Ala Asp Ser Gln His Ala Ala Pro Pro
1               5                   10                  15
 
Lys Lys Lys Arg Lys Val
             20
<210> 32
   <211> 5
   <212> PRT
   <213> Artificial sequence
<220>
   <223> Vp3
<400> 32 
Lys Lys Lys Arg Lys
1               5
<210> 33
   <211> 16
   <212> PRT
   <213> Artificial sequence
<220>
   <223> Nucleoplasmin
<400> 33 
Lys Arg Pro Ala Ala Thr Lys Lys Ala Gly Gln Ala Lys Lys Lys Lys
1               5                   10                  15
<210> 34
   <211> 10
   <212> PRT
   <213> Artificial sequence
<220>
   <223> Histone 2B NLS
<400> 34 
Asp Gly Lys Lys Arg Lys Arg Ser Arg Lys
1               5                   10
<210> 35
   <211> 24
   <212> PRT
   <213> Artificial sequence
<220>
   <223> Xenopus N1
<400> 35 
Val Arg Lys Lys Arg Lys Thr Glu Glu Glu Ser Pro Leu Lys Asp Lys
1               5                   10                  15

Asp Ala Lys Lys Ser Lys Gln Glu
            20
<210> 36
   <211> 19
   <212> PRT
   <213> Artificial sequence
<220>
   <223> PARP
<400> 36 
Lys Arg Lys Gly Asp Glu Val Asp Gly Val Asp Glu Cys Ala Lys Lys
1               5                   10                  15

Ser Lys Lys
<210> 37
   <211> 7
   <212> PRT
   <213> Artificial sequence
<220>
   <223> PDX-1
<400> 37 
Arg Arg Met Lys Trp Lys Lys
1               5
<210> 38
   <211> 7
   <212> PRT
   <213> Artificial sequence
<220>
   <223> QKI-5
<400> 38 
Arg Val His Pro Tyr Gln Arg
1               5
<210> 39
   <211> 24
   <212> PRT
   <213> Artificial sequence
<220>
   <223> HCDA
<400> 39 
Lys Arg Pro Ala Cys Thr Leu Lys Pro Glu Cys Val Gln Gln Leu Leu
1               5                   10                  15
 
Val Cys Ser Gln Glu Ala Lys Lys
            20
<210> 40
   <211> 7
   <212> PRT
   <213> Artificial sequence
<220>
   <223> H2B
<400> 40 
Gly Lys Lys Arg Ser Lys Ala
1               5
<210> 41
   <211> 6
   <212> PRT
   <213> Artificial sequence
<220>
   <223> v-Rel
<400> 41 
Lys Ala Lys Arg Gln Arg
1               5
<210> 42
   <211> 5
   <212> PRT
   <213> Artificial sequence
<220>
   <223> Amida
<400> 42 
Arg Lys Arg Arg Arg
1               5
<210> 43
   <211> 9
   <212> PRT
   <213> Artificial sequence
<220>
   <223> RanBP3
<400> 43 
Pro Pro Val Lys Arg Glu Arg Thr Ser
1               5
<210> 44
   <211> 10
   <212> PRT
   <213> Artificial sequence
<220>
   <223> Pho4p
<400> 44 
Pro Tyr Leu Asn Lys Arg Lys Gly Lys Pro
1               5                   10
<210> 45
   <211> 9
   <212> PRT
   <213> Artificial sequence
<220>
   <223> LEF-1
<400> 45 
Lys Lys Lys Lys Arg Lys Arg Glu Lys
1               5
<210> 46
   <211> 9
   <212> PRT
   <213> Artificial sequence
<220>
   <223> TCF-1
<400> 46 
Lys Lys Lys Arg Arg Ser Arg Glu Lys
1               5
<210> 47
   <211> 8
   <212> PRT
   <213> Artificial sequence
<220>
   <223> BDV-P
<400> 47 
Pro Arg Pro Arg Lys Ile Pro Arg
1               5
<210> 48
   <211> 20
   <212> PRT
   <213> Artificial sequence
<220>
   <223> TR2
<400> 48 
Lys Asp Cys Val Ile Asn Lys His His Arg Asn Arg Cys Gln Tyr Cys
1               5                   10                  15

Arg Leu Gln Arg
            20
<210> 49
   <211> 5
   <212> PRT
   <213> Artificial sequence
<220>
   <223> SOX9
<400> 49 
Pro Arg Arg Arg Lys
1               5
<210> 50
   <211> 8
   <212> PRT
   <213> Artificial sequence
<220>
   <223> Max
<400> 50 
Pro Gln Ser Arg Lys Lys Leu Arg
1               5
<210> 51
   <211> 9
   <212> PRT
   <213> Artificial sequence
<220>
   <223> Mitochondrial signal sequence from Tim9
<400> 51 
Asn Leu Val Glu Arg Cys Phe Thr Asp
1               5
<210> 52
   <211> 12
   <212> PRT
   <213> Artificial sequence
<220>
   <223> Mitochondrial signal sequence from Yeast cytochrome c oxidase subunit IV
<400> 52 
Met Leu Ser Leu Arg Gln Ser Ile Arg Phe Phe Lys
1               5                   10
<210> 53
   <211> 16
   <212> PRT
   <213> Artificial sequence
<220>
   <223> Mitochondrial signal sequence from 18S rRNA
<400> 53 
Met Leu Ile Ser Arg Cys Lys Trp Ser Arg Phe Pro Gly Asn Gln Arg
1               5                   10                  15
<210> 54
   <211> 3
   <212> PRT
   <213> Artificial sequence
<220>
   <223> Peroxisome signal sequence - PTS1
<400> 54 
Ser Lys Leu
1
<210> 55
   <211> 17
   <212> PRT
   <213> Artificial sequence
<220>
   <223> Nucleolar signal sequence from BIRC5
<400> 55 
Met Gln Arg Lys Pro Thr Ile Arg Arg Lys Asn Leu Arg Leu Arg Arg
1               5                   10                  15

Lys
<210> 56
   <211> 11
   <212> PRT
   <213> Artificial sequence
<220>
   <223> Nucleolar signal sequence from RECQL4
<400> 56 
Lys Gln Ala Trp Lys Gln Lys Trp Arg Lys Lys
1               5                   10
<210> 57
   <211> 29
   <212> PRT
   <213> Artificial sequence
<220>
   <223> CM18-TAT
<400> 57 
Lys Trp Lys Leu Phe Lys Lys Ile Gly Ala Val Leu Lys Val Leu Thr
1               5                   10                  15

Thr Gly Tyr Gly Arg Lys Lys Arg Arg Gln Arg Arg Arg
            20                  25
<210> 58
   <211> 34
   <212> PRT
   <213> Artificial sequence
<220>
   <223> CM18-Penetratin
<400> 58 
Lys Trp Lys Leu Phe Lys Lys Ile Gly Ala Val Leu Lys Val Leu Thr
1               5                   10                  15

Thr Gly Arg Gln Ile Lys Ile Trp Phe Gln Asn Arg Arg Met Lys Trp
            20                  25                  30

Lys Lys
<210> 59
   <211> 36
   <212> PRT
   <213> Artificial sequence
<220>
   <223> His-CM18-TAT
<400> 59 
Met His His His His His His Lys Trp Lys Leu Phe Lys Lys Ile Gly
1               5                   10                  15

Ala Val Leu Lys Val Leu Thr Thr Gly Tyr Gly Arg Lys Lys Arg Arg
            20                  25                  30

Gln Arg Arg Arg
        35
<210> 60
   <211> 290
   <212> PRT
   <213> Artificial sequence
<220>
   <223> GFP
<400> 60 
Met His His His His His His Gly Gly Gly Gly Ser Gly Gly Gly Gly
1               5                   10                  15

Ser Gly Gly Ala Ser Thr Gly Thr Gly Ile Arg Met Val Ser Lys Gly
            20                  25                  30

Glu Glu Leu Phe Thr Gly Val Val Pro Ile Leu Val Glu Leu Asp Gly
        35                  40                  45

Asp Val Asn Gly His Lys Phe Ser Val Ser Gly Glu Gly Glu Gly Asp
    50                  55                  60

Ala Thr Tyr Gly Lys Leu Thr Leu Lys Phe Ile Cys Thr Thr Gly Lys
65                  70                  75                  80

Leu Pro Val Pro Trp Pro Thr Leu Val Thr Thr Leu Thr Tyr Gly Val
                85                  90                  95

Gln Cys Phe Ser Arg Tyr Pro Asp His Met Lys Gln His Asp Phe Phe
            100                 105                 110

Lys Ser Ala Met Pro Glu Gly Tyr Val Gln Glu Arg Thr Ile Phe Phe
        115                 120                 125

Lys Asp Asp Gly Asn Tyr Lys Thr Arg Ala Glu Val Lys Phe Glu Gly
    130                 135                 140

Asp Thr Leu Val Asn Arg Ile Glu Leu Lys Gly Ile Asp Phe Lys Glu
145                 150                 155                 160

Asp Gly Asn Ile Leu Gly His Lys Leu Glu Tyr Asn Tyr Asn Ser His
                165                 170                 175

Asn Val Tyr Ile Met Ala Asp Lys Gln Lys Asn Gly Ile Lys Val Asn
            180                 185                 190

Phe Lys Ile Arg His Asn Ile Glu Asp Gly Ser Val Gln Leu Ala Asp
        195                 200                 205

His Tyr Gln Gln Asn Thr Pro Ile Gly Asp Gly Pro Val Leu Leu Pro
    210                 215                 220
 
Asp Asn His Tyr Leu Ser Thr Gln Ser Ala Leu Ser Lys Asp Pro Asn
225                 230                 235                 240

Glu Lys Arg Asp His Met Val Leu Leu Glu Phe Val Thr Ala Ala Gly
                245                 250                 255

Ile Thr Leu Gly Met Asp Glu Leu Tyr Lys Gly Gly Ser Gly Gly Gly
            260                 265                 270

Ser Gly Gly Gly Ser Gly Trp Ile Arg Ala Ser Ser Gly Gly Arg Glu
        275                 280                 285

Ile Ser
    290
<210> 61
   <211> 317
   <212> PRT
   <213> Artificial sequence
<220>
   <223> TAT-GFP
<400> 61 
   Met His His His His His His Gly Gly Gly Gly Ser Gly Gly Gly Gly
   1               5                   10                  15
   Ser Gly Gly Ala Ser Thr Gly Thr Gly Arg Lys Lys Arg Arg Gln Arg
               20                  25                  30
   Arg Arg Pro Pro Gln Gly Gly Gly Gly Ser Gly Gly Gly Gly Ser Gly
           35                  40                  45
   Gly Gly Thr Gly Ile Arg Met Val Ser Lys Gly Glu Glu Leu Phe Thr
       50                  55                  60
   Gly Val Val Pro Ile Leu Val Glu Leu Asp Gly Asp Val Asn Gly His
   65                  70                  75                  80
   Lys Phe Ser Val Ser Gly Glu Gly Glu Gly Asp Ala Thr Tyr Gly Lys
                   85                  90                  95
   Leu Thr Leu Lys Phe Ile Cys Thr Thr Gly Lys Leu Pro Val Pro Trp
               100                 105                 110
   Pro Thr Leu Val Thr Thr Leu Thr Tyr Gly Val Gln Cys Phe Ser Arg
           115                 120                 125
   Tyr Pro Asp His Met Lys Gln His Asp Phe Phe Lys Ser Ala Met Pro
       130                 135                 140
   Glu Gly Tyr Val Gln Glu Arg Thr Ile Phe Phe Lys Asp Asp Gly Asn
   145                 150                 155                 160
   Tyr Lys Thr Arg Ala Glu Val Lys Phe Glu Gly Asp Thr Leu Val Asn
                   165                 170                 175
   Arg Ile Glu Leu Lys Gly Ile Asp Phe Lys Glu Asp Gly Asn Ile Leu
               180                 185                 190
   Gly His Lys Leu Glu Tyr Asn Tyr Asn Ser His Asn Val Tyr Ile Met
           195                 200                 205
   Ala Asp Lys Gln Lys Asn Gly Ile Lys Val Asn Phe Lys Ile Arg His
       210                 215                 220
   Asn Ile Glu Asp Gly Ser Val Gln Leu Ala Asp His Tyr Gln Gln Asn
   225                 230                 235                 240
   Thr Pro Ile Gly Asp Gly Pro Val Leu Leu Pro Asp Asn His Tyr Leu
                   245                 250                 255
   Ser Thr Gln Ser Ala Leu Ser Lys Asp Pro Asn Glu Lys Arg Asp His
               260                 265                 270
   Met Val Leu Leu Glu Phe Val Thr Ala Ala Gly Ile Thr Leu Gly Met
           275                 280                 285
   Asp Glu Leu Tyr Lys Gly Gly Ser Gly Gly Gly Ser Gly Gly Gly Ser
       290                 295                 300
   Gly Trp Ile Arg Ala Ser Ser Gly Gly Arg Glu Ile Ser
   305                 310                 315
<210> 62
   <211> 327
   <212> PRT
   <213> Artificial sequence
<220>
   <223> GFP-NLS
<400> 62 
Met His His His His His His Gly Gly Gly Gly Ser Gly Gly Gly Gly
1               5                   10                  15

Ser Gly Gly Ala Ser Thr Gly Ile Arg Met Val Ser Lys Gly Glu Glu
 
20                  25                  30

Leu Phe Thr Gly Val Val Pro Ile Leu Val Glu Leu Asp Gly Asp Val
        35                  40                  45

Asn Gly His Lys Phe Ser Val Ser Gly Glu Gly Glu Gly Asp Ala Thr
    50                  55                  60

Tyr Gly Lys Leu Thr Leu Lys Phe Ile Cys Thr Thr Gly Lys Leu Pro
65                  70                  75                  80

Val Pro Trp Pro Thr Leu Val Thr Thr Leu Thr Tyr Gly Val Gln Cys
                85                  90                  95

Phe Ser Arg Tyr Pro Asp His Met Lys Gln His Asp Phe Phe Lys Ser
            100                 105                 110

Ala Met Pro Glu Gly Tyr Val Gln Glu Arg Thr Ile Phe Phe Lys Asp
        115                 120                 125

Asp Gly Asn Tyr Lys Thr Arg Ala Glu Val Lys Phe Glu Gly Asp Thr
    130                 135                 140

Leu Val Asn Arg Ile Glu Leu Lys Gly Ile Asp Phe Lys Glu Asp Gly
145                 150                 155                 160

Asn Ile Leu Gly His Lys Leu Glu Tyr Asn Tyr Asn Ser His Asn Val
                165                 170                 175

Tyr Ile Met Ala Asp Lys Gln Lys Asn Gly Ile Lys Val Asn Phe Lys
            180                 185                 190

Ile Arg His Asn Ile Glu Asp Gly Ser Val Gln Leu Ala Asp His Tyr
        195                 200                 205

Gln Gln Asn Thr Pro Ile Gly Asp Gly Pro Val Leu Leu Pro Asp Asn
    210                 215                 220

His Tyr Leu Ser Thr Gln Ser Ala Leu Ser Lys Asp Pro Asn Glu Lys
225                 230                 235                 240

Arg Asp His Met Val Leu Leu Glu Phe Val Thr Ala Ala Gly Ile Thr
                245                 250                 255

Leu Gly Met Asp Glu Leu Tyr Lys Gly Gly Ser Gly Gly Gly Ser Gly
            260                 265                 270
 
Gly Gly Ser Gly Trp Ile Arg Ala Ser Ser Gly Gly Arg Ser Ser Asp
        275                 280                 285

Asp Glu Ala Thr Ala Asp Ser Gln His Ala Ala Pro Pro Lys Lys Lys
    290                 295                 300

Arg Lys Val Gly Gly Ser Gly Gly Gly Ser Gly Gly Gly Ser Gly Gly
305                 310                 315                 320

Gly Arg Gly Thr Glu Ile Ser
                325
<210> 63
   <211> 14
   <212> PRT
   <213> Artificial Sequence
<220>
   <223> C(LLKK)3C
<400> 63 
Cys Leu Leu Lys Lys Leu Leu Lys Lys Leu Leu Lys Lys Cys
1               5                   10
<210> 64
   <211> 14
   <212> PRT
   <213> Artificial Sequence
<220>
   <223> G(LLKK)3G
<400> 64 
Gly Leu Leu Lys Lys Leu Leu Lys Lys Leu Leu Lys Lys Gly
1               5                   10
<210> 65
   <211> 11
   <212> PRT
   <213> Artificial Sequence
<220>
   <223> PTD4
<400> 65 
Tyr Ala Arg Ala Ala Ala Arg Gln Ala Arg Ala
1               5                   10
<210> 66
   <211> 30
   <212> PRT
   <213> Artificial Sequence
<220>
   <223> TAT-CM18
<400> 66 
Tyr Gly Arg Lys Lys Arg Arg Gln Arg Arg Arg Cys Lys Trp Lys Leu
1               5                   10                  15

Phe Lys Lys Ile Gly Ala Val Leu Lys Val Leu Thr Thr Gly
            20                  25                  30
<210> 67
   <211> 42
   <212> PRT
   <213> Artificial Sequence
<220>
   <223> TAT-KALA
<400> 67 
Tyr Gly Arg Lys Lys Arg Arg Gln Arg Arg Arg Cys Trp Glu Ala Lys
1               5                   10                  15

Leu Ala Lys Ala Leu Ala Lys Ala Leu Ala Lys His Leu Ala Lys Ala
            20                  25                  30

Leu Ala Lys Ala Leu Lys Ala Cys Glu Ala
        35                  40
<210> 68
   <211> 35
   <212> PRT
   <213> Artificial Sequence
<220>
   <223> His-CM18-PTD4
<400> 68 
His His His His His His Lys Trp Lys Leu Phe Lys Lys Ile Gly Ala
1               5                   10                  15

Val Leu Lys Val Leu Thr Thr Gly Tyr Ala Arg Ala Ala Ala Arg Gln
            20                  25                  30

Ala Arg Ala
        35
<210> 69
   <211> 33
   <212> PRT
   <213> Artificial Sequence
<220>
   <223> His-CM18-9Arg
<400> 69 
His His His His His His Lys Trp Lys Leu Phe Lys Lys Ile Gly Ala
1               5                   10                  15

Val Leu Lys Val Leu Thr Thr Gly Arg Arg Arg Arg Arg Arg Arg Arg
            20                  25                  30

Arg
<210> 70
   <211> 50
   <212> PRT
   <213> Artificial Sequence
<220>
   <223> His-CM18-Transportan
<400> 70 
His His His His His His Lys Trp Lys Leu Phe Lys Lys Ile Gly Ala
1               5                   10                  15

Val Leu Lys Val Leu Thr Thr Gly Gly Trp Thr Leu Asn Ser Ala Gly
            20                  25                  30

Tyr Leu Leu Lys Ile Asn Leu Lys Ala Leu Ala Ala Leu Ala Lys Lys
        35                  40                  45

Ile Leu
    50
<210> 71
   <211> 43
   <212> PRT
   <213> Artificial Sequence
<220>
   <223> His-LAH4-PDT4
<400> 71 
His His His His His His Lys Lys Ala Leu Leu Ala Leu Ala Leu His
1               5                   10                  15

His Leu Ala His Leu Ala Leu His Leu Ala Leu Ala Leu Lys Lys Ala
            20                  25                  30

Tyr Ala Arg Ala Ala Ala Arg Gln Ala Arg Ala
        35                  40
<210> 72
   <211> 31
   <212> PRT
   <213> Artificial Sequence
<220>
   <223> His-C(LLKK)3C-PDT4
<400> 72 
His His His His His His Cys Leu Leu Lys Lys Leu Leu Lys Lys Leu
1               5                   10                  15

Leu Lys Lys Cys Tyr Ala Arg Ala Ala Ala Arg Gln Ala Arg Ala
            20                  25                  30
<210> 73
   <211> 324
   <212> PRT
   <213> Artificial Sequence
<220>
   <223> mCherry-NLS
<400> 73 
Met His His His His His His Gly Gly Gly Gly Ser Gly Gly Gly Gly
1               5                   10                  15

Ser Gly Gly Ala Ser Thr Gly Ile Arg Met Val Ser Lys Cys Glu Glu
            20                  25                  30

Asp Asn Met Ala Ile Ile Lys Glu Phe Met Arg Phe Lys Val His Met
        35                  40                  45

Glu Gly Ser Val Asn Gly His Glu Phe Glu Ile Glu Gly Glu Gly Glu
    50                  55                  60

Gly Arg Pro Tyr Glu Gly Thr Gln Thr Ala Lys Leu Lys Val Thr Lys
65                  70                  75                  80

Gly Gly Pro Leu Pro Phe Ala Trp Asp Ile Leu Ser Pro Gln Phe Met
                85                  90                  95

Tyr Gly Ser Lys Ala Tyr Val Lys His Pro Ala Asp Ile Pro Asp Tyr
            100                 105                 110

Leu Lys Leu Ser Phe Pro Glu Gly Phe Lys Trp Glu Arg Val Met Asn
        115                 120                 125

Phe Glu Asp Gly Gly Val Val Thr Val Thr Gln Asp Ser Ser Leu Gln
    130                 135                 140
 
Asp Gly Glu Phe Ile Tyr Lys Val Lys Leu Arg Gly Thr Asn Phe Pro
145                 150                 155                 160

Ser Asp Gly Gln Val Met Gln Lys Lys Thr Met Gly Trp Glu Ala Ser
                165                 170                 175

Ser Glu Arg Met Tyr Pro Glu Asp Gly Ala Leu Lys Gly Glu Ile Lys
            180                 185                 190

Gln Arg Leu Lys Leu Lys Asp Gly Gly His Tyr Asp Ala Glu Val Lys
        195                 200                 205

Thr Thr Tyr Lys Ala Lys Lys Pro Val Gln Leu Pro Gly Ala Tyr Asn
    210                 215                 220

Val Asn Ile Lys Leu Asp Ile Thr Ser His Asn Glu Asp Tyr Thr Ile
225                 230                 235                 240

Val Glu Gln Tyr Glu Arg Ala Glu Gly Arg His Ser Thr Gly Gly Met
                245                 250                 255

Asp Glu Leu Tyr Lys Gly Gly Ser Gly Gly Gly Ser Gly Gly Gly Ser
            260                 265                 270

Gly Trp Ile Arg Ala Ser Ser Gly Gly Arg Ser Ser Asp Asp Glu Ala
        275                 280                 285

Thr Ala Asp Ser Gln His Ala Ala Pro Pro Lys Lys Lys Arg Lys Val
    290                 295                 300

Gly Gly Ser Gly Gly Gly Ser Gly Gly Gly Ser Gly Gly Gly Arg Gly
305                 310                 315                 320

Thr Glu Ile Ser
<210> 74
   <211> 1414
   <212> PRT
   <213> Artificial Sequence
<220>
   <223> Cas9-NLS
<400> 74 
Met Asp Lys Lys Tyr Ser Ile Gly Leu Asp Ile Gly Thr Asn Ser Val
1               5                   10                  15
 
Gly Trp Ala Val Ile Thr Asp Glu Tyr Lys Val Pro Ser Lys Lys Phe
            20                  25                  30

Lys Val Leu Gly Asn Thr Asp Arg His Ser Ile Lys Lys Asn Leu Ile
        35                  40                  45

Gly Ala Leu Leu Phe Asp Ser Gly Glu Thr Ala Glu Ala Thr Arg Leu
    50                  55                  60

Lys Arg Thr Ala Arg Arg Arg Tyr Thr Arg Arg Lys Asn Arg Ile Cys
65                  70                  75                  80

Tyr Leu Gln Glu Ile Phe Ser Asn Glu Met Ala Lys Val Asp Asp Ser
                85                  90                  95

Phe Phe His Arg Leu Glu Glu Ser Phe Leu Val Glu Glu Asp Lys Lys
            100                 105                 110

His Glu Arg His Pro Ile Phe Gly Asn Ile Val Asp Glu Val Ala Tyr
        115                 120                 125

His Glu Lys Tyr Pro Thr Ile Tyr His Leu Arg Lys Lys Leu Val Asp
    130                 135                 140

Ser Thr Asp Lys Ala Asp Leu Arg Leu Ile Tyr Leu Ala Leu Ala His
145                 150                 155                 160

Met Ile Lys Phe Arg Gly His Phe Leu Ile Glu Gly Asp Leu Asn Pro
                165                 170                 175

Asp Asn Ser Asp Val Asp Lys Leu Phe Ile Gln Leu Val Gln Thr Tyr
            180                 185                 190

Asn Gln Leu Phe Glu Glu Asn Pro Ile Asn Ala Ser Gly Val Asp Ala
        195                 200                 205

Lys Ala Ile Leu Ser Ala Arg Leu Ser Lys Ser Arg Arg Leu Glu Asn
    210                 215                 220

Leu Ile Ala Gln Leu Pro Gly Glu Lys Lys Asn Gly Leu Phe Gly Asn
225                 230                 235                 240

Leu Ile Ala Leu Ser Leu Gly Leu Thr Pro Asn Phe Lys Ser Asn Phe
                245                 250                 255

Asp Leu Ala Glu Asp Ala Lys Leu Gln Leu Ser Lys Asp Thr Tyr Asp
            260                 265                 270
 
Asp Asp Leu Asp Asn Leu Leu Ala Gln Ile Gly Asp Gln Tyr Ala Asp
        275                 280                 285

Leu Phe Leu Ala Ala Lys Asn Leu Ser Asp Ala Ile Leu Leu Ser Asp
    290                 295                 300

Ile Leu Arg Val Asn Thr Glu Ile Thr Lys Ala Pro Leu Ser Ala Ser
305                 310                 315                 320

Met Ile Lys Arg Tyr Asp Glu His His Gln Asp Leu Thr Leu Leu Lys
                325                 330                 335

Ala Leu Val Arg Gln Gln Leu Pro Glu Lys Tyr Lys Glu Ile Phe Phe
            340                 345                 350

Asp Gln Ser Lys Asn Gly Tyr Ala Gly Tyr Ile Asp Gly Gly Ala Ser
        355                 360                 365

Gln Glu Glu Phe Tyr Lys Phe Ile Lys Pro Ile Leu Glu Lys Met Asp
    370                 375                 380

Gly Thr Glu Glu Leu Leu Val Lys Leu Asn Arg Glu Asp Leu Leu Arg
385                 390                 395                 400

Lys Gln Arg Thr Phe Asp Asn Gly Ser Ile Pro His Gln Ile His Leu
                405                 410                 415

Gly Glu Leu His Ala Ile Leu Arg Arg Gln Glu Asp Phe Tyr Pro Phe
            420                 425                 430

Leu Lys Asp Asn Arg Glu Lys Ile Glu Lys Ile Leu Thr Phe Arg Ile
        435                 440                 445

Pro Tyr Tyr Val Gly Pro Leu Ala Arg Gly Asn Ser Arg Phe Ala Trp
    450                 455                 460

Met Thr Arg Lys Ser Glu Glu Thr Ile Thr Pro Trp Asn Phe Glu Glu
465                 470                 475                 480

Val Val Asp Lys Gly Ala Ser Ala Gln Ser Phe Ile Glu Arg Met Thr
                485                 490                 495

Asn Phe Asp Lys Asn Leu Pro Asn Glu Lys Val Leu Pro Lys His Ser
            500                 505                 510

Leu Leu Tyr Glu Tyr Phe Thr Val Tyr Asn Glu Leu Thr Lys Val Lys
 
        515                 520                 525

Tyr Val Thr Glu Gly Met Arg Lys Pro Ala Phe Leu Ser Gly Glu Gln
    530                 535                 540

Lys Lys Ala Ile Val Asp Leu Leu Phe Lys Thr Asn Arg Lys Val Thr
545                 550                 555                 560

Val Lys Gln Leu Lys Glu Asp Tyr Phe Lys Lys Ile Glu Cys Phe Asp
                565                 570                 575

Ser Val Glu Ile Ser Gly Val Glu Asp Arg Phe Asn Ala Ser Leu Gly
            580                 585                 590

Thr Tyr His Asp Leu Leu Lys Ile Ile Lys Asp Lys Asp Phe Leu Asp
        595                 600                 605

Asn Glu Glu Asn Glu Asp Ile Leu Glu Asp Ile Val Leu Thr Leu Thr
    610                 615                 620

Leu Phe Glu Asp Arg Glu Met Ile Glu Glu Arg Leu Lys Thr Tyr Ala
625                 630                 635                 640

His Leu Phe Asp Asp Lys Val Met Lys Gln Leu Lys Arg Arg Arg Tyr
                645                 650                 655

Thr Gly Trp Gly Arg Leu Ser Arg Lys Leu Ile Asn Gly Ile Arg Asp
            660                 665                 670

Lys Gln Ser Gly Lys Thr Ile Leu Asp Phe Leu Lys Ser Asp Gly Phe
        675                 680                 685

Ala Asn Arg Asn Phe Met Gln Leu Ile His Asp Asp Ser Leu Thr Phe
    690                 695                 700

Lys Glu Asp Ile Gln Lys Ala Gln Val Ser Gly Gln Gly Asp Ser Leu
705                 710                 715                 720

His Glu His Ile Ala Asn Leu Ala Gly Ser Pro Ala Ile Lys Lys Gly
                725                 730                 735

Ile Leu Gln Thr Val Lys Val Val Asp Glu Leu Val Lys Val Met Gly
            740                 745                 750

Arg His Lys Pro Glu Asn Ile Val Ile Glu Met Ala Arg Glu Asn Gln
        755                 760                 765
 
Thr Thr Gln Lys Gly Gln Lys Asn Ser Arg Glu Arg Met Lys Arg Ile
    770                 775                 780

Glu Glu Gly Ile Lys Glu Leu Gly Ser Gln Ile Leu Lys Glu His Pro
785                 790                 795                 800

Val Glu Asn Thr Gln Leu Gln Asn Glu Lys Leu Tyr Leu Tyr Tyr Leu
                805                 810                 815

Gln Asn Gly Arg Asp Met Tyr Val Asp Gln Glu Leu Asp Ile Asn Arg
            820                 825                 830

Leu Ser Asp Tyr Asp Val Asp His Ile Val Pro Gln Ser Phe Leu Lys
        835                 840                 845

Asp Asp Ser Ile Asp Asn Lys Val Leu Thr Arg Ser Asp Lys Asn Arg
    850                 855                 860

Gly Lys Ser Asp Asn Val Pro Ser Glu Glu Val Val Lys Lys Met Lys
865                 870                 875                 880

Asn Tyr Trp Arg Gln Leu Leu Asn Ala Lys Leu Ile Thr Gln Arg Lys
                885                 890                 895

Phe Asp Asn Leu Thr Lys Ala Glu Arg Gly Gly Leu Ser Glu Leu Asp
            900                 905                 910

Lys Ala Gly Phe Ile Lys Arg Gln Leu Val Glu Thr Arg Gln Ile Thr
        915                 920                 925

Lys His Val Ala Gln Ile Leu Asp Ser Arg Met Asn Thr Lys Tyr Asp
    930                 935                 940

Glu Asn Asp Lys Leu Ile Arg Glu Val Lys Val Ile Thr Leu Lys Ser
945                 950                 955                 960

Lys Leu Val Ser Asp Phe Arg Lys Asp Phe Gln Phe Tyr Lys Val Arg
                965                 970                 975

Glu Ile Asn Asn Tyr His His Ala His Asp Ala Tyr Leu Asn Ala Val
            980                 985                 990

Val Gly Thr Ala Leu Ile Lys Lys  Tyr Pro Lys Leu Glu  Ser Glu Phe
        995                 1000                1005

Val Tyr  Gly Asp Tyr Lys Val  Tyr Asp Val Arg Lys  Met Ile Ala
    1010                1015                1020
 
Lys Ser Glu Gln Glu Ile Gly Lys Ala Thr Ala Lys Tyr Phe Phe
    1025                1030                1035

Tyr Ser Asn Ile Met Asn Phe Phe Lys Thr Glu Ile Thr Leu Ala
    1040                1045                1050

Asn Gly Glu Ile Arg Lys Arg Pro Leu Ile Glu Thr Asn Gly Glu
    1055                1060                1065

Thr Gly Glu Ile Val Trp Asp Lys Gly Arg Asp Phe Ala Thr Val
    1070                1075                1080

Arg Lys Val Leu Ser Met Pro Gln Val Asn Ile Val Lys Lys Thr
    1085                1090                1095

Glu Val Gln Thr Gly Gly Phe Ser Lys Glu Ser Ile Leu Pro Lys
    1100                1105                1110

Arg Asn Ser Asp Lys Leu Ile Ala Arg Lys Lys Asp Trp Asp Pro
    1115                1120                1125

Lys Lys Tyr Gly Gly Phe Asp Ser Pro Thr Val Ala Tyr Ser Val
    1130                1135                1140

Leu Val Val Ala Lys Val Glu Lys Gly Lys Ser Lys Lys Leu Lys
    1145                1150                1155

Ser Val Lys Glu Leu Leu Gly Ile Thr Ile Met Glu Arg Ser Ser
    1160                1165                1170

Phe Glu Lys Asn Pro Ile Asp Phe Leu Glu Ala Lys Gly Tyr Lys
    1175                1180                1185

Glu Val Lys Lys Asp Leu Ile Ile Lys Leu Pro Lys Tyr Ser Leu
    1190                1195                1200

Phe Glu Leu Glu Asn Gly Arg Lys Arg Met Leu Ala Ser Ala Gly
    1205                1210                1215

Glu Leu Gln Lys Gly Asn Glu Leu Ala Leu Pro Ser Lys Tyr Val
    1220                1225                1230

Asn Phe Leu Tyr Leu Ala Ser His Tyr Glu Lys Leu Lys Gly Ser
    1235                1240                1245

Pro Glu Asp Asn Glu Gln Lys Gln Leu Phe Val Glu Gln His Lys
    1250                1255                1260
 
His Tyr Leu Asp Glu Ile Ile Glu Gln Ile Ser Glu Phe Ser Lys
    1265                1270                1275

Arg Val Ile Leu Ala Asp Ala Asn Leu Asp Lys Val Leu Ser Ala
    1280                1285                1290

Tyr Asn Lys His Arg Asp Lys Pro Ile Arg Glu Gln Ala Glu Asn
    1295                1300                1305

Ile Ile His Leu Phe Thr Leu Thr Asn Leu Gly Ala Pro Ala Ala
    1310                1315                1320

Phe Lys Tyr Phe Asp Thr Thr Ile Asp Arg Lys Arg Tyr Thr Ser
    1325                1330                1335

Thr Lys Glu Val Leu Asp Ala Thr Leu Ile His Gln Ser Ile Thr
    1340                1345                1350

Gly Leu Tyr Glu Thr Arg Ile Asp Leu Ser Gln Leu Gly Gly Asp
    1355                1360                1365

Gly Gly Arg Ser Ser Asp Asp Glu Ala Thr Ala Asp Ser Gln His
    1370                1375                1380

Ala Ala Pro Pro Lys Lys Lys Arg Lys Val Gly Gly Ser Gly Gly
    1385                1390                1395

Gly Ser Gly Gly Gly Ser Gly Gly Gly Arg His His His His His
    1400                1405                1410

His
<210> 75
   <211> 42
   <212> RNA
   <213> Artificial Sequence
<220>
   <223> crRNA (Example 13.3)
<400> 75
   gaguccgagc agaagaagaa guuuuagagc uaugcuguuu ug 42
<210> 76
   <211> 69
   <212> RNA
   <213> Artificial Sequence
<220>
   <223> tracrRNA (Example 13.3)
<400> 76 
aaacagcaua gcaaguuaaa auaaggcuag uccguuauca acuugaaaaa guggcaccga 60

gucggugcu 69
<210> 77
   <211> 69
   <212> RNA
   <213> Artificial Sequence
<220>
   <223> tracrRNA (Example 13.5)
<400> 77 
aaacagcaua gcaaguuaaa auaaggcuag uccguuauca acuugaaaaa guggcaccga 60

gucggugcu 69
<210> 78
   <211> 42
   <212> RNA
   <213> Artificial Sequence
<220>
   <223> PPIB crRNA (Example 13.5)
<400> 78
   guguauuuug accuacgaau guuuuagagc uaugcuguuu ug 42
<210> 79
   <211> 74
   <212> RNA
   <213> Artificial Sequence
<220>
   <223> PPIB tracrRNA (Example 13.5)
<400> 79 
aacagcauag caaguuaaaa uaaggcuagu ccguuaucaa cuugaaaaag uggcaccgag 60

ucggugcuuu uuuu 74
<210> 80
   <211> 246
   <212> PRT
   <213> Artificial Sequence
<220>
   <223> HOXB4-WT
<400> 80 
Met His His His His His His Met Ala Met Ser Ser Phe Leu Ile Asn
1               5                   10                  15
 
Ser Asn Tyr Val Asp Pro Lys Phe Pro Pro Cys Glu Glu Tyr Ser Gln
        20                  25                  30

Ser Asp Tyr Leu Pro Ser Asp His Ser Pro Gly Tyr Tyr Ala Gly Gly
    35                  40                  45

Gln Arg Arg Glu Ser Ser Phe Gln Pro Glu Ala Gly Phe Gly Arg Arg
    50                  55                  60

Ala Ala Cys Thr Val Gln Arg Tyr Pro Pro Pro Pro Pro Pro Pro Pro
65                  70                  75                  80

Pro Pro Gly Leu Ser Pro Arg Ala Pro Ala Pro Pro Pro Ala Gly Ala
                85                  90                  95

Leu Leu Pro Glu Pro Gly Gln Arg Cys Glu Ala Val Ser Ser Ser Pro
            100                 105                 110

Pro Pro Pro Pro Cys Ala Gln Asn Pro Leu His Pro Ser Pro Ser His
        115                 120                 125

Ser Ala Cys Lys Glu Pro Val Val Tyr Pro Trp Met Arg Lys Val His
    130                 135                 140

Val Ser Thr Val Asn Pro Asn Tyr Ala Gly Gly Glu Pro Lys Arg Ser
145                 150                 155                 160

Arg Thr Ala Tyr Thr Arg Gln Gln Val Leu Glu Leu Glu Lys Glu Phe
                165                 170                 175

His Tyr Asn Arg Tyr Leu Thr Arg Arg Arg Arg Val Glu Ile Ala His
            180                 185                 190

Ala Leu Cys Leu Ser Glu Arg Gln Ile Lys Ile Trp Phe Gln Asn Arg
        195                 200                 205

Arg Met Lys Trp Lys Lys Asp His Lys Leu Pro Asn Thr Lys Ile Arg
    210                 215                 220

Ser Gly Gly Ala Ala Gly Ser Ala Gly Gly Pro Pro Gly Arg Pro Asn
225                 230                 235                 240

Gly Gly Pro Arg Ala Leu
                245
<210> 81
   <211> 17
   <212> PRT
   <213> Artificial Sequence
<220>
   <223> His-PTD4
<400> 81 
His His His His His His Tyr Ala Arg Ala Ala Ala Arg Gln Ala Arg
1               5                   10                  15

Ala
<210> 82
   <211> 41
   <212> PRT
   <213> Artificial Sequence
<220>
   <223> PTD4-KALA
<400> 82 
Tyr Ala Arg Ala Ala Ala Arg Gln Ala Arg Ala Trp Glu Ala Lys Leu
1               5                   10                  15

Ala Lys Ala Leu Ala Lys Ala Leu Ala Lys His Leu Ala Lys Ala Leu
            20                  25                  30

Ala Lys Ala Leu Lys Ala Cys Glu Ala
        35                  40
<210> 83
   <211> 39
   <212> PRT
   <213> Artificial Sequence
<220>
   <223> 9Arg-KALA
<400> 83 
Arg Arg Arg Arg Arg Arg Arg Arg Arg Trp Glu Ala Lys Leu Ala Lys
1               5                   10                  15

Ala Leu Ala Lys Ala Leu Ala Lys His Leu Ala Lys Ala Leu Ala Lys
            20                  25                  30

Ala Leu Lys Ala Cys Glu Ala
        35
<210> 84
   <211> 51
   <212> PRT
   <213> Artificial Sequence
<220>
   <223> Pep1-KALA
<400> 84 
Lys Glu Thr Trp Trp Glu Thr Trp Trp Thr Glu Trp Ser Gln Pro Lys
1               5                   10                  15

Lys Lys Arg Lys Val Trp Glu Ala Lys Leu Ala Lys Ala Leu Ala Lys
            20                  25                  30

Ala Leu Ala Lys His Leu Ala Lys Ala Leu Ala Lys Ala Leu Lys Ala
        35                  40                  45

Cys Glu Ala
    50
<210> 85
   <211> 37
   <212> PRT
   <213> Artificial Sequence
<220>
   <223> Xentry-KALA
<400> 85 
Leu Cys Leu Arg Pro Val Gly Trp Glu Ala Lys Leu Ala Lys Ala Leu
1               5                   10                  15

Ala Lys Ala Leu Ala Lys His Leu Ala Lys Ala Leu Ala Lys Ala Leu
            20                  25                  30

Lys Ala Cys Glu Ala
        35
<210> 86
   <211> 40
   <212> PRT
   <213> Artificial Sequence
<220>
   <223> SynB3-KALA
<400> 86 
Arg Arg Leu Ser Tyr Ser Arg Arg Arg Phe Trp Glu Ala Lys Leu Ala
1               5                   10                  15

Lys Ala Leu Ala Lys Ala Leu Ala Lys His Leu Ala Lys Ala Leu Ala
            20                  25                  30

Lys Ala Leu Lys Ala Cys Glu Ala
 35                                                    40
<210> 87
   <211> 36
   <212> PRT
   <213> Artificial Sequence
<220>
   <223> VSVG-PTD4
<400> 87 
Lys Phe Thr Ile Val Phe Pro His Asn Gln Lys Gly Asn Trp Lys Asn
1               5                   10                  15

Val Pro Ser Asn Tyr His Tyr Cys Pro Tyr Ala Arg Ala Ala Ala Arg
            20                  25                  30

Gln Ala Arg Ala
        35
<210> 88
   <211> 34
   <212> PRT
   <213> Artificial Sequence
<220>
   <223> EB1-PTD4
<400> 88 
Leu Ile Arg Leu Trp Ser His Leu Ile His Ile Trp Phe Gln Asn Arg
1               5                   10                  15

Arg Leu Lys Trp Lys Lys Lys Tyr Ala Arg Ala Ala Ala Arg Gln Ala
            20                  25                  30

Arg Ala
<210> 89
   <211> 31
   <212> PRT
   <213> Artificial Sequence
<220>
   <223> JST-PTD4
<400> 89 
Gly Leu Phe Glu Ala Leu Leu Glu Leu Leu Glu Ser Leu Trp Glu Leu
1               5                   10                  15

Leu Leu Glu Ala Tyr Ala Arg Ala Ala Ala Arg Gln Ala Arg Ala
            20                  25                  30
<210> 90
   <211> 29
   <212> PRT
   <213> Artificial Sequence
<220>
   <223> CM18-PTD4
<400> 90 
Lys Trp Lys Leu Phe Lys Lys Ile Gly Ala Val Leu Lys Val Leu Thr
1               5                   10                  15

Thr Gly Tyr Ala Arg Ala Ala Ala Arg Gln Ala Arg Ala
            20                  25
<210> 91
   <211> 35
   <212> PRT
   <213> Artificial Sequence
<220>
   <223> 6Cys-CM18-PTD4
<400> 91 
Cys Cys Cys Cys Cys Cys Lys Trp Lys Leu Phe Lys Lys Ile Gly Ala
1               5                   10                  15

Val Leu Lys Val Leu Thr Thr Gly Tyr Ala Arg Ala Ala Ala Arg Gln
            20                  25                  30

Ala Arg Ala
        35
<210> 92
   <211> 32
   <212> PRT
   <213> Artificial Sequence
<220>
   <223> CM18-L1-PTD4
<400> 92 
Lys Trp Lys Leu Phe Lys Lys Ile Gly Ala Val Leu Lys Val Leu Thr
1               5                   10                  15

Thr Gly Gly Gly Ser Tyr Ala Arg Ala Ala Ala Arg Gln Ala Arg Ala
            20                  25                  30
<210> 93
   <211> 36
   <212> PRT
   <213> Artificial Sequence
<220>
   <223> CM18-L2-PTD4
<400> 93 
Lys Trp Lys Leu Phe Lys Lys Ile Gly Ala Val Leu Lys Val Leu Thr
1               5                   10                  15

Thr Gly Gly Gly Ser Gly Gly Gly Ser Tyr Ala Arg Ala Ala Ala Arg
            20                  25                  30

Gln Ala Arg Ala
        35
<210> 94
   <211> 41
   <212> PRT
   <213> Artificial Sequence
<220>
   <223> CM18-L3-PTD4
<400> 94 
Lys Trp Lys Leu Phe Lys Lys Ile Gly Ala Val Leu Lys Val Leu Thr
1               5                   10                  15

Thr Gly Gly Gly Ser Gly Gly Gly Ser Gly Gly Gly Ser Gly Tyr Ala
            20                  25                  30

Arg Ala Ala Ala Arg Gln Ala Arg Ala
        35                  40
<210> 95
   <211> 35
   <212> PRT
   <213> Artificial Sequence
<220>
   <223> His-CM18-TAT
<400> 95 
His His His His His His Lys Trp Lys Leu Phe Lys Lys Ile Gly Ala
1               5                   10                  15

Val Leu Lys Val Leu Thr Thr Gly Tyr Gly Arg Lys Lys Arg Arg Gln
            20                  25                  30

Arg Arg Arg
        35
<210> 96
   <211> 41
   <212> PRT
   <213> Artificial Sequence
<220>
   <223> His-CM18-PTD4-6Cys
<400> 96 
His His His His His His Lys Trp Lys Leu Phe Lys Lys Ile Gly Ala
1               5                   10                  15

Val Leu Lys Val Leu Thr Thr Gly Tyr Ala Arg Ala Ala Ala Arg Gln
            20                  25                  30

Ala Arg Ala Cys Cys Cys Cys Cys Cys
        35                  40
<210> 97
   <211> 32
   <212> PRT
   <213> Artificial Sequence
<220>
   <223> 3His-CM18-PTD4
<400> 97 
His His His Lys Trp Lys Leu Phe Lys Lys Ile Gly Ala Val Leu Lys
1               5                   10                  15

Val Leu Thr Thr Gly Tyr Ala Arg Ala Ala Ala Arg Gln Ala Arg Ala
            20                  25                  30
<210> 98
   <211> 41
   <212> PRT
   <213> Artificial Sequence
<220>
   <223> 12His-CM18-PTD4
<400> 98 
His His His His His His His His His His His His Lys Trp Lys Leu
1               5                   10                  15

Phe Lys Lys Ile Gly Ala Val Leu Lys Val Leu Thr Thr Gly Tyr Ala
            20                  25                  30

Arg Ala Ala Ala Arg Gln Ala Arg Ala
        35                  40
<210> 99
   <211> 36
   <212> PRT
   <213> Artificial Sequence
<220>
   <223> HA-CM18-PTD4
<400> 99 
His His His Ala His His His Lys Trp Lys Leu Phe Lys Lys Ile Gly
1               5                   10                  15

Ala Val Leu Lys Val Leu Thr Thr Gly Tyr Ala Arg Ala Ala Ala Arg
            20                  25                  30

Gln Ala Arg Ala
        35
<210> 100
   <211> 38
   <212> PRT
   <213> Artificial Sequence
<220>
   <223> 3HA-CM18-PTD4
<400> 100 
His Ala His His Ala His His Ala His Lys Trp Lys Leu Phe Lys Lys
1               5                   10                  15

Ile Gly Ala Val Leu Lys Val Leu Thr Thr Gly Tyr Ala Arg Ala Ala
            20                  25                  30

Ala Arg Gln Ala Arg Ala
        35
<210> 101
   <211> 35
   <212> PRT
   <213> Artificial Sequence
<220>
   <223> CM18-His-PTD4
<400> 101 
Lys Trp Lys Leu Phe Lys Lys Ile Gly Ala Val Leu Lys Val Leu Thr
1               5                   10                  15

Thr Gly His His His His His His Tyr Ala Arg Ala Ala Ala Arg Gln
            20                  25                  30

Ala Arg Ala
        35
<210> 102
   <211> 41
   <212> PRT
   <213> Artificial Sequence
<220>
   <223> His-CM18-PTD4-His
<400> 102 
His His His His His His Lys Trp Lys Leu Phe Lys Lys Ile Gly Ala
1               5                   10                  15

Val Leu Lys Val Leu Thr Thr Gly Tyr Ala Arg Ala Ala Ala Arg Gln
            20                  25                  30

Ala Arg Ala His His His His His His
        35                  40
<210> 103
   <211> 36
   <212> RNA
   <213> Artificial Sequence
<220>
   <223> HPRT crRNA
<400> 103
   aauuaugggg auuacuagga guuuuagagc uaugcu 36